(19) Europäisches Patentamt
European Patent Office
Office européen des brevets

(11) EP 0 819 135 B1

(12) # EUROPÄISCHE PATENTSCHRIFT

(45) Veröffentlichungstag und Bekanntmachung des Hinweises auf die Patenterteilung:
**17.11.1999 Patentblatt 1999/46**

(21) Anmeldenummer: **96910926.3**

(22) Anmeldetag: **22.03.1996**

(51) Int Cl.[6]: **C07K 9/00**, C07D 487/04, A61K 31/02, C07D 401/04, C07H 15/203

(86) Internationale Anmeldenummer:
**PCT/EP96/01279**

(87) Internationale Veröffentlichungsnummer:
**WO 96/31532 (10.10.1996 Gazette 1996/45)**

(54) **KOHLENHYDRATMODIFIZIERTE CYTOSTATIKA**
SUGAR-MODIFIED CYTOSTATICS
CYTOSTATIQUES MODIFIES AUX GLUCIDES

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **04.04.1995 DE 19512484**

(43) Veröffentlichungstag der Anmeldung:
**21.01.1998 Patentblatt 1998/04**

(73) Patentinhaber: **BAYER AG
51368 Leverkusen (DE)**

(72) Erfinder:
- **LERCHEN, Hans-Georg
D-51061 Köln (DE)**
- **VON DEM BRUCH, Karsten
D-51375 Leverkusen (DE)**
- **PETERSEN, Uwe
D-51375 Leverkusen (DE)**
- **BAUMGARTEN, Jörg
D-42115 Wuppertal (DE)**
- **PIEL, Norbert
D-40699 Erkrath (DE)**
- **ANTONICEK, Horst-Peter
D-51467 Bergisch Gladbach (DE)**
- **WEICHEL, Walter
D-51519 Odenthal (DE)**
- **SPERZEL, Michael
D-42275 Wuppertal (DE)**
- **BREMM, Klaus, Dieter
D-45661 Recklinghausen (DE)**

(56) Entgegenhaltungen:
**EP-A- 0 284 071**
**EP-A- 0 501 250**
**EP-A- 0 520 240**
**EP-A- 0 595 133**
**WO-A-95/10296**

- **CHEMICAL ABSTRACTS, vol. 121, no. 17, 24.Oktober 1994 Columbus, Ohio, US; abstract no. 205995t, K.DZIERZBICKA ET AL: "Synthesis of Muramyl Dipeptide Conjugated with Acridine Derivatives Showing Anti-HIV-1 and Anticancer Activity" Seite 1244; Spalte 1; XP002010852 & J.POL.CHEM., Bd. 68, Nr. 1, 1994, Seiten 37-45,**
- **ONCOLOGY, Bd. 12, 1989, Seiten 175-181, XP002010851 H.-J.GABIUS: "Endogene Lektine in Tumoren und ihre mögliche Bedeutung für Diagnose und Therapie von Krebserkrankungen." in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 819 135 B1

Printed by Jouve, 75001 PARIS (FR)

**Beschreibung**

Die Erfindung betrifft Cytostatika, die durch Modifikation mit Kohlenhydraten tumorspezifisch sind. Geeignete Spacer gewährleisten die Serumstabilität und gleichzeitig intrazelluläre Wirkung.

Die Chemotherapie bei Tumorerkrankungen ist begleitet von meist schwerwiegenden Nebenwirkungen, bedingt durch die Toxizität von Chemotherapeutika auf proliferierende Zellen anderer Gewebe. Seit vielen Jahren beschäftigen sich Wissenschaftler mit dem Problem der Verbesserung der Selektivität von eingesetzten Wirkstoffen. Ein vielfach verfolgter Ansatz ist die Synthese von Prodrugs, die mehr oder weniger selektiv im Zielgewebe beispielsweise durch Veränderung des pH-Wertes (Tietze et al., z.B. DE-4 229 903), durch Enzyme (z.B. Glucuronidasen; Jacquesy et al., EP-511 917; Bosslet et al., EP-595 133) oder durch Antikörper-Enzym-Konjugate (Bagshawe et al. WO 88/07378; Senter et al., US-PS 4 975 278; Bosslet et al., EP-595 133) freigesetzt werden. Problematisch bei diesen Ansätzen ist u.a. die mangelnde Stabilität der Konjugate in anderen Geweben und Organen, und insbesondere die ubiquitäre Wirkstoffverteilung, die sich an die extrazelluläre Wirkstofffreisetzung im Tumorgewebe anschließt.

Das ausgeprägte Lektinmuster auf Tumorzelloberflächen (Gabius; Onkologie 12, (1989), 175) eröffnet die prinzipielle Möglichkeit, durch Anknüpfen der korrespondierenden Kohlenhydratbausteine an Cytostatika diese gezielt an Tumorzellen zu adressieren. Eingeschränkt wird diese Perspektive dadurch, daß auch in anderen Geweben, insbesondere in der Leber, Lektine mit ähnlichen Kohlenhydratspezifitäten (Galactose, Lactose, Mannose, N-Acetyl-glucosamin, Fucose etc.) vorkommen (Ashwell et al., Annu.Rev.Biochem. 46 (1982), 531; Stahl et al.. Proc.Natl.Acad. Sci. USA 74 (1977), 1521; Hill et al., J.Biol. Chem. 262 (1986), 7433; Jansen et al., J.Biol.Chem. 266 (1991), 3343). Demzufolge muß mit einer deutlichen Anreicherung von wirkstoffhaltigen Glycokonjugaten in der Leber und anderen lektinreichen Organen gerechnet werden, wenn solche unmodifizierten Zucker verwendet werden.

Das heterocyclische Amin Batracylin **(1)** zeigt in verschiedenen Darmkrebs-Model(en eine gute Antitumorwirkung (US-PS 4 757 072).

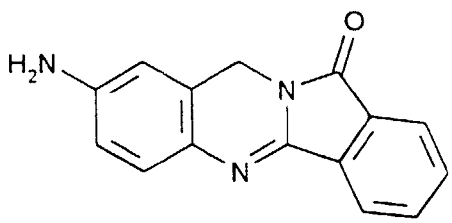

(1)

Peptidkonjugate von (1) mit guter In-vitro-Wirkung und günstigeren Löslichkeitseigenschaften (US-4 180 343) sind im Tierversuch schlechter verträglich als Batracylin. Die in EP-501 250 beschriebenen Fucose-Konjugate reichern sich sehr stark in der Leber an.

Das Chinolon-a **(2)** 7-[(3aRS, 4RS, 7aSR)-4-Amino-1,3,3a,4,7,7a-hexahydro-isoindol-2-yl]-8-chlor-1-cyclopropyl-6-fluor-1,4-dihydro-4-oxo-3-chinolincarbonsäure zeigt neben seiner hervorragenden antibakteriellen Aktivität auch eine sehr gute Wirksamkeit gegenüber verschiedenen Tumorzellinien (EP-520 240, JP-4 253 973). Dem stehen jedoch erhebliche toxikologische Probleme gegenüber (z.B Genotoxizität, Knochenmarks-Toxizität, hohe akute Toxizität in vivo etc.).

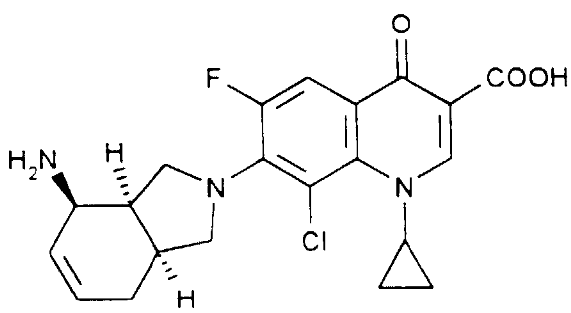

(2) (Chinolon-a)

Durch eine neuartige Modifizierung von Cytostatika fanden wir überraschend eine neue Klasse von Konjugaten, die sich durch folgende Eigenschaften auszeichnet. Eine neuartige Verknüpfung von Kohlenhydraten mit Cytostatika (beispielsweise Batracylin, Chinolon-a) führt zu Glycokonjugaten, die serumstabil sind. Die Wirkung hängt nicht von einer extrazellulären Freisetzung des Wirkstoffs ab. Die In-vitro-Aktivitäten gegenüber verschiedenen Tumorzellinien sind mit der des zugrundeliegenden Cytostatikums vergleichbar. Die zellspezifische Aufnahme ist kohlenhydratabhängig.

Durch die regioselektiven Modifizierungen im Kohlenhydratteil der beschriebenen Konjugate wird die Zell- und Gewebeselektivität (insbesondere Tumor zu Leber) deutlich verbessert.

In vivo zeichnen sich die erfindungsgemäßen Konjugate durch eine gegenüber dem Wirkstoff und den entsprechenden Peptidkonjugaten deutlich verbesserte Verträglichkeit aus.

Darüber hinaus zeigen die erfindungsgemäßen Konjugate im Vergleich zu den zugrundeliegenden Cytostatika wesentlich verbesserte Löslichkeitseigenschaften.

Die erfindungsgemäßen Verbindungen werden durch folgende allgemeine Formel beschrieben:

$$K - Sp - L - AA1 - AA2 - C \qquad (I)$$

mit

K = unsubstituierter oder regioselektiv modifizierter Kohlenhydratrest
Sp = gegebenenfalls substituiertes Arylen oder Alkylen
L =

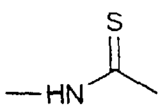
oder
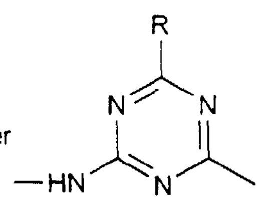

mit R = Chlor oder Hydroxyalkylamino,

wobei die Verknüpfung zu Sp über die NH-Gruppe erfolgt.

AA1 = ist ein Aminosäure-Rest in der D- oder L-Konfiguration, der gegebenenfalls eine zweite Gruppierung K-Sp-L- trägt, in der K, Sp, L unabhängig von der anderen Gruppierung K-Sp-L- die oben angegebenen Bedeutungen haben können, oder eine direkte Bindung. Ein Aminosäurerest kann sowohl über die α-Aminogruppe als auch gegebenenfalls über Seitenketten-amino- oder -hydroxyfunktionen und auch über beide Funktionen mit L verknüpft sein. Falls AA1 weitere funktionelle Gruppen trägt, so können diese deblockiert oder mit bekannten Schutzgruppen geschützt vorliegen. Als Schutzgruppen geeignet sind z.B. Acetyl, Allyloxycarbonyl, Benzyloxycarbonyl, Fluorenylmethoxycarbonyl, t-Butoxycarbonyl, Allyl, Benzyl, Methyl oder tert.- Butyl.

AA2 = ist ein Aminosäure-Rest in der D- oder L-Konfiguration, der gegebenenfalls eine zweite Gruppierung K-Sp-L- trägt, in der K, Sp, L unabhängig von der anderen Gruppierung K-Sp-L- die oben angegebenen Bedeutungen haben können, oder eine direkte Bindung. Ein Aminosäurerest kann sowohl über die α-Aminogruppe als auch gegebenenfalls über Seitenketten-amino- oder -hydroxyfunktionen und auch über beide Funktionen mit AA1 verknüpft sein. Falls AA2 weitere funktionelle Gruppen trägt, so können diese deblockiert oder mit bekannten Schutzgruppen geschützt vorliegen. Als Schutzgruppen geeignet sind z.B. Benzyloxycarbonyl, Acetyl, Allyloxycarbonyl, Fluorenylmethoxycarbonyl, t-Butoxycarbonyl, Allyl, Benzyl, Methyl oder tert- Butyl.

C = Reste eines Cytostatikums oder eines Cytostatikum-Derivates, das zusätzlich eine Amino- oder Hydroxygruppe tragen kann. C kann eine interkalierende Substanz, ein Topoisomerase-Inhibitor, ein Antimetabolit, ein Alkylanz, ein Tubulinhemmer, ein Tyrosinphosphokinase-Inhibitor, ein Proteinkinase-C-Inhibitor oder ein Wirkstoff mit einem anderen oder unbekannten cytostatischen oder cytotoxischen Wirkmechanismus sein. C kann beispielsweise ein Nucleosid, ein Endiin-Antibiotikum, eine Chinolon- oder Naphtyridoncarbonäure oder ein cytotoxisches Peptidantibiotikum z.B aus der Klasse der Dolastatine sein C kann Batracylin, Chinolon-a, 5-Fluoruracil, Cytosinarabinosid, Methotrexat, Etoposid, Camptothecin, Daunomycin, Doxorubicin, Taxol, Vinblastin, Vincristin, Dynemicin, Calicheamycin, Esperamycin, Quercetin, Suramin, Erbstatin, Cyclophosphamid, Mitomycin C, Melphalan, Cisplatin, Bleomycin, Staurosporin oder ein anderer antineoplastisch aktiver Wirkstoff sein.

Das Strukturelement -Sp-L-AA1-AA2- insgesamt stellt den Spacer dar, der K und C verbindet.

Bevorzugt sind Verbindungen der Formel (1), bei denen

K = Kohlenhydratrest mit der allgemeinen Formel

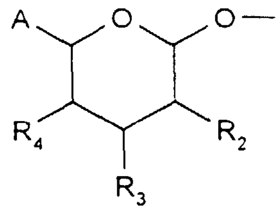

(II),

wobei

A = Methyl, Hydroxymethyl, Carboxy sowie davon abgeleitete Ester und Amide, Alkoxymethyl, Acyloxymethyl oder Carboxyalkyl oxymethyl sowie davon abgeleitete Ester und Amide. A kann auch $CH_2$-B sein, wobei B wiederum ein über das anomere Zentrum verknüpfter Kohlenhydratrest der allgemeinen Formel (II) sein kann.

$R_2$, $R_3$, $R_4$ = einzeln oder zusammen gleich H, Hydroxy, Alkyloxy, Carboxyalkyloxy sowie davon abgeleitete Ester und Amide, Hydroxyalkyloxy, Aminoalkyloxy, Acyloxy, Carboxyalkylcarbonyloxy, Sulfato, Phosphato, Halogen, oder ein weiterer, im gleichen Rahmen modifizierter und über das anomere Zentrum verknüpfter Kohlenhydratrest (II). $R_2$ kann zusätzlich auch Amino oder Acylamino sein.
Zwei der Reste $R_2$, $R_3$, $R_4$ können auch zusammen eine Epoxygruppe bedeuten.

Die Stereochemie am anomeren Zentrum des Kohlenhydratbausteins kann α oder β sein. Durch die Stereochemie an den anderen Zentren kann sich die gluco, manno-, galacto-, gulo-, rhamno- oder fuco-Konfiguration ergeben.

Sp = Arylenrest, der ortho-, meta- oder para-ständig mit K und L modifiziert ist und darüber hinaus noch 1 bis 4 weitere Substituenten tragen kann, die unabhängig oder gleich H, Methyl, Methoxy, Hydroxy, Carboxy, Methyloxycarbonyl, Cyano, Nitro, Halogen, Sulfonyl oder Sulfonamid sein konnen;
Sp kann auch ein linearer oder verzweigter Alkylen-Rest sein

L =

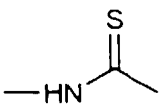

oder

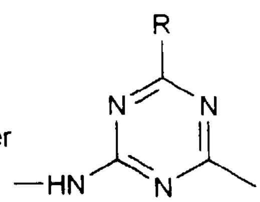

mit R = Chlor oder Hydroxyalkylamino,

AA1 = ist ein Aminosäure-Rest in der D- oder L-Konfiguration, der gegebenenfalls eine zweite Gruppierung K-Sp-L- trägt, in der K, Sp, L unabhängig von der anderen Gruppierung K-Sp-L- die oben angegebenen Bedeutungen haben können, oder eine direkte Bindung. Ein Aminosäurerest kann sowohl über die α-Aminogruppe als auch gegebenenfalls über Seitenketten-amino- oder -hydroxyfunktionen und auch über beide Funktionen mit L verknüpft sein. Falls AA1 weitere funktionelle Gruppen trägt, so können diese deblockiert oder mit bekannten Schutzgruppen geschützt vorliegen. Als Schutzgruppen geeignet sind z.B. Acetyl, Allyloxycarbonyl, Benzyloxycarbonyl, Fluorenylmethoxycarbonyl, t-Butoxycarbonyl, Allyl, Benzyl, Methyl oder tert.- Butyl.

AA2 = ist ein Aminosäure-Rest in der D- oder L-Konfiguration, der gegebenenfalls eine zweite Gruppierung K-Sp-L- trägt, in der K, Sp, L unabhängig von der anderen Gruppierung K-Sp-L- die oben angegebenen Bedeutungen haben können, oder eine direkte Bindung. Ein Aminosäurerest kann sowohl über die α-Aminogruppe als auch gegebenenfalls über Seitenketten-amino- oder -hydroxyfunktionen und auch über beide Funktionen mit AA1 verknüpft sein Falls AA2 weitere funktionelle Gruppen trägt, so können diese deblockiert oder mit bekannten Schutzgruppen geschützt vorliegen. Als Schutzgruppen geeignet sind z.B. Benzyloxycarbonyl, Allyloxycarbonyl, Acetyl, Fluorenylmethoxycarbonyl, t-Butoxycarbonyl, Allyl, Benzyl, Methyl oder tert.- Butyl.

C kann beispielsweise der Rest eines Nucleosids, eines Endiin-Antibiotikum oder ein cytotoxisches Peptidantibiotikum z.B. aus der Klasse der Dolastatine oder eine wie unten definierte Chinolon- oder Naphthyridoncatonsaure sein. C kann beispielsweise Batracylin, 5-Fluorouracil, Cytosinarabinosid, Methotrexat, Etoposid, Camptothecin, Daunomycin, Doxorubicin, Taxol, Vinblastin, Vincristin, Dynemicin, Calicheamycin, Esperamycin, Quercetin, Suramin, Erbstatin, Cyclophosphamid, Mitomycin C, Melphalan, Cisplatin, Bleomycin, Staurosporin oder ein anderer antineoplastisch aktiver Wirkstoff sein. Das Cytostatikum ist über Amino-oder Hydroxyfunktionen mit AA2 verknüpft.

Ganz besonders bevorzugt sind Verbindungen der Formel (I), bei denen

K = Kohlenhydratrest mit der allgemeinen Formel

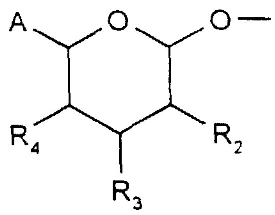

(II),

wobei

A = Methyl, Hydroxymethyl, Carboxy, und Methoxycarbonylmethyl sowie $CH_2$-B, wobei B wiederum ein über das anomere Zentrum verknüpfter Kohlenhydratrest der allgemeinen Formel (II) sein kann.

$R_2$, $R_3$, $R_4$ = einzeln oder zusammen gleich H, Hydroxy, $C_1$-$C_3$-Alkyloxy, Carboxy-$C_1$-$C_3$-alkyloxy sowie davon abgeleitete $C_1$-$C_3$-Alkylester und Amide, Hydroxyalkyloxy, Acyloxy, Carboxy($C_1$-$C_3$-alkyl)-carbonyloxy, Sulfato, oder ein weiterer, im gleichen Rahmen modifizierter und über das anomere Zentrum verknüpfter Kohlenhydratrest in Position $R_3$ oder $R_4$.
Zwei der Reste $R_2$, $R_3$, $R_4$ können auch zusammen eine Epoxygruppe bedeuten.

Die Stereochemie am anomeren Zentrum kann α oder β sein. Durch die Stereochemie an den anderen Zentren kann sich die D-manno-, D-galacto-, L-gulo-, D-gluco-, L-rhamno- oder L-fuco-Konfiguration ergeben.

Sp = Arylenrest, der ortho- oder para-ständig mit K und L modifiziert ist und darüber hinaus neben Wasserstoffatomen noch einen weiteren Substituenten tragen kann, der Methoxy, Nitro oder Chlor sein kann;

L =

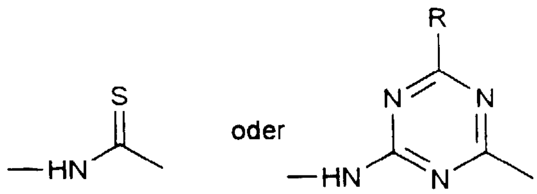

oder

mit R = Chlor oder Hydroxyalkylamino,

AA1 = ist ein Aminosäure-Rest wie Lysin, Alanin, Asparaginsäure, Glutaminsäure, Glycin, Ornithin, Tyrosin, Valin oder Serin in der D- oder L-Konfiguration oder eine direkte Bindung. Der Aminosäurerest kann sowohl über die α-Aminogruppe als auch gegebenenfalls über die Seitenketten-Aminofunktionen und auch über beide Funktionen mit L verknüpft sein und somit gegebenenfalls eine weitere Gruppierung K-Sp-L- tragen, die mit der ersten gleich oder verschieden ist. Falls AA1 weitere funktionelle Gruppen trägt, so sind diese bevorzugt deblockiert

AA2 = ist ein Aminosäure-Rest wie Alanin, Lysin, Glycin, Serin, Ornithin, Diaminopropionsäure in der D- oder L-Konfiguration oder eine direkte Bindung. Der Aminosäurerest kann sowohl über die α-Aminogruppe als auch gegebenenfalls über die Seitenketten-Aminofunktionen und auch über beide Funktionen mit AA1 verknüpft sein und somit gegebenenfalls eine weitere Gruppierung K-Sp-L- tragen, die mit der ersten gleich oder verschieden ist. Falls AA2 weitere funktionelle Gruppen trägt, so sind diese bevorzugt deblockiert.

C kann Batracylin, Methotrexat, Chinolon-a, Etoposid, Melphalan, Taxol, Camptothecin, Daunomycin oder Doxorubicin oder eine wie unten definierte Chinolonoder Naphthyridoncarbonsäure sein. Das Cytostatikum ist über

eine Amino- oder Hydroxyfunktion mit AA2 verknüpft.

Die als Edukte verwendeten Chinolon- oder Naphthyridoncarbonsaurebausteine C lassen sich durch die allgemeine Struktur der Formel (III) darstellen,

$$\text{T-Q} \qquad \text{(III)}$$

in welcher

Q einen Rest der Formeln

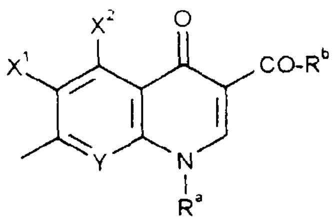
,
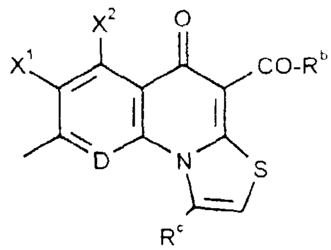
,
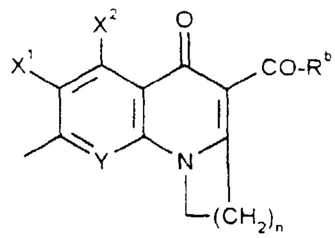

oder

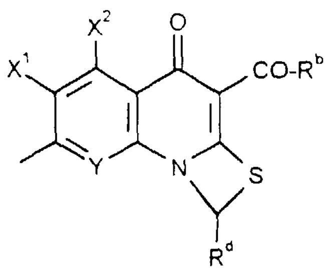

bedeutet, worin

$R^a$ für gegebenenfalls durch Halogen oder Hydroxy ein- oder zweifach substituiertes Alkyl mit 1 bis 4 Kohlenstoffatomen, Vinyl, gegebenenfalls durch 1 oder 2 Fluoratome substituiertes Cycloalkyl mit 3 bis 6 Kohlenstoffatomen, Bicyclo[1.1.1]pent-1-yl, 1,1-Dimethylpropargyl, 3-Oxetanyl, Methoxy, Amino, Methylamino, Dimethylamino, gegebenenfalls durch Halogen, Amino oder Hydroxy einoder zweifach substituiertes Phenyl steht oder auch gemeinsam mit $R^e$ eine dort beschriebene Brücke bilden kann,

$R^b$ für Hydroxy, Alkoxy mit 1 bis 3 Kohlenstoffatomen, Nitromethyl,

$R^c$ für Wasserstoff oder Methyl steht oder auch gemeinsam mit $R^g$ eine dort beschriebene Brücke bilden kann,

$R^d$ für Wasserstoff, $CH_3$, $CH_2F$ oder $=CH_2$,

$X^1$ für Wasserstoff, Halogen oder Nitro,

$X^2$ für Wasserstoff, Halogen, Amino, Hydroxy, Methoxy, Mercapto, Methyl, Halogenmethyl oder Vinyl,

Y für N oder C-$R^c$ steht, worin

$R^c$ für Wasserstoff, Halogen, $CF_3$, $OCH_3$, $OCHF_2$, $CH_3$, CN, $CH=CH_2$ oder $C\equiv CH$ steht oder auch gemeinsam mit $R^a$ eine Brücke der Struktur $-{}^*O-CH_2-CH-CH_3$, $-{}^*S-CH_2-CH_2-$, $-{}^*S-CH_2-CH-CH_3$, $-{}^*CH_2-CH_2-CH-CH_3$ oder $-{}^*O-CH_2-N-R^f$, wobei das mit * markierte Atom mit dem Kohlenstoffatom von Y verknüpft ist und worin

$R^f$ Wasserstoff, Methyl oder Formyl bedeutet,

bilden kann, und

D für N oder C-$R^g$ steht, worin

$R^g$ für Wasserstoff, Halogen, $CF_3$, $OCH_3$, $OCHF_2$ oder $CH_3$ steht oder auch gemeinsam mit $R^c$ eine Brücke der Struktur $-{*}O-CH_2-$, $-{*}NH-CH_2-$, $-{*}N(CH_3)-CH_2-$, $-{*}N(C_2H_5)-CH_2-$, $-{*}N(C_3H_5)-CH_2-$ oder $-{*}S-CH_2-$ bilden kann, wobei das mit * markierte Atom mit dem Kohlenstoffatom von D verknüpft ist,

n 1, 2 oder 3 und

T einen Rest der Formel

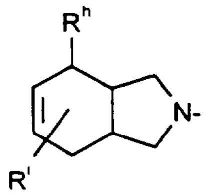
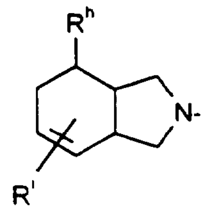
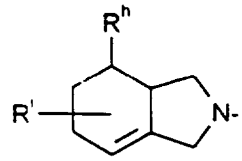

bedeutet, worin

$R^h$ für O-,

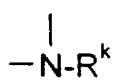 . $CH_2$-O- oder 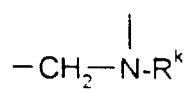

steht, wobei

$R^k$ für Wasserstoff oder Methyl und

$R^i$ für Wasserstoff, $C_1$-$C_3$-Alkyl oder Cyclopropyl steht

Besonders bevorzugt als Cytostatikum C sind die Verbindungen der Formel (III), in welcher

Q einen Rest der Formel

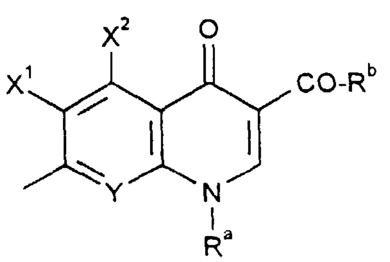

oder

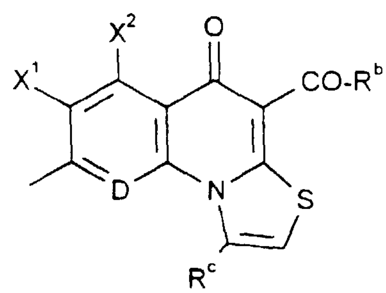

bedeutet, worin

$R^a$ für gegebenenfalls durch 1 Fluoratom substituiertes Alkyl mit 2 bis 4 Kohlenstoffatomen, gegebenenfalls durch 1 Fluoratom substituiertes Cyclopropyl, gegebenenfalls durch Fluor ein- oder zweifach substituiertes Phenyl,

$R^b$ für Hydroxy, Alkoxy mit 1 bis 2 Kohlenstoffatomen,

$R^c$ für Wasserstoff, Methyl steht oder zusammen mit $R^g$ eine dort beschriebene Brücke bilden kann,

$X^1$ für Fluor,

$X^2$ für Wasserstoff oder Amino,

Y für N oder C-$R^e$ steht, worin

$R^e$ für Wasserstoff, Fluor, Chlor, $CF_3$, $OCH_3$, $OCHF_2$ oder C≡CH steht oder auch gemeinsam mit $R^a$ eine Brücke der Struktur -*O-$CH_2$-CH-$CH_3$ oder -*O-$CH_2$-N-$R^f$, wobei das mit * markierte Atom mit dem Kohlenstoffatom von Y verknüpft ist und worin

$R^f$ Methyl bedeutet,

bilden kann,

D für N oder C-$R^g$ steht, worin

$R^g$ für Wasserstoff, Fluor, Chlor, $CF_3$, $OCH_3$ oder $CH_3$ steht oder auch gemeinsam mit $R^c$ eine Brücke der Struktur -*O-$CH_2$-, -*NH-$CH_2$-, -*N($CH_3$)-$CH_2$- oder -*S-$CH_2$- bilden kann, wobei das mit * markierte Atom mit dem Kohlenstoffatom von D verknüpft ist und

T einen Rest der Formel

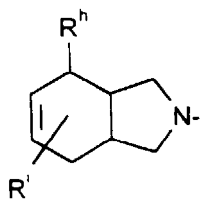
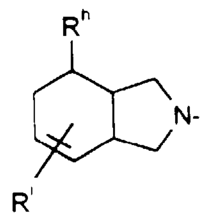
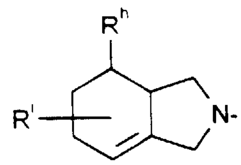

bedeutet, worin

$R^h$ für

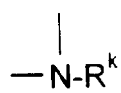

steht, worin

$R^k$ für Wasserstoff oder Methyl steht,

$R^i$ für Wasserstoff oder Methyl steht.

Ebenfalls besonders bevorzugt sind Glycokonjugate mit Camptothecin oder dessen Derivaten.

Von besonderer Bedeutung sind weiterhin die Verbindungen der allgemeinen Formel I, bei denen K, Sp und L Wasserstoff bedeuten und C für Camptothecin steht. Diese Stoffe sind neu, können als Zwischenprodukte zu weiteren Derivaten der allgemeinen Formel I umgesetzt werden und zeigen ihrerseits bereits ein interessantes pharmazeutisches Wirkungsspektrum insbesondere als Cytostatika.

Die erfindungsgemäßen Verbindungen können in stereoisomeren Formen, beispielsweise als Enantiomere oder Diastereomere, oder als deren Gemische, beispielsweise als Racemat, vorliegen. Die Erfindung betrifft sowohl die reinen Stereoisomere als auch deren Gemische.

Die Stereoisomerengemische können, falls erforderlich, in bekannter Weise in die stereoisomer einheitlichen Bestandteile getrennt werden, beispielsweise durch Chromatographie oder durch Kristallisationsverfahren.

Die erfindungsgemäßen Verbindungen können auch in Form ihrer Salze vorliegen Im allgemeinen seien hier Salze mit organischen oder anorganischen Basen oder Säuren sowie innere Salze genannt.

Zu den Säuren, die addiert werden können, gehören vorzugsweise Halogenwasserstoffsäuren, wie z.B. die

Chlorwasserstoffsäure und die Bromwasserstoffsaure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethynolamin, Dicyclohexylamin, Dimethylaminoethanol, Arginin, Lysin, Ethylendiamin oder Phenethylamin.

**Beispielserie A:** **Biologische Testung**

**Beispiel A.1**

**Wachstumsinhibitionstest zur Bestimmung der cytotoxischen Eigenschaften von Glycokonjugaten des Batracylins und des Chinolons a:**

Die humanen Dickdarmzellinien SW 480 und HT 29 (ATCC-Nr. CCL 228 und HBT-38) sowie die Maus-Melanom-Zellinie B 16 F 10 wurden in Rouxschalen in RPMI 1640 Medium unter Zusatz von 10 % FCS gezogen. Anschließend wurde trypsiniert und in RPMI plus 10 % FCS zu einer Zellzahl von 50.000 Zellen/ml aufgenommen. 100 µl Zellsuspension/Well wurden in eine 96 Mikrowellplatte gegeben und 1 Tag bei 37°C im $CO_2$ Brutschrank inkubiert. Anschließend wurden weitere 100 µl RPMI Medium und 1 µl DMSO mit den Prüfsubstanzen zugesetzt Das Wachstum wurde nach Tag 3 und Tag 6 überprüft. Dazu wurde zu jedem Mikrowell 40 µl MTT-Lösung (3-(4,5-Dimethylthiazol-2-yl)-2,5-diphenyltetrazolinbromid) mit einer Ausgangskonzentration von 5 mg/ml $H_2O$ zugesetzt. Es wurde 5 Stunden im $CO_2$-Brutschrank bei 37°C inkubiert. Anschließend wurde das Medium abgesaugt und 100 µl i-Propanol/Well zugesetzt. Nach 30 min Schütteln mit 100 µl $H_2O$ wurde die Extinktion bei 540 nm mit einem Titertek Multiskan MCC/340 (Flow) gemessen.

Die cytotoxische Wirkung der beschriebenen Glycokonjugate des Batracylins ist in der Tabelle 1a als $IC_{50}$-Wert jeweils für die SW 480- und HT 29-Zellinie angegeben.

In der Tabelle 1b sind die $IC_{50}$-Werte für die Chinolon-a-Glycokonjugate an der SW 480-, HT 29- und B 16 F 10-Zellinie zusammengefaßt.

Tabelle 1a

| Substanz | IC 50 [µM] SW 480 | IC 50 [µM] HT 29 |
|---|---|---|
| Batracylin | 25 | 20 |
| 3.2 | 100 | 75 |
| 3.4 | 100 | 65 |
| 3.7 | 55 | n.g. |
| 3.9 | 40 | 55 |
| 3.10 | 100 | 125 |
| 3.11 | 85 | n.g. |
| 3.14 | 40 | n.g. |
| 3.18 | 15 | n.g. |
| 3.19 | 75 | n.g. |
| 3.20 | 100 | n.g. |
| 3.21 | 90 | n.g. |
| 3.23 | 50 | n.g. |
| 3.24 | 95 | n.g. |

Tabelle 1a (fortgesetzt)

| Substanz | IC 50 [µM] SW 480 | IC 50 [µM] HT 29 |
|---|---|---|
| 3.26 | 50 | n.g |
| 3.27 | 110 | n.g. |
| 3.28 | 60 | n.g. |
| 3.29 | 110 | n.g. |
| 3.30 | >250 | n.g. |
| 3.33 | 90 | 70 |
| 4.1 | 25 | 30 |
| 4.3 | 20 | 20 |
| 4.4 | 30 | 25 |
| 4.5 | 15 | 15 |
| 4.6 | 10 | 10 |
| 4.7 | 15 | 15 |
| 4.8 | 50 | 40 |
| 4.9 | 20 | 30 |
| 4.10 | 30 | 30 |
| 4.11 | 15 | 15 |
| 4.12 | 15 | 9 |
| 5.1 | 55 | 45 |
| 5.2 | 20 | 55 |
| 5.6 | >250 | n.g. |
| 5.8 | 100 | >250 |
| 5.9 | 70 | 70 |
| 5.12 | 20 | 20 |
| 5.13 | 20 | 40 |
| 5.14 | 20 | 30 |
| 5.15 | >250 | 70 |
| 5.19 | 35 | 25 |
| 5.20 | 50 | 30 |
| 5.21 | 60 | 80 |
| 5.22 | 30 | 40 |
| 5.23 | 25 | 35 |
| 6.2 | 40 | 50 |
| 6.3 | 70 | 105 |
| 6.7 | 60 | >250 |
| 6.10 | 50 | 50 |
| 6.12 | 35 | 50 |
| 6.15 | >250 | >250 |

Tabelle 1a (fortgesetzt)

| Substanz | IC 50 [µM] SW 480 | IC 50 [µM] HT 29 |
|---|---|---|
| 6.20 | 80 | n.g. |
| 6.21 | 150 | n.g. |
| 6.23 | 107 | 45 |
| 6.25 | 50 | 40 |
| 6.28 | 40 | 25 |
| 6.29 | 95 | 130 |
| 6.30 | 60 | 70 |
| 6.32 | 60 | 60 |
| 6.34 | 50 | n.g. |
| 6.35 | 20 | n.g. |
| 6.36 | 70 | 70 |
| 6.40 | 170 | 60 |
| 6.43 | 90 | 80 |
| 6.46 | 120 | 100 |
| 6.59 | 50 | 50 |
| 6.60 | 50 | 40 |
| 6.80 | 40 | 25 |
| 6.81 | 30 | 30 |
| 6.82 | 125 | n.g. |
| 6.83 | 90 | n.g. |
| 6.85 | 22 | n.g. |
| 7.1 | 40 | 40 |
| 7.2 | 40 | 30 |
| 7.3 | 50 | n.g. |
| 7.5 | 80 | n.g. |
| 7.7 | 100 | >250 |
| 7.8 | 40 | 30 |
| 7.11 | 30 | 25 |
| 7.12 | 10 | 10 |
| 8.10 | 70 | n.g. |
| 8.11 | 45 | n.g. |
| 8.12 | 30 | n.g. |

Tabelle 1b

| Substanz | IC 50 [µM] SW 480 | IC 50 [µM] HT 29 | IC 50 [µM] B 16 F 10 |
|---|---|---|---|
| 10.1 | 50 | >250 | 15 |

Tabelle 1b (fortgesetzt)

| Substanz | IC 50 [µM] SW 480 | IC 50 [µM] HT 29 | IC 50 [µM] B 16 F 10 |
|---|---|---|---|
| 10.2 | 4 | 3 | 5 |
| 10.3 | 5 | 4 | 0,7 |
| 11.2 | 30 | n.g. | 9 |
| 11.6 | 8 | 9 | 5 |
| 11.7 | 12 | 13 | 15 |
| 11.8 | 12 | 16 | 15 |
| 11.9 | 12 | 12 | 9 |
| 11.10 | 8 | 20 | 2 |
| 11.16 | 10 | 10 | 1,5 |
| 11.17 | 75 | 75 | 8 |
| 11.18 | 4,5 | 3,5 | 0,5 |
| 12.1 | 1 | 1,5 | 0,1 |
| 12.2 | 4 | n.d. | 0,8 |
| 12.3 | 2 | n.d. | 0,3 |
| 12.5 | 1 | 4 | 0,2 |
| 12.6 | 4 | 7 | 0,3 |
| 12.7 | 60 | >250 | 20 |
| 12.8 | 8 | 7 | 1 |
| 12.9 | 4 | 8 | 2 |
| 12.10 | 15 | 15 | 4 |
| 12.11 | 2 | | 0.5 |
| 12.12 | 8 | 13 | 0,5 |
| 12.13 | 35 | 100 | 1 |
| 12.14 | 1 | 2 | 0,3 |
| 12.15 | 0,3 | 1 | 0,1 |
| 14.1 | 0,8 | 1 | 1,5 |
| 14.2 | 1 | 6 | 1,5 |
| 14.3 | 8 | 4 | 4 |
| 14.4 | 1,5 | 1 | 0,4 |
| 15.1 | 20 | 20 | 2 |
| 15.2 | 50 | 70 | 15 |
| 16.1 | 50 | 100 | 200 |
| 16.2 | 50 | 60 | 80 |
| 17.1 | 10 | 5 | 5 |
| 17.2 | 4 | 4 | 4 |
| 18.1 | 0,03 | 0,01 | 0,2 |
| 18.2 | 0,02 | 0,02 | 0,2 |

Tabelle 1b (fortgesetzt)

| Substanz | IC 50 [µM] SW 480 | IC 50 [µM] HT 29 | IC 50 [µM] B 16 F 10 |
|---|---|---|---|
| 18.4 | 0,02 | 0,02 | 0,3 |
| 18.5 | 0,2 | 0,2 | 1 |
| 18.9 | 0,08 | 0,06 | 0,7 |
| 18.14 | 0,015 | 0,01 | 0,08 |

Die Kohlenhydratabhängigkeit der biologischen Wirkung ist zusätzlich durch die Unwirksamkeit der den Beispielserien 5, 6 und 11 zugrundeliegenden kohlenhydratfreien Vergleichsverbindungen N-[$N^{\alpha}$,$N^{\varepsilon}$-bis-(4-hydroxyphenylamino-thio-carbonyl)-lysyl]-batracylin und N-[$N^{\alpha}$,$N^{\varepsilon}$-bis-(4-Hydroxyphenylamino-thiocarbonyl)-lysyl-D-alanyl]-batracylin bzw. N-[$N^{\alpha}$,$N^{\varepsilon}$-bis-(4-Hydroxyphenylaminothiocarbonyl)-D-lysyl-chinolon-a belegt ($IC_{50}$-Werte >250).

**Beispiel A.2**

**In-vitro-Untersuchung der Spaltbarkeit der Glycokonjugate**

**Spaltungskinetik mit Humanblut**

1,225 ml Humanblut werden zusammen mit 1,25 ml PBS und 25 µl einer Substratstammlösung (1 mg/ml in 3 % DMSO in PBS) bei 37°C inkubiert. Nach 1 h und 24 h werden jeweils Proben von 1 ml entnommen, mit 1 ml Ethanol gemischt und 20 min bei 4°C stehengelassen. Nach Zentrifugation (5 min bei 3500 U/min) werden 100 µl Überstand für die HPLC-Analyse entnommen.

**Spaltungskinetik mit Zellen**

2,25 ml PBS werden zusammen mit 225 µl einer Zellsuspension (30 mg/ml) und 25 µl Substratstammlösung (1 mg/ml in 3 % DMSO in PBS) bei 37°C inkubiert. Nach 1 h und 24 h werden jeweils Proben von 1 ml entnommen, mit 1 ml Ethanol gemischt und 20 min bei 4°C stehengelassen. Nach Zentrifugation (5 min bei 3500 U/min) werden 100 µl Überstand für die HPLC-Analyse entnommen.

| **HPLC-Bedingungen:** | | |
|---|---|---|
| **Gerät** | Waters-Anlage | |
| **Säule** | Bischoff Hypersil OCS RP 18 5 um 250 x 4 mm | |
| **Eluent** | A: 10mM Kaliumphosphatpuffer pH 4,5 | |
| | B: 80 % Acetonitril/20 % Wasser | |
| **Flow** | 1 ml/min | |
| **Wellenlänge** | 372 nm | |
| **Gradient** | 0 min | 10 % B |
| | 10 min | 60 % B |
| | 15 min | 60 % B |
| | 18 min | 10 %1B |
| | 20 min | 60 % B |

**Eluent für Chinolon-a-Konjugate:**

A: 100 % Methanol
B: 10 mM Kaliumphosphatpuffer pH 2,2;

10 mM Heptansulfonsäure

Tabelle 2a

| Beispiel | %-Spaltung in Human-Blut | | %-Spaltung in SW-480 | | %-Spaltung in Hepatoma | |
|---|---|---|---|---|---|---|
| | 1 h | 24 h | 1 h | 24 h | 1 h | 24 h |
| 3.4 | n.d. | n.d. | 74* | n.d. | 98* | n.d. |
| 3.9 | 0 | 54* | 28* | 100* | 32* | 100* |
| 4.4 | 0 | 0 | 0 | 0 | 0 | 0 |
| 5.9 | 0 | 0 | 0 | 0 | 0 | 0 |
| 6.2 | n.d. | n.d. | 0 | 0 | 0 | 0 |
| 6.12 | n.d. | n.d. | 0 | 0 | 0 | 0 |
| 7.3 | 0 | 0 | 0 | 0 | 0 | 0 |

*: Spaltprodukt ist N-[D-Alanyl]-batracylin

Tabelle 2b

| | %-Spaltung in Human-Blut | | %-Spaltung in SW-480 | | %-Spaltung in Hepatoma | |
|---|---|---|---|---|---|---|
| Beispiel | 1 h | 24 h | 1 h | 24 h | 1 h | 24 h |
| 11.2 | 0 | 0 | 0 | 0 | 0 | 0 |
| 11.6 | 0 | 11 % | 0 | 8 % | 0 | 12% |
| 11.7 | 0 | 0 | 0 | 0 | 0 | 0 |
| 12.1 | 0 | 0 | 0 | 0 | 0 | 0 |
| 12.3 | 0 | 0 | 0 | 0 | 0 | 0 |
| 12.8 | 0 | 0 | 0 | 0 | 0 | 0 |

**Beispiel A.3**

**Untersuchung zur Organverteilung**

Für alle Experimente wurden athymische Nacktmäuse (Stamm NMRI nu/nu), die im "Drug Development Laboratory, Oncotest GmbH", Prof. H.H. Fiebig, Freiburg, gezüchtet werden, benutzt. Die Tiere wurden in Macrolonkäfigen unter Laminar Flow Bedingungen gehalten. Als Tumormaterial wurde Gewebe der Zellinie SW 480 genutzt, das vorher in mehreren Passagen in den Nacktmäusen angezogen wurde.

Den 6 bis 8 Wochen alten Nacktmäusen wurden je Tier zwei Tumore subcutan in die beiden Flanken implantiert. Bis zum Zeitpunkt der Randomisation wurden die Tiere 26 bis 27 Tage gehalten. Die mittlere Tumorgröße war dann 500 mg, entsprechend einem Tumordurchmesser von ca. 10 mm.

Die Pharmakokinetik selbst lief wie folgt ab: den Nacktmäusen wurde die zu untersuchende Substanz injiziert und die Mäuse dann bis zur Probenentnahme nach einer 1/2 h bzw. nach 4 h zurück in die Käfige gesetzt. Die Probenentnahme selbst begann mit der Blutentnahme. Hierzu wurde die Maus mittels Narkoseether betäubt (Dauer 1/2 bis eine Minute). 0,5 h bzw. 4 h nach Substanzinjektion wurde dann der Bauchraum geöffnet und die Maus in Narkose über die Vena cava caudalis innerhalb von 1 bis 2 Minuten entblutet und anschließend durch Genickbruch getötet. Hierdurch kam es zum zentralen Kreislaufstillstand und zur Unterbindung der Organperfusion. Im weiteren wurden dann die einzelnen Organe freipräpariert und entnommen, ein Vorgang, der ca. 5 Minuten in Anspruch nahm. Sofort danach wurden die Organproben und danach der Restkörper gewogen und in flüssigem Stickstoff eingefroren.

Die Substanz "Konjugat 1" wird mit 300 mg/kg Körpergewicht i.p. und die Substanz "Konjugat 2" mit 100 mg/kg iv in die Schwanzvene verabreicht. Je Substanz und Zeitpunkt werden 5 Tiere benutzt.

Die Verteilungsergebnisse sind für Konjugat 1 in der Tabelle 3 und für Konjugat 2 in der Tabelle 4 zusammengefaßt.

**A. Eichreihe:**

5, 10, 50, 100 und 200 µg Substanz gelöst in Ethanol-Wasser (1:1, v/v), wurden zu 1 g Rinderleber gegeben. Anschließend wurde mit 1 g Seesand und 2,5 ml gekühltem Ethanol-Wasser (1:1, v/v) zermörsert, die Proben bei 3500 U/min 2 min zentrifugiert. Nach Abnahme des Überstandes wurde der Rückstand erneut mit 2,5 ml Ethanol-Wasser vermischt, zentrifugiert und die Überstände vereinigt. Jeweils 100 µl wurden entnommen und am HPLC analysiert.

| **HPLC-Bedingungen:** | | |
|---|---|---|
| **Gerät** | Waters Anlage | |
| **Säule** | Bischoff Hypersil ODS RP18 5 µm 250 x 4 mm | |
| **Eluent** | A: 80 % Acetonitril 20 % Wasser | |
| | B: 10 mM Kaliumphosphatpuffer pH 4,5 | |
| **Gradient** | 0 min | 90 % B |
| | 10 min | 40 % B |
| | 15 min | 40 % B |
| | 18 min | 90 % B |
| | 20 min | 90 % B |
| **Flow** | 1 ml/min | |
| **Wellenlänge** | 372 nm | |

**B. Aufarbeitung der Organe**

Die Aufarbeitung der Organe erfolgte analog A, wobei die gesamten Organe mit 2,5 ml Ethanol-Wasser aufgeschlossen und extrahiert wurden

**C. Aufarbeitung des Blutes**

Die entnommene Blutmenge wurde mit 2 ml Ethanol-Wasser (1:1, v/v) vermischt, 2 Min. bei 3500 U/min zentrifugiert und Überstand dekantiert. Zu dem Rückstand wurde erneut 2 ml Ethanol-Wasser (1:1, v/v) gegeben, zentrifugiert und die Überstände vereinigt. Jeweils 100 µl der vereinigten Überstände wurden an HPLC analysiert. Es wurden die unter A verwendeten HPLC-Bedingungen benutzt.

Konjugat 1 (EP 501 250-A1)

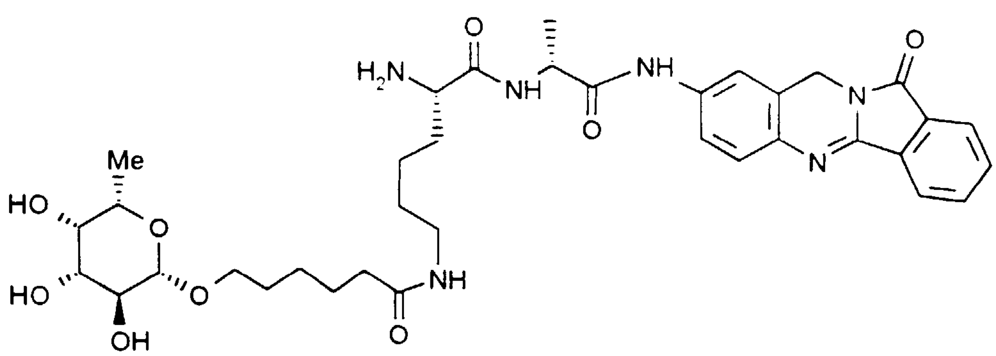

Konjugat 2 (Beispiel 3.9)

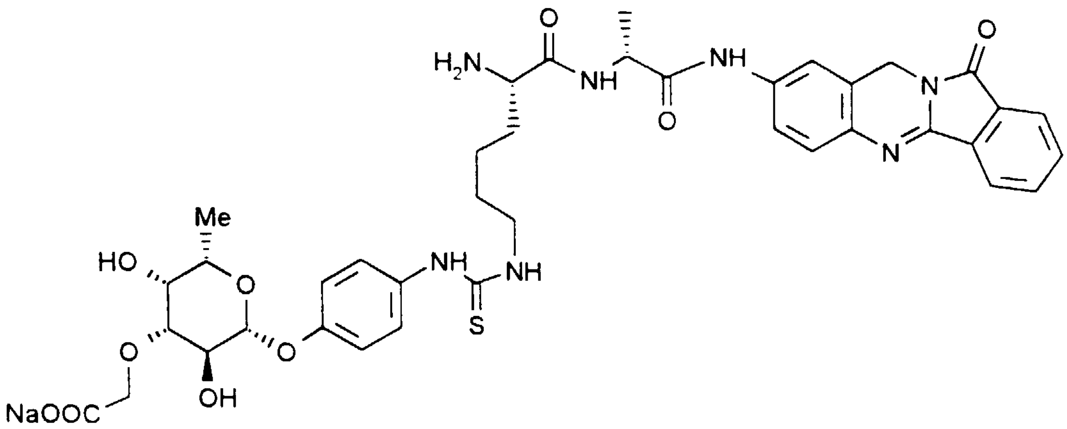

Tabelle 3

| Auswertung Organproben | | | |
|---|---|---|---|
| Organ | Substanz | Mittelwert 1 h (µg/g Organ) | Mittelwert 4 h (µg/g Organ) |
| Blut | Konjugat 1 | 31,2 | - |
| | Batracylin | - | - |
| Tumor | Konjugat 1 | 56,6 | 24,4 |
| | Batracylin | - | - |
| Leber | Konjugat 1 | 770 | 126 |
| | Batracylin | 34,1 | 22,4 |
| Niere | Konjugat 1 | 81 | 78,4 |
| | Batracylin | 26,1 | 27,5 |
| Hirn | Konjugat 1 | - | - |
| | Batracylin | - | - |

Tabelle 4

| Auswertung Organproben | | | |
|---|---|---|---|
| Organ | Substanz | Mittelwert 0,5 h (µg/g Organ) | Mittelwert 4 h (µg/g Organ) |
| Blut | Konjugat 2 | 6,5 | - |
| | Batracylin | - | - |
| Tumor | Konjugat 2 | 2,0 | - |
| | Batracylin | 2,5 | 3,12 |
| Leber | Konjugat 2 | 0,9 | 1,2 |
| | Batracylin | - | - |
| Niere | Konjugat 2 | 17,5 | 0,5 |
| | Batracylin | 10,7 | 0,8 |

Tabelle 4 (fortgesetzt)

| Auswertung Organproben | | | |
|---|---|---|---|
| Organ | Substanz | Mittelwert 0,5 h (µg/g Organ) | Mittelwert 4 h (µg/g Organ) |
| Hirn | Konjugat 2 | - | - |
| | Batracylin | - | - |

**Beispiel A.4**

**Bestimmung der akuten Toxizität von Glycokonjugaten des Batracylins (Einmalapplikation)**

Die akute Toxizität der Batracylin-Derivate wurde an nu/nu Nacktmäusen bestimmt.

Die i.v.-applizierten Substanzen wurden als wäßrige Lösungen, die i.p.-applizierten Substanzen als DMSO Lösungen in Konzentrationen bis zu 400 mg Substanz pro kg Maus einmalig gespritzt.

Die tolerierte Einzeldosis wurde anhand der Gewichtsabnahme der Tiere bis zum Tag 21 nach Applikation und nach der Überlebendszahl der Tiere kalkuliert.

Die tolerierbare Einzeldosis für die Substanzen ist der Tabelle 5 zu entnehmen.

Sie lag bei den Substanzen 3.16; 3.33; 3.9; 6.12; 6.14; 6.2; 6.81; 8.2 über 200 mg Substanz pro kg Maus. Bei den Substanzen 3.33; 6.12; 6.14; 6.2; 6.81 konnte auch mit einer Dosis von 400 mg/kg noch keine akute Toxizität nachgewiesen werden.

Dagegen war das zuckerfreie Lysyl-D-alanyl-batracylin (2.13) schon bei Einzeldosen zwischen 25 und 50 mg kg Maus deutlich toxisch.

Tabelle 5

| **Maximal tolerierbare Einzeldosis von Batracylinderivaten und Chinolon-a-Derivaten** | | |
|---|---|---|
| Beispiel | Tolerierte Einzeldosis in mg/kg Maus | |
| | i.p. | i.v. |
| 2.13 | 50 | 25 |
| 3.4 | 200 | <100 (2 Tiere verstorben) |
| 3.9 | 200 | 200 |
| 3.16 | >200 | n.d. |
| 3.33 | n.g. | >400 |
| 6.2 | >400 | n.g. |
| 6.12 | n.g. | >400 |
| 6.14 | >400 | n.g. |
| 6.81 | n.g. | >400 |
| 8.2 | >200 | n.d. |
| Chinolon-a | n.d. | <25 |
| 12.3 | n.d. | >200 |
| | | |

**Beispiel A.5**

**Bestimmung der akuten Toxizität nach Mehrfachapplikation**

Die Substanzen wurden zum Teil i.v. und zum Teil i.p. appliziert, entweder täglich an den Tagen 1 bis 4 und 7 bis 10 oder an den Tagen 1, 5 und 9. Die Dosierungen lagen bei 400, 200 und 100 mg/kg/Tag. Die Auswertung erfolgte nach Gewichtsabnahme bis zum Tag 21 und nach der Zahl der überlebenden Tiere. Für die Versuche wurden je Substanz und Dosis 5 Tiere eingesetzt. Die Ergebnisse sind in der Tabelle 6 zusammengefaßt.

Tabelle 6

| Maximal tolerierbare Dosis bei Mehrfachapplikation | | | |
|---|---|---|---|
| | Applikation | Dosierung am Tag | MTD mg/kg/Tag |
| 3.9 | i.v. | 1-4, 7-10<br>1-4 | ~50<br>~100 |
| 3.33 | i.v. | 1, 5, 9 | >400 |
| 6.2 | i.p. | 1-4, 7-10<br>1, 5, 9 | ~400<br>>400 |
| 6.12 | i.v. | 1-4, 7-10<br>1, 5, 9 | >400<br>>400 |
| 6.14 | i.p. | 1, 5, 9 | >400 |
| 6.81 | i.v. | 1-4, 7-10<br>1, 5, 9 | ~200<br>>400 |
| Batracylin | i.p. | 1, 5, 9 | ~100 |

**Beispiel A.6**

**Hämatopoetische Aktivität von Glycokonjugaten des Chinolons-a im Vergleich zum zugrundeliegenden Wirkstoff:**

**Material und Methoden**

in vitro:

Knochenmarkzellen werden aus dem Femur der Maus gespült. $10^5$-Zellen werden in McCoy 5A-Medium (0,3 % Agar) zusammen mit rekombinanten murinen GMCSF (Genzyme; Stammzellen-Kolonienbildung) und den Substanzen ($10^{-4}$ bis 100 µg/ml) bei 37°C und 7 % $CO_2$ inkubiert. 7 Tage später werden die Kolonien (<50 Zellen) und Kluster (17-50 Zellen) ausgezählt.

in vivo:

Mäuse werden subkutan mit 1, 3, 10, 30 mg/kg der Verbindungen behandelt. Zu verschiedenen Zeiten (3, 24, 48, 72 p. inj.) werden die Femura entfernt und die Knochenmarkzellen isoliert. 2 x $10^5$-Zellen werden wie oben beschrieben mit GM-CSF inkubiert und nach 7 Tagen die Kolonien und Kluster ausgezählt.

**Ergebnisse:**

Wie in Tab. 7 abgebildet, zeigen die untersuchten Glycokonjugate eine gegenüber Chinolon-a um Faktor $10^5$ bis $10^3$ verminderte Hemmung der Knochenmarkstammzellproliferation.

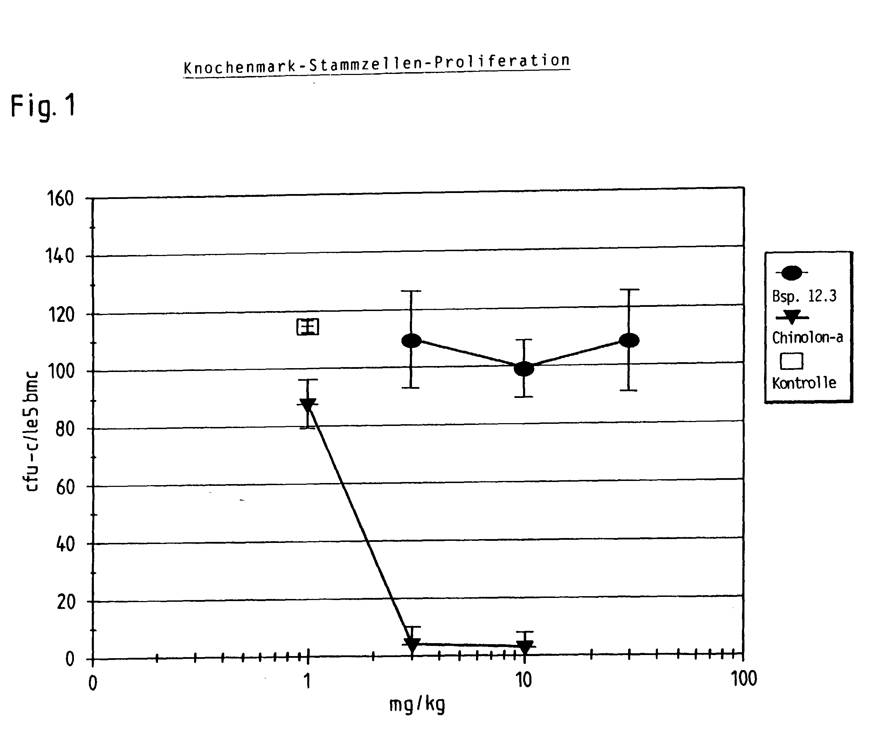

Auch in vivo war im Vergleich zu Chinolon-a keine Hemmung der Stammzellproliferation durch Verbindung 12.3 bis 30 mg/kg zu beobachten. Schon mit 3 mg/kg Chinolon-a wurde eine massive Suppression der Stammzellproliferation induziert (Abb. 1).

Tabelle 7

| CSF-induzierte Proliferation von Knochenmarkstammzellen der Maus | |
|---|---|
| Beispiele Chinolon-a | $IC_{50}$ [µg/ml] 0,0002 |
| 11.2 | 22,5 |
| 11.7 | 2,9 |
| 12.1 | 0,21 |
| 12.3 | 0,27 |

Tabelle 7 (fortgesetzt)

| CSF-induzierte Proliferation von Knochenmarkstammzellen der Maus | |
|---|---|
| Beispiele Chinolon-a | $IC_{50}$ [µg/ml] 0,0002 |
| 12.6 | 0,3 |
| 12.8 | 0,3 |
| 14.1 | 2,9 |
| 14.2 | 3,6 |
| 14.4 | 3,6 |

**Beispiel A.7**

**Antineoplastische Aktivität von Chinolon-a-Konjugaten**

Die In-vitro-Aktivität von Glycokonjugaten des Chinolons-a wurde an humanen Tumorxenografts in einem Zweischicht-Weichagar-Kultur-System nach Hamburger und Salmon bestimmt (Science 197: 461-463).

Die soliden Tumore wurden zunächst in der athymischen Nacktmaus (NMRI nu/nu) angezogen, operativ gewonnen und mechanisch zerkleinert. Einzelzellen wurden anschließend durch Inkubation in einem Enzymgemisch von Collagenase 0,05 %, DNAse 0,07 % und Hyaluronidase 0,1 % in RPMI bei 37°C für 30 Minuten gewonnen. Die Zellen wurden 2 x gewaschen und anschließend durch ein Sieb mit 200 µm und 20 µm Maschenweite gegeben.

Es wurde folgende Kulturmethode angewandt:

Die Bodenschicht enthält 0,2 ml Iscoves's Modified Dulbeccos Medium mit 20 % fötalem Kälberserum und 0,7 % Agar. Auf diese Schicht wurden 40 000 bis 200 000 Zellen in 0,2 ml desselben Mediums und 0,4 % Agar in 24 Multiwell-Platten aufgetragen. Die cytostatischen Substanzen wurden in 0,2 ml Medium zugesetzt.

Die Kulturen wurden bei 37°C in einer 7 %igen $CO_2$-Atmosphäre für 6 bis 15 Tage inkubiert. Anschließend wurden die gewachsenen Kolonien im Invertmikroskop ausgezählt, wobei 24 Stunden vor der Auswertung der lebenden Kolonien mit einem Tetrazoliumchlorid-Farbstoff angefärbt wurden.

Der Wirkstoffeffekt wird in Prozent überlebender Kolonien im Vergleich mit der Koloniezahl unbehandelter Platten (T/C = Koloniezahl behandelt x 100/Kontrollzahl unbehandelt) ausgedrückt.

Eine Substanz ist aktiv, wenn der T/C-Wert ≤30 % ist.

In Tabelle 8 ist dieser Wert als $IC_{70}$-Wert in µg/ml angegeben.

Tabelle 8

| | **$IC_{70}$ / µg/ml** | | | | |
|---|---|---|---|---|---|
| Beispiel | 11.7 | 12.1 | 12.3 | 12.5 | Chinolon-a |
| CXF 280 | >100 | 70 | 5 | <0,3 | 6 |
| HT 29 | 56 | 6 | 5 | 11 | 20 |
| SW 480 | 18 | 8 | 11 | 10 | 3 |
| LXFL 529 | 4 | 0,9 | 2 | 2 | 3 |
| LXFS 538 | 18 | 0,4 | 0,5 | <0,3 | <0,3 |
| MEXF 989 | 3 | <0,3 | <0,3 | 0,5 | <0,3 |
| OVXF 899 | 234 | 45 | 162 | 55 | 2 |
| OVXF 1023 | 136 | 12 | 10 | 9 | 0,5 |

**Beispiel A.8:**

**In-vivo-Versuche:**

Methode:

Mäuse werden am Tag 0 mit $5x10^6$ B16F10 Tumorzellen inokuliert. Die tumortransplantierten Tiere entwickeln

solide peritoneale Tumoren und werden dann täglich mit Testsubstanzen bzw. mit der Vehikel-Kontrolle behandelt. In der Kontrollgruppe sterben normalerweise 50% der Tiere zwischen Tag 14 und 20. Die Testsubstanzen werden in Puffer oder einem organischen Lösemittelsystem bestehend aus 20% Methanol und 20% Dimethylsulfoxid in 0,7%iger Kochsalzlösung appliziert.

Die Gabe des Vehikels zeigte keinen Einfluß auf die Überlebenszeit der Tiere. Der therapeutische Erfolg ergibt sich aus der Verlängerung der Überlebenszeit der behan-delten Tiere. Die Verträglichkeit der Verbindungen wird parallel an nicht tumor-tragenden Tieren analysiert. Aus Verträglichkeit und Verlängerung der Überlebenszeit kann ein therapeutischer Index abgeschätzt werden.

Tabelle 9:

| % Überlebende | | | | | |
|---|---|---|---|---|---|
| | Tag 0 | Tag 20 | Tag 25 | Tag 30 | Tag 35 |
| Kontrolle | 100 | 70 | 30 | 10 | 10 |
| 11.12 1mg/kg | 100 | 90 | 60 | 30 | 30 |
| 11.12 (100mg/kg) | 100 | 100 | 100 | 90 | 90 |
| Chinolon-a (0,1 mg/kg) | 100 | 100 | 100 | 60 | 40 |
| Etoposid (5mg/kg) | 100 | 90 | 80 | 80 | 70 |

Tabelle 9 zeigt die therapeutische Wirksamkeit der Verbindung aus Beispiel 11.12 an Mäusen, die mit einem B16F10 Tumor transplantiert wurden.

**Beispiel A.9:**

**In-vivo-Hemmung des Tumorwachstums unter Verwendung eines Nacktmaus-Modells**

Material:

Für alle in-vivo-Experimente zur Untersuchung der Hemmung des Tumorwachstums wurden athymische Nacktmäuse (NMRI nu/nu-Stamm) verwendet. Das ausgewählte großzellige Lungenkarzinom LXFL 529 wurde durch serielle Passage in Nacktmäusen entwickelt. Der menschliche Ursprung des Tumors wurde durch isoenzymatische und immunohistochemische Methoden belegt.

Experimenteller Aufbau:

Der Tumor wurde subcutan in beide Flanken von 6 bis 8 Wochen alten nu/nu-Nacktmäusen implantiert. Die Behandlung wurde, abhängig von der Verdopplungszeit, gestartet sobald die Tumoren einen Durchmesser von 5 - 7 mm erreicht hatten. Die Mäuse wurden der Behandlungsgruppe und der Kontrollgruppe (5 Mäuse pro Gruppe mit 8 - 10 auswertbaren Tumoren) durch Randomisieren zugeteilt. Die einzelnen Tumoren der Kontrollgruppe wuchsen alle progressiv. Die Größe der Tumoren wurde in zwei Dimensionen mittels Schieblehre vermessen. Das Tumorvolumen, das gut mit der Zellzahl korreliert, wurde anschließend für alle Auswertungen verwendet. Das Volumen wurde gemäß der Formel "Länge x Breite x Breite / 2" berechnet ($[a \times b^2] / 2$, a bzw. b stehen für zwei rechtwinklig angeordnete Durchmesser).
Die Werte des relativen Tumorvolumens (RTV) wurden für jeden einzelnen Tumor durch Dividieren der Tumorgröße am Tag X mit der Tumorgröße des Tages 0 (zum Zeitpunkt der Randomisierung) berechnet. Die mittleren Werte des RTV wurden dann für die weitere Auswertung verwendet.
Die Hemmung der Zunahme des Tumorvolumens (Tumorvolumen der Testgruppe/ Kontrollgruppe, T/C, in Prozent) war der abschließende Meßwert.

Behandlung:

Alle Verbindungen wurden gemäß einem intermittierenden Plan jeweils am Tag 1, 5 und 9 verabreicht. Weiterhin wurden alle Verbindungen intraperitoneal (i.p) unter Verwendung von Wasser als Lösungsmittel appliziert

Tabelle 10

| Therapie | Dosis[a)][mg/kg/ Tag] | Überlebenszeit [Tage] | | Zahl der Tumoren | relatives Tumorvolumen [% des Tages 0] [b)] | Optimales T/C[b)] |
|---|---|---|---|---|---|---|
| Kontrollgruppe | - | 19<br>>21<br>14 | >21<br>>21 | 10 | 1552 | 100 % |
| 12.6 | 100 | 23<br>26<br>>26 | >26<br>>26 | 8 | 300.7 | 19.4 % |
| 12.8 | 50 | >21<br>>21<br>>21 | >21<br>>21 | 9 | 502.2 | 32.3 % |
| 12.14 | 25 | 23<br>19<br>>26 | >26<br>26 | 8 | 519.5 | 33.5% |

a) maximal tolerierte Dosis (MTD)
b) an Tag 19

Die Camptothecin-Verbindungen der Beispielserie 18 zeigten in diesem Test in der Regel vergleichbare oder bessere Wirkung.

**Beispielserie B: Synthesebeispiele**

**Beispiel 1.1**

**p-Aminophenyl-2-O-methyl-β-L-fucosid**

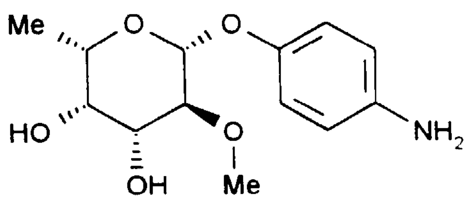

**1.1.a) p-Nitrophenyl-3,4-O-isopropyliden-β-L-fucosid:**

Eine Lösung von p-Nitrophenyl-β-L-fucosid (750mg, 2,63mmol) in 40ml DMF/Dioxan 1:2 wird bei 0°C mit 65mg p-Toluolsulfonsäure und in 30 min-Abständen mit 5x100 µl 2-Methoxypropen versetzt. Nach 16h Rühren bei 20°C wird eingeengt und durch Flash-Chromatographie (Dichlormethan/Methanol 99:1) gereinigt. Nach Einengen erhält man 710mg (83%) eines weißen Feststoffs.

**1.1.b) p-Nitrophenyl-2-O-methyl-3,4-O-isopropyliden-β-L-fucosid:**

100mg (0,307mmol) der Verbindung aus Beispiel 1.1.a werden zusammen mit 96µl Methyljodid in 10ml THF vorgelegt und dann portionsweise mit 11mg Natriumhydrid (80%ig) versetzt. Nach 3h Rühren bei 20°C werden nochmals 96µl Methyljodid und 11mg Natriumhydrid nachgesetzt. Nach weiteren 16h Rühren bei 20°C werden etwas Wasser und 100ml Dichlormethan zugesetzt. Der Ansatz wird zweimal mit Wasser ausgeschüttelt, die organische Phase eingeengt und das Produkt anschließend säulenchromatographisch (Petrolether/Essigester 8:1) gereinigt. Ausb.: 78mg (75%)

**1.1) p-Aminophenyl-2-O-methyl-β-L-fucosid:**

78mg (0,23mmol) p-Nitrophenyl-2-O-methyl-3,4-O-isopropyliden-β-L-fucosid werden 16h bei 20°C in 3ml 80%iger Essigsäure gerührt. Anschließend wird die Essigsäure i.vac. entfernt, der Ansatz mit 10ml Methanol versetzt und nach Zusatz von Platin-dioxid in einer Wasserstoffatmosphäre unter geringem Überdruck hydriert. Die Suspension wird über Celite filtriet und das Filtergut mit Methanol gewaschen. Nach chromatographischer Reinigung (Dichlormethan/Methanol 97,5:2,5) erhält man 77mg (80%) des Zielproduktes. [DC: Dichlormethan/Methanol 9:1 $R_f$= 0,42].

**Beispiel 1.2**

**p-Aminophenyl-3-O-methyl-β-L-fucosid**

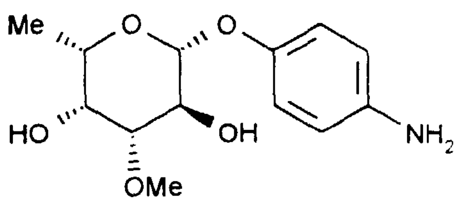

**1.2.a) p-Nitrophenyl-3-O-methyl-β-L-fucosid:**

6g (21mmol) p-Nitrophenyl-β-L-fucosid in 300ml absol. Methanol werden mit 7,84g (31,5mmol) Dibutylzinnoxid versetzt und 2h unter Rückfluß erhitzt. Anschließend wird eingeengt, der Rückstand getrocknet und dann in 300ml DMF aufgenommen. Nach Zugabe von 15,7ml Methyljodid wird der Ansatz 40h bei 70°C gerührt. Das Lösungsmittel wird i. vac. entfernt und der Rückstand in 300ml Dichlormethan aufgenommen. Die Suspension wird filtriert, die verbleibende Lösung erneut eingeengt und einer Flash-Chromatographie (Dichlormethan/Methanol 99:1) unterzogen. Nach Einengen erhält man 3815mg (61%) des Zielproduktes.

**1.2) p-Aminophenyl-3-O-methyl-β-L-fucosid:**

3,81g (12,73mmol) p-Nitrophenyl-3-O-methyl-β-L-fucosid werden analog zu Beispiel 1.1 hydriert. Ausb.: 3g (88%). [DC: Dichlormethan/Methanol 9:1 $R_f$= 0,53].

**Beispiel 1.3**

**p-Aminophenyl-3-O-methyl-α-L-fucosid**

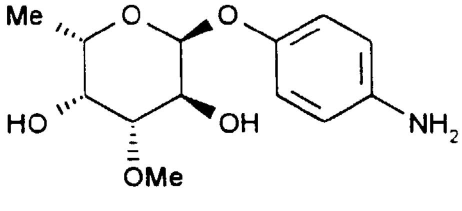

Herstellung analog zu Beispiel 1.2 ausgehend von p-Nitrophenyl-a-L-fucosid. Ausb.: 63% über 2 Stufen. [DC

Dichlormethan/Methanol 9:1 $R_f$= 0,39].

**Beispiel 1.4**

**p-Aminophenyl-4-O-methyl-β-L-fucosid**

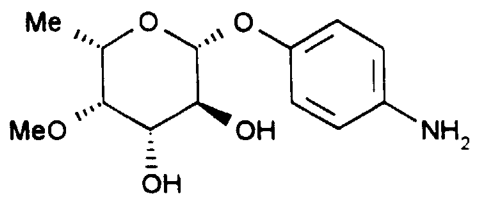

**1.4.a) p-Nitrophenyl-2-O-benzyl-4-O-acetyl-β-L-fucosid:**

1g (3,5mmol) p-Nitrophenyl-β-L-fucosid in 100ml absol. THF werden mit 31mg p-Toluolsulfonsäure und 1134mg (7mmol) Triethylorthoacetat versetzt. Nach 15min Rühren bei 20°C wird das Lösungsmittel i. vac. abdestilliert. Der Rückstand wird in 50ml THF und 3ml DMF aufgenommen und mit 4165µl Benzylbromid sowie 210mg Natriumhydrid (60%ig) versetzt. Nach 1h Rühren bei 20°C werden 10ml 80%ige Essigsäure zugegeben, eingeengt und der Rückstand durch Flash-Chromatographie (Dichlormethan/Methanol 99:1) gereinigt. Nach Einengen und Trocknen erhält man 1236mg (85%) des Zielproduktes.

**1.4.b) p-Nitrophenyl-2-O-benzyl-3-O-acetyl-4-O-methyl-β-L-fucosid:**

1000mg (2,39mmol) p-Nitrophenyl-2-O-benzyl-4-O-acetyl-β-L-fucosid werden in 60ml Benzol gelöst. Nach Zugabe von 2988µl Methyljodid und 1109mg Silberoxid wird der Ansatz 8h unter Rückfluß erhitzt. Das entstehende Produktgemisch wird durch Flash-Chromatographie (Dichlormethan/Methanol 99:1) in die Komponenten aufgetrennt. 239mg (23%) p-Nitrophenyl-2-O-benzyl-3-O-acetyl-4-O-methyl-β-L-fucosid werden neben 653mg (63%) des isomeren p-Nitrophenyl-2-O-benzyl-3-O-methyl-4-O-acetyl-β-L-fucosids als weißer Feststoff isoliert.

**1.4) p-Aminophenyl-4-O-methyl-β-L-fucosid:**

224mg (0,52mmol) p-Nitrophenyl-2-O-benzyl-3-O-acetyl-4-O-methyl-β-L-fucosid werden in 20ml Methanol gelöst und mit 390µl einer 1N Natriummethylat-Lösung versetzt. Nach 16h Rühren bei 20°C wird mit 80%iger Essigsäure neutralisiert, eingeengt und in Dichlormethan aufgenommen. Die organische Phase wird mit 1N Natriumhydrogencarbonat-Lösung sowie mit Wasser gewaschen, getrocknet und eingeengt. Der Rückstand wird in 20ml Methanol aufgenommen und über Palladium/Aktivkohle in Analogie zu Beispiel 1.1 hydriert. Nach Einengen wird das Produkt in Wasser aufgenommen und lyophilisiert. 119 mg (88%) eines weißen amorphen Feststoffes werden isoliert. [DC: Dichlormethan/Methanol 9:1 $R_f$= 0,38]

**Beispiel 1.5**

**p-Aminophenyl-3-O-n-propyl-β-L-fucosid**

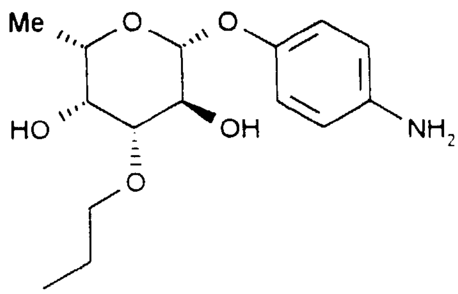

**1.5.a) p-Nitrophenyl-2-O-benzyl-3-O-n-propyl-4-O-acetyl-β-L-fucosid:**

In Analogie zu Beispiel 1.4.b werden aus Verbindung 1.4.a mit Propyljodid die isomeren 3- und 4-Propylierungsprodukte hergestellt und chromatographisch aufgetrennt. Man erhält p-Nitrophenyl-2-O-benzyl-3-O-n-propyl-4-O-acetyl-β-L-fucosid in 49%iger Ausbeute neben p-Nitrophenyl-2-O-benzyl-3-O-acetyl-4-O-n-propyl-β-L-fucosid in 29%iger Ausbeute.

**1.5.b) p-Aminophenyl-3-O-n-propyl-β-L-fucosid:**

Synthese aus Beispiel 1.5.a) Fraktion 1 in Analogie zu Beispiel 1.4. Ausb.: 78% [DC Dichlormethan/Methanol 9:1 $R_f$= 0,42]

**Beispiel 1.6**

**p-Aminophenyl-3-desoxy-β-L-fucosid**

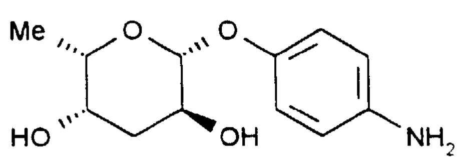

**1.6.a) p-Nitrophenyl-3,6-didesoxy-3-chlor-4-O-acetyl-β-L-gulosid:**

1g (3,5mmol) p-Nitrophenyl-β-L-fucosid in 100ml THF werden mit 31mg p-Toluolsulfonsäure und 1134mg (7mmol) Triethylorthoacetat versetzt. Nach 15min Rühren bei 20°C wird das Lösungsmittel i.vac. abdestilliert. Man gibt 100ml einer gesättigten Lösung von Chlorwasserstoff in Dichlormethan zu. Nach 10min Reaktionszeit wird eingeengt und das Produkt durch Flash-Chromatographie (Dichlormethan/Methanol 99:1) gereinigt. Man erhält 793 mg (65%) des Zielproduktes. [DC: Dichlormethan/Methanol 97,5:2,5 $R_f$= 0,36]

**1.6.b) p-Nitrophenyl-3,6-didesoxy-3-chlor-β-L-gulosid:**

375mg (1,08mmol) p-Nitrophenyl-3,6-didesoxy-3-chlor-4-O-acetyl-β-L-gulosid werden in 25ml Methanol gelost und mit 10 Tropfen 1N Natriummethylat-Lösung versetzt. Nach 20min wird mit Essigsäure angesauert, eingeengt und zwischen 400ml Dichlormethan und 60ml Wasser verteilt. Die organische Phase wird getrocknet, eingeengt und

aus Dichlormethan/Ether gefällt. Man erhält 315mg (96%) des Zielproduktes.

**1.6) p-Aminophenyl-3-desoxy-β-L-fucosid:**

315mg (1,04mmol) p-Nitrophenyl-3,6-didesoxy-3-chlor-β-L-gulosid werden in 40ml Methanol gelöst, mit 200mg Palladium auf Aktivkohle sowie mit 290µl Triethylamin versetzt und in einer Wasserstoffatmosphäre bei geringem Überdruck über 4 Tage hydriert. Die Suspension wird filtriert, gewaschen, eingeengt und das Produkt durch Flash-Chromatographie (Dichlormethan/Methanol 97,5:2,5) gereinigt. Man erhält 160mg (65%) der Desoxy-Verbindung. [DC: Dichlormethan/Methanol 95:5 $R_f$= 0,18]

**Beispiel 1.7**

**p-Aminophenyl-3,4-didesoxy-β-L-fucosid**

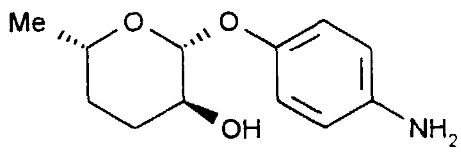

400mg (1,16mmol) p-Nitrophenyl-3,6-didesoxy-3-chlor-4-O-acetyl-β-L-gulosid (Beispiel 1.6.a) werden in 55ml Methanol gelöst, mit 323µl Triethylamin versetzt und in einer Wasserstoffatmosphäre bei geringem Überdruck über Palladium/Aktivkohle (10%) hydriert. Nach 16h Rühren bei 20°C wird über Celite filtriert, nachgewaschen, eingeengt und erneut in 100ml Methanol aufgenommen. Man setzt 1,5ml einer 1N Natriummethylat-Lösung zu und rührt 16h bei Raumtemperatur. Man neutralisiert mit Essigsäure, engt ein und trennt die entstandenen Produkte durch Flash-Chromatographie (Dichlormethan/Methanol 97,5:2,5). Nach Einengen der entsprechenden Fraktionen und Umfällen aus Methanol/Ether erhält man 120mg (46%) der Zielverbindung [DC: Dichlormethan/Methanol 95:5 $R_f$= 0,31] neben 77mg (28%) von p-Aminophenyl-3-desoxy-β-L-fucosid [DC: Dichlormethan/Methanol 95:5 $R_f$= 0,18].

**Beispiel 1.8**

**p-Aminophenyl-3,4-epoxy-β-L-fucosid**

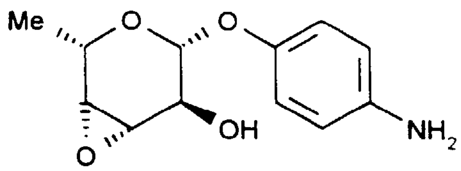

80mg (0,23mmol) p-Nitrophenyl-3,6-didesoxy-3-chlor-4-O-acetyl-β-L-gulosid (Beispiel 1.6.a) werden in 10ml Methanol aufgenommen und mit 345µl 1N Natriummethylat-Lösung versetzt. Nach 1h Ultraschallbehandlung säuert man mit 80%iger Essigsäure an, engt ein und chromatographiert mit Dichlormethan/Methanol 99:1. Nach Einengen der relevanten Fraktionen wird in Methanol aufgenommen und über Palladium/Aktivkohle in Analogie zu Beispiel 1.1 hydriert. Man erhält 46mg (75%) der Zielverbindung. FAB-MS: m/e = 238 = M+1.

**Beispiel 1.9**

**p-Aminophenyl-4-desoxy-β-L-fucosid**

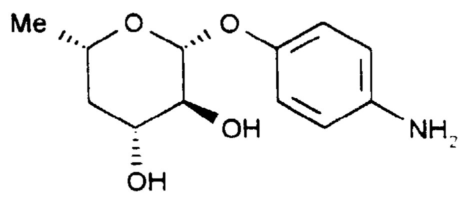

Diese Verbindung wurde in Analogie zur Vorschrift von T.Lindhorst und J.Thiem in Carbohydr. Res. 209 (1991), 119 ausgehend von p-Nitrophenyl-β-L-fucosid über p-Nitrophenyl-2,3-di-O-benzoyl-4,6-didesoxy-4-jodo-β-L-fucosid hergestellt. [DC: Dichlormethan/Methanol 90:10 $R_f$= 0,3].

**Beispiel 1.10**

**p-Aminophenyl-3-O-carboxymethyl-β-L-fucosid**

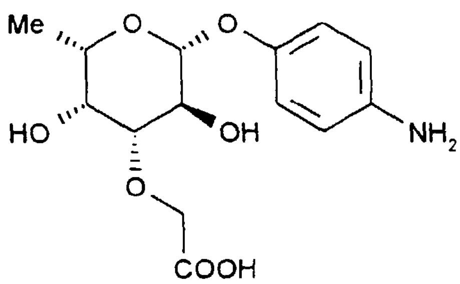

**1.10.a) p-Nitrophenyl-3-O-methoxycarbonylmethyl-β-L-fucosid:**

1g (3,5mmol) p-Nitrophenyl-β-L-fucosid und 1,3g (5,2mmol) Dibutylzinnoxid werden in 50ml Methanol 2h unter Rückfluß erhitzt. Die Lösung wird eingeengt, der Rückstand in 50ml Dioxan aufgenommmen, mit 2ml Bromessigsäure-methylester sowie 100mg Tetrabutylammoniumjodid versetzt und 16h unter Rückfluß erhitzt. Das Lösungsmittel wird abgedampft und das Produkt durch Flash-Chromatographie (Dichlormethan/Methanol 99:1) gereinigt. Nach Einengen der entsprechenden Fraktionen und Umfällen aus Methanol/Ether erhält man 455mg (37%) der Zielverbindung.

**1.10) p-Aminophenyl-3-O-carboxymethyl-β-L-fucosid:**

282mg (0,79mmol) p-Nitrophenyl-3-methoxycarbonylmethyl-β-L-fucosid werden in 20ml Methanol gelöst und mit 440µl einer 2N Lithiumhydroxid-Lösung versetzt. Nach 2h Rühren bei 20°C wird mit saurem Ionenaustauscher SC108 auf pH3 eingestellt und filtriert. Zum Filtrat werden 250mg Palladium auf Aktivkohle zugesetzt. Anschließend wird 1,5h mit Wasserstoff bei geringem Überdruck hydriert, der Katalysator abgetrennt und mit Methanol gewaschen. Einengen, Aufnehmen in Wasser und Gefriertrocknung führt in 86%iger Ausbeute zum Zielprodukt (212mg). [DC: Acetonitril/Wasser/Eisessig 5:1:0,2 $R_f$= 0,24]

**Beispiel 1.11**

**p-Aminophenyl-3-O-methoxycarbonylmethyl-β-L-fucosid**

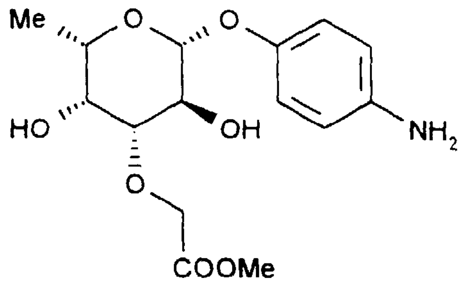

250mg (0,7mmol) p-Nitrophenyl-3-methoxycarbonylmethyl-β-L-fucosid (Beispiel 1.10.a) werden in 20ml Methanol gelöst und 1,5h mit Wasserstoff über Palladium auf Aktivkohle bei geringem Überdruck hydriert. Der Katalysator wird abgetrennt und mit Methanol gewaschen. Einengen, Aufnehmen in Wasser und Gefriertrocknung führen zu 195mg (85%) Zielprodukt.
[DC Dichlormethan/Methanol 9:1 $R_f$=0,43; FAB-MS: m/e = 328 = M+1.]

**Beispiel 1.12**

**p-Aminophenyl-3-O-hydroxyethyl-β-L-fucosid**

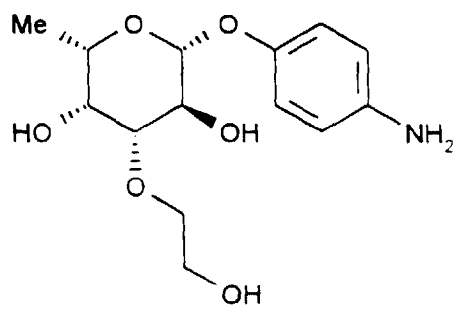

**1.12.a) p-Nitrophenyl-3-O-hydroxyethyl-β-L-fucosid:**

1000mg (2,8mmol) p-Nitrophenyl-3-methoxycarbonylmethyl-β-L-fucosid werden in einer Mischung aus 160ml THF und 40ml Wasser gelöst und mit 53mg Natriumborhydrid versetzt. Nach 10min wird das Lösungsmittel abgedampft und der Rückstand durch Flash-Chromatographie (Dichlormethan/Methanol 95:5) gereinigt. Nach Einengen der entsprechenden Fraktionen, Aufnehmen in Wasser und Gefriertrocknung erhält man 362mg (40%) des Zielproduktes.

**1.12) p-Aminophenyl-3-O-hydroxyethyl-β-L-fucosid:**

Nach Hydrierung von 362mg der Verbindung aus Beispiel 1.12.a) in Analogie zu Beispiel 1.1 erhält man 270mg (82%) des Zielproduktes. [DC: Acetonitril/Wasser 10:1 $R_f$= 0,43]

**Beispiel 1.13**

**p-Aminophenyl-2-O-carboxymethyl-β-L-fucosid**

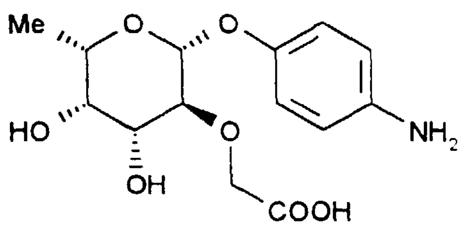

**1.13.a) p-Nitrophenyl-2-O-methoxycarbonylmethyl-β-L-fucosid:**

250mg (0,88mmol) p-Nitrophenyl-β-L-fucosid werden in 25ml abs. THF und 3ml DMF gelöst. Man gibt 80mg (2,64mmol) 80%iges Natriumhydrid zu und nach 10min Rühren bei 20°C 35μl Bromessigsäurebenzylester. In 10min-Abständen werden 3 weitere Zugaben von je 35μl Bromessigsäurebenzylester gemacht. Man läßt 30min nachrühren und quencht mit Methanol. Nach weiteren 10min wird mit 5ml 80%iger Essigsäure angesäuert. Man engt ein und destilliert mit Dichlormethan nach. Die Reinigung mittels Flash-Chromatographie wird mit dem Laufmittelsystem Dichlormethan/Methanol/Eisessig 90:10:1 begonnen. Später steigt man im gleichen System auf das Verhältnis 80:20:2 um. Nach dem Einengen der entsprechenden Fraktionen werden die Rückstände mit Ether digeriert, und man erhält aus dem frühen Eluat 157mg (42%) der Zielverbindung. [DC: Acetonitril/Wasser/Eisessig 5:1:0,2 $R_f$= 0,65] Aus den späten Eluaten wird die isomere 3-O-alkylierte Verbindung erhalten (33%). [DC: Acetonitril/Wasser/Eisessig 5:1:0,2 $R_f$= 0,54]

**1.13) p-Aminophenyl-2-O-carboxymethyl-β-L-fucosid:**

Die Verseifung und Hydrierung von 150mg p-Nitrophenyl-2-O-methoxycarbonylmethyl-β-L-fucosid führt nach der im Beispiel 1 10 beschrieben Vorgehensweise in 83%iger Ausbeute zu 109mg des Zielproduktes [DC: Acetonitril/Wasser/Eisessig 5:1:0,2 $R_f$= 0,35].

**Beispiel 1.14.a**

Synthese der regioisomeren Monosuccinylierungsprodukte von p-Nitrophenyl-β-L-fucosid:
1100mg (3,86mmol) p-Nitrophenyl-β-L-fucosid werden in 50ml Pyridin gelöst und mit 580mg (5,79mmol) Bernsteinsäureanhydrid versetzt. Nach 16h Rühren bei 20°C engt man ein und destilliert zweimal mit Dichlormethan nach. Man fällt aus Dichlormethan/Ether und erhält 1g eines nicht trennbaren Substanzgemisches. Dieses wird in Methanol/Wasser aufgenommen und mit 846mg (2,6mmol) Caesiumcarbonat versetzt. Man dampft das Lösungsmittel ab und destilliert mit DMF nach. Der Rückstand wird in DMF aufgenommen und mit 618μl Benzylbromid versetzt. Nach 1h Ultraschallbehandlung wird Caesiumbromid abfiltriert und das Filtrat eingeengt. Man verteilt zwischen 500ml Essigester und 50ml Wasser. Die organische Phase wird getrocknet und eingeengt. Die flashchromatographische Trennung der Komponenten gelingt im Laufmittelsystem Dichlormethan/Methanol 99:1. Man erhält:

Fr.1: 87mg (4,8%) p-Nitrophenyl-3-O-(3-benzyloxycarbonylpropionyl)-β-L-fucosid [DC: Dichlormethan/Methanol 95:5 $R_f$ = 0,45].

Fr.2: 27mg (1,5%) p-Nitrophenyl-2-O-(3-benzyl oxycarbonylpropionyl)-β-L-fucosid [DC: Dichlormethan/Methanol 95:5 $R_f$= 0,34].

Fr.3: 190mg (10,3%) p-Nitrophenyl-4-O-(3-benzyloxycarbonylpropionyl)-β-L-fucosid [DC: Dichlormethan/Methanol 95:5 $R_f$= 0,28].

**Beispiel 1.14**

**p-Aminophenyl-3-O-succinyl-β-L-fucosid**

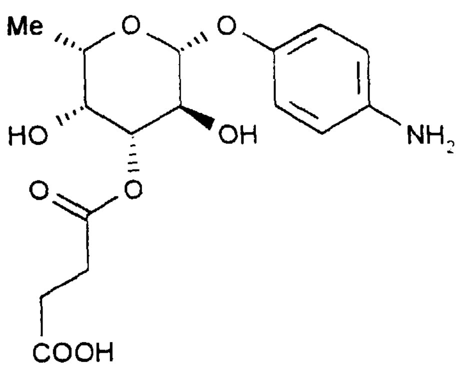

85mg (0,17mmol) der Fraktion 1 aus Beispiel 1.14.a werden in 5ml THF und 1ml Wasser gelöst. Man gibt 20mg Platindioxid zu und hydriert für 8h. Der Katalysator wird abfiltriert, mit THF/Wasser gewaschen und das Filtrat eingeengt. Der Rückstand wird in Wasser aufgenommen und lyophilisiert. Man erhält 57mg (94%) des Zielproduktes. [DC: Acetonitril/Wasser/Eisessig 5:1:0,2 $R_f$= 0,65]

**Beispiel 1.15**

**p-Aminophenyl-2-O-succinyl-β-L-fucosid**

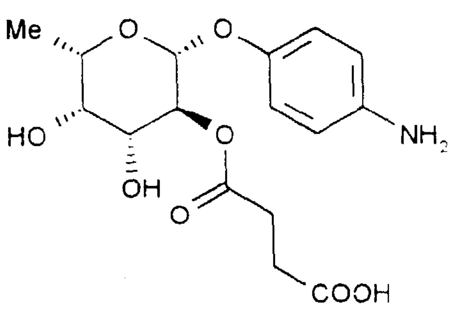

Fraktion 2 aus Beispiel 1.14.a wird analog zur Vorschrift in Beispiel 1.14 hydriert. Ausb.: 16mg (87%) [DC: Acetonitril/Wasser/Eisessig 5:1 0,2 $R_f$= 0,62]

**Beispiel 1.16**

**p-Aminophenyl-4-O-succinyl-β-L-fucosid**

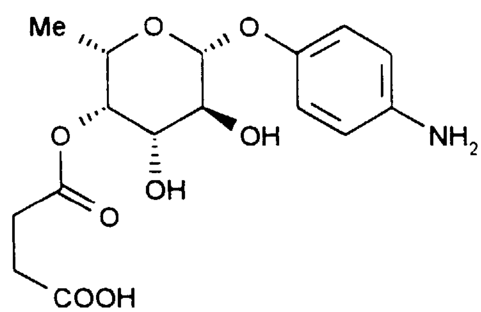

Fraktion 3 aus Beispiel 1.14.a wird analog zur Vorschrift in Beispiel 1.14 hydriert. Ausb.: 125mg (88%) [DC: Acetonitril/Wasser/Eisessig 5:1:0,2 $R_f$ = 0,63]

**Beispiel 1.17**

**p-Aminophenyl-3,4-di-O-methyl-β-L-fucosid**

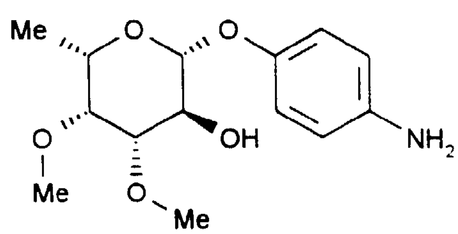

**1.17.a) p-Nitrophenyl-2-O-benzyl-3.4-O-isopropyliden-β-L-fucosid:**

377mg (1,16mmol) der Verbindung aus Beispiel 1.1.a werden in 30ml abs. THF gelöst und nacheinander mit 690µl Benzylbromid und 52mg Natriumhydrid versetzt und bei 20°C gerührt. Nach 4h und 6h werden nochmals 690µl Benzylbromid und Natriumhydrid nachgesetzt. Der Ansatz wird analog zu Beispiel 1.1. b aufgearbeitet. Man erhält 245mg (51%) der Zielverbindung.

**1.17.b) p-Nitrophenyl-2-O-benzyl-3,4-di-O-methyl-β-L-fucosid:**

245mg (0,59mmol) p-Nitrophenyl-2-O-benzyl-3,4-O-isopropyliden-β-L-fucosid werden 16h bei 20°C in 80%iger Essigsäure gerührt. Man engt ein und verruhrt den Rückstand mit Ether/Pentan Nach dem Absaugen und Trocknen wird das verbleibende Produkt in 20ml abs. THF aufgenommen, 45mg 80%iges Natriumhydrid zugegeben und nach 15min 160µl Methyljodid eingespritzt. Nach 20h Rühren bei 20°C wird mit Methanol und Eisessig gequencht, eingeengt und der Rückstand zwischen Dichlormethan und Wasser verteilt. Die organische Phase wird getrocknet, eingeengt und anschließend durch Flash-Chromatographie (Dichlormethan/Methanol 100:1) gereinigt. Nach Einengen und Trocknen der entsprechenden Fraktionen erhält man 188 mg (79%) des Zielproduktes.

**1.17) p-Aminophenyl-3,4-di-O-methyl-β-L-fucosid:**

180mg (0,45mmol) der Verbindung aus Beispiel 1.17.b werden in einer Mischung aus 15ml Methanol und

3ml Dichlormethan nach Zusatz von 50mg Palladium auf Aktivkohle über 2d bei Raumtemperatur hydriert. Der Katalysator wird abfiltriert, das Filtrat wird eingengt und durch Flash-Chromatographie (Dichlormethan/Methanol 97,5:2,5) gereinigt. Man erhält 86mg (68%) der Zielverbindung. [DC: Dichlormethan/Methanol 95:5 $R_f$ = 0,21]

**Beispiel 1.18**

**p-Aminophenyl-3-O-carbamoylmethyl-β-L-fucosid**

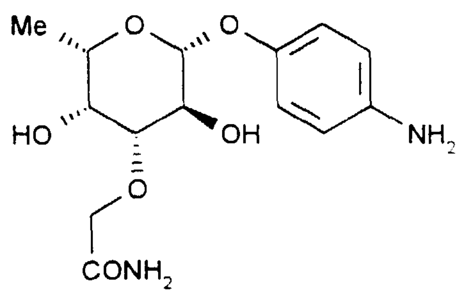

100mg (0,305mmol) der Verbindung aus Beispiel 1.11 werden in 10ml Methanol gelöst und mit 0,5ml 17%iger Ammoniumhydroxyd-Lösung versetzt. Nach 4h wird eingeengt, in Wasser aufgenommen und lyophilisiert Man erhält 95mg (quant.) der Zielverbindung. [DC: Acetonitril/Wasser 10:1 $R_f$ = 0.43]

**Beispiel 1.19**

**p-Aminophenyl-2-O-hydroxyethyl-β-L-fucosid**

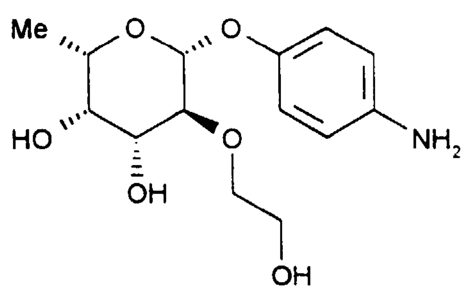

Diese Verbindung wurde ausgehend von 200mg (0,56mmol) p-Nitrophenyl-2-O-methoxycarbonylmethyl-β-L-fucosid (Beispiel 1.13.a) in Analogie zu den Beispielen 1.12.a und 1.12 hergestellt. Ausb.: 76mg (45% über 2 Stufen) [DC: Dichlormethan/Methanol 9:1 $R_f$= 0,2]

**Beispiel 1.20**

**p-Aminophenyl-3,6-didesoxy-3-chlor-β-L-gulosid**

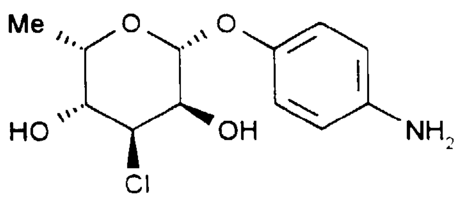

50mg (0,165mmol) der Verbindung aus Beispiel 1.6.b werden in 5ml Methanol über Palladium/Aktivkohle 1h hydnert. Man filtriert vom Katalysator ab. wäscht nach, engt ein, nimmt in Wasser auf und lyophilisiert. Man erhält 45mg (89%) der Zielverbindung.
[DC: Dichlormethan/Methanol 9:1 $R_f$ = 0,35]

**Beispiel 1.21**

**p-Aminophenyl-α-L-rhamnosid**

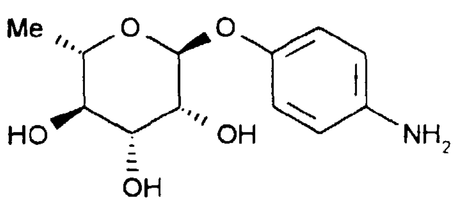

Diese Verbindung wurde ausgehend von 300mg p-Nitrophenyl-α-L-rhamnosid (Sigma) in Analogie zu Beispiel 1.1 hergestellt. Ausb.: 96%

**Beispiel 1.22**

**p-Aminophenyl-3-O-carboxymethyl-α-L-rhamnosid**

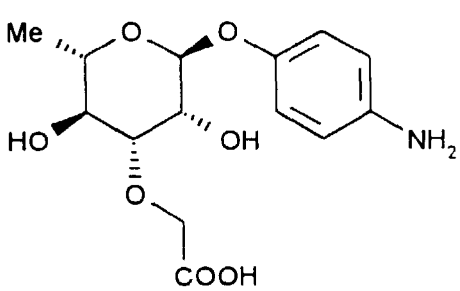

**1.22.a) p-Nitrophenyl-3-O-methoxycarbonylmethyl-α-L-rhamnosid**

481mg (1,63mmol) p-Nitrophenyl-α-L-rhamnosid werden 30ml Methanol aufgenommen und mit 629mg (2,45mmol) Dibutylzinnoxid versetzt. Man erhitzt 2h unter Rückfluß, engt ein und nimmt in 30ml Dioxan auf. Man gibt 85mg Tetrabutyl-ammoniumjodid und 950µl Bromessigsäuremethylester zu und erhitzt 16h unter Rückfluß. Gegebenenfalls wird nochmals 1ml Bromessigsäuremethylester nachgesetzt und die Reaktionszeit verlängert. Man engt ein und reinigt den Rückstand durch Flash-Chromatographie. Mit Dichlormethan/Methanol 99:1 wird p-Nitrophenyl-3-O-methoxycarbonylmethyl-α-L-rhamnosid eluiert und nach dem Trocknen erhält man 408mg (70%). [DC: Dichlormethan/ Methanol 95:5 $R_f$ = 0,36]

**1.22) p-Aminophenyl-3-O-carboxymethyl-α-L-rhamnosid:**

Synthese völlig analog zu Beispiel 1.10 ausgehend von p-Nitrophenyl-3-O-methoxy-carbonylmethyl-α-L-rhamnosid. Man erhält das Zielprodukt in 80%iger Ausbeute.
[DC: Acetonitril/Wasser/Eisessig 5:1:0,2 Rf = 0,26]

__Beispiel 1.23__

**p-Aminophenyl-β-D-galactopyranosid**

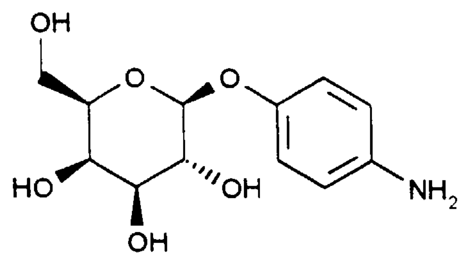

p-Nitrophenyl-β-D-galactopyranosid (3,0 g, 10 mmol) wird in Methanol/Wasser 1:1 (50 ml) gelöst und nach Zugabe von Palladium auf Aktivkohle (10% Pd, 200 mg) für 3 h in einer Wasserstoffatmosphäre unter geringem Überdruck hydriert. Die Suspension wird über Celite filtriert und das Filtergut mit heißem Methanol/Wasser 1:1 (100 ml) gewaschen. Einengen des Filtrats im Vakuum und Umkristallisation aus Methanol ergibt farblose Kristalle (2,11 g, 78 %); DC [Methanol]: $R_f$= 0,62; $[\alpha]^{20}$ = -39,5° (c = 1,0 / $H_2O$); Schmp. = 166 °C.

__Beispiel 1.24__

**p-Aminophenyl-2-O-methyl-β-D-galactopyranosid**

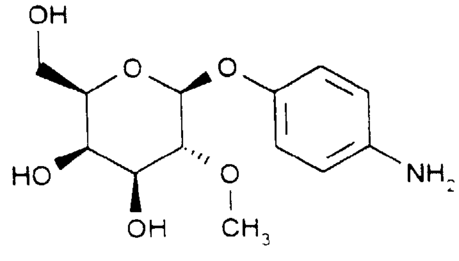

**1.24.a) p-Nitrophenyl-6-O-triphenylmethyl-β-D-galactopyranosid:**

Eine Lösung von p-Nitrophenyl-β-D-galactopyranosid (9,0 g, 30 mmol), Chlortriphenylmethan (16,7 g, 60 mmol) und N,N-Dimethylaminopyridin (609 mg, 5 mmol) in absolutem Pyridin (100 ml) wird für 4 h auf 60°C erhitzt. Nach Einengen im Vakuum wird der Rückstand durch Flashchromatographie [Petrolether/Ethylacetat 2:1 → 3:2, jeweils mit 0,5 % Triethylamin] gereinigt. Man erhält farblose Kristalle (9,23 g, 57 %); DC [Dichlormethan/Methanol/Ammoniak (25 %) 15:3:0,2]: $R_f$ = 0,55; Schmp. = 82 °C.

**1.24.b) p-Nitrophenyl-3,4-O-isopropyliden-6-O-triphenylmethyl-β-D-galactopyranosid:**

Die obige Verbindung (8,7 g, 16 mmol) wird mit Dimethoxypropan (400 ml) und einer katalytischen Menge an (±)-Campher-10-sulfonsäure (400 mg, 1,7 mmol) versetzt. Nach 1h bei Raumtemperatur beendet man die Reaktion durch Zugabe von Triethylamin (240 ml, 1,7 mmol) und engt im Vakuum ein. Durch Flash-chromatographie [Petrolether/ Ethylacetat 2:1] erhält man einen farblosen Schaum (6,2 g, 66 %); DC [Petrolether/Ethylacetat 1:1]: $R_f$= 0,46; $[\alpha]^{20}$ = -42,1° (c = 0,94/$CH_2Cl_2$).

**1.24.c) p-Nitrophenyl-2-O-methyl-3,4-O-isopropyliden-6-O-triphenylmethyl-β-D-galactopyranosid:**

Man löst Verbindung 1.24.b (5,83 g, 10 mmol) in Dimethylformamid (100 ml) und versetzt mit Methyljodid (2,5 ml, 40 mmol) und portionsweise mit einer 80%-igen Suspension von Natriumhydrid in Mineralöl (450 mg, 15 mmol). Nach 2 h bei Raumtemperatur beendet man die Reaktion durch Zutropfen von Methanol (10 ml) und engt im Vakuum ein. Der Rückstand wird in Dichlormethan (1000 ml) aufgenommen und die Lösung kräftig mit Wasser (500 ml) verrührt. Man trocknet die organische Phase über Magnesiumsulfat (50 g), engt im Vakuum ein und reinigt durch Flashchromatographie [Petrolether/Ethylacetat 12:1 → 8:1]. Man erhält einen farblosen Schaum (4,72 g, 79 %); DC [Petrolether/ Ethylacetat 1:1]: $R_f$ = 0,72; $[\alpha]^{20}$ = -35,7° (c = 1,0 / $CH_3OH$).

**1.24.d) p-Nitrophenyl-2-O-methyl-β-D-galactopyranosid:**

Eine Lösung der obigen Verbindung (4,48 g, 7,5 mmol) in Dichlormethan (200 ml) wird mit 99%-iger Trifluoressigsäure (20 ml) versetzt und für 3 h bei Raumtemperatur gerührt. Nach Einengen im Vakuum wird der Rückstand durch Flashchromatographie [Petrolether/Ethylacetat 5:1 → 2:1] gereinigt. Man erhält farblose Kristalle (1,09 g, 46 %); DC [Dichlormethan/Methanol/Ammoniak (25%) 15:3:0,2]: $R_f$= 0,42; Schmp. = 177 °C.

**1.24) p-Aminophenyl-2-O-methyl-β-D-galactopyranosid:**

Verbindung 1.24.d (946 mg, 3 mmol) wird in Methanol (50 ml) gelöst und nach Zugabe von Wasser (0,5 ml) und basischem Raney-Nickel (ca. 200 mg) für 2 h in einer Wasserstoffatmosphäre unter geringem Überdruck hydriert. Die Suspension wird über Celite filtriert und das Filtergut gründlich mit Methanol (100 ml) gewaschen. Einengen des Filtrats im Vakuum ergibt einen bräunlichen Schaum (579 mg, 68 %); DC [Dichlormethan/Methanol/Ammoniak (25 %) 15:3:0,2]: $R_f$ = 0,28; $[\alpha]^{20}$ = -39,3° (c = 0,15 / $CH_3OH$).

<u>**Beispiel 1.25**</u>

**p-Aminophenyl-3-O-methyl-β-D-galactopyranosid**

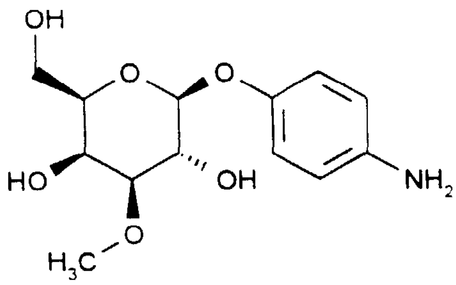

**1.25.a) p-Nitrophenyl-3-O-methyl-β-D-galactopyranosid:**

Eine Lösung von p-Nitrophenyl-β-D-galactopyranosid (1,5 g, 5,0 mmol) in absolutem Methanol (40 ml) wird mit Dibutylzinnoxid (1,87 g, 7,5 mmol) versetzt und unter Ruckfluß erhitzt. Nach 3 h wird im Vakuum eingeengt und der Ruckstand für 1 h im Ölpumpenvakuum getrocknet. Man nimmt mit absolutem Dioxan (40 ml) auf, versetzt die resultierende Lösung mit Methyljodid (1,9 ml, 30 mmol) und rührt den Ansatz für 16 h bei 100°C Badtemperatur. Anschließend wird das Lösungsmittel im Vakuum abdestilliert und der Rückstand durch Flashchromatographie [Ethylacetat/Petrolether 2:1 → Ethylacetat] gereinigt. Man erhält farblose Kristalle (1,32 g, 84 %); DC [Dichlormethan/Methanol/Ammoniak (25 %) 15:3:0,2]: $R_f$= 0,34; Schmp. = 196 °C; $[\alpha]^{20}$ = -53,3° (c = 1,0 / $CH_3OH$).

**1.25) p-Aminophenyl-3-O-methyl-β-D-galactopyranosid:**

Die obige Verbindung (946 mg, 3 mmol) wird wie in Beispiel 1.24 beschrieben reduziert und aufgearbeitet. Man erhält bräunliche Kristalle (656 mg, 77 %); DC [Dichlormethan/Methanol/Ammoniak (25 %) 15:3:0,2]: $R_f$ = 0,21; Schmp. = 196°C; $[\alpha]^{20}$ = -25,2° (c = 1,0 / $CH_3OH$).

__Beispiel 1.26__

**p-Aminophenyl-4-O-methyl-β-D-galactopyranosid, Acetat**

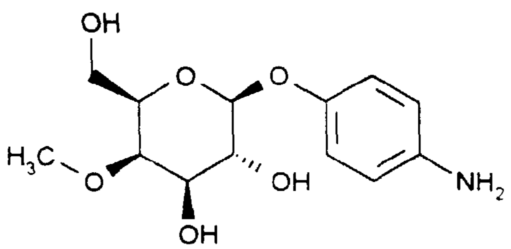

**1.26.a) p-Nitrophenyl-3-O-benzyl-β-D-galactopyranosid:**

Eine Lösung von p-Nitrophenyl-β-D-galactopyranosid (1,5 g, 5,0 mmol) in absolutem Dioxan (40 ml) wird mit Dibutylzinnoxid (1,87 g, 7,5 mmol) versetzt und unter Rückfluß erhitzt. Nach 3 h versetzt man die erhaltene Lösung mit Benzylbromid (3,6 ml, 30 mmol) und rührt den Ansatz für weitere 48 h unter Rückfluß. Anschließend wird das Lösungsmittel im Vakuum abdestilliert und der Rückstand durch Flashchromatographie [Ethylacetat/Petrolether 2:1 → 1:1] gereinigt. Man erhält farblose Kristalle (1,58 g, 81 %); DC [Dichlormethan/Methanol/Ammoniak (25 %) 15:3:0,2]: $R_f$= 0,69; Schmp = 127 °C.

**1.26.b) p-Nitrophenyl-3-O-benzyl-4,6-O-isopropyliden-β-D-galactopyranosid:**

Verbindung 1.26.a (6,26 g, 16 mmol) wird wie in Beispiel 1.24.b beschrieben umgesetzt. Nach Flashchromatographie [Petrolether/Ethylacetat 5:1 → 3:1] erhält man einen farblosen Schaum (6,54 g, 95 %); DC [Petrolether/Ethylacetat 1:1]: $R_f$ = 0,34; $[\alpha]^{20}$ = -38,9° (c = 1,0 / $CH_2Cl_2$).

**1.26.c) p-Nitrophenyl-2,3-di-O-benzyl-4,6-O-isopropyliden-β-D-galactopyranosid:**

Man löst Verbindung 1.26.b (4,31 g, 10 mmol) in Dimethylformamid (100 ml) und versetzt mit Benzylbromid (12 ml, 100 mmol) und portionsweise mit einer 80%-igen Suspension von Natriumhydrid in Mineralöl (450 mg, 15 mmol). Nach 2 h bei Raumtemperatur beendet man die Reaktion durch Zutropfen von Methanol (10 ml) und engt im Vakuum ein. Der Rückstand wird in Dichlormethan (1000 ml) aufgenommen und die Lösung kräftig mit Wasser (500 ml) verrührt Man trocknet die organische Phase über Magnesiumsulfat (50 g), engt im Vakuum ein und reinigt durch Flashchromatographie [Petrolether/Ethylacetat 20:1 → 15:1 → 10:1]. Man erhält ein farbloses Öl (2,72 g, 52 %), das noch verunreinigt ist; DC [Petrolether/Ethylacetat 1:1]: $R_f$ = 0,62.

**1.26.d) p-Nitrophenyl-2,3-di-O-benzyl-β-D-galactopyranosid:**

Die obige Verbindung (2,6 g, 5 mmol) wird wie in Beispiel 1.24.d beschrieben umgesetzt. Nach Einengen im Vakuum und Auskochen des Rückstands mit Diethylether erhält man farblose Kristalle (805 mg, 33 %); DC [Petrolether/Ethylacetat 1:1]: $R_f$= 0,23; Smp. = 160°C.

**1.26.e) p-Nitrophenyl-2,3-di-O-benzyl-6-O-triphenylmethyl-β-D-galactopyranosid:**

Verbindung 1.26.d (722 mg, 1,5 mmol) wird wie in Beispiel 1.24.a beschrieben trityliert. Nach Flashchromatographie [Petrolether/Ethylacetat 15:1 → 10:1 → 5:1] erhält man einen farblosen Schaum (880 mg, 81 %), DC [Petrolether/Ethylacetat 2:1]: $R_f$= 0,79; $[\alpha]^{20}$ = -25,3° (c = 0,3 / $CH_2Cl_2$).

**1.26.f) p-Nitrophenyl-2,3-di-O-benzyl-4-O-methyl-6-O-tnphenylmethyl-β-D galactopyranosid:**

Die obige Verbindung (724 mg, 1 mmol) wird wie in Beispiel 1.24.c beschrieben methyliert. Nach Flashchromatographie [Petrolether/Ethylacetat 10:1 → 5:1] erhält man einen farblosen Schaum (662 mg, 90 %); DC [Petrolether/Ethylacetat 5:1]: $R_f$ = 0,66; $[\alpha]^{20}$ = -38,7° (c = 0,2 / $CH_2Cl_2$).

**1.26) p-Aminophenyl-4-O-methyl-β-D-galactopyranosid, Acetat:**

Die obige Verbindung (590 mg, 0,8 mmol) wird in 90%-iger Essigsäure (50 ml) gelöst und nach Zugabe von Palladium auf Aktivkohle (10% Pd, 200 mg) für 16 h in einer Wasserstoffatmosphäre unter geringem Überdruck hydriert. Die Suspension wird über Celite filtriert und das Filtergut gründlich mit Methanol (100 ml) gewaschen. Einengen des Filtrats im Vakuum und Umfällen des Rückstands aus Diethylether/Petrolether ergibt farblose Kristalle (253 mg, 92 %); DC [Dichlormethan/Methanol 5:1]: $R_f$ = 0,12.

**Beispiel 1.27**

**p-Aminophenyl-6-O-methyl-β-D-galactopyranosid**

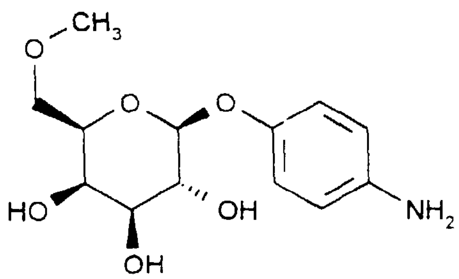

**1.27.a) Benzylierung von Verbindung 1.24.b:**

Verbindung 1.24.b (5,84 g, 10 mmol) wird wie in Beispiel 1.26.c beschrieben benzyliert. Nach Flashchromatographie [Petrolether/Ethylacetat 15:1 → 12:1 → 5:1 → Ethylacetat, jeweils mit 0,5 % Triethylamin] erhält man zwei Produktfraktionen:

Fraktion 1: p-Nitrophenyl-2-O-benzyl-3,4-O-isopropyliden-6-O-triphenylmethyl-β-D-galactopyranosid; gelblicher Schaum (1,71 g. 25 %); DC [Petrolether/Ethylacetat 2:1]: $R_f$ = 0,72; $[\alpha]^{20}$ = -8,1° (c = 1,0/$CH_2Cl_2$)

Fraktion 2: p-Nitrophenyl-2-O-benzyl-3,4-O-isopropyliden-β-D-galactopyranosid; gelbliches Öl (806 mg, 19 %); DC [Petrolether/Ethylacetat 2:1]: $R_f$ = 0,45; $[\alpha]^{20}$ = +2,8° (c = 1,2/$CH_3OH$).

**1.27.b) p-Nitrophenyl-2-O-benzyl-3,4-O-isopropyliden-6-O-methyl-β-D-galactopyranosid:**

Fraktion 2 aus Beispiel 1.27.a (777 mg, 1,8 mmol) wird wie in Beispiel 1.24.c beschrieben methyliert. Nach Flashchromatographie [Petrolether/Ethylacetat 10:1 → 8:1] erhält man ein bräunliches Öl (730 mg, 91 %); DC [Petrolether/Ethylacetat 2:1]: $R_f$= 0,54; $[\alpha]^{20}$ = -11,6° (c = 1,1/$CH_2Cl_2$).

**1.27.c) p-Nitrophenyl-2-O-benzyl-6-O-methyl-β-D-galactopyranosid:**

Die obige Verbindung (668 mg, 1,5 mmol) wird wie in Beispiel 1.24.d beschrieben umgesetzt. Einengen des Filtrats im Vakuum und Auskochen des Rückstands mit wenig Diethylether ergibt nach Abkühlung auf Raumtemperatur hellbeige Kristalle (388 mg, 64 %); DC [Petrolether/Ethylacetat 1:1]: $R_f$ = 0,15, Schmp. = 143°C.

**1.27) p-Aminophenyl-6-O-methyl-β-D-galactopyranosid**

Verbindung 1.27.c (324 mg, 0,8 mmol) wird wie in Beispiel 1.24 beschrieben für 16 h reduziert. Nach Einengen des Filtrats im Vakuum und Auskochen des Rückstands mit wenig Diethylether erhält man beige Kristalle (184 mg, 81 %); DC [Ethylacetat]: $R_f$ = 0,05; Schmp. = 115°C (Zers.).

**Beispiel 1.28**

**p-Aminophenyl-2,3-di-O-methyl-β-D-galactopyranosid**

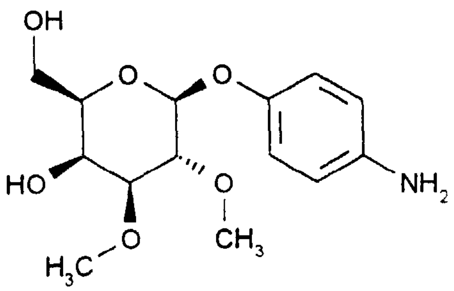

**1.28.a) Isopropylidenierung von p-Nitrophenyl-β-D-galactopyranosid:**

Eine Lösung von p-Nitrophenyl-β-D-galactopyranosid (7,5 g, 25 mmol) in absolutem Aceton (1000 ml) wird mit wasserfreier Toluolsulfonsäure (500 mg) versetzt. Man destilliert unter Normaldruck innerhalb von 30 min Aceton (250 ml) ab und neutralisiert anschließend sofort durch Zugabe vom Kaliumcarbonat (500 mg). Nach Einengen im Vakuum wird der Rückstand mit Diethylether (1000 ml) verrührt. Man filtriert ab, engt das Filtrat ein und reinigt durch Flash-chromatographie [Petrolether/Ethylacetat 2:1 → 1:1 → 3:2]. Dabei fallen zwei Produktfraktionen an:

Fraktion 1: p-Nitrophenyl-3,4-O-isopropyliden-β-D-galactopyranosid; farbloser Schaum (3,74 g, 44 %); DC [Dichlormethan/Methanol/Ammoniak (25 %) 15:3:0,2]: $R_f$ = 0,59; $[\alpha]^{20}$ = -54,2° (c = 0,38/$CH_3OH$).
Fraktion 2: p-Nitrophenyl-4,6-O-isopropyliden-β-D-galactopyranosid; farbloser Schaum (4,3 g, 50 %); DC [Dichlormethan/Methanol/Ammoniak (25 %) 15:3:0,2]: $R_f$ = 0,54; $[\alpha]^{20}$ = -81,0° (c = 0,31/$CH_3OH$).

**1.28.b) p-Nitrophenyl-2,3-di-O-methyl-4,6-O-isopropyliden-β-D-galactopyranosid:**

Fraktion 2 aus Beispiel 1.28.a (4,1 g, 12 mmol) wird wie in Beispiel 1.24.c beschrieben methyliert. Nach Flashchromatographie [Petrolether/Ethylacetat 5:1 → 2:1] erhält man einen farblosen Schaum (2,93 g. 66 %). DC [Petrolether/Ethylacetat 1:1]: $R_f$ = 0.42; $[\alpha]^{20}$ = -52,6° (c = 0,34/$CH_3OH$).

**1.28.c) p-N itrophenyl-2,3-di-O-methyl-β-D-galactopyranosid:**

Die obige Verbindung (2,77 g, 7,5 mmol) wird wie in Beispiel 1.24.d beschrieben umgesetzt. Nach 1 h bei Raumtemperatur wird im Vakuum eingeengt und der Rückstand für 1 h im Ölpumpenvakuum getrocknet. Durch Digerieren mit Diethylether/Petrolether 1:1 (100 ml) erhält man farblose Kristalle (1,11 g, 45 %); DC [Ethylacetat]: $R_f$ = 0,24; Schmp. = 156°C.

**1.28) p-Aminophenyl-2,3-di-O-methyl-β-D-galactopyranosid:**

Verbindung 1.38.c (989 mg, 3 mmol) wird wie in Beispiel 1.24 beschrieben reduziert. Man erhält ein farbloses Öl (396 mg, 44 %); DC [Dichlormethan/Methanol/Ammoniak (25 %) 15:3:0,2]: $R_f$ = 0,50; $[\alpha]^{20}$ = -19,4° (c = 0,16/$CH_3OH$)

**Beispiel 1.29**

**p-Aminophenyl-2,4-di-O-methyl-β-D-galactopyranosid**

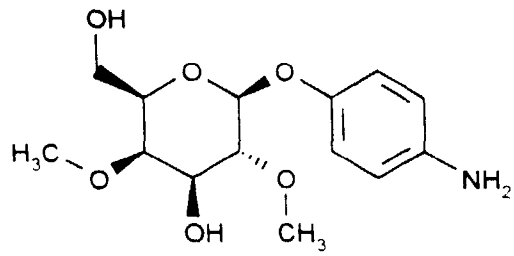

**1.29.a) Tritylierung von Verbindung 1.26.a:**

Verbindung 1.26.a (11,7 g, 30 mmol) wird wie in Beispiel 1.24.a beschrieben trityliert. Nach Flashchromatographie [Petrolether/Ethylacetat 10:1 → 7:1 → 5:1; jeweils mit 0,5 % Triethylamin] erhält man zwei Produkte:

Fraktion 1: Triphenylmethyl-2-O-(p-nitrophenyl)-3-O-benzyl-6-O-triphenylmethyl-β-D-galactopyranosid, farbloser Schaum (8,5 g, 32 %); DC [Petrolether/Ethylacetat 2:1]; $R_f$ = 0,68; $[\alpha]^{20}$ = +42,8° (c 1.0/$CH_2Cl_2$).
Fraktion 2: p-Nitrophenyl-3-O-benzyl-6-O-triphenylmethyl-β-D-galactopyranosid; farbloser Schaum (9,0 g, 47 %); DC [Petrolether/Ethylacetat 2:1], $R_f$ = 0,22, $[\alpha]^{20}$ = -22,6° (c = 1,03/$CH_2Cl_2$)

**1.29.b) p-Nitrophenyl-2,4-di-O-methyl-3-O-benzyl-6-O-triphenylmethyl-β-D-galactopyranosid:**

Fraktion 2 aus Beispiel 1.29.a (7,6 g, 12 mmol) wird wie in Beispiel 1.24.c beschrieben methyliert. Nach Flashchromatographie [Petrolether/Ethylacetat 15:1 → 10:1] erhält man einen farblosen Schaum (7,07 g, 89 %); DC [Petrolether/Ethylacetat 2:1]: $R_f$ = 0,79; $[\alpha]^{20}$ = -35,8° (c = 1,09/$CH_3OH$).

**1.29.c) p-Aminophenyl-2,4-di-O-methyl-3-O-benzyl-β-D-galactopyranosid:**

Die obige Verbindung (6,0 g, 9 mmol) wird für 48 h wie in Beispiel 1.26 beschrieben hydriert. Man erhält farblose Kristalle (1,39 g, 40 %); DC [Ethylacetat/Petrolether 2:1]: $R_f$ = 0,20; Schmp. = 148°C.

**1.29) p-Aminophenyl-2,4-di-O-methyl-β-D-galactopyranosid:**

Verbindung 1.29.c (779 mg, 2 mmol) wird für 5 d wie in Beispiel 1.24 beschrieben hydriert. Nach Eindampfen der vereinigten Filtrate im Vakuum und Auskochen des Rückstands mit Diethylether (2 x 50 ml) erhält man leicht grünliche Kristalle (391 mg, 65 %); DC [Ethylacetat]: $R_f$ = 0,16; Schmp = 260°C (Zers.).

**Beispiel 1.30**

**p-Aminophenyl-2,6-di-O-methyl-β-D-galactopyranosid**

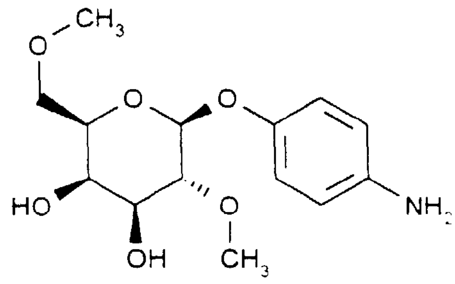

**1.30.a) p-Nitrophenyl-2,6-di-O-methyl-3,4-O-isopropyliden-β-D-galactopyranosid:**

Fraktion 1 aus Beispiel 1.28.a (4,1 g, 12 mmol) wird wie in Beispiel 1.24.c beschrieben methyliert. Nach Flashchromatographie [Petrolether/Ethylacetat 10:1 → 8:1 → 5:1] erhält man ein farbloses Öl (3,25 g, 73 %); DC [Petrolether/Ethylacetat 1:1]: $R_f$ = = 0,65.

**1.30.b) p-Nitrophenyl-2,6-di-O-methyl-β-D-galactopyranosid:**

Die obige Verbindung (2,77 g, 7,5 mmol) wird wie in Beispiel 1.24.d beschrieben umgesetzt. Nach Flashchromatographie [Ethylacetat/Petrolether 2:1] erhält man farblose Kristalle (1,63 g, 66 %); DC [Ethylacetat]: $R_f$ = 0,31; Schmp. = 222°C.

**1.30) p-Aminophenyl-2,6-di-O-methyl-β-D-galactopyranosid:**

Verbindung 1.30.b (989 mg, 3 mmol) wird wie in Beispiel 1.24 beschrieben reduziert. Man erhält ein farbloses Öl (597 mg, 66 %); DC [Dichlormethan/Methanol/Ammoniak (25 %) 15:3:0,2]: $R_f$ = 0,56; $[\alpha]^{20}$ = -53,1° (c = 0,49/$CH_3OH$).

**Beispiel 1.31**

**p-Aminophenyl-3,4-di-O-methy-β-D-galactopyranosid, Acetat**

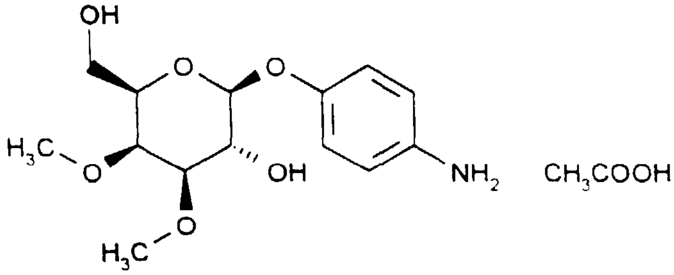

**1.31.a) p-Nitrophenyl-2,6-di-O-benzyl-3,4-O-isopropyliden-β-D-galactopyranosid:**

Fraktion 1 aus Beispiel 1.28.a (4.1 g, 12 mmol) wird wie in Beispiel 1.26.c beschrieben benzyliert. Nach Flashchromatographie [Petrolether/Ethylacetat 6:1] erhält man ein gelbliches Öl (5,3 g, 85 %); DC [Ethylacetat/Petrolether 2:1]: $R_f$ = 0,76; $[\alpha]^{20}$ = +8,8° (c = 1,2/$CH_3OH$).

**1.31.b) p-Nitrophenyl-2,6-di-O-benzyl-β-D-galactopyranosid:**

Obige Verbindung (4,69 g, 9 mmol) wird wie in Beispiel 1.24.d beschrieben umgesetzt. Nach 30 min. bei Raumtemperatur wird im Vakuum eingeengt und der Rückstand für 1 h im Ölpumpenvakuum getrocknet. Durch Umkristallisation aus Ethanol erhält man farblose Kristalle (2,89 g, 67 %); DC [Ethylacetat/Petrolether 2:1]: $R_f$ = 0,42; Schmp. = 133°C; $[\alpha]^{20}$ = -64,2° (c = 1,0 /$CH_3OH$)

**1.31.c) p-Nitrophenyl-2,6-di-O-benzyl-3,4-di-O-methyl-β-D-galactopyranosid:**

Die obige Verbindung (2,4 g, 5 mmol) wird wie in Beispiel 1.24.c beschrieben methyliert. Nach Umkristallisation aus Ethanol/n-Hexan erhält man farblose Kristalle (1,74 g, 69 %); DC [Petrolether/Ethylacetat 1:1]: $R_f$ = 0,74; Schmp = 149°C.

**1.31) p-Aminophenyl-3,4-di-O-methy-β-D-galactopyranosid, Acetat:**

Verbindung 1.31.c (1,52 g, 3 mmol) wird wie in Beispiel 1.26 beschrieben hydriert. Man erhält farblose Kristalle (664 mg, 62 %); DC [Dichlormethan/Methanol/Ammoniak (25 %) 15:3:0,2]: $R_f$ = 0,47; Schmp. = 140°C (Zers.).

__**Beispiel 1.32**__

**p-Aminophenyl-3,6-di-O-methy-β-D-galactopyranosid**

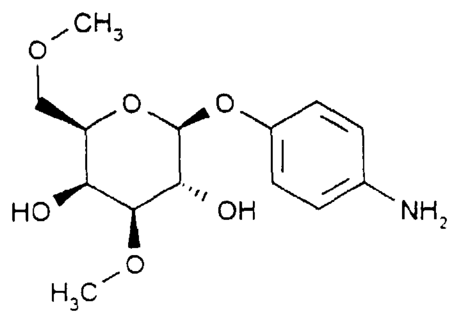

**1.32.a) p-Nitrophenyl-3-O-methyl-6-O-(tert-butyldimethylsilyl)-β-D-galactopyranosid:**

Eine Lösung von Verbindung 1.25.a (1.58 g, 5 mmol) in Dimethylformamid (150 ml) wird mit Imidazol (1 g, 15 mmol) und tert-Butyldimethylsilyl-chlorid (1,25 g, 8 mmol) versetzt und für 24h bei Raumtemperatur gerührt Anschließend wird die Reaktion durch Zugabe von Wasser (100 ml) abgebrochen. Man verdünnt mit Dichlormethan (1000 ml), wäscht die organische Phase mit Wasser (2 x 1000 ml), trocknet über Magnesiumsulfat (20 g) und engt im Vakuum ein. Nach Flashchromatographie [Petrolether/Ethylacetat 15:1 → 10:1 → 5:1] erhält man einen gelblichen Schaum (826 mg, 38 %), der noch leicht verunreinigt ist; DC [Ethylacetat]: $R_f$= 0,59; $[\alpha]^{20}$ = -56,3° (c = 1,0 / $CH_2Cl_2$).

**1.32.b) p-Nitrophenyl-2,4-di-O-benzyl-3-O-methyl-6-O-(tert-butyldimethylsilyl)-β-D-galactopyranosid:**

Obige Verbindung (773 mg, 1,8 mmol) wird wie in Beispiel 1.26.c beschrieben benzyliert. Nach Flashchromatographie [Petrolether/Ethylacetat 30:1 → 5:1] erhält man einen farblosen Schaum (810 mg, 74 %); DC [Petrolether/Ethylacetat 5:1]: $R_f$ = 0,58.

**1.32.c) p-Nitrophenyl-2,4-di-O-benzyl-3-O-methyl-β-D-galactopyranosid:**

Verbindung 1.32.b (732 mg, 1,2 mmol) wird in Tetrahydrofuran (6 ml) gelöst und bei 0°C mit einer 1M-Lösung von Tetrabutylammoniumfluorid in THF (2,4 ml) versetzt. Man rührt für 40 min. bei Raumtemperatur und engt dann im Vakuum ein. Nach Flashchromatographie [Petrolether/Ethylacetat 5:1 → 3:1 → 2:1] erhält man farblose Kristalle (512 mg, 86 %); DC [Petrolether/Ethylacetat 1:1]: $R_f$ = 0,36; Schmp. = 177°C.

**1.32.d) p-Nitrophenyl-2,4-di-O-benzyl-3,6-di-O-methyl-β-D-galactopyranosid:**

Die obige Verbindung (446 mg, 0,9 mmol) wird wie in Beispiel 1.24.c beschrieben methyliert. Nach Flash-chromatographie [Petrolether/Ethylacetat 20:1 → 10:1 → 8:1] erhält man ein farbloses Öl (401 mg, 87 %); DC [Petrolether/Ethylacetat 1:1]: $R_f$ = 0,70; $[\alpha]^{20}$ = -56.5° (c = 0,96 / $CH_2Cl_2$).

**1.32) p-Aminophenyl-3,6-di-O-methyl-β-D-galactopyranosid:**

Verbindung 1.32.d (357 mg, 0,7 mmol) wird wie in Beispiel 1.24 beschrieben hydriert. Nach Eindampfen der vereinigten Filtrate im Vakuum und Auskochen des Rückstands mit Diethylether (20 ml) erhält man farblose Kristalle (207 mg, 99 %); DC [Petrolether/Ethylacetat 1:1]: $R_f$ = 0,02; Schmp = >280 °C (Zers.).

<u>**Beispiel 1.33**</u>

**p-Aminophenyl-4,6-di-O-methyl-β-D-galactopyranosid**

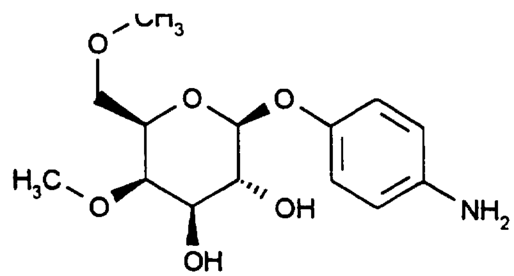

**1.33.a) p-Nitrophenyl-2,3-di-O-benzyl-4,6-di-O-methyl-β-D-galactopyranosid:**

Verbindung 1.26.d (2,4 g, 5 mmol) wird wie in Beispiel 1.24.c beschrieben methyliert. Nach Flashchromatographie [Petrolether/Ethylacetat 5:1 → 3:1] erhält man farblose Kristalle (1,89 g, 74 %); DC [Petrolether/Ethylacetat 1:1]: $R_f$ = 0,76, Smp. = 100°C.

**1.33) p-Aminophenyl-4,6-di-O-methy-β-D-galactopyranosid:**

Verbindung 1.33.a (1,53 g, 3 mmol) wird wie in Beispiel 1.24 beschrieben hydriert. Man erhält farblose Kristalle (890 mg, 99 %); DC [Methanol/Ethylacetat 1:1]: $R_f$ = 0,71; Schmp. = 180°C (Zers.).

**Beispiel 1.34**

**p-Aminophenyl-2,3.4-tri-O-methyl-β-D-galactopyranosid**

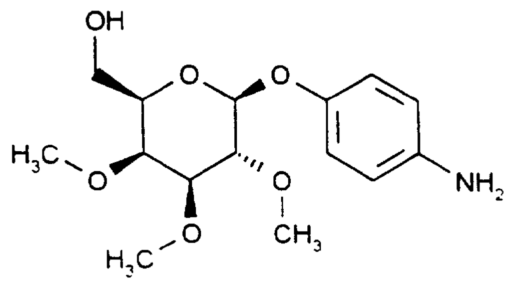

**1.34.a) p-Nitrophenyl-2,3,4-tri-O-methyl-6-O-triphenylmethyl-β-D-galactopyranosid:**

Verbindung 1.24.a (1,63 g, 3 mmol) wird wie in Beispiel 1.24.c beschrieben methyliert. Nach Flashchromatographie [Petrolether/Ethylacetat 5:1 → 3:1] erhält man einen farblosen Schaum (1,24 g, 71 %); DC [Petrolether/Ethylacetat 1:1]: $R_f$ = 0,54; $[\alpha]^{20}$ = -53,6° (c = 0,3 / $CH_3OH$).

**1.34.b) p-Nitrophenyl-2,3,4-tri-O-methyl-β-D-galactopyranosid:**

Die obige Verbindung (1,17 g, 2 mmol) wird wie in Beispiel 1.24.d beschrieben umgesetzt. Nach Flashchromatographie [Petrolether/Ethylacetat 3:1 → 2:1] erhält man farblose Kristalle (468 mg, 68 %); DC [Petrolether/Ethylacetat 1:1]: $R_f$ = 0,12; Schmp. = 104°C; $[\alpha]^{20}$ = -68,2° (c = 0,47 / $CH_3OH$).

**1.34) p-Aminophenyl-2,3,4-tri-O-methyl-β-D-galactopyranosid:**

Verbindung 1.34.b (343 mg, 1 mmol) wird wie in Beispiel 1.24 beschrieben reduziert. Man erhält beige Kristalle (224 mg, 71 %); DC [Dichlormethan/Methanol/Ammoniak (25 %) 15:3:0,2]: $R_f$ = 0,67; Schmp. = 138°C.

**Beispiel 1.35**

**p-Aminophenyl-2,3,6-tri-O-methyl-β-D-galactopyranosid**

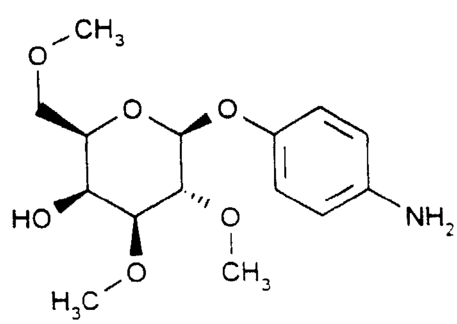

**1.35.a) p-Nitrophenyl-2,3,6-tri-O-methyl-β-D-galactopyranosid:**

Verbindung 1.30.b (2,63 g, 3 mmol) wird wie in Beispiel 1.25. a beschrieben selektiv methyliert. Nach Flashchromatographie [Petrolether/Ethylacetat 5:1 → 2:1] erhält man ein bräunliches Öl (890 mg, 32 %); DC [Ethylacetat]: $R_f$ = 0,37; $[\alpha]^{20}$ = -63,3° (c = 0,9 / $CH_2Cl_2$).

**1.35) p-Aminophenyl-2,3,6-tri-O-methyl-β-D-galactopyranosid:**

Die obige Verbindung (858 mg, 2,5 mmol) wird wie in Beispiel 1.24 beschrieben reduziert. Man erhält einen beigen Schaum (519 mg, 66 %); DC [Ethylacetat]: $R_f$ = 0,23; $[\alpha]^{20}$ = -34,5° (c = 0,86 / $CH_3OH$).

<u>**Beispiel 1.36**</u>

**p-Aminophenyl-2,4,6-tri-O-methyl-β-D-galactopyranosid**

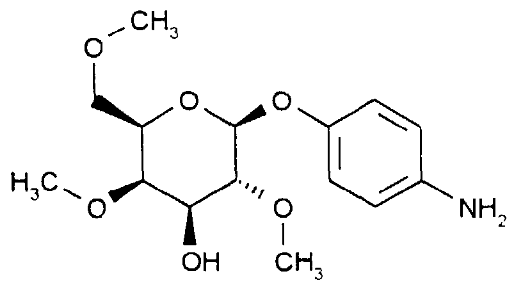

**1.36.a) p-Nitrophenyl-2,4,6-tri-O-methyl-3-O-benyzl-β-D-galactopyranosid:**

Verbindung 1.26.a (1,96 g, 5 mmol) wird wie in Beispiel 1.24.c beschrieben methyliert. Nach Flashchromatographie [Petrolether/Ethylacetat 10:1 → 8:1] erhält man farblose Kristalle (1,47 g, 68 %); DC [Petrolether/Ethylacetat 2:1]: $R_f$ = 0,46; Schmp. = 164°C.

**1.36) p-Aminophenyl-2,4,6-tri-O-methyl-β-D-galactopyranosid:**

Die obige Verbindung (1,3 g, 3 mmol) wird wie in Beispiel 1.26 beschrieben reduziert. Man erhält farblose Kristalle (642 mg, 68 %), DC [Ethylacetat/Petrolether 2:1]: $R_f$ = 0,12; Schmp = 147°C (Zers.).

<u>**Beispiel 1.37**</u>

**p-Aminophenyl-3,4,6-tri-O-methyl-β-D-galactopyranosid**

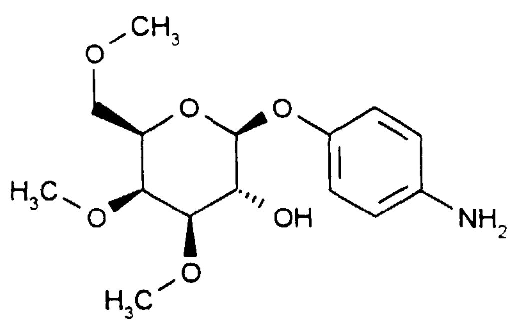

**1.37.a) p-Nitrophenyl-2-O-benzyl-β-D-galactopyranosid:**

Fraktion 1 aus Beispiel 1.27.a (1,17 g, 2 mmol) wird wie in Beispiel 1.24.d beschrieben umgesetzt. Nach Flashchromatographie [Petrolether/Ethylacetat 3:1 → 2:1] erhält man farblose Kristalle (468 mg, 68 %); DC [Petrolether/Ethylacetat 1:1]: $R_f$ = 0,12; Schmp. = 104 °C; $[\alpha]^{20}$ = -68,2° (c = 0,47 / $CH_3OH$).

**1.37.b) p-Nitrophenyl-2-O-benzyl-3,4,6-tri-O-methyl-β-D-galactopyranosid:**

Die obige Verbindung (391 mg, 1 mmol) wird wie in Beispiel 1.24.c beschrieben methyliert. Nach Flashchromatographie [Petrolether/Ethylacetat 10:1 → 5:1] erhält man hellgelbe Kristalle (303 mg, 70 %); DC [Ethylacetat]: $R_f$ = 0,81; $[\alpha]^{20}$ = -76,5° (c = 1,1 /$CH_2Cl_2$)

**1.37) p-Aminophenyl-3,4,6-tri-O-methyl-β-D-galactopyranosid:**

Verbindung 1.37.b (260 mg, 0,6 mmol) wird wie in Beispiel 1.24 beschrieben hydriert Man erhält beige Kristalle (161 mg, 86 %); DC [Ethylacetat]: $R_f$ = 0,20; Schmp = 132°C.

<u>**Beispiel 1.38**</u>

**p-Aminophenyl-2,3,4,6-tetra-O-methyl-β-D-galactopyranosid**

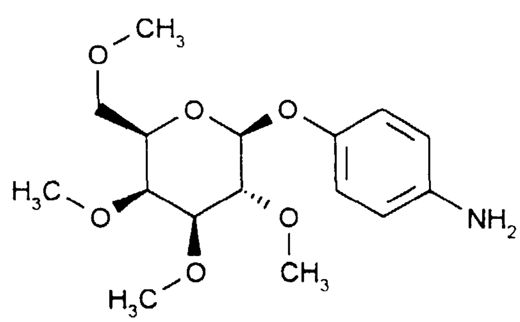

**1.38.a) p-Nitrophenyl-2,3,4,6-tetra-O-methyl-β-D-galactopyranosid:**

p-Nitrophenyl-β-D-galactopyranosid (904 mg, 3 mmol) wird wie in Beispiel 1.24.c beschrieben methyliert. Nach Flashchromatographie [Petrolether/Ethylacetat 8:1 → 6:1 → 4:1 → 2:1] erhält man einen farblosen, wachsartigen Feststoff (633 mg, 59 %); DC [Ethylacetat]: $R_f$ = 0,67; $[\alpha]^{20}$ = -55,7° (c = 0,9 / $CH_2Cl_2$).

**1.38) p-Aminophenyl-2,3,4,6-tetra-O-methyl-β-D-galactopyranosid:**

Verbindung 1.33.a (536 mg, 1,5 mmol) wird wie in Beispiel 1.24 beschrieben hydriert. Nach Eindampfen der vereinigten Filtrate im Vakuum und Auskochen des Rückstands mit Diethylether (20 ml) erhält man farblose Kristalle (412 mg, 84 %); DC [Ethylacetat]: $R_f$ = 0,42; Schmp. = 204°C (Zers.).

**Beispiel 1.39**

**p-Aminophenyl-α-D-mannopyranosid**

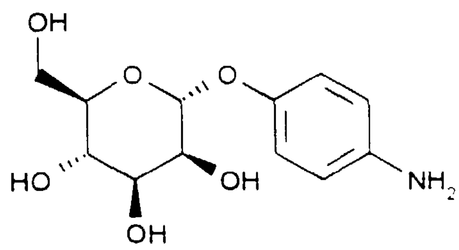

p-Nitrophenyl-α-D-mannopyranosid (3,0 g, 10 mmol) wird wie in Beispiel 1.23 beschrieben hydriert. Umfallen aus Methanol/Diethylether ergibt farblose Kristalle (2,03 g, 75 %); DC [Methanol]: $R_f$ = 0,69; $[\alpha]^{20}$ = +102,7° (c = 1,0 / $H_2O$); Schmp. = 161°C.

**Beispiel 1.40**

**p-Aminophenyl-3-O-methyl-α-D-mannopyranosid**

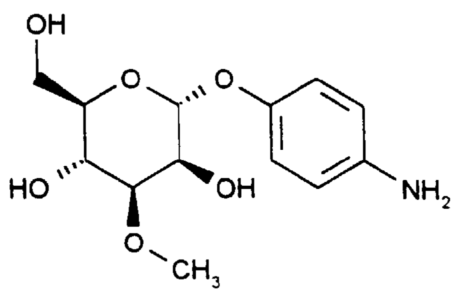

**1.40.a) p-Nitrophenyl-6-O-triphenylmethyl-α-D-mannopyranosid:**

p-Nitrophenyl-α-D-mannopyranosid (3,0 g, 10 mmol) wird wie in Beispiel 1.24.a beschrieben trityliert. Man erhält farblose Kristalle (4,35 g, 80 %); DC [Dichlormethan/Methanol/Ammoniak (25 %) 15:3:0,2]: $R_f$ = 0,52; $[\alpha]^{20}$ = +104,0° (c = 1,0 / $CH_3OH$); Schmp. = 102-104°C.

**1.40.b) p-Nitrophenyl-3-O-methyl-6-O-triphenylmethyl-α-D-mannopyranosid:**

Die obige Verbindung (2,72 g, 5 mmol) wird wie in Beispiel 1.26.a beschrieben mit Methyliodid (2 ml, 30 mmol) umgesetzt Nach Flashchromatographie [Petrolether/Ethylacetat 2:1] und Umfällen aus Ethanol/n-Hexan erhält man farblose Kristalle (1,83 g, 66 %); DC [Dichlormethan/Methanol/Ammoniak (25 %) 15:3:0,2]: $R_f$ = 0,68; $[\alpha]^{20}$ = +106,4° (c = 1,0 / $CH_3OH$); Schmp. = 104°C.

**1.40) p-Aminophenyl-3-O-methyl-α-D-mannopyranosid:**

Verbindung 1.40.b (1,4 g, 2,5 mmol) wird in Methanol (50 ml) gelöst und nach Zugabe von Palladium auf Aktivkohle (10% Pd. 300 mg) für 24 h in einer Wasserstoffatmosphäre unter geringem Überdruck hydriert. Die Suspension wird über Celite filtriert und das Filtergut gründlich mit Methanol (100 ml) gewaschen. Nach Einengen des Filtrats im Vakuum zieht man den Rückstand mit Wasser (50 ml) aus, filtriert ab und lyophylisiert das Filtrat. Man erhält

einen bräunlichen amorphen Feststoff (709 mg, 99 %); DC [Dichlormethan/Methanol/Ammoniak (25 %) 15:3:0,2]: $R_f$ = 0,33; $[\alpha]^{20}$ = +92,9° (c = 1,1 / $CH_3OH$).

**Beispiel 1.41**

**p-Aminophenyl-2,3-di-O-methyl-α-D-mannopyranosid**

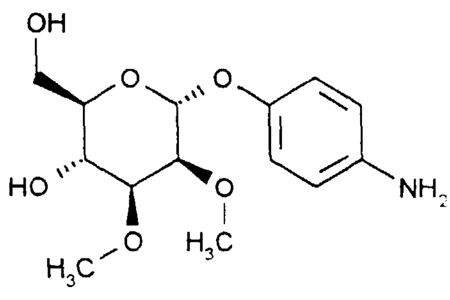

**1.41.a) p-Nitrophenyl-4,6-O-benzyliden-α-D-mannopyranosid:**

Eine Lösung von p-Nitrophenyl-α-D-mannopyranosid (6,0 g, 20 mmol) in Dimethylformamid (120 ml) wird mit Benzaldehyd-dimethylacetal (3,2 ml, 21,4 mmol) und einer 54 %igen Lösung von Tetrafluorborsäure in Diethylether (2,7 ml, 20 mmol) versetzt. Man rührt für 5 h bei Raumtemperatur, bricht dann die Reaktion durch Zugabe von Triethylamin (2,8 ml, 20 mmol) ab und engt im Vakuum ein. Nach Flashchromatographie [Toluol → Toluol/Ethanol 20:1] erhält man farblose Kristalle (6,48 g, 83 %); DC [Dichlormethan/Methanol/Ammoniak (25 %) 15:3:0,2]: $R_f$ = 0,82; $[\alpha]^{20}$ = +170,7° (c = 1,0 / $CH_2Cl_2$); Schmp = 116°C.

**1.41.b) p-Nitrophenyl-2,3-di-O-methyl-4,6-O-benzyliden-α-D-mannopyranosid:**

Obige Verbindung (3,9 g, 10 mmol) wird wie in Beispiel 1.24.c beschrieben methyliert. Nach Flashchromatographie [Petrolether/Ethylacetat 20:1 → 7:1] und Umfällen aus Ethylacetat/n-Hexan erhält man einen farblosen Schaum (3,2 g, 77 %); DC [Ethylacetat/Petrolether 2:1]: $R_f$ = 0,67; $[\alpha]^{20}$ = +167,3° (c = 1,05 / $CH_3OH$).

**1.41) p-Aminophenyl-2,3-di-O-methyl-α-D-mannopyranosid:**

Verbindung 1.41.b (1,25 g, 3 mmol) wird wie in Beispiel 1.26 beschrieben hydriert. Nach Flashchromatographie [Ethylacetat/Petrolether 2:1 → Ethylacetat, jeweils mit 0,5 % Triethylamin] erhält man einen rötlich-braunen Schaum (480 mg, 53 %); DC [Dichlormethan/Methanol/Ammoniak (25 %) 15:3.0,2]: $R_f$ = 0,31; $[\alpha]^{20}$ = +83,6° (c = 0,76 / $CH_3OH$).

**Beispiel 1.42**

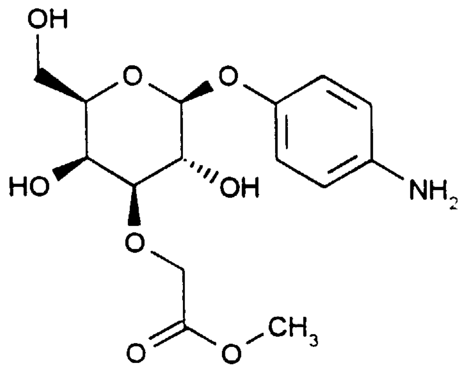

**1.42.a) p-Nitrophenyl-3-O-methoxycarbonylmethyl-β-D-galactopyranosid:**

Eine Lösung von p-Nitrophenyl-β-D-galactopyranosid (7,53 g, 25 mmol) in absolutem Dioxan (180 ml) wird mit Dibutylzinnoxid (9,3 g, 37,5 mmol) versetzt und unter Rückfluß erhitzt. Nach 4 h versetzt man die erhaltene Lösung mit Bromessigsäure-methylester (8,3 ml, 90 mmol) und Tetrabutylammoniumiodid (9,25 g, 25 mmol) und rührt den Ansatz für weitere 3 h unter Rückfluß. Anschließend wird das Lösungsmittel im Vakuum abdestilliert und der Rückstand durch Flashchromatographie [Dichlormethan/Methanol 50:1 → 20:1] gereinigt. Man erhält neben einigen Nebenprodukten die Verbindung 1.42.a als farblose Kristalle (4,05 g, 43 %), DC [Dichlormethan/Methanol/Ammoniak (25 %) 15: 3:0,2]: $R_f$ = 0,54; $[\alpha]^{20}$ = -62,0° (c = 1,0 / $CH_3OH$); Schmp. = 176°C.

**1.42) p-Aminophenyl-3-O-methoxycarbonylmethyl-β-D-galactopyranosid:**

Verbindung 1.42.a (3,73 g, 10 mmol) wird wie in Beispiel 1.24 beschrieben hydriert. Nach Umfällen aus Ethanol/n-Hexan erhält man farblose Kristalle (2,98 g, 87 %); DC [Dichlormethan/Methanol/Ammoniak (25 %) 15:3:0,2]: $R_f$ = 0,39; $[\alpha]^{20}$ = -36,3° (c = 1,07 / $CH_3OH$); Schmp. = 155 °C.

**Beispiel 1.43**

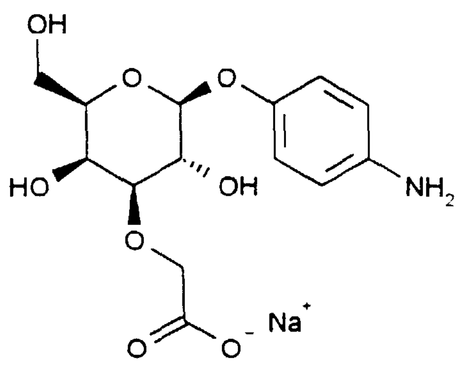

**1.43.a) p-Nitrophenyl-3-O-carboxymethyl-β-D-galactopyranosid, Natriumsalz:**

Eine Lösung von Verbindung 1.42.a (3,73 g, 10 mmol) in Methanol (100 ml) wird mit einer Lösung von Natriumhydroxid (400 mg, 10 mmol) in Wasser (5 ml) versetzt und für 2 h bei Raumtemperatur gerührt. Nach Einengen im Vakuum wird der Rückstand für 2 h im Ölpumpenvakuum getrocknet und dann mit Ethanol (100 ml) versetzt. Man kocht für 5 min unter Rückfluß, filtriert nach Abkühlung im Eisbad ab und erhält farblose Kristalle (3,66 g, 96 %); DC

[Methanol]: $R_f$ = 0,62; $[\alpha]^{20}$ = -50,0° (c = 1,0 / $CH_3OH$); Schmp. = 180-185°C.

**1.43) p-Aminophenyl-3-O-carboxymethyl-β-D-galactopyranosid, Natriumsalz:**

Die obige Verbindung (3,05 g, 8 mmol) wird wie in Beispiel 1.24 beschrieben hydriert. Nach Auskochen mit Ethanol (50 ml) erhält man farblose Kristalle (2,03 g, 72 %); DC [Methanol]: $R_f$ = 0,70. $[\alpha]^{20}$ = -22,4° (c = 1,0 / $CH_3OH$); Schmp. = 180-182°C.

**Beispiel 1.44**

**p-Aminophenyl-3-O-carbamoylmethyl-β-D-galactopyranosid**

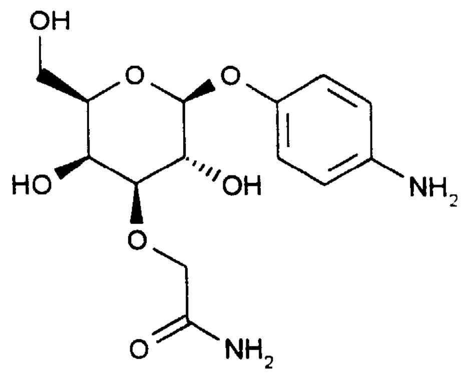

**1.44.a) p-Nitrophenyl-3-O-carbamoylmethyl-β-D-galactopyranosid:**

Eine Lösung von Verbindung 1.42.a (373 mg, 1 mmol) in Methanol (30 ml) wird mit einer 25 %-igen wäßrigen Lösung von Ammoniak (10 ml) versetzt und für 15 min. bei Raumtemperatur gerührt. Nach Einengen im Vakuum wird der Rückstand für 2 h im Ölpumpenvakuum getrocknet und dann mit Ethanol (30 ml) versetzt. Man kocht für 5 min. unter Rückfluß, filtriert nach Abkühlung im Eisbad ab und erhält farblose Kristalle (306 mg, 85 %); DC [Dichlormethan/Methanol/Ammoniak (25 %) 15:3:0,2]: $R_f$ = 0,14; $[\alpha]^{20}$ = -41,7° (c = 1,0 / $CH_3OH$); Schmp. = 229 °C (Zers.).

**1.44) p-Aminophenyl-3-O-carbamoylmethyl-β-D-galactopyranosid:**

Obige Verbindung (287 mg, 0,8 mmol) wird wie in Beispiel 1.24 beschrieben hydriert. Nach Umfällen aus Methanol/Diethylether erhält man farblose Kristalle (207 mg, 79 %); DC [Dichlormethan/Methanol/Ammoniak (25 %) 15:3:0,2]: $R_f$ = 0,10; Schmp. = 205°C (Zers.).

**Beispiel 1.45**

**p-Aminophenyl-3-O-(N-methyl-carbamoylmethyl)-β-D-galactopyranosid**

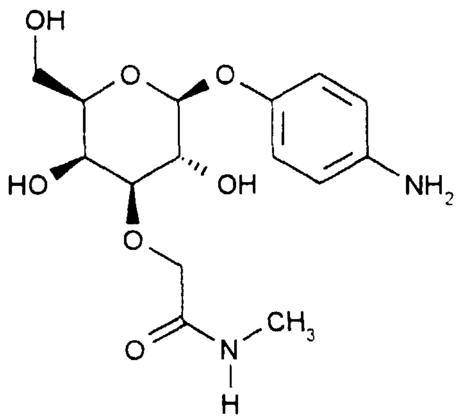

**1.45.a) p-Nitrophenyl-3-O-(N-methyl-carbamoylmethyl)-β-D-galactopyranosid**

Eine Lösung von Verbindung 1.42.a (373 mg, 1 mmol) in Methanol (30 ml) wird mit einer 30 %-igen wäßrigen Lösung von Methylamin (10 ml) versetzt und für 2 h bei Raumtemperatur gerührt. Nach Einengen im Vakuum wird der Rückstand für 2 h im Ölpumpenvakuum getrocknet und dann aus Ethanol umkristallisiert. Man erhält farblose Kristalle (372 mg, 100 %); DC [Dichlormethan/Methanol/Ammoniak (25 %) 15:3:0,2]: $R_f = 0,33$; $[\alpha]^{20} = -36,7°$ (c = 1,0 / $CH_3OH$); Schmp. = 205°C.

**1.45) p-Aminophenyl-3-O-(N-methyl-carbamoylmethyl)-β-D-galactopyranosid:**

Verbindung 1.45.a (298 mg, 0,8 mmol) wird wie in Beispiel 1.24 beschrieben hydriert. Nach Umfällen aus Methanol/Diethylether erhält man farblose Kristalle (180 mg, 66 %); DC [Dichlormethan/Methanol/Ammoniak (25 %) 15:3:0,2]: $R_f = 0.16$; Schmp. = 239 °C.

**Beispiel 1.46**

**p-Aminophenyl-3-O-(N-propyl-carbamoylmethyl)-β-D-galactopyranosid**

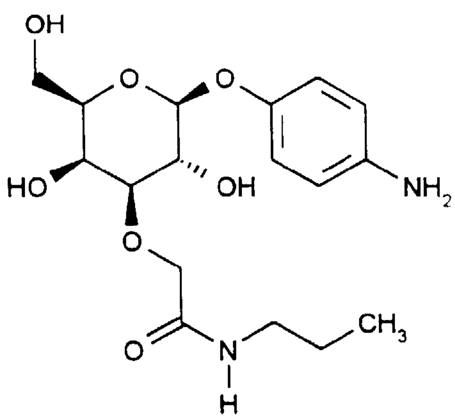

**1.46.a) p-Nitrophenyl-3-O-(N-propyl-carbamoylmethyl)-β-D-galactopyranosid:**

Verbindung 1.42.a (373 mg, 1 mmol) wird wie in Beispiel 1.45.a beschrieben mit mit n-Propylamin (823 µl, 10 mmol) umgesetzt. Nach Einengen im Vakuum wird der Rückstand aus Ethanol/n-Hexan umgefällt. Man erhält farblose Kristalle (340 mg, 85 %); DC [Dichlormethan/Methanol/Ammoniak (25 %) 15:3:0,2]: $R_f$ = 0,49; $[\alpha]^{20}$ = -32,4° (c = 1,0 / $CH_3OH$); Schmp. = 155°C.

**1.46) p-Aminophenyl-3-O-(N-propyl-carbamoylmethyl)-β-D-galactopyranosid:**

Verbindung 1.46.a (320 mg, 0,8 mmol) wird wie in Beispiel 1.24 beschrieben hydriert. Nach Umfällen aus Methanol/Diethylether erhält man farblose Kristalle (188 mg, 63 %); DC [Dichlormethan/Methanol/Ammoniak (25 %) 15:3;0:2]; $R_f$ = 0,31; Schmp. = 154°C.

__Beispiel 1.47__

**p-Aminophenyl-3-O-(N-butyl-carbamoylmethyl)-β-D-galactopyranosid**

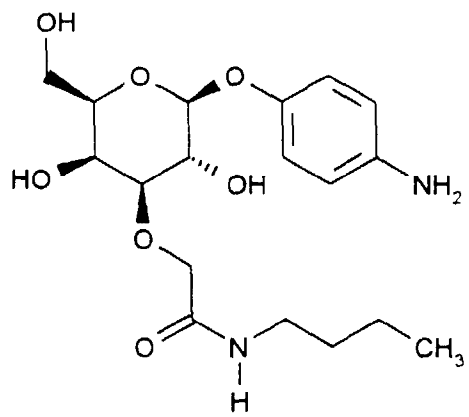

**1.47.a) p-Nitrophenyl-3-O-(N-butyl-carbamoylmethyl)-β-D-galactopyranosid:**

Verbindung 1.42.a (373 mg, 1 mmol) wird wie in Beispiel 1.45.a beschrieben mit n-Butylamin (900 µl, 10 mmol) umgesetzt. Nach Einengen im Vakuum wird der Rückstand aus Ethanol/n-Hexan umgefällt. Man erhält farblose Kristalle (413 mg, 100 %); DC [Dichlormethan/Methanol/Ammoniak (25 %) 15:3:0,2]: $R_f$ = 0,51; $[\alpha]^{20}$ = -26,8° (c = 1,0 / $CH_3OH$); Schmp. = 92°C.

**1.47) p-Aminophenyl-3-O-(N-butyl-carbamoylmethyl)-β-D-galactopyranosid:**

Verbindung 1.47.a (332 mg, 0,8 mmol) wird wie in Beispiel 1.24 beschrieben hydriert. Nach Umfällen aus Ethanol/n-Hexan erhält man farblose Kristalle (105 mg, 34 %); DC [Dichlormethan/Methanol/Ammoniak (25 %) 15:3; 0:2]: $R_f$ = 0,32; Schmp. = 135°C.

Beispiel 1.48

**p-Aminophenyl-3-O-methoxycarbonylmethyl-α-D-mannopyranosid**

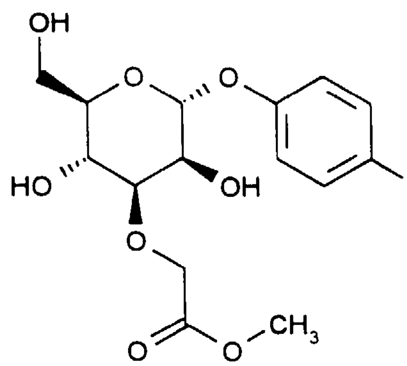

**1.48.a) p-Nitrophenyl-3-O-methoxycarbonylmethyl-6-O-triphenylmethyl-α-D-mannopyranosid:**

Verbindung 1.40.a (13,6 g, 25 mmol) wird wie in Beispiel 1.42.a beschrieben umgesetzt. Nach Flashchromatographie [Petrolether/Ethylacetat 10:1] erhält man neben einigen Nebenprodukten farblose Kristalle (2,79 g, 18 %), DC [Ethylacetat/Petrolether 2:1]: $R_f$ = 0,50; Schmp. = 95-97°C.

**1.48) p-Aminophenyl-3-O-methoxycarbonylmethyl-α-D-mannopyranosid:**

Verbindung 1.48.a (1,23 g, 2 mmol) wird wie in Beispiel 1.40 beschrieben hydriert und aufgearbeitet. Man erhält man einen bräunlichen amorphen Feststoff (250 mg, 36 %); DC [Dichlormethan/Methanol/Ammoniak (25 %) 15: 3;0:2]; $R_f$ = 0,45.

Beispiel 1.49

**p-Aminophenyl-3-O-carboxymethyl-α-D-mannopyranosid**

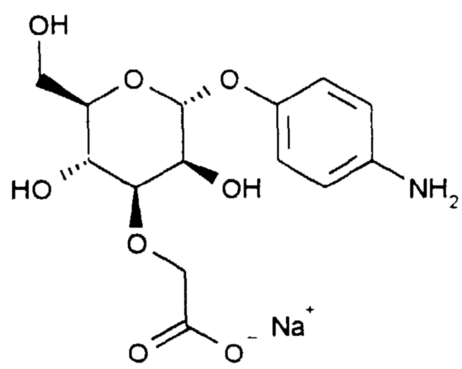

**1.49.a) p-Nitrophenyl-3-O-benzoxycarbonylmethyl-6-O-triphenylmethyl-α-D-mannopyranosid:**

Verbindung 1.40.a (13,6 g, 25 mmol) wird wie in Beispiel 1.42.a beschrieben mit Bromessigsäure-benzylester (14,4 ml, 90 mmol) umgesetzt. Nach Flashchromatographie [Petrolether/Ethylacetat 20:1 → 10:1] erhält man neben einigen Nebenprodukten einen gelblichen Schaum (5,0 g, 29 %); DC [Ethylacetat/Petrolether 2:1]: $R_f$ = 0,66; $[\alpha]^{20}$ = +74,8° (c = 1,0 / $CH_2Cl_2$).

**1.49) p-Aminophenyl-3-O-carboxymethyl-α-D-mannopyranosid:**

Die obige Verbindung (2,08 g, 3 mmol) wird wie in Beispiel 1.40 beschrieben fur 36 h hydriert. Nach Einengen des Filtrats wird der Rückstand aus Ethanol/n-Hexan umgefällt. Durch Waschen mit Ethylacetat und erneutes Umfällen aus Ethanol/Diethylether erhält man farblose Kristalle (495 mg, 50 %); DC [Methanol]: $R_f$ = 0,53; Schmp. = 205-207°C.

__Beispiel 1.50__

**p-Aminophenyl-3-O-carbamoylmethyl-α-D-mannopyranosid**

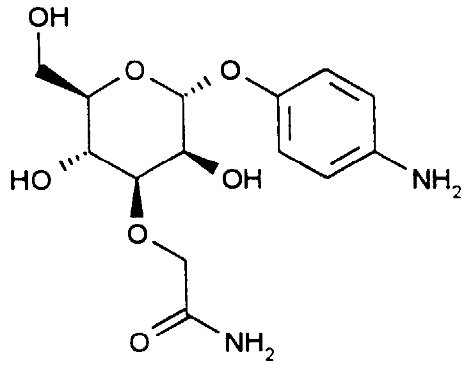

**1.50.a) p-Nitrophenyl-3-O-carbamoylmethyl-6-O-triphenylmethyl-α-D-mannopyranosid:**

Verbindung 1.49.a (1,04 g, 1,5 mmol) wird wie in Beispiel 1.44.a beschrieben umgesetzt. Nach Trocknung im Ölpumpenvakuum wird der Rückstand durch Flashchromatographie [Petrolether/Ethylacetat 2:3] gereinigt. Man erhält farblose Kristalle (561 mg, 62 %); DC [Dichlormethan/Methanol/Ammoniak (25 %) 15:3:0,2]: $R_f$ = 0,67; $[\alpha]^{20}$ = +91,3° (c = 1,0 / $CH_2Cl_2$); Schmp. = 125-127°C.

**1.50) p-Aminophenyl-3-O-carbamoylmethyl-α-D-mannopyranosid:**

Die obige Verbindung (541 g, 0,9 mmol) wird wie in Beispiel 1.40 beschrieben für 48 h hydriert. Nach Einengen des Filtrats wäscht man den Rückstand gründlich mit Methanol und erhält farblose Kristalle (134 mg, 45 %); DC [Dichlormethan/Methanol/Ammoniak (25 %) 15:3:0,2]: $R_f$ = 0,21; Schmp. = 126-128°C.

__Beispiel 1.51__

**p-Aminophenyl-3-desoxy-β-D-galactopyranosid**

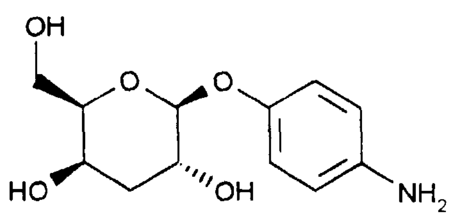

**1.51.a) p-Nitrophenyl-2,6-di-O-benzyl-4-O-acetyl-β-D-galactopyranosid:**

Eine Lösung von Verbindung 1.31.b (2,7 g, 5,6 mmol) in Dichlormethan (20 ml) wird mit Triethylorthoacetat

(3 ml, 16,3 mmol) und Toluolsulfonsäure (20 mg) versetzt. Nach 30 min. bei Raumtemperatur verdünnt man den Ansatz mit Dichlormethan (200 ml), wäscht mit gesättigter Natriumhydrogencarbonat-Lösung (50 ml), trocknet die organische Phase über Magnesiumsulfat und engt nach Filtration im Vakuum ein. Der resultierende farblose Schaum wird in 80%-iger Essigsaure (15 ml) gelöst. Nach weiteren 30 min. bei Raumtemperatur gießt man den Ansatz in gesättigte Natriumhydrogencarbonat-Lösung (200 ml), extrahiert mit Chloroform (3x 75 ml), wäscht die vereinigten organischen Phasen mit Wasser (100 ml), trocknet über Magnesiumsulfat und engt nach Filtration im Vakuum ein Durch Umfällen aus Ethanol/Petrolether erhält man farblose Kristalle (2,43 g, 83 %); DC [Ethylacetat/Petrolether 2:1]: $R_f$ = 0,64; $[\alpha]^{20}$ = -62,1° (c = = 1,0 / $CH_2Cl_2$); Schmp. = 83°C.

**1.51.b) p-Nitrophenyl-2,6-di-O-benzyl-3-O-trifluormethansulfonyl-4-O-acetyl-β-D-galactopyranosid:**

Eine Lösung von Verbindung 1.51.a (2,3 g, 4,4 mmol) in einer Mischung aus Dichlormethan (30 ml) und Pyridin (3 ml) wird unter Argon bei -20°C tropfenweise mit einer Lösung von Trifluormethansulfonsäure-anhydrid (2 ml, 11,8 mmol) in Dichlormethan (30 ml) versetzt. Nach 1 h bei -20°C gießt man den Ansatz in gesättigte Natriumhydrogencarbonat-Lösung (200 ml), trennt die organische Phase ab, trocknet über Magnesiumsulfat und engt nach Filtration im Vakuum ein. Nach Flashchromatographie [Toluol → Toluol/Ethylacetat 20:1] erhält man farblose Kristalle (2,39 g, 83 %); DC [Toluol/Ethylacetat 5:1]: $R_f$ = 0,55; $[\alpha]^{20}$ = -61,4° (c = 1,0 / $CH_2Cl_2$); Schmp. = 105°C.

**1.51.c) p-Nitrophenyl-2,6-di-O-benzyl-3-desoxy-β-D-galactopyranosid:**

Obige Verbindung (1,31 g, 2 mmol) wird in Toluol (25 ml) gelöst und mit Tetrabutylammonium-tetraborhydrat (1,54 g, 6 mmol) versetzt. Nach 2 h bei 80°C verdünnt man den Ansatz mit Dichlormethan (200 ml), wäscht einmal mit Wasser (50 ml), trocknet die organische Phase über Magnesiumsulfat und engt nach Filtration im Vakuum ein. Nach Flashchromatographie [Toluol/Ethylacetat 7:1] erhält man farblose Kristalle (596 mg, 64 %); DC [Toluol/Ethylacetat 5:1]: $R_f$ = 0,10; $[\alpha]^{20}$ = -81,3° (c = 1,0 / $CH_2Cl_2$); Schmp. = 114°C.

**1.51) p-Aminophenyl-3-desoxy-β-D-galactopyranosid:**

Verbindung 1.51.c (465 mg, 1 mmol) wird wie in Beispiel 1.40 beschrieben für 6 h hydriert. Nach Einengen des Filtrats fällt man den Rückstand aus Ethanol/Petrolether um und erhält farblose Kristalle (206 mg, 81 %); DC [Dichlormethan/Methanol/Ammoniak (25 %) 15:3:0,2]: $R_f$ = 0,22.

**<u>Beispiel 1.52</u>**

**p-Aminophenyl-3,4-didesoxy-β-D-galactopyranosid**

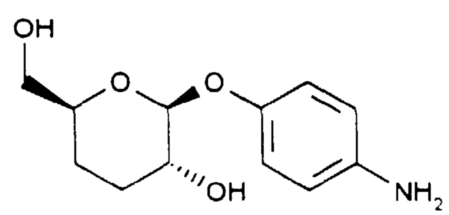

**1.52.a) p-Nitrophenyl-2,6-di-O-benzyl-3,4-di-O-trifluormethansulfonyl-β-D-galactopyranosid:**

Verbindung 1.31.b (2,12 g, 4,4 mmol) wird wie in Beispiel 1.51.b mit Trifluormethansulfonsäure-anhydrid (4 ml, 23,6 mmol) umgesetzt. Nach Flashchromatographie [Toluol → Toluol/Ethylacetat 50:1] erhält man ein gelbliches Öl (2,75 g, 84 %); DC [Toluol/Ethylacetat 5:1]: $R_f$ = 0,67; $[\alpha]^{20}$ = -22,5° (c = 1,0/$CH_2Cl_2$).

**1.52.b) p-Nitrophenyl-2,6-di-O-benzyl-3,4-didesoxy-β-D-galactopyranosid:**

Verbindung 1.52.a (1,49 g, 2 mmol) wird wie in Beispiel 1.51.c mit Tetrabutylammonium-tetraborhydrat (2,31 g, 9 mmol) umgesetzt. Nach Flashchromatographie [Toluol → Toluol/Ethylacetat 50:1] erhält man farblose Kristalle

(629 mg, 70 %); DC [Toluol/Ethylacetat 5:1]: $R_f$ = 0,53; $[\alpha]^{20}$ = -79,1° (c= 1,0 / $CH_2Cl_2$); Schmp. = 89°C.

**1.52) p-Aminophenyl-3,4-didesoxy-β-D-galactopyranosid:**

Verbindung 1.52.b (450 mg, 1 mmol) wird wie in Beispiel 1.40 beschrieben für 5 h hydriert. Nach Einengen des Filtrats fällt man den Rückstand aus Ethanol/Petrolether um und erhält farblose Kristalle (183 mg, 76 %); DC [Dichlormethan/Methanol/Ammoniak (25 %) 15:3:0,2]: $R_f$ = 0,47; $[\alpha]^{20}$ = -115,1° (c = 1,0 / $CH_3OH$); Schmp. = 187°C.

<u>**Beispiel 1.53**</u>

**p-Aminophenyl-6-O-acetyl-β-D-galactopyranosid**

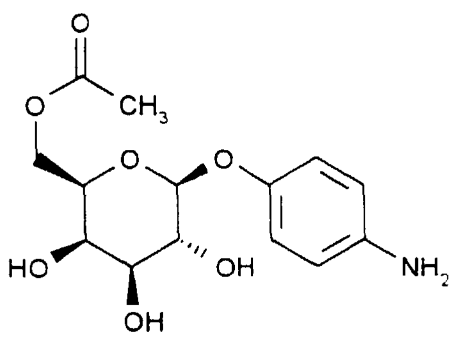

**1.53.a) p-Nitrophenyl-6-O-acetyl-β-D-galactopyranosid:**

Eine Lösung von p-Nitrophenyl-β-D-galactopyranosid (7,53 g, 25 mmol) in absolutem Acetonitril (80 ml) wird bei 0°C tropfenweise mit einer frisch angesetzten Lösung aus Pyridin (2 ml, 25 mmol) und Acetylchlorid (1,85 ml, 26 mmol) in Acetonitril (20 ml) versetzt. Man rührt für 30 min. bei 0°C und engt anschließend im Vakuum ein. Nach Flashchromatographie [Dichlormethan/Methanol 50:1 → 20:1] erhält man farblose Kristalle (4,02 g, 47 %); DC [Dichlormethan/Methanol/Ammoniak (25 %) 15:3:0,2]: $R_f$ = 0,50.

**1.53) p-Aminophenyl-6-O-acetyl-β-D-galactopyranosid:**

Verbindung 1.53.a (1,72 g, 5 mmol) wird wie in Beispiel 1.40 beschrieben für 2 h hydriert. Nach Einengen des Filtrats fällt man den Rückstand aus Methanol/Diethylether um und erhält farblose Kristalle (1,34 g, 86 %); DC [Dichlormethan/Methanol/Ammoniak (25 %) 15:3:0,2]: $R_f$ = 0,34; $[\alpha]^{20}$ = -41,0° (c = 0,56 / $CH_3OH$); Schmp. = 180°C (Zers.).

**Beispiel 1.54**

**p-Aminophenyl-3,4-di-O-methoxycarbonylmethyl-β-D-galactopyranosid**

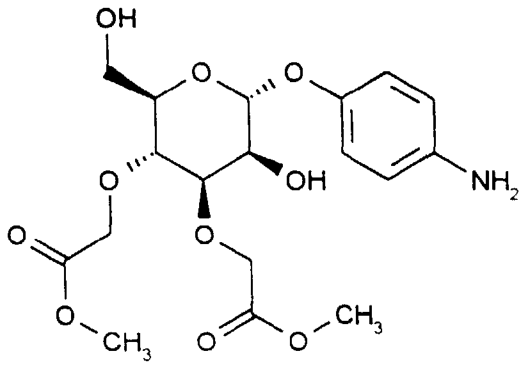

**1.54.a) Acylierung von Verbindung 1.24.a:**

Verbindung 1.24.a (1,36 g, 3 mmol) wird in Dimethylformamid (25 ml) gelöst und mit Bromessigsäure-methylester (1 ml, 10,6 mmol) und portionsweise mit einer 80%-igen Suspension von Natriumhydrid in Mineralöl (300 mg, 10 mmol) versetzt. Nach 3,5 h bei Raumtemperatur setzt man erneut Bromessigsäure-methylester (250 µl, 2,65 mmol) und Natriumhydnd in Mineralöl (75 mg, 2,5 mmol) zu. Nach weiteren 2 h beendet man die Reaktion durch Zutropfen von Methanol (5 ml) und engt im Vakuum ein Der Rückstand wird in Dichlormethan (250 ml) aufgenommen und die Lösung kräftig mit Wasser (100 ml) verrührt. Man trocknet die organische Phase über Magnesiumsulfat (10 g), engt im Vakuum ein und reinigt durch Flashchromatographie [Petrolether/Ethylacetat 10:1 → 7:1 → 5:1 → 3:1]. Man erhalt drei Produktfraktionen:

Fraktion 1:p-Nitrophenyl-2,3,4-tri-O-methoxycarbonylmethyl-β-D-galactopyranosid; farbloser Schaum (401 mg, 21 %); DC [Petrolether/Ethylacetat 1:1] $R_f$ = 0,47; $[\alpha]^{20}$ = -51,9° (c = 0,26 / $CH_3OH$)

Fraktion 2: nicht identifiziert; farbloser Schaum (88 mg); DC [Petrolether/Ethylacetat 1:1]: $R_f$ = 0,39; $[\alpha]^{20}$ = -61,5° (c = 0,26 / $CH_3OH$).

Fraktion 3: p-Nitrophenyl-3 ,4-di-O-methoxycarbonylmethyl-β-D-galactopyranosid; farbloser Schaum (275 mg, 16 %); DC [Petrolether/Ethylacetat 1:1]: $R_f$ = 0,30; $[\alpha]^{20}$ = -38,6° (c = 0,28 / $CH_3OH$).

**1.54) p-Aminophenyl-3,4-di-O-methoxycarbonylmethyl-β-D-galactopyranosid:**

Fraktion 3 aus Beispiel 1.54.a (206 mg, 0,3 mmol) wird wie in Beispiel 1.40 beschrieben für 16 h hydriert. Nach Einengen des Filtrats kocht man den Rückstand mit Diethylether (20 ml) aus und erhält graue Kristalle (45,6 mg, 37 %); DC [Petrolether/Ethylacetat 1:1]: $R_f$ = 0,22; Schmp. = 155°C (Zers.).

**Beispiel 1.55**

**p-Aminophenyl-2,3,4-tri-O-methoxycarbonylmethyl-β-D-galactopyranosid**

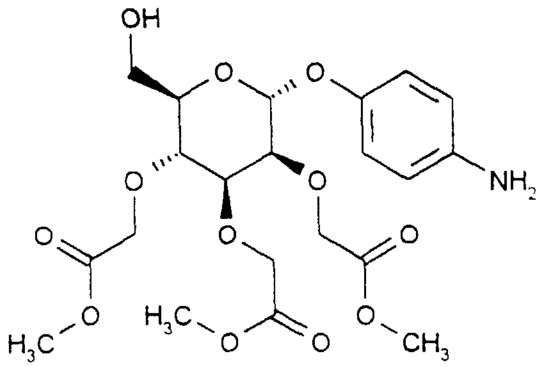

Fraktion 1 aus Beispiel 1.54.a (380 mg, 0,5 mmol) wird wie in Beispiel 1.40 beschrieben für 16 h hydriert. Nach Einengen des Filtrats kocht man den Rückstand mit Diethylether (20 ml) aus und erhält ein gelb-braunes Öl (46,8 mg, 19 %); DC [Petrolether/Ethylacetat 1:1]: $R_f$ = 0,29; Schmp. = 106°C (Zers.).

**Beispiel 1.56**

**p-Aminophenyl-4-O-(β-D-galactopyranosyl)-β-D-glucopyranosid**

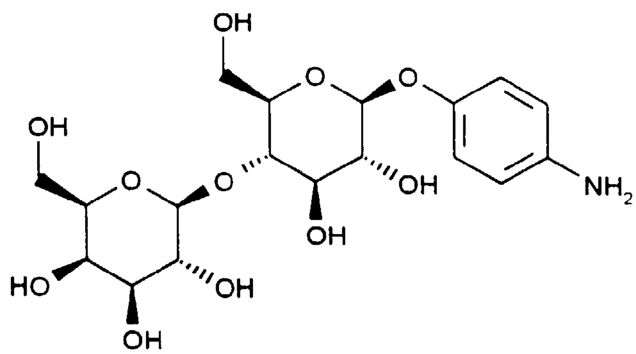

p-Nitrophenyl-4-O-(β-D-galactopyranosyl)-β-D-galactopyranosid (4,63 g, 10 mmol) wird wie in Beispiel 1.23 beschrieben hydriert. Man erhält farblose Kristalle (3,04 g, 70 %); DC [Methanol]: $R_f$ = 0,55; Schmp. = 235-237°C (Zers.).

**Beispiel 1.57**

**p-Aminophenyl-4-O-(3'-sulfat-β-D-galactopyranosyl)-β-D-glucopyranosid, Natriumsalz**

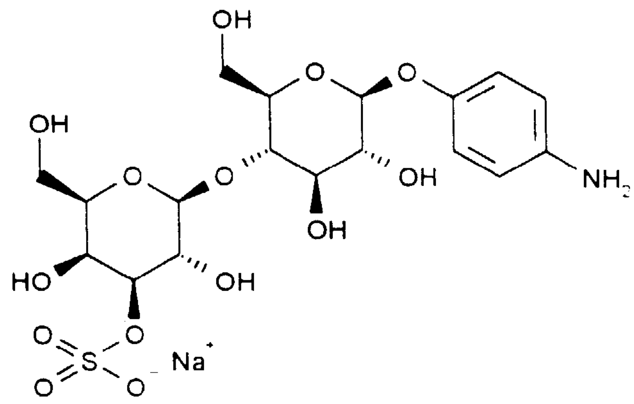

**1.57.a) p-Nitrophenyl-4-O-(3',4'-O-isopropyliden-β-D-galactopyranosyl)-β-D-glucopyranosid:**

p-Nitrophenyl-4-O-(β-D-galactopyranosyl)-β-D-glucopyranosid (23,2 g, 50 mmol) wird mit Dimethoxypropan (400 ml) und einer katalytischen Menge an (±)-Campher-10-sulfonsäure (400 mg, 1,7 mmol) versetzt. Nach 3 d bei Raumtemperatur beendet man die Reaktion durch Zugabe von Triethylamin (240 µl, 1,7 mmol), engt im Vakuum ein und trocknet für 2 h im Ölpumpenvakuum. Die resultierenden Kristalle werden in Methanol/Wasser 10:1 (500 ml) aufgenommen und für 6 h unter Rückfluß gekocht. Nach Einengen im Vakuum und Flashchromatographie [Dichlormethan/Methanol 25:1 → 10:1, jeweils mit 0,5 % Triethylamin] erhält man farblose Kristalle (15,2 g, 60 %); DC [Dichlormethan/Methanol 5:1]: $R_f$ = 0,49; Schmp. = 253-255°C (Zers.).

**1.57,b) p-Nitrophenyl-2,3,6-tri-O-benzoyl-4-O-(2',6'-di-O-benzoyl-3',4'-O-isopropyliden-β-D-galactopyranosyl)-β-D-glucopyranosid:**

Zu einer Lösung von Verbindung 1.57.a (15,1 g, 30 mmol) in Pyridin (300 ml) wird bei 0 °C innerhalb von 30 min. Benzoylchlorid (50 ml, 430 mmol) langsam zugetropft. Anschließend läßt man für weitere 2 h bei Raumtemperatur rühren und gießt den Ansatz dann unter Rühren in Eiswasser (2000 ml) Nach 15 min. werden die ausgefallenen Kristalle abfiltriert und in Dichlormethan (1500 ml) aufgenommen. Man wäscht die Lösung mit Wasser (2x 500 ml) und 1N-Natriumhydrogencarbonat-Lösung (2x 500 ml), trocknet die organische Phase über Magnesiumsulfat (50 g), engt im Vakuum ein und reinigt durch Umkristallisation aus Methanol. Man erhält farblose Kristalle (26,7 g, 87 %); DC [Dichlormethan/Methanol 50:1]: $R_f$ = 0,49; $[\alpha]^{20}$ = +23,6° (c = 1,08 / $CH_2Cl_2$); Schmp = 272-274°C.

**1.57.c) p-Nitrophenyl-2,3,6-tri-O-benzoyl-4-O-(2',6'-di-O-benzoyl-β-D-galactopyranosyl)-β-D-glucopyranosid:**

Eine Lösung von Verbindung 1.57.b (20,5 g, 20 mmol) in Dichlormethan (400 ml) wird mit 99%-iger Trifluoressigsäure (20 ml) versetzt und für 20 min bei Raumtemperatur gerührt. Anschließend wäscht man die Lösung mit 1N-Natriumhydrogencarbonat-Lösung (2x 200 ml), trocknet die organische Phase über Magnesiumsulfat (10 g), engt im Vakuum ein und reinigt den Rückstand durch Umfällen aus Dichlormethan/Diethylether. Man erhält farblose Kristalle (18,0 g, 91 %); DC [Dichlormethan/Methanol 20:1] $R_f$ = 0,18; Schmp. = 234°C.

**1.57.d) p-Nitrophenyl-2,3,6-tri-O-benzoyl-4-O-(2',6'-di-O-benzoyl-4'-O-acetyl-β-D-galactopyranosyl)-β-D-glucopyranosid:**

Verbindung 1.57.c (5,5 g, 5,6 mmol) wird wie in Beispiel 1.51.a beschrieben umgesetzt. Nach Flashchromatographie [Petrolether/Ethylacetat 3:1 → 1:1] erhält man farblose Kristalle (4,03 g, 70 %); DC [Dichlormethan/Methanol 20:1]; $R_f$ = 0,67; Schmp. = 118°C.

**1.57.e) p-Nitrophenyl-2,3,6-tri-O-benzoyl-4-O-(2',6'-di-O-benzoyl-3'-sulfat-4'-O-acetyl-β-D-galactopyranosyl)-β-D-glucopyranosid, Natriumsalz:**

Eine Lösung von Verbindung 1.57.d (3,59 g, 3,5 mmol) in Pyridin (200 ml) wird mit Schwefeltrioxid-Pyridin-Komplex (4,5 g, 28 mmol) versetzt und zuerst für 2 h bei 60°C und dann für 16 h bei Raumtemperatur gerührt. Anschließend beendet man die Reaktion durch Zutropfen von Methanol (50 ml) und engt im Vakuum ein. Der Rückstand wird durch Flashchromatographie [Dichlormethan/Methanol 10:1] gereinigt. Man erhält ein festes Produkt, das in Dichlormethan/Methanol 1:1 (200 ml) aufgenommen und mit Amberlite IR120 ($Na^+$-Form, 10 g) versetzt wird. Diese Mischung wird für 1 h bei Raumtemperatur gerührt, filtriert und das Filtrat im Vakuum eingeengt. Es fallen farblose Kristalle (3,64 g, 92 %) an; DC [Dichlormethan/Methanol 2:1]: $R_f$ = 0,87; Schmp. = 168°C.

**1.57.f) p-Nitrophenyl-4-O-(3'-sulfat-β-D-galactopyranosyl)-β-D-glucopyranosid, Natriumsalz:**

Verbindung 1.57.e (3,4 g, 3 mmol) wird in absolutem Methanol (150 ml) gelöst, mit Natriummethylat (200 mg) versetzt und für 7 h bei 60°C gerührt. Nach Abkühlung auf Raumtemperatur wird mit Lewatit SC108 ($H^+$-Form) neutralisiert und dann filtriert. Man erhöht den pH-Wert des Filtrats durch Zutropfen von 1N-Natronlauge auf pH 7-8, dampft im Vakuum ein und erhält durch Umfällen aus Methanol/Diethylether leicht bräunliche Kristalle (1.30 g, 77 %), DC [Dichlormethan/Methanol 2:1]: $R_f$ = 0,55; Schmp. = 230°C (Zers.).

**1.57) p-Aminophenyl-4-O-(3'-sulfat-β-D-galactopyranosyl)-β-D-glucopyranosid, Natriumsalz:**

Die obige Verbindung (1,13 g, 2 mmol) wird wie in Beispiel 1.24 beschrieben reduziert. Nach Auskochen mit Diethylether (50 ml) erhält man farblose Kristalle (983 mg, 92 %); DC [Dichlormethan/Methanol 2:1]: $R_f$ = 0,22; Schmp. = 176°C (Zers.).

**<u>Beispiel 1.58</u>**

**p-Aminophenyl-4-O-(3'-O-methyl-β-D-galactopyranosyl)-β-D-glucopyranosid**

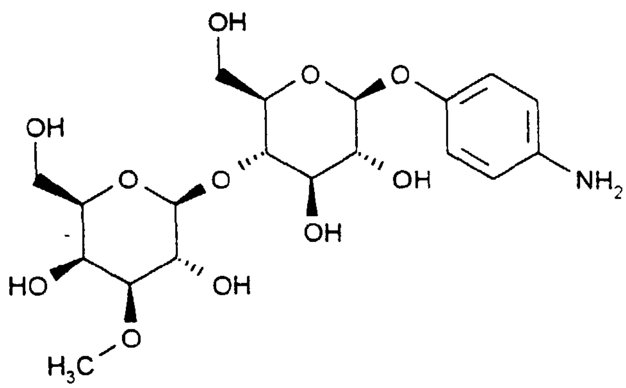

**1.58.a) selektive Methylierung von p-Nitrophenyl-4-O-(β-D-galactopyranosyl)-β-D-glucopyranosid:**

p-Nitrophenyl-4-O-(β-D-galactopyranosyl)-β-D-glucopyranosid (2,3 g, 5 mmol) wird wie in Beispiel 1.25.a beschrieben methyliert Durch Flashchromatographie [Dichlormethan/Methanol 20:1 → 10:1 → 5:1] erhält man zwei Produkte:

Fraktion 1: p-Nitrophenyl-2-O-methyl-4-O-(3'-O-methyl-β-D-galactopyranosyl)-β-D-glucopyranosid; farblose Kristalle (264 mg, 11 %); DC [Dichlormethan/Methanol 5:1]: $R_f$ = 0,46; $[\alpha]^{20}$ = -73,9° (c = 1,0 / $CH_3OH$); Schmp. = 228°C (Zers.).

Fraktion 2: p-Nitrophenyl-4-O-(3'-O-methyl-β-D-galactopyranosyl)-β-D-glucopyranosid; farblose Kristalle (1,0 g, 42 %), DC [Dichlormethan/Methanol 5:1]: $R_f$ = 0,29; $[\alpha]^{20}$ = -65,3° (c = 1,1 / $CH_3OH$); Schmp. = 220°C.

**1.58) p-Aminophenyl-4-O-(3'-O-methyl-β-D-galactopyranosyl)-β-D-glucopyranosid:**

Fraktion 2 aus Beispiel 1.58.a (955 mg, 2 mmol) wird wie in Beispiel 1.24 beschrieben reduziert. Nach Waschen mit Diethylether (50 ml) erhält man farblose Kristalle (894 mg, 100 %); DC [Dichlormethan/Methanol 5:1]: $R_f$ = 0,08; Schmp. = 129 °C (Zers.).

**Beispiel 1.59**

**p-Aminophenyl-2-O-methyl-4-O-(3'-O-methyl-β-D-galactopyranosyl)-β-D-glucopyranosid**

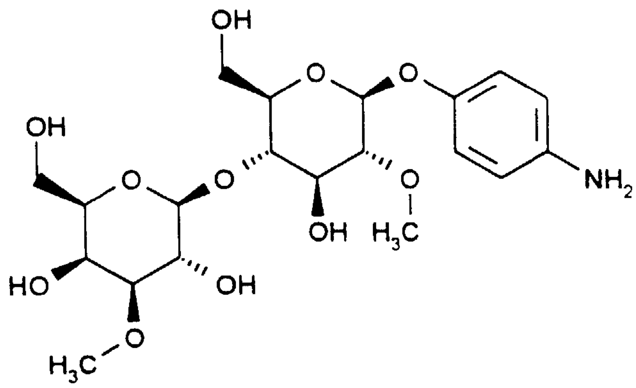

Fraktion 1 aus Beispiel 1.58.a (246 mg, 0,5 mmol) wird wie in Beispiel 1.24 beschrieben reduziert. Nach Waschen mit Diethylether (20 ml) erhält man farblose Kristalle (186 mg, 81 %); DC [Dichlormethan/Methanol 5:1]: $R_f$ = 0,13; $[\alpha]^{20}$ = -3,6° (c = 1,0 / $CH_3OH$); Schmp. = 105 °C.

**Beispiel 1.60**

**p-Aminophenyl-4-O-(3',4'-di-O-methyl-β-D-galactopyranosyl)-β-D-gluco pyranosid**

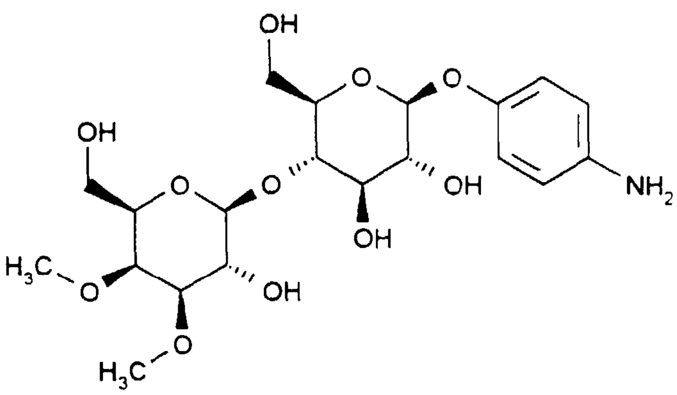

**1.60.a) p-Nitrophenyl-2,3,6-tri-O-benzyl-4-O-(2',6'-di-O-benzyl-3',4'-O-isopropyliden-β-D-galactopyranosyl)-β-D-glucopyranosid:**

Verbindung 1.57.a (5,0 g, 10 mmol) wird wie in Beispiel 1.26.c beschrieben mit Benzylbromid (30 ml, 250 mmol) für 16 h umgesetzt. Nach Einengen im Vakuum wird der Rückstand in Ethylacetat (300 ml) aufgenommen und die Lösung mit Wasser (200 ml) gewaschen. Man trocknet die organische Phase über Magnesiumsulfat (10 g), engt

im Vakuum ein und reinigt durch Flashchromatographie [Dichlormethan/Petrolether 5:1 → Dichlormethan]. Man erhält ein bräunliches Öl (5,3 g, 56 %); DC [Dichlormethan/Methanol 50:1]: $R_f$ = 0,70; $[\alpha]^{20}$ = -23,2° (c = 1,08 / $CH_2Cl_2$)

**1.60.b) p-Nitrophenyl-2,3,6-tri-O-benzyl-4-O-(2',6'-di-O-benzyl-β-D-galactopyranosyl)-β-D-glucopyranosid:**

Die obige Verbindung (4,77 g, 5 mmol) wird wie in Beispiel 1.57.c beschrieben umgesetzt. Nach Einengen im Vakuum und Umfällen aus Diethylether/Petrolether erhält man farblose Kristalle (3,94 g, 86 %); DC [Dichlormethan/Methanol 50:1]: $R_f$ = 0,36; Schmp. = 116°C.

**1.60.c) p-Nitrophenyl-2,3,6-tri-O-benzyl-4-O-(2',6'-di-O-benzyl-3',4'-di-O-methyl-β-D-galactopyranosyl)-β-D-glucopyranosid:**

Verbindung 1.60.b (1,8 g, 2 mmol) wird wie in Beispiel 1.24.c beschrieben methyliert. Durch Umfällen aus Dichlormethan/Petrolether erhält man farblose Kristalle (1,55 g, 82 %); DC [Dichlormethan/Methanol 50:1]: $R_f$ = 0,74; Schmp. = 161-162°C.

**1.60) p-Aminophenyl-4-O-(3',4'-di-O-methyl-β-D-galactopyranosyl)-β-D-gluco-pyranosid:**

Verbindung 1.60.c (1,41 g, 1,5 mmol) wird in Methanol (50 ml) gelöst und nach Zugabe von Palladiumhydroxid auf Kohle (feucht, 20% Pd, 500 mg) für 6 d in einer Wasserstoffatmosphäre unter geringem Überdruck hydriert. Die Suspension wird über Celite filtriert und das Filtergut gründlich mit Methanol (100 ml) gewaschen. Einengen des Filtrats im Vakuum und Waschen des Rückstands mit Dichlormethan ergibt bräunliche Kristalle (425 mg, 61 %); Schmp. = 124°C (Zers.).

<u>**Beispiel 2.1**</u>

**N-Alanyl-batracylin**

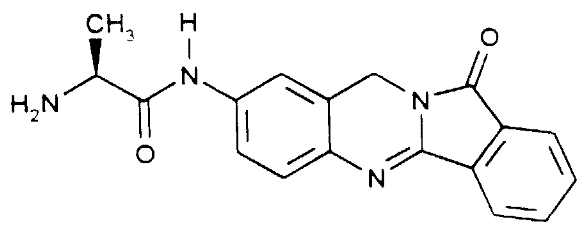

**2.1.a) N-[N-(tert-Butoxycarbonyl)-alanyl]-batracylin:**

N-(tert-Butoxycarbonyl)-alanin (3,3 g, 17,5 mmol) und 2-isobutoxy-1-isobutoxy-carbonyl-1,2-dihydro-chinolin (6,8 ml, 23 mmol) werden in 100 ml Dichlormethan gelöst. Nach 20 min, Rühren bei Raumtemperatur setzt man eine Lösung von Batracylin (4,1 g, 16,5 mmol) in absolutem Dimethylformamid (350 ml) zu und ruhrt den Ansatz für weitere 48 h bei Raumtemperatur. Anschließend wird im Vakuum auf 50 ml eingeengt, mit Ethylacetat auf 300 ml aufgefüllt und sofort für 10 min. zum Sieden erhitzt. Anschließend läßt man auf Raumtemperatur abkühlen, filtriert ab und kocht das Filtergut erneut mit Ethylacetat (200 ml) aus. Abkühlung unter Rühren auf 0°C und Filtration ergibt gelbe Kristalle (6,18 g, 84 %); DC [Ethylacetat]: $R_f$ = 0,57; Schmp. = 246-247°C (Zers.).

**2.1) N-Alanyl-batracylin:**

Eine Lösung von Verbindung 2.1.a (10,5 g, 25 mmol) in wasserfreier Trifluoressigsäure (150 ml) wird für 15 min. bei Raumtemperatur gerührt. Nach Einengen im Vakuum auf 30 ml wird der Ansatz unter kräftigem Rühren in gesättigte Natriumhydrogencarbonat-Lösung (1000 ml) gegossen. Man rührt für 10 min. weiter, filtriert ab und wäscht mit Wasser, wenig Isopropanol und Diethylether. Das Produkt fällt in gelben Kristallen (7,15 g, 89 %) an; DC [Ethylacetat]: $R_f$ = 0,06; Schmp. = 261-262°C (Zers.).

**Beispiel 2.2**

**N-[Lysyl-alanyl]-batracylin, Di-Trifluoracetat**

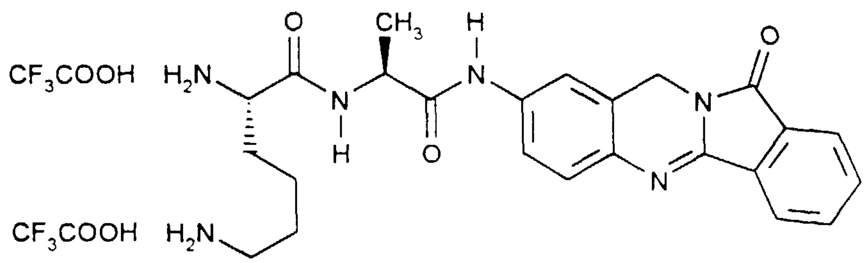

**2.2.a) N-[N$^{\alpha}$,N$^{\varepsilon}$-Di-(tert-butoxycarbonyl)-lysyl-alanyl]-batracylin:**

N,N-Di-(tert-butoxycarbonyl)-lysin (2,1 g, 6 mmol) und 2-Isobutoxy-1-isobutoxy-carbonyl-1,2-dihydro-chinolin (2,4 ml, 8 mmol) werden in 20 ml Dichlormethan gelöst. Nach 20 min. Rühren bei Raumtemperatur setzt man eine Lösung aus Verbindung 2.1 (1,6 g, 5 mmol) in Dimethylformamid (40 ml) zu und rührt den Ansatz für weitere 16 h bei Raumtemperatur. Anschließend wird im Vakuum eingeengt und der Rückstand durch Flashchromatographie [Petrolether/Ethylacetat 2:1 → 1:1 → Ethylacetat] gereinigt. Man erhält gelbe Kristalle (2,89 g, 89 %); DC [Ethylacetat]: $R_f$ = 0,52; Schmp = 203-204°C.

**2.2) N-[Lysyl-alanyll-batracylin, Di-Trifluoracetat:**

Eine Suspension der obigen Verbindung (2,6 g, 4 mmol) in Dichlormethan (25 ml) wird mit wasserfreier Trifluoressigsäure (10 ml) versetzt und die resultierende Lösung für 30 min. bei Raumtemperatur gerührt. Nach Einengen im Vakuum wird der Rückstand durch Zugabe von Diethylether (100 ml) kristallisiert. Der Niederschlag wird abfiltriert und intensiv mit Diethylether gewaschen. Man erhält gelbe Kristalle (2,68 g, 99 %); DC [Ethylacetat]: $R_f$ = 0,05; Schmp. = 144-146°C (Zers.).

**Beispiel 2.3**

**N-[D-Alanyl]-batracylin**

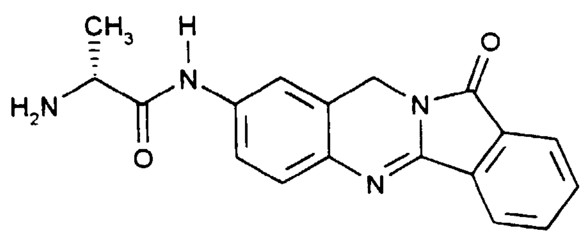

**2.3.a) N-[N-Benzyloxycarbonyl-D-alanyl]-batracylin:**

N-Benzyloxycarbonyl-D-alanin (3,9 g, 17,5 mmol) wird wie in Beispiel 2.1.a beschrieben umgesetzt und aufgearbeitet. Die resultierenden gelben Kristalle (6,4 g, 80 %) werden durch Filtration abgetrennt und die vereinigten Filtrate nach Einengen im Vakuum durch Flashchromatographie [Petrolether/Ethylacetat 3:2 → 1:1] gereinigt. Man erhält weitere 1,35 g (17 %), DC [Ethylacetat]: $R_f$ = 0,45; Schmp. = 256°C; $[\alpha]^{20}$ = +75,1° (c = 1,0 / $CH_2Cl_2$ + 0,5 % $CH_3OH$).

**2.3) N-[D-Alanyl]-batracylin:**

Verbindung 2.3.a (11,4 g, 25 mmol) wird in einer 33%-igen Lösung von Bromwasserstoff in Eisessig (100 ml) gelöst. Nach 30 min bei Raumtemperatur wird der Ansatz im Vakuum auf 30 ml eingeengt und anschließend unter kräftigem Rühren in gesättigte Natriumhydrogencarbonat-Lösung (1000 ml) gegossen. Man rührt für 10 min weiter, filtriert ab und wäscht mit Wasser, wenig Isopropanol und

Diethylether. Das Produkt fällt in gelben Kristallen (7,87 g, 98 %) an; DC [Ethylacetat]: $R_f$ = 0,06; Schmp. = 267°C (Zers.).

**Beispiel 2.4**

**N-[N$^{\alpha}$-(tert-Butoxycarbonyl)-lysyl-D-alanyl]-batracylin**

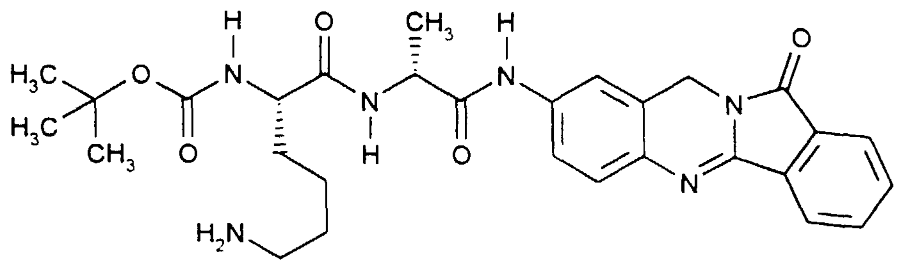

**2.4.a) N-[N$^{\alpha}$-(tert-Butoxycarbonyl)-N$^{\varepsilon}$-(fluorenyl-9-methoxycarbonyl)-lysyl-D-alanyl]-batracylin:**

N$^{\alpha}$-(tert-Butoxycarbonyl)-N$^{\varepsilon}$-(fluorenyl-9-methoxycarbonyl)-lysin (5,3 g, 11,3 mmol) und 2-Isobutoxy-1-isobutoxycarbonyl-1,2-dihydro-chinolin (4 ml, 14 mmol) werden in 40 ml Dichlormethan gelöst. Nach 20 min Rühren bei Raumtemperatur setzt man eine Lösung von Verbindung 2.3 (3,2 g, 10 mmol) in Dimethylformamid (80 ml) zu und rührt den Ansatz für weitere 16 h bei Raumtemperatur. Anschließend wird im Vakuum eingeengt und der Rückstand in Dichlormethan (100 ml) suspendiert. Die erhaltene Suspension wird mit Diethylether (300 ml) aufgefüllt. Nach Abfiltrieren und Waschen des Filterguts mit Diethylether erhält man gelbe Kristalle (5,65 g, 65 %); DC [Ethylacetat]: $R_f$ = 0,45; Schmp. = 186°C.

**2.4) N-[N$^{\alpha}$-(tert-Butoxycarbonyl)-lysyl-D-alanyl]-batracylin:**

Die obige Verbindung (5,6 g, 7,3 mmol) wird in Dimethylformamid (50 ml) gelöst. Nach Zugabe von Piperidin (50 ml) rührt man für 3 h bei Raumtemperatur, engt dann im Vakuum ein und reinigt den Rückstand durch Flashchromatographie [Dichlormethan/Methanol/Ammoniak (25 %) 15:3:0,1 → 15:5:0,1]. Man erhält gelbe Kristalle (2,5 g, 62 %); Schmp. = 217°C (Zers.).

**Beispiel 2.5**

**N-[N$^{\alpha}$-(Fluorenyl-9-methoxycarbonyl)-lysyl-D-alanyl]-batracylin, Trifluoracetat**

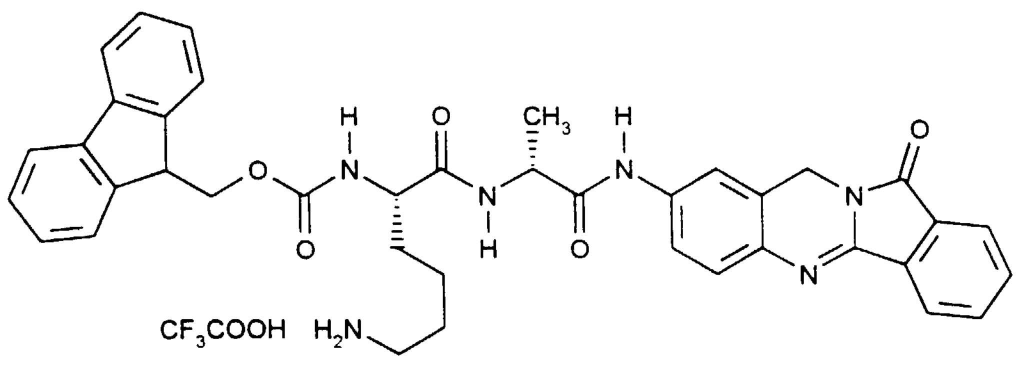

**2.5.a) N-[N$^{\alpha}$-(Fluorenyl-9-methoxycarbonyl)-N$^{\varepsilon}$-(tert-butoxycarbonyl)-lysyl-D-alanyl]-batracylin:**

N$^{\alpha}$-(Fluorenyl-9-methoxycarbonyl)-N$^{\varepsilon}$-(tert-butoxycarbonyl)-lysin (5,3 g, 11,3 mmol) wird wie in Beispiel 2.4.a beschrieben umgesetzt und gereinigt. Man erhält gelbe Kristalle (7,0 g, 80 %); DC [Ethylacetat]: $R_f$ = 0,51; Schmp. = 223°C.

**2.5) N-[N$^{\alpha}$-(Fluorenyl-9-methoxycarbonyl)-lysyl-D-alanyl]-batracylin, Trifluoracetat:**

Verbindung 2.5.a (6,17 g, 8 mmol) wird wie in Beispiel 2.2 beschrieben umgesetzt. Nach Einengen im Vakuum fällt man den Rückstand aus Dichlormethan/Diethylether um und erhält gelbe Kristalle (6,08 g, 97 %), DC [Ethylacetat]: $R_f$ = 0,05; Schmp. = 224°C (Zers.).

**Beispiel 2.6**

**N-[N$^{\varepsilon}$-(Fluorenyl-9-methoxycarbonyl)-lysyl-D-alanyl]-batracylin, Trifluoracetat**

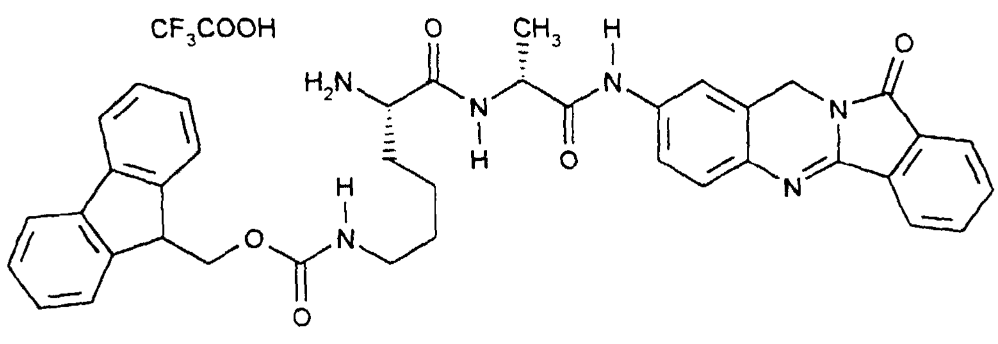

Verbindung 2.4.a (6,17 g, 8 mmol) wird wie in Beispiel 2.2 beschrieben umgesetzt. Nach Einengen im Vakuum fällt man den Rückstand aus Dichlormethan/Diethylether um und erhält gelbe Kristalle (5,97 g, 95 %); DC [Ethylacetat]: $R_f$ = 0,04; Schmp. = 188°C (Zers.).

**Beispiel 2.7**

**N-[Lysyl-D-asparagyl]-batracylin, Di-Trifluoracetat**

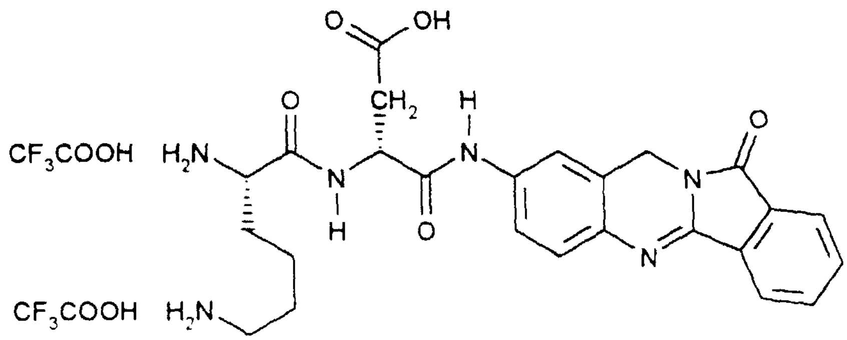

**2.7.a) N-[N-(Fluorenyl-9-methoxycarbonyl)-D-asparagyl-(ß-tert-butylester))-batracylin:**

N-(Fluorenyl-9-methoxycarbonyl)-D-asparagyl-(β-tert-butylester) (7,2 g, 17,5 mmol) wird wie im Beispiel 2.1.a beschrieben umgesetzt. Nach Einengen im Vakuum wird der Rückstand in Dichlormethan (1000 ml) aufgenommen und mit 1N-Salzsäure (2x 200 ml) und mit 1N-Natriumhydrogencarbonat-Lösung (1x 200 ml) gewaschen. Nach Trocknung über Magnesiumsulfat (20 g), Filtration, Einengen auf 100 ml und Zugabe von Petrolether fällt die Verbindung 2.7.a in Form von gelben Kristallen (9,7 g, 86 %) an; DC [Ethylacetat]: $R_f$ = 0,71, Schmp. = 195°C.

**2.7.b) N-[D-Asparagyl-(β-tert-butylester)]-batracylin:**

Die obige Verbindung (6,4 g, 10 mmol) wird in Dichlormethan (100 ml) gelöst. Nach Zugabe von Morpholin (50 ml) rührt man für 5 h bei Raumtemperatur, engt dann im Vakuum ein und reinigt den Rückstand durch Flashchromatographie [Ethylacetat/Petrolether 4:1 → Ethylacetat → Ethylacetat/Ethanol 10:1]. Man erhält gelbe Kristalle (3,44 g, 82 %); DC [Ethylacetat]: $R_f$ = 0,21; Schmp. = 209°C (Zers.).

**2.7.c) N-[$N^{\alpha}$,$N^{\varepsilon}$-Di-(tert-butoxycarbonyl)-lysyl-D-asparagyl-(β-tert-butylester)]-batracylin:**

Verbindung 2.7.b (2,1 g, 5 mmol) wird wie in Beispiel 2.2.a beschrieben umgesetzt. Man erhält gelbe Kristalle (1,71 g, 46 %); DC [Ethylacetat]: $R_f$ = 0,61, Schmp. = 142°C.

**2.7) N-[Lysyl-D-asparagyl]-batracylin, Di-Trifluoracetat:**

Verbindung 2.7.c (1,65 g, 2,2 mmol) wird wie in Beispiel 2.2 beschrieben umgesetzt und gereinigt. Man erhält gelbe Kristalle (1,5 g, 95 %), DC [Methanol/Essigsäure 10:1]: $R_f$ = 0,29; Schmp. = 154-155°C (Zers.).

**Beispiel 2.8**

**N-[Lysyl-D-glutamyl]-batracylin, Di-Hydrobromid**

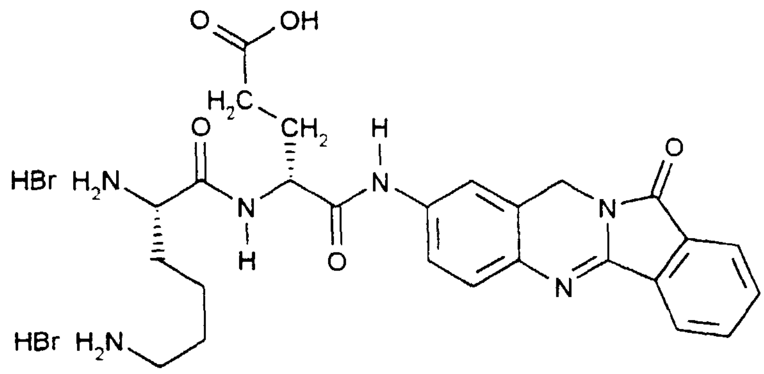

**2.8.a) N-[N-(tert-Butoxycarbonyl)-D-glutamyl-(β-benzylester)]-batracylin:**

N-(tert-Butoxycarbonyl)-D-glutamyl-(β-benzylester) (5,9 g, 17,5 mmol) wird wie im Beispiel 2.1.a beschrieben umgesetzt. Nach Einengen im Vakuum wird der Rückstand durch Flashchromatographie [Petrolether/Ethylacetat 1:1] zu gelben Kristallen (9,45 g, 95 %) gereinigt; DC [Ethylacetat]: $R_f$ = 0,61, $[\alpha]^{20}$ = +53,1° (c = 1,0 / $CH_2Cl_2$); Schmp. = 159°C.

**2.8.b) N-[D-Glutamyl-(β-benzylester)]-batracylin:**

Verbindung 2.8.a (9,1 g, 10 mmol) wird in Ameisensäure (100 ml) gelöst und die Lösung für 6 h bei Raumtemperatur gerührt. Nach Einengen im Vakuum wird der Rückstand in Methanol (100 ml) aufgenommen und der pH-Wert der Lösung durch vorsichtige Zugabe von 25 %-iger wäßriger Ammoniaklösung auf pH 8 erhöht. Nach erneutem Einengen im Vakuum und durch anschließende Flash-chromatographie [Ethylacetat/Ethanol 10:1] erhält man ein gelbes Öl (4,2 g, 56 %), DC [Ethylacetat]: $R_f$ = 0,06.

**2.8.c) N-[$N^{\alpha}$,$N^{\varepsilon}$-Di-(tert-butoxycarbonyl)-lysyl-D-glutamyl-(β-benzylester)]-batracylin:**

Die obige Verbindung (3,75 g, 8 mmol) wird wie in Beispiel 2.2.a beschrieben umgesetzt und gereinigt. Man erhält einen gelben amorphen Feststoff (2.26 g, 35 %), DC [Ethylacetat]: $R_f$ = 0,40; $[\alpha]^{20}$ = +32,1° (c = 1,2 /$CH_2Cl_2$).

**2.8) N-[Lysyl-D-glutamyl)-batracylin, Di-Hydrobromid:**

Verbindung 2.8.c (2,0 g, 2,5 mmol) wird in einer 33%-igen Lösung von Bromwasserstoff in Eisessig (50 ml) gelöst. Nach 1 h bei Raumtemperatur wird der Ansatz im Vakuum eingeengt und der Rückstand gründlich mit Diethylether gewaschen. Das Produkt fällt in gelb-roten Kristallen (1,63 g, 98 %) an; Schmp. = 207-209°C (Zers.).

**Beispiel 2.9**

**N-[Lysyl-glycyl]-batracylin, Di-Trifluoracetat**

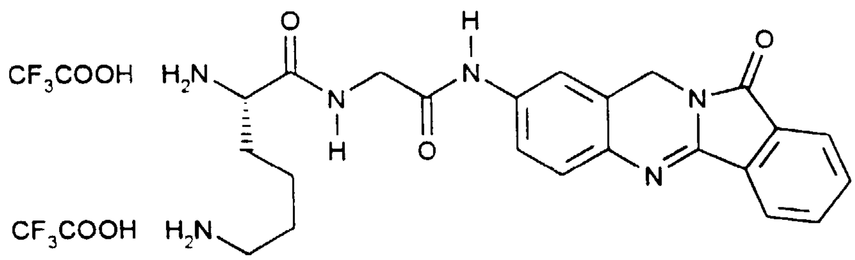

**2.9.a) N-[N-(tert-Butoxycarbonyl)-glycyl]-batracylin:**

N-(tert-Butoxycarbonyl)-glycin (3,07 g, 17,5 mmol) wird wie im Beispiel 2.1.a beschrieben umgesetzt. Nach 3 d bei Raumtemperatur wird im Vakuum eingeengt und der Rückstand in Ethanol (200 ml) aufgenommen. Nach 30 min. Rühren unter Rückfluß und Filtration nach Abkühlung fällt die Zielverbindung in Form von gelben Kristallen (4,73 g, 66 %) an; DC [Ethylacetat]: $R_f$ = 0,44; Schmp. = 279°C (Zers.).

**2.9.b) N-Glycyl-batracylin, Hydrochlorid**

Die obige Verbindung (4,1 g, 10 mmol) wird unter Erwärmen im Ultraschallbad in Dichlormethan (1200 ml) gelöst. Nach Zugabe von Chlorwasserstoff in Diethylether (100 ml) rührt man 30 min. bei Raumtemperatur, engt im Vakuum ein und versetzt den Rückstand mit Ethanol (200 ml). Nach 10 min Rühren unter Rückfluß und Filtration nach Abkühlung fällt das Produkt in Form von gelben Kristallen (3,21 g, 94%) an; Schmp. = 297-299°C (Zers.).

**2.9.c) N-[$N^{\alpha}$,$N^{\varepsilon}$-Di-(tert-butoxycarbonyl)-lysyl-glycyl)-batracylin:**

N,N-Di-(tert-butoxycarbonyl)-lysin (2,1 g, 6 mmol) und 2-Isobutoxy-1-isobutoxy-carbonyl-1,2-dihydro-chinolin (2,4 ml, 8 mmol) werden in 20 ml Dichlormethan gelöst. Nach 20 min. Rühren bei Raumtemperatur setzt man eine Lösung aus Verbindung 2.9.b (1,71 g, 5 mmol), Ethyldiisopropylamin (0,86 ml, 5 mmol) und Dimethylformamid (40 ml) zu und rührt den Ansatz für weitere 16 h bei Raumtemperatur. Anschließend wird im Vakuum eingeengt und durch Flash-chromatographie [Ethylacetat/Petrolether 1:1 → Ethylacetat] gereinigt. Man erhält gelbe Kristalle (1,69 g, 53 %); DC [Ethylacetat]: $R_f$ = 0,31; Schmp. = 211°C (Zers.).

**2.9) N-[Lysyl-glycyll-batracylin, Di-Trifluoracetat:**

Verbindung 2.9.c (1,4 g, 2,2 mmol) wird wie in Beispiel 2.2 beschrieben umgesetzt und gereinigt. Man erhält gelbe Kristalle (1,33 g, 91 %); Schmp. = 153°C (Zers.).

**Beispiel 2.10**

**N-[Lysyl-seryl]-batracylin, Di-Trifluoracetat**

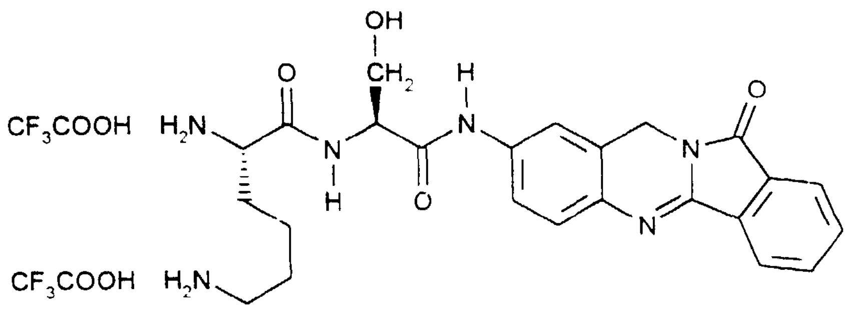

**2.10.a) N-[N-(tert-Butoxycarbonyl)-seryl)-batracylin:**

N-(tert-Butoxycarbonyl)-serin (3,6 g, 17,5 mmol) wird wie im Beispiel 2.1.a beschrieben umgesetzt. Nach 48 h wird im Vakuum auf 100 ml eingeengt und mit 2 1 Dichlormethan versetzt. Man wäscht die resultierende Lösung mit Wasser (1 x 500 ml), mit 0,5N-Salzsäure (2 x 250 ml) und gesättigter Natriumhydrogencarbonat-Lösung (1 x 250 ml) Trocknung über Magnesiumsulfat (50 g), Abdestillieren des Lösungsmittels im Vakuum und Flashchromatographie [Petrolether/Ethylacetat 1:1] des Rückstands ergibt Verbindung 2.10.a (4,6 g, 64 %) in Form von gelben Kristallen; DC [Ethylacetat/Essigsäure 100:1]: $R_f$ = 0,38; Schmp. = 219°C (Zers.); $[\alpha]^{20}$ = -61,0° (c = 0,5 / $CH_2Cl_2$ + 0,5 % $CH_3OH$).

**2.10.b) N-Seryl-batracylin, Hydrochlorid:**

Eine Suspension der obigen Verbindung (4,6 g, 10,4 mmol) in Dioxan (70 ml) wird unter Rühren mit konzentrierter Salzsäure (10 ml) versetzt und dann für 1 h kräftig bei Raumtemperatur gerührt. Anschließend engt man im Vakuum ein und trocknet den Rückstand für 2 h im Ölpumpenvakuum. Nach Zugabe von Ethanol (100 ml) wird für 15 min. unter Rückfluß gekocht. Abkühlen und Absaugen ergibt orange Kristalle (1,97 g, 96 %); DC [Ethylacetat]: $R_f$ = 0,05; $[\alpha]^{20}$ = +51,8° (c = 1,0 / $H_2O$); Schmp. >270°C (Zers.).

**2.10.c) N-[$N^{\alpha}$,$N^{\varepsilon}$-Di-(tert-butoxycarbonyl)-lysyl-seryl]-batracylin:**

Verbindung 2.10.b (1,86 g, 5 mmol) wird wie in Beispiel 2.9.c beschrieben umgesetzt. Nach Flashchromatographie [Ethylacetat/Petrolether 2:1 → Ethylacetat] erhält man gelbe Kristalle (1,18 g, 36 %); DC [Ethylacetat/Essigsäure 100:1]: $R_f$ = 0,24; Schmp. = 188°C (Zers.); $[\alpha]^{20}$ = -13,1° (c = 0,5 / $CH_2Cl_2$ + 0,5 % $CH_3OH$).

**2.10) N-[Lysyl-seryl)-batracylin, Di-Trifluoracetat:**

Verbindung 2.10.c (1,0 g, 1,5 mmol) wird wie in Beispiel 2.2 beschrieben umgesetzt und gereinigt. Man erhält gelbe Kristalle (1,0 g, 96 %); DC [Dichlormethan/Methanol/Ammoniak (25 %) 15:3:0,2]: $R_f$ = 0,10; Schmp. = 188-190°C (Zers.).

**Beispiel 2.11**

**N-[Lysyl-D-seryl]-batracylin, Di-Hydrobromid**

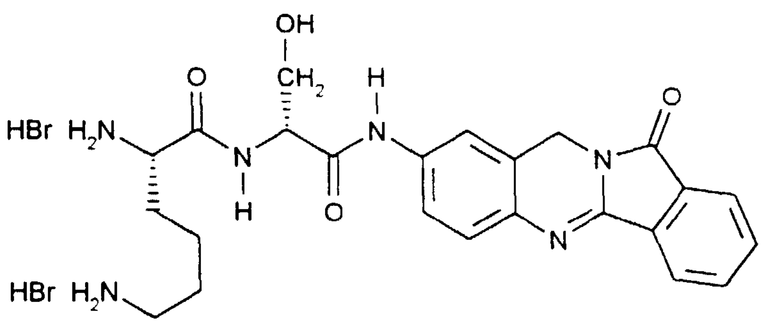

**2.11.a) N-[N-(Fluorenyl-9-methoxycarbonyl)-O-(tert-butyl)-D-seryl]-batracylin:**

N-(Fluorenyl-9-methoxycarbonyl)-O-(tert-butyl)-D-serin (6,7 g, 17,5 mmol) wird wie im Beispiel 2.1.a beschrieben umgesetzt. Einengen im Vakuum und Flash-chromatographie [Petrolether/Ethylacetat 1:1] liefert die Verbindung 2.11.a (5,64 g, 52 %) in Form von gelben Kristallen; DC [Dichlormethan/Methanol/Ammoniak (25 %) 15:3:0,2]: $R_f$ = 0,87; Schmp. = 225-226°C (Zers.)

**2.11.b) N-[O-(tert-Butyl)-D-seryl]-batracylin:**

Die obige Verbindung (2,89 g, 4,7 mmol) wird wie in Beispiel 2.7.b beschrieben umgesetzt. Durch Flashchromatographie [Petrolether/Ethylacetat 3:2 $\rightarrow$ Ethylacetat] erhält man das Produkt als gelbe Kristalle (1,15 g, 62 %); DC [Ethylacetat]: $R_f$ = 0,11; Schmp. = 197°C.

**2.11.c) N-[$N^{\alpha}$,$N^{\varepsilon}$-Di-(tert-butoxycarbonyl)-lysyl-O-(tert-butyl)-D-seryl]-batracylin:**

Obige Verbindung (1,1 g, 2,8 mmol) wird wie in Beispiel 2.2.a beschrieben umgesetzt. Man erhält gelbe Kristalle (1,94 g, 96 %); DC [Ethylacetat]: $R_f$ = 0,59; Schmp. = 208°C.

**2.11.d) N-[Lysyl-O-(tert-butyl)-D-seryl]-batracylin, Di-Trifluoracetat:**

Verbindung 2.11.c (1,08 g, 1,5 mmol) wird wie in Beispiel 2.2 beschrieben umgesetzt und gereinigt. Man erhält gelbe Kristalle (1,1 g, 98 %); DC [Methanol/Essigsäure 10:1]: $R_f$ = 0,30; Schmp. = 128°C.

**2.11) N-]Lysyl-D-seryl]-batracylin, Di-Hydrobromid:**

Verbindung 2.11.d (1,05 g, 1,4 mmol) wird wie in Beispiel 2.8 beschrieben umgesetzt und gereinigt. Man erhält gelb-rote Kristalle (846 mg, 96 %); Schmp. = 247-248°C.

**Beispiel 2.12**

**N-[Lysyl-D-threonyl]-batracylin, Di-Hydrobromid**

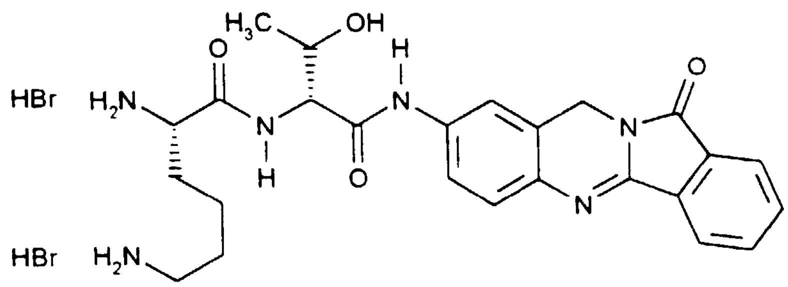

**2.12.a) N-[N-(Fluorenyl-9-methoxycarbonyl)-O-(tert-butyl)-D-threonyl)- batracylin:**

N-(Fluorenyl-9-methoxycarbonyl)-O-(tert-butyl)-D-threonin (6,96 g, 17,5 mmol) wird wie im Beispiel 2.1.a beschrieben umgesetzt. Einengen im Vakuum und Flashchromatographie [Petrolether/Ethylacetat 1:1] liefert die Verbindung 2.12.a (7,45 g, 68 %) in Form von gelben Kristallen; DC [Ethylacetat]: $R_f$ = 0.63; Schmp. = 225-226°C.

**2.12.b) N-[O-(tert-Butyl)-D-threonyl]-batracylin:**

Verbindung 2.12.a (3,8 g, 6 mmol) wird wie in Beispiel 2.7.b beschrieben umgesetzt. Nach Einengen im Vakuum erhält man das Produkt als gelbe Kristalle (1,9 g, 78 %); DC [Ethylacetat]: $R_f$ = 0,21; Schmp. = 110-111°C.

**2.12.c) N-[N$^{\alpha}$,N$^{\varepsilon}$-Di-(tert-butoxycarbonyl)-lysyl-O-(tert-butyl)-D-threonyl]-batracylin**

Verbindung 2.12.b (1,8 g, 4,5 mmol) wird wie in Beispiel 2.2.a beschrieben umgesetzt. Man erhält gelbe Kristalle (2,6 g, 79 %); DC [Ethylacetat]: $R_f$ = 0,59; Schmp. = 212°C.

**2.12.d) N-[Lysyl-O-(tert-butyl)-D-threonyl]-batracylin, Di-Trifluoracetat:**

Die obige Verbindung (2,5 g, 3,4 mmol) wird wie in Beispiel 2.2 beschrieben umgesetzt und gereinigt. Man erhält gelbe Kristalle (2,5 g, 96 %); DC [Methanol/Essigsäure 10:1]: $R_f$ = 0,30; Schmp. = 142°C (Zers.).

**2.12) N-[Lysyl-D-threonyl]-batracylin, Di-Hydrobromid:**

Verbindung 2.12.d (2,29 g, 3 mmol) wird wie in Beispiel 2.8 beschrieben umgesetzt und gereinigt. Man erhält gelb-rote Kristalle (1,86 g, 97 %); Schmp. = 232°C (Zers.).

Beispiel 2.13

**N-[Lysyl-D-alanyl]-batracylin, Di-Trifluoracetat**

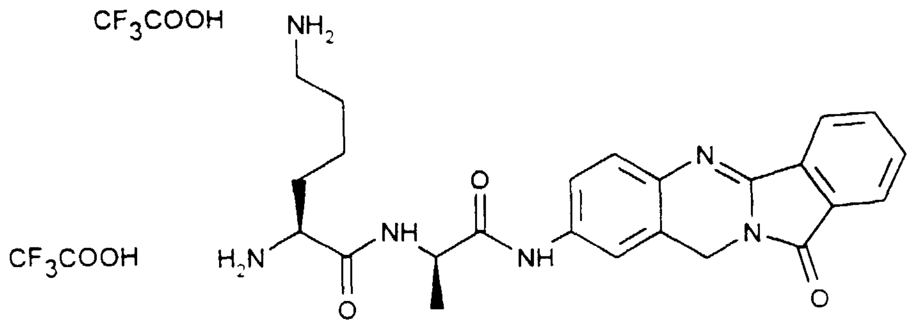

**2.13.a) N-[N$^{\alpha}$,N$^{\varepsilon}$-bis-(tert-Butoxycarbonyl)-lysyl-D-alanyl]-batracylin:**

6 g (17,3 mmol) N$^{\alpha}$,N$^{\varepsilon}$-bis-(tert-Butoxycarbonyl)-lysin werden in 75 ml DMF gelöst und bei 0°C mit 3 g (26 mmol) N-Hydroxysuccinimid und 4,29 g (20,8 mmol) N,N'-Dicyclohexylcarbodiimid versetzt. Nach 3 h filtriert man den entstandenen Harnstoff ab, gibt zum Filtrat 5 g (15,6 mmol) N-[D-Alanyl]-batracylin (Beispiel 2.3) und rührt 16 h bei 20°C. Restlicher Harnstoff wird abfiltriert und verworfen. Das Filtrat wird eingeengt und der Rückstand mit Methanol verrührt und filtriert. Das Filtrat wird erneut eingeengt und nochmals mit Methanol behandelt. Man filtriert wiederum und vereinigt die Filterrückstände. Sie werden in Dichlormethan/Methanol 10:1 gelöst und mit Ether gefällt. Man erhält 8,22 g (81 %) des kristallinen Zielproduktes.

**2.13) N-[Lysyl-D-alanyll-batracylin, Di-Trifluoracetat:**

Darstellung aus 8,2 g der Verbindung 2.13.a in Analogie zu Beispiel 2.2. Ausb.: 7,58g (89%)

Beispiel 2.14

**N-[N$^{\varepsilon}$-(Fluorenyl-9-methoxycarbonyl)-lysyl]-batracylin, Trifluoracetat**

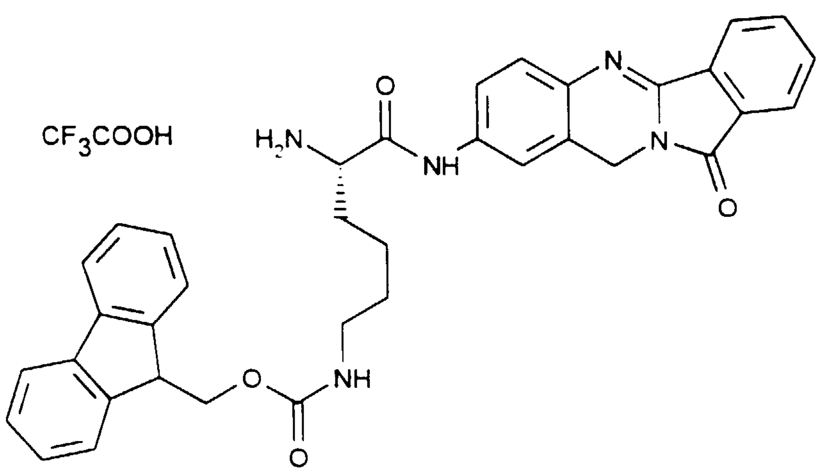

**2.14.a) N-[N$^{\alpha}$-(tert-Butoxycarbonyl)-N$^{\varepsilon}$-(fluorenyl-9-methoxycarbonyl)-lysyl]-batracylin:**

Darstellung in Analogie zu Beispiel 2.4.a aus N$^{\alpha}$-(tert-Butoxycarbonyl)-N$^{\varepsilon}$-(fluorenyl-9-methoxycarbonyl)-lysin und Batracylin. Ausb.: 78 %

**2.14) N-[N$^{\varepsilon}$-(Fluorenyl-9-methoxycarbonyl)-lysyl]-batracylin, Trifluoracetat:**

Darstellung in Analogie zu Beispiel 2.5 aus Verbindung 2.14.a.
Ausb.: 90%

**Beispiel 2.15**

**N-[Lysyl-N$^{\varepsilon}$-(fluorenyl-9-methoxycarbonyl)-lysyl]-batracylin, Di-Trifluoracetat**

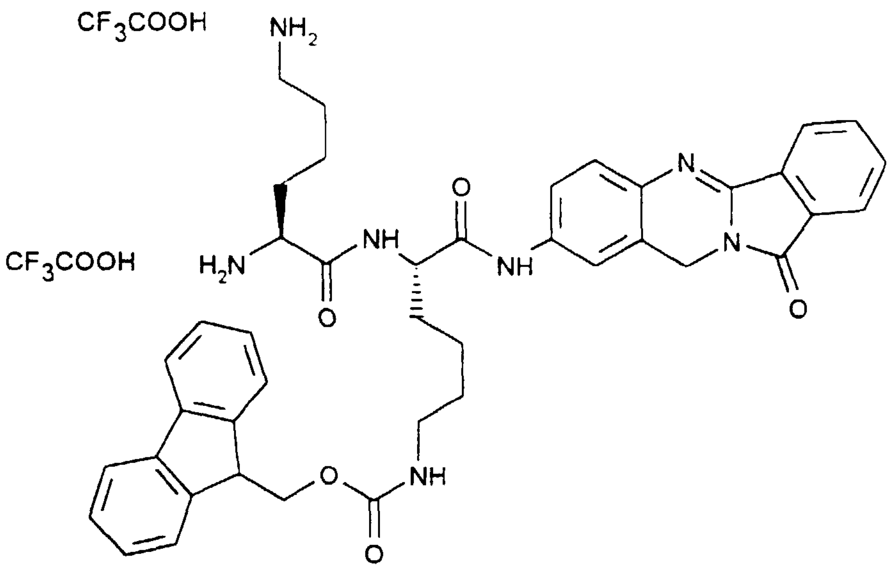

**2.15.a) N-[N$^{\alpha}$,N$^{\varepsilon}$-di-(tert-Butoxycarbonyl)-lysyl-N$^{\varepsilon}$-(fluorenyl-9-methoxy-carbonyl)-lysyl]-batracylin:**

3240 mg (4,54 mmol) der Verbindung 2.14 werden in 50 ml Dimethylformamid gelöst und mit 2550 mg (5,45 mmol) N$^{\alpha}$,N$^{\varepsilon}$-di-(tert-Butoxycarbonyl)-lysin-p-nitrophenylester sowie mit 938 µl Ethyldiisopropylamin versetzt. Man rührt 16 h bei 20°C, engt ein und verrührt den Rückstand zunächst mit Ether. Man filtriert und verrührt den Filterrückstand nochmals mit Methanol/Ether 1:1. So erhält man nach Absaugen und Trocknen 3881 mg (92 %) des Zielproduktes.

**2.15) N-[Lysyl-N$^{\varepsilon}$-(fluorenyl-9-methoxycarbonyl)-lysyl]-batracylin, Di-Trifluor-acetat:**

Deblockierung der Verbindung 2.15.a mit wasserfreier Trifluoressigsäure/Di-chlormethan 1:1 in Analogie zu Beispiel 2.2. Ausb.: 95 % [DC: Dichlormethan/Methanol/Ammoniak (17 %ig) 15:6:0,6 $R_f$ = 0,08]

**Beispiel 2.16**

**N-[Lysyl-N$^{\beta}$-(fluorenyl-9-methoxycarbonyl)-$\alpha$,$\beta$-diaminopropionyl]-batracylin, Di-Trifluoracetat**

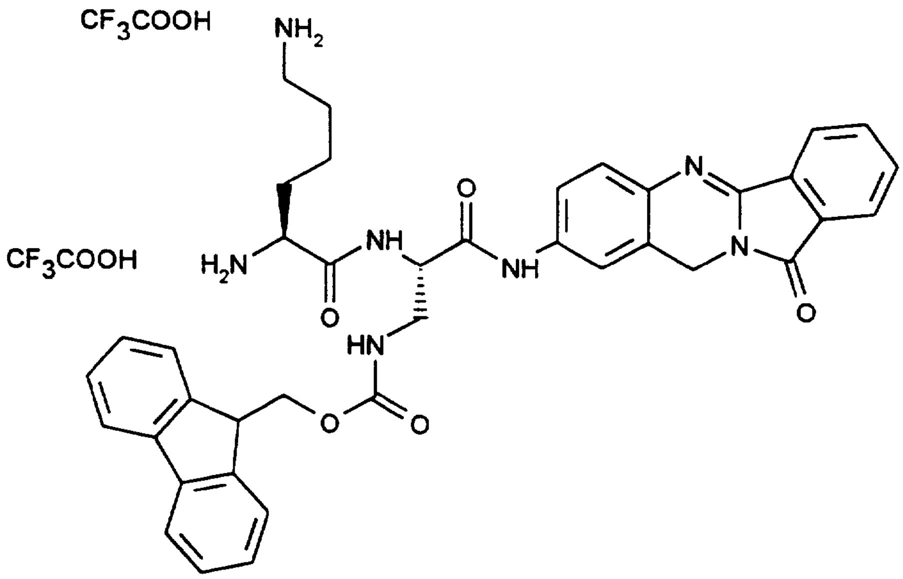

Dieses Peptidkonjugat wurde in Analogie zu den Beispielen 2.14 und 2.15 über 4 Stufen aus Batracylin und N$^{\alpha}$-(tert-Butoxycarbonyl)-N$^{\beta}$-(fluorenyl-9-methoxy-carbonyl)-diaminopropionsäure hergestellt.
[DC: Dichlormethan/Methanol/Eisessig 5:1:0,2 $R_f$ = 0,15]

**Beispiel 2.17**

**N-[Lysyl]-batracylin**

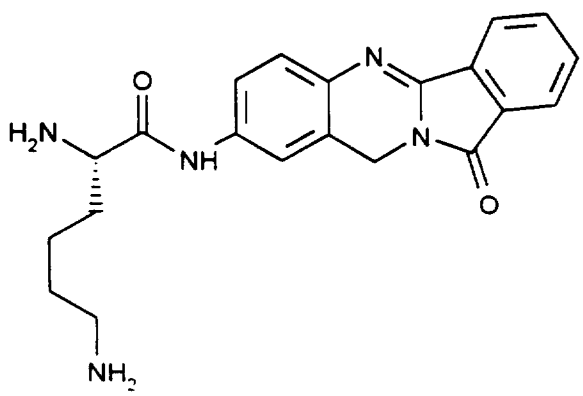

Fmoc-Abspaltung in Analogie zu Beispiel 2.4 aus N-[N$^{\varepsilon}$-(Fluorenyl-9-methoxy-carbonyl)-lysyl]-batracylin Trifluoracetat (Beispiel 2.14). Ausb.: 65 %

**Beispiel 2.18**

**N-[Seryl-D-alanyl]-batracylin, Trifluoracetat**

$CF_3COOH$

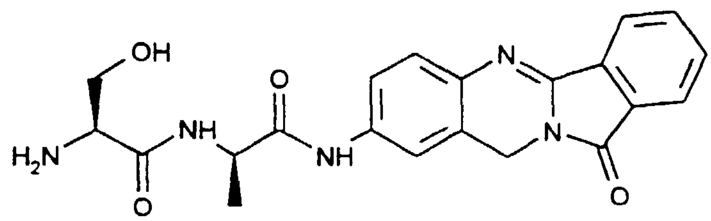

**2.18.a) N-[N-(tert-Butoxycarbonyl)-seryl-D-alanyl]-batracylin:**

Darstellung in Analogie zu Beispiel 2.13.a aus N-(tert-Butoxycarbonyl)-serin und N-[D-Alanyl]-batracylin (Beispiel 2.3). Ausb.: 77 %

**2.18) N-[Seryl-D-alanyl]-batracylin, Trifluoracetat:**

Darstellung in Analogie zu Beispiel 2.2 aus Verbindung 2.18.a. Ausb.: 98 %

**Beispiel 2.19**

**N-[D-Alanyl-D-alanyl]-batracylin, Trifluoracetat**

$CF_3COOH$

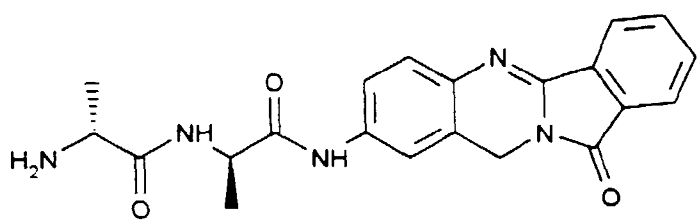

Darstellung über 2 Stufen in Analogie zu Beispiel 2.18.

**Beispiel 2.20**

**N-[Glutamyl-D-alanyl]-batracylin**

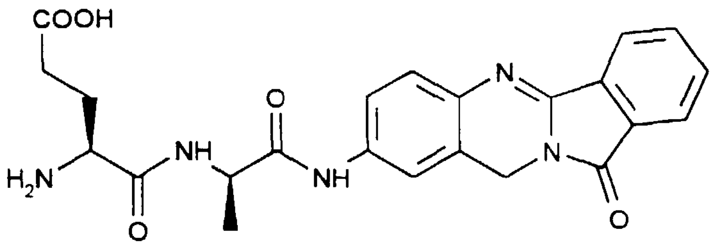

Darstellung über 2 Stufen in Analogie zu Beispielen 2.18 ausgehend von N-tert-Butoxycarbonyl-glutamyl-δ-tert-butylester und N-[D-Alanyl]-batracylin (Beispiel 2.3). Nach der Boc-Abspaltung wird eingeengt, in Wasser aufgenommen, mit 0,1NNatronlauge auf pH 7 gebracht und das Betain abgesaugt.

**Beispiele 3.1 - 3.34**

**Allgemeine Formel**

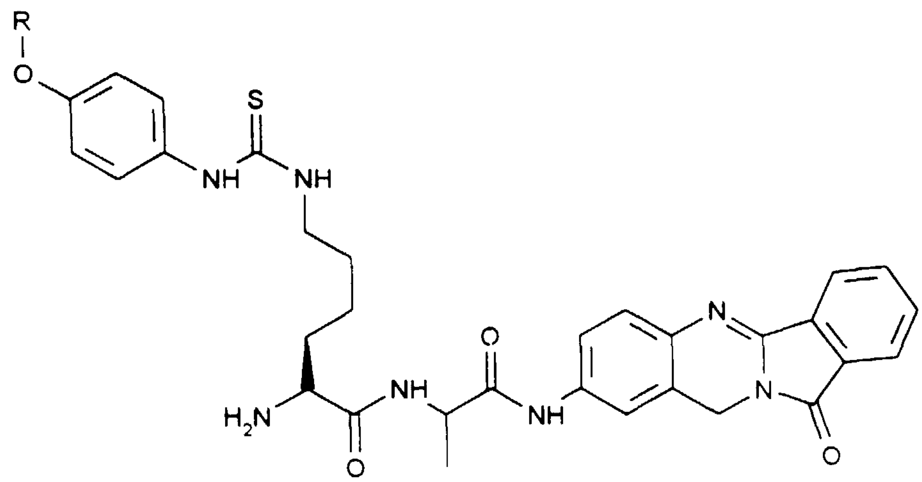

**Beispiel 3.1**

**N-{N$^{\varepsilon}$-[O-(β-L-Fucosyl)-4-hydroxy-phenylaminothio-carbonyl]-lysyl-D-alanyl}-batracylin**

**3.1.a) N-{N$^{\alpha}$-(tert-Butoxycarbonyl)-N$^{\varepsilon}$-[O-(β-L-fucosyl)-4-hydroxy-phenyl- amino-thiocarbonyl]-lysyl-D-alanyl}-batracylin:**

55 mg (0,22 mmol) p-Aminophenyl-β-L-fucosid werden in 10 ml Dioxan/Wasser 1:1 unter Rühren mit Thiophosgen (34 µl, 0,44 mmol) versetzt. Nach 10 min engt man im Vakuum ein und trocknet den Rückstand für 1 h im Hochvakuum. Das erhaltene Senföl wird anschließend in absolutem Dimethylformamid mit 109 mg (0,21 mmol) N-[N$^{\alpha}$-(tert-Butoxycarbonyl)-lysyl-D-alanyl]-batracylin (Beispiel 2.4) in Gegenwart von 115 µl Ethyldiisopropylamin gekuppelt. Nach zweimaliger Fällung des Rohproduktes aus Methanol/Isopropanol erhält man 132 mg (75 %) des Zielproduktes. [DC: Dichlormethan/Methanol 9:1 $R_f$ = 0,15].

**3.1) N-{$N^{\varepsilon}$-[O-(β-L-Fucosyl)-4-hydroxy-phenylamino-thiocarbonyl]-lysyl-D-alanyl}-batracylin:**

127 mg (0,15 mmol) der Verbindung aus Beispiel 3.1.a werden in 10 ml Dichlormethan mit 6 ml wasserfreier Trifluoressigsäure 2 h bei 0°C gerührt. Man engt ein, destilliert dreimal mit 5 ml Dichlormethan nach und chromatographiert mit Dichlormethan/Methanol/Ammoniak (17 %ig) 15:2:0,2. Nach anschließender Gefriertrocknung erhält man 80 mg (71 %) des Zielproduktes. [DC: Dichlormethan/Methanol/Ammoniak (17 %ig) 15:4:0,4 $R_f$ = 0,3].

**In Analogie zu Beispiel 3.1 werden aus dem teilgeschützten Peptidkonjugat in Beispiel 2.4 bzw. aus dem analog herzustellenden isomeren N-[$N^{\alpha}$-(tert-Butoxycarbonyl)-lysyl-alanyl]-batracylin folgende Glycokonjugate hergestellt:**

**Beispiel 3.2**

**N-{$N^{\varepsilon}$-[O-(2-O-Methyl-β-L-fucosyl)-4-hydroxy-phenylamino-thiocarbonyl]-lysyl-D-alanyl}-batracylin:**

Edukt: Kohlenhydrat aus Beispiel 1.1
Flashchromatographische Reinigung der Zwischenstufe mit Dichlormethan/Methanol 95:5 und der Endstufe mit Dichlormethan/Methanol/Ammoniak (17 %ig) 15.2:0,2. Ausb.: 55 % [DC: Dichlormethan/Methanol/Eisessig 5:1:0.2 $R_f$ = 0,4].

**Beispiel 3.3**

**N-{$N^{\varepsilon}$-[O-(2-O-Methyl-β-L-fucosyl)-4-hydroxy-phenylamino-thiocarbonyl]-lysyl-alanyl}-batracylin:**

Edukt: Kohlenhydrat aus Beispiel 1.1
Reinigung der Zwischenstufe durch Fällung aus Dichlormethan/Methanol 1:1 mit Ether und flashchromatographische Reinigung der Endstufe mit Dichlormethan/Methanol/Ammoniak (17 %ig) 15:2:0,2. Ausb.: 65 % [DC: Dichlormethan/Methanol/Ammoniak (17 %ig) 15:2:0,2 $R^f$ 0,21].

**Beispiel 3.4**

**N-{$N^{\varepsilon}$-[O-(3-O-Methyl-β-L-fucosyl)-4-hydroxy-phenylamino-thiocarbonyl]-lysyl-D-alanyl}-batracylin, Trifluoracetat:**

Edukt: Kohlenhydrat aus Beispiel 1.2
Flashchromatographische Reinigung der Zwischenstufe mit Dichlormethan/Methanol 97,5:2,5 und Fällung der Endstufe aus Methanol mit Ether; Ausb.: 59 % [DC : Dichlormethan/Methanol/Ammoniak (17 %ig) 15:2:0,2 $R_f$ = 0,19].

**Beispiel 3.5**

**N-{$N^{\varepsilon}$-[O-(3-O-Methyl-β-L-fucosyl)-4-hydroxy-phenylamino-thiocarbonyl]-lysyl-alanyl}-batracylin:**

Edukt: Kohlenhydrat aus Beispiel 1.2
Reinigung der Zwischenstufe durch Fällung aus Dichlormethan/Methanol 1:1 mit Ether und flashchromatographische Reinigung der Endstufe mit Dichlormethan/Methanol/Ammoniak (17 %ig) 15:2:0,2. Ausb.: 36% [DC Dichlormethan/Methanol/Ammoniak (17%ig) 15:4:0,5 $R_f$ = 0,57].

**Beispiel 3.6**

**N-{$N^{\varepsilon}$-[O-(3-O-Methyl-α-L-fucosyl)-4-hydroxy-phenylamino-thiocarbonyl]-lysyl-D-alanyl}-batracylin:**

Edukt: Kohlenhydrat aus Beispiel 1.3
Reinigung der Zwischenstufe durch Fällung aus Methanol mit Ether und flashchromatographische Reinigung der Endstufe mit Dichlormethan/Methanol/Ammoniak (17%ig) 15:2:0,2. Ausb.: 44% [DC: Dichlormethan/Methanol/Ammoniak (17 %ig) 15:2:0,2 $R_f$ = 0,15].

**Beispiel 3.7**

**N-{N$^{\varepsilon}$-[O-(3-Desoxy-β-L-fucosyl)-4-hydroxy-phenylamino-thiocarbonyl]-lysyl-D-alanyl}-batracylin, Trifluoracetat:**

Edukt: Kohlenhydrat aus Beispiel 1.6
Flashchromatographische Reinigung der Zwischenstufe mit Dichlormethan/Methanol 95:5 und Fällung der Endstufe aus Methanol mit Ether. Ausb.: 35 % [DC: Acetonitril/Wasser/Eisessig 5:1:0,2 $R_f$ = 0,42].

**Beispiel 3.8**

**N-{N$^{\varepsilon}$-[O-(3,4-Epoxy-β-L-fucosyl)-4-hydroxy-phenylamino-thiocarbonyl]-lysyl-D-alanyl}-batracylin, Trifluoracetat:**

Edukt: Kohlenhydrat aus Beispiel 1.8
Flashchromatographische Reinigung der Zwischenstufe mit Dichlormethan/Methanol 95:5. Mehrmalige Fällung der Endstufe aus Methanol mit Ether und anschließendes Verrühren mit Essigester. [DC: Acetonitril/Wasser/Eisessig 5:1:0,2 $R_f$ = 0,49].

**Beispiel 3.9**

**N-{N$^{\varepsilon}$-[O-(3-O-Carboxymethyl-β-L-fucosyl)-4-hydroxy-phenylamino-thiocarbonyl]-lysyl-D-alanyl}-batracylin, Natriumsalz:**

Edukt: Kohlenhydrat aus Beispiel 1.10
Reinigung der Zwischenstufe durch Verrühren mit Methanol und Vervollständigung der Fällung mit Ether. Flashchromatographische Reinigung der Endstufe mit Dichlormethan/Methanol/Ammoniak (17 %ig) 15:3:0,3; später im gleichen System mit 15:6:0,6. Die entsprechenden Fraktionen werden eingeengt, in Wasser aufgenommen und mit 0,1N Natronlauge auf pH 7 gebracht. Man saugt ab, nimmt den Filterrückstand in Dimethylformamid/Wasser 1:3 auf und setzt ein Äquivalent einer 0,1N Natronlauge zu. Man engt ein, nimmt das Natriumsalz in Wasser auf und lyophilisiert. Ausb.: 43%. [DC: Dichlormethan/Methanol/Ammoniak (17%ig) 15:4:0,5 $R_f$ = 0,15].

**Beispiel 3.10**

**N-{N$^{\varepsilon}$-[O-(3-O-Carbamoylmethyl-β-L-fucosyl)-4-hydroxy-phenylaminothiocarbonyl]-lysyl-D-alanyl}-batracylin, Natriumsalz:**

Edukt: Kohlenhydrat aus Beispiel 1.18
Reinigung der Zwischenstufe durch Verrühren mit Methanol und Vervollständigung der Fällung mit Ether. Flashchromatographische Reinigung der Endstufe mit Dichlormethan/Methanol/Ammoniak (17%ig) 15:2:0,2. [DC: Dichlormethan/Methanol/Ammoniak (17 %ig) 15:3:0,3 $R_f$ = 0,38]; Schmp.: 190° (Zers.)

**Beispiel 3.11**

**N-{N$^{\varepsilon}$-[O-(4-O-Methyl-β-L-fucosyl)-4-hydroxy-phenylamino-thiocarbonyl]-lysyl-D-alanyl}-batracylin, Acetat:**

Edukt: Kohlenhydrat aus Beispiel 1.4
Flashchromatographische Reinigung der Zwischenstufe mit Dichlormethan/Methanol 95:5 und der Endstufe mit Dichlormethan/Methanol/Ammoniak (17 %ig) 15:2:0,2. Nach dem Einengen wird der Ruckstand mit einem Äquivalent Eisessig und 10 ml Wasser versetzt und lyophilisiert. Ausb.: 52 % [DC: Acetonitril/Wasser/Eisessig 5:1:0,2 $R^f$ 0,43].
FAB-MS: m/z = 760 = M+1.

**Beispiel 3.12**

**N-{N$^{\varepsilon}$-[O-(α-D-Glucosyl)-4-hydroxy-phenylamino-thiocarbonyl]-lysyl-D-alanyl}-batracylin, Trifluoracetat:**

Edukt: p-Aminophenyl-α-D-glucosid
Reinigung der Zwischenstufe und der Endstufe durch Verrühren mit Methanol und Vervollständigung der Fällung mit

Ether. Ausb.: 83%. [DC: Dichlormethan/Methanol/Ammoniak (17% ig) 15:8:0,8 $R_f$ = 0,48].

**Beispiel 3.13**

**N-{$N^{\varepsilon}$-[O-(α-D-Glucosyl)-4-hydroxy-phenylamino-thiocarbonyl]-lysyl-alanyl}-batracylin, Trifluoracetat:**

Edukt: p-Aminophenyl-α-D-glucosid
Analoge Darstellung wie das Isomer in Beispiel 3.12

**Beispiel 3.14**

**N-{$N^{\varepsilon}$-[O-(3-O-Methyl-β-D-galactopyranosyl)-4-hydroxy-phenylamino-thiocarbonyl]-lysyl-D-alanyl}-batracylin, Trifluoracetat**

**3.14.a) N-{$N^{\alpha}$-(tert-Butoxycarbonyl)-$N^{\varepsilon}$-[O-(3-O-methyl-β-D-galactopyranosyl)-4-hydroxy-phenylamino-thiocarbonyl]-lysyl-D-alanyl}-batracylin:**

Eine Lösung von Verbindung 1.25 (62,8 mg, 0,22 mmol) in Dioxan/Wasser 1:1 (10 ml) wird unter Rühren mit Thiophosgen (33,5 ml, 0.44 mmol) versetzt. Nach 10 min. engt man im Vakuum ein und trocknet den Rückstand für 1 h im Ölpumpenvakuum. Das erhaltene Isothiocyanat wird in absolutem Dimethylformamid (10 ml) gelöst und mit Verbindung 2 4 (109.7 mg, 0,2 mmol) und mit Ethyldiisopropylamin (0,5 ml) versetzt. Man rührt für 16 h bei Raumtemperatur, engt dann im Vakuum ein und reinigt den Rückstand durch Flashchromatographie [Dichlormethan/Methanol 20:1]. Man erhält gelbe Kristalle (108,3 mg, 62 %); DC [Dichlormethan/Methanol 5:1]: $R_f$ = 0,42; Schmp = 194-195°C (Zers.).

**3.14) N-{$N^{\varepsilon}$-[O-(3-O-Methyl-β-D-galactopyranosyl)-4-hydroxy-phenylaminothiocarbonyl]-lysyl-D-alanyl}-batracylin, Trifluoracetat:**

Aus Verbindung 3.14.a (105,1 mg, 0,12 mmol) wird wie in Beispiel 2.2 beschrieben die tert-Butoxycarbonyl-Gruppe abgespalten. Nach Einengen im Vakuum und Umfällen aus Methanol/Diethylether erhält man gelbe Kristalle (57,4 mg, 54 %); DC [Dichlormethan/ Methanol 5:1]: $R_f$ = 0,16; Schmp. = 188-189°C (Zers.).

**In Analogie zu den Beispiel 3.14.a und 3.14 werden aus dem Peptidkonjugat 2.4 (je 109,7 mg, 0,2 mmol) die folgenden Glycokonjugate hergestellt:**

**Beispiel 3.15**

**N-{$N^{\varepsilon}$-[O-(β-D-Galactopyranosyl)-4-hydroxy-phenylamino-thiocarbonyl]-lysyl-D-alanyl}-batracylin, Trifluoracetat:**

Edukt: Kohlenhydrat 1.23 (59,7 mg, 0,22 mmol)
Reinigung der Zwischenstufe durch Flashchromatographie [Dichlormethan/Methanol 10:1] ergibt gelbe Kristalle (79,5 mg, 46 %); DC [Methanol]: $R_f$ = 0,74; Schmp. = 182°C.
Reinigung des Endprodukts wie in Beispiel 3.14 beschrieben ergibt gelbe Kristalle (77,1 mg, 44 %); DC [Methanol]: $R_f$ = 0,27; Schmp. = 191-192°C (Zers.).

**Beispiel 3.16**

**N-{$N^{\varepsilon}$-[O-(3,4-di-O-Methyl-β-D-galactopyranosyl)-4-hydroxy-phenylaminothiocarbonyl]-lysyl-D-alanyl}-batracylin, Trifluoracetat:**

Edukt: Kohlenhydat 1.31 (79 mg, 0,22 mmol)
Reinigung der Zwischenstufe durch Flashchromatographie [Dichlormethan/Methanol 30:1 → 20:1] ergibt gelbe Kristalle (150,7 mg, 85 %); DC [Dichlormethan/-Methanol 10:1]: $R_f$ = 0,35; Schmp. = 197-199°C (Zers.)
Reinigung des Endprodukts wie in Beispiel 3.14 beschrieben ergibt gelbe Kristalle (137 mg, 76 %); DC [Dichlormethan/ Methanol 10:1]: $R_f$ = 0,13; Schmp. = 184 - 186°C (Zers.).

**Beispiel 3.17**

**N-{N$^{\varepsilon}$-[O-(3-O-Methoxycarbonylmethyl-β-D-galactopyranosyl)-4-hydroxy-phenylamino-thiocarbonyl]-lysyl-D-alanyl}-batracylin, Trifluoracetat:**

Edukt: Kohlenhydrat 1.42 (75,5 mg, 0,22 mmol)
Reinigung der Zwischenstufe durch Flashchromatographie [Dichlormethan/Methanol 30:1 → 25:1] ergibt gelbe Kristalle (124,1 mg, 66 %); DC [Dichlormethan/-Methanol/Ammoniak (25 %) 15:3:0,2]: $R_f$ = 0,50; Schmp. = 165°C.
Reinigung des Endprodukts wie in Beispiel 3.14 beschrieben ergibt gelbe Kristalle (107,8 mg, 57 %); DC [Dichlormethan/Methanol 4:1]: $R_f$ = 0,53; Schmp. = 183°C (Zers.).

**Beispiel 3.18**

**N-{N$^{\varepsilon}$-[O-(3-O-Carboxymethyl-β-D-galactopyranosyl)-4-hydroxy-phenyl-amino-thiocarbonyl]-lysyl-D-alanyl}-batracylin, Trifluoracetat:**

Edukt: Verbindung 1.43 (77,3 mg, 0,22 mmol)
Reinigung der Zwischenstufe durch Umfällen aus Ethanol/Diethylether ergibt das Natriumsalz als gelbe Kristalle (172 mg, 91 %); DC [Methanol]: $R_f$ = 0,71; Schmp. = 225-228°C.
Reinigung des Endprodukts wie in Beispiel 3.14 beschrieben ergibt gelbe Kristalle (136,5 mg, 73 %); DC [Methanol]: $R_f$ = 0,12; Schmp. = 217-220 °C (Zers.).

**Beispiel 3.19**

**N-{N$^{\varepsilon}$-[O-(3-O-Carbamoylmethyl-β-D-galactopyranosyl)-4-hydroxy-phenyl-amino-thiocarbonyl]-lysyl-D-alanyl}-batracylin, Trifluoracetat:**

Edukt: Kohlenhydrat 1.44 (72,2 mg. 0,22 mmol)
Reinigung der Zwischenstufe durch Flashchromatographie [Dichlormethan/Methanol 10:1] ergibt gelbe Kristalle (137,7 mg, 75 %); DC [Dichlormethan/Methanol 4:1]: $R_f$ = 0,41; Schmp. = 198-201°C (Zers.).
Reinigung des Endprodukts wie in Beispiel 3.14 ergibt gelbe Kristalle (140,2 mg, 75 %); DC [Dichlormethan/Methanol 4:1]: $R_f$ = 0,16; Schmp. = 188-190°C (Zers.).

**Beispiel 3.20**

**N-{N$^{\varepsilon}$-[O-(3-O-(N-Methyl-carbamoylmethyl)-β-D-galactopyranosyl)-4-hydroxy-phenylamino-thiocarbonyl]-lysyl-D-alanyl}-batracylin, Trifluoracetat:**

Edukt: Kohlenhydrat 1.45 (75,3 mg, 0,22 mmol)
Reinigung der Zwischenstufe durch Flashchromatographie [Dichlormethan/Methanol/Ammoniak (25 %) 7:1:0,1] ergibt gelbe Kristalle (158,4 mg, 85 %); DC [Dichlormethan/Methanol/Ammoniak (25 %) 15:3:0,2]: $R_f$ = 0,25; Schmp. = 161 - 163°C (Zers.).
Reinigung des Endprodukts wie in Beispiel 3.14 beschrieben ergibt gelbe Kristalle (132,5 mg, 70 %); DC [Dichlormethan/Methanol/Ammoniak (25 %) 15:3:0,2]: $R_f$ = 0,10; Schmp. = 191-193 °C (Zers.).

**Beispiel 3.21**

**N-{N$^{\varepsilon}$-[O-(3-O-(N-Propyl-carbamoylmethyl)-β-D-galactopyranosyl)-4-hydroxy-phenylamino-thiocarbonyl]-lysyl-D-alanyl}-batracylin, Trifluoracetat:**

Edukt: Kohlenhydrat 1.46 (81,5 mg, 0,22 mmol)
Reinigung der Zwischenstufe durch Flashchromatographie [Dichlormethan/Methanol/Ammoniak (25 %) 8:1:0,1] ergibt gelbe Kristalle (153,1 mg, 80 %); DC [Dichlormethan/Methanol/Ammoniak (25 %) 15:3:0,2]: $R_f$ = 0,33; Schmp. = 187°C (Zers.).
Reinigung des Endprodukts wie in Beispiel 3.14 beschrieben ergibt gelbe Kristalle (154,9 mg, 79 %); DC [Dichlormethan/Methanol/Ammoniak (25 %) 15:3:0,2]: $R_f$ = 0,21; Schmp. = 179°C.

**Beispiel 3.22**

**N-{N$^{\varepsilon}$-[O-(3-O-(N-Butyl-carbamoylmethyl)-β-D-galactopyranosyl)-4-hydroxy-phenylamino-thiocarbonyl]-lysyl-D-alanyl}-batracylin, Trifluoracetat:**

Edukt: Kohlenhydrat 1.47 (84,6 mg, 0,22 mmol)
Reinigung der Zwischenstufe durch Flashchromatographie [Dichlormethan/Methanol 12:1] ergibt gelbe Kristalle (132,7 mg, 68 %); DC [Dichlormethan/Methanol/Ammoniak (25 %) 15:3:0,2]: $R_f$ = 0,54; Schmp. = 180-182°C.
Reinigung des Endprodukts wie in Beispiel 3.14 beschrieben ergibt gelbe Kristalle (115,2 mg, 58 %); DC [Dichlormethan/Methanol/Ammoniak (25 %) 15:3:0,2]: $R_f$ = 0,30; Schmp. = 176 °C.

**Beispiel 3.23**

**N-{N$^{\varepsilon}$-[O-(3,4-Didesoxy-β-D-galactopyranosyl)-4-hydroxy-phenylamino-thiocarbonyl]-lysyl-D-alanyl}-batracylin, Trifluoracetat:**

Edukt: Kohlenhydrat 1.52 (52,6 mg, 0,22 mmol)
Reinigung der Zwischenstufe durch Flashchromatographie [Dichlormethan/Methanol 25:1] ergibt gelbe Kristalle (127,4 mg, 77 %); DC [Dichlormethan/Methanol/Ammoniak (25 %) 15:3:0,2]: $R_f$ = 0,60; Schmp. = 166-167°C.
Reinigung des Endprodukts wie in Beispiel 3.14 beschrieben ergibt gelbe Kristalle (103,1 mg, 61 %); DC [Dichlormethan/Methanol/Ammoniak (25 %) 15:3:0,2]: $R_f$ = 0,44; Schmp. = 173-175 °C (Zers.).

**Beispiel 3.24**

**N-{N$^{\varepsilon}$-[O-(6-O-Acetyl-β-D-galactopyranosyl)-4-hydroxy-phenylamino-thiocarbonyl]-lysyl-D-alanyl}-batracylin, Trifluoracetat:**

Edukt: Kohlenhydrat 1.53 (78,2 mg, 0,22 mmol)
Reinigung der Zwischenstufe durch Flashchromatographie [Dichlormethan/Methanol 25:1] ergibt gelbe Kristalle (88,3 mg. 49 %); DC [Dichlormethan/Methanol 4:1]: $R_f$ = 0,61; Schmp. = 196-199°C (Zers.).
Reinigung des Endprodukts wie in Beispiel 3.14 beschrieben ergibt gelbe Kristalle (89,6 mg, 49 %); DC [Dichlormethan/Methanol 4:1]: $R_f$ = 0,31; Schmp. = 186 °C (Zers.).

**Beispiel 3.25**

**N-{N$^{\varepsilon}$-[O-(α-D-Mannopyranosyl)-4-hydroxy-phenylamino-thiocarbonyl]-lysyl-D-alanyl}-batracylin, Trifluoracetat:**

Edukt: Kohlenhydrat 1.39 (59,7 mg, 0,22 mmol)
Reinigung der Zwischenstufe durch Flashchromatographie [Dichlormethan/Methanol 10:1] ergibt gelbe Kristalle (101,7 mg, 59 %); DC [Methanol]: $R_f$ = 0,79; Schmp. = 180°C (Zers.).
Reinigung des Endprodukts wie in Beispiel 3.14 beschrieben ergibt gelbe Kristalle (103,1 mg, 59 %); DC [Methanol]: $R_f$ = 0,34; Schmp. = 177-178°C (Zers.).

**Beispiel 3.26**

**N-{N$^{\varepsilon}$-[O-(3-O-Methyl-α-D-mannopyranosyl)-4-hydroxy-phenylamino-thiocarbonyl]-lysyl-D-alanyl}-batracylin, Trifluoracetat:**

Edukt: Kohlenhydrat 1.40 (62,8 mg, 0,22 mmol)
Reinigung der Zwischenstufe durch Flashchromatographie [Dichlormethan/Methanol 20:1] ergibt gelbe Kristalle (56,6 mg, 32 %); DC [Dichlormethan/Methanol 5:1]: $R_f$ = 0,38; Schmp. = 191-192°C (Zers.).
Reinigung des Endprodukts wie in Beispiel 3.14 beschrieben ergibt gelbe Kristalle (46,6 mg, 26 %); DC [Dichlormethan/Methanol 5:1]: $R_f$ = 0,13; Schmp. = 190 - 191°C (Zers.).

**Beispiel 3.27**

**N-{$N^{\varepsilon}$-[O-(2,3-di-O-Methyl-α-D-mannopyranosyl)-4-hydroxy-phenylaminothiocarbonyl]-lysyl-D-alanyl}-batracylin, Trifluoracetat:**

Edukt Kohlenhydrat 1.41 (66 mg, 0,22 mmol)
Reinigung der Zwischenstufe durch Flashchromatographie [Dichlormethan/Methanol 25:1] ergibt gelbe Kristalle (77,8 mg, 44 %); DC [Dichlormethan/Methanol 4:1]: $R_f$ = 0,65; Schmp. = 182-183°C (Zers.).
Reinigung des Endprodukts wie in Beispiel 3.14 beschrieben ergibt gelbe Kristalle (66,1 mg, 37 %); DC [Dichlormethan/Methanol 4:1]: $R_f$ = 0,40; Schmp. = 181°C.

**Beispiel 3.28**

**N-{$N^{\varepsilon}$-[O-(3-O-Methoxycarbonylmethyl-α-D-mannopyranosyl)-4-hydroxyphenylamino-thiocarbonyl]-lysyl-D-alanyl}-batracylin, Trifluoracetat:**

Edukt: Kohlenhydrat 1.48 (75,5 mg, 0,22 mmol)
Reinigung der Zwischenstufe durch Flashchromatographie [Dichlormethan/Methanol 18:1] ergibt gelbe Kristalle (62,1 mg, 33 %); DC [Dichlormethan/Methanol/Ammoniak (25 %) 15:3:0,2]: $R_f$ = 0,66; Schmp. = 165°C.
Reinigung des Endprodukts wie in Beispiel 3.14 beschrieben ergibt gelbe Kristalle (57,6 mg, 30 %); DC [Dichlormethan/Methanol/Ammoniak (25 %) 15:3:0,2]: $R_f$ = 0,43; Schmp. = 183-184°C.

**Beispiel 3.29**

**N-{$N^{\varepsilon}$- [O-(3-O-Carboxymethyl-α-D-mannopyranosyl)-4-hydroxy-phenylaminothiocarbonyl]-lysyl-D-alanyl}-batracylin, Trifluoracetat:**

Edukt: Kohlenhydrat 1.49 (77,3 mg, 0,22 mmol)
Reinigung der Zwischenstufe durch Umfällen aus Ethanol/Diethylether ergibt das Natriumsalz als gelbe Kristalle (173,4 mg, 92 %); DC [Methanol]: $R_f$ = 0,57; Schmp. = 201- 205°C (Zers.)
Reinigung des Endprodukts wie in Beispiel 3.14 beschrieben ergibt gelbe Kristalle (171,7 mg, 92 %); DC [Methanol]: $R_f$ = 0,29; Schmp = 196-198 °C (Zers.).

**Beispiel 3.30**

**N-{$N^{\varepsilon}$-[O-(3-O-Carbamoylmethyl-α-D-mannopyranosyl)-4-hydroxyphenylamino-thiocarbonyl]-lysyl-D-alanyl}-batracylin, Trifluoracetat:**

Edukt: Kohlenhydrat 1.50 (72,2 mg, 0,22 mmol)
Reinigung der Zwischenstufe durch Flashchromatographie [Dichlormethan/Methanol 10:1] ergibt gelbe Kristalle (106,6 mg, 58 %); DC [Dichlormethan/Methanol 4:1]: $R_f$ = 0,34; Schmp. = 192-194°C (Zers.).
Reinigung des Endprodukts wie in Beispiel 3.14 beschrieben ergibt gelbe Kristalle (107,7 mg, 58 %); DC [Dichlormethan/Methanol 4:1]: $R_f$ = 0,13; Schmp. = 186-187°C (Zers.).

**Beispiel 3.31**

**N-{$N^{\varepsilon}$-[O-(3,4-di-O-Methyl-β-D-galactopyranosyl)-4-hydroxy-phenylaminothiocarbonyl]-lysyl-D-alanyl}-batracylin**

**3.31.a) N-{$N^{\alpha}$-(Fluorenyl-9-methoxycarbonyl)-$N^{\varepsilon}$-[O-(3,4-di-O-methyl-β-D-galactopyranosyl)-4-hydroxy-phenylamino-thiocarbonyl]-lysyl-D-alanyl}-batracylin:**

Eine Lösung von Verbindung 1.31 (79 mg, 0,22 mmol) in Dioxan/Wasser 1:1 (10 ml) wird unter Rühren mit Thiophosgen (33,5 ml, 0,44 mmol) versetzt. Nach 10 min. engt man im Vakuum ein und trocknet den Rückstand für 1 h im Olpumpenvakuum. Das erhaltene Isothiocyanat wird in absolutem Dimethylformamid (10 ml) gelöst und mit Verbindung 2.5 (157 mg, 0,2 mmol) und mit Ethyldiisopropylamin (0,5 ml) versetzt. Man rührt für 16 h bei Raumtemperatur, engt dann im Vakuum ein und nimmt mit Dichlormethan/Methanol 1:1 auf Das Produkt wird durch Zugabe von Diethylether ausgefällt und mit wenig eiskaltem Methanol gewaschen. Man erhält gelbe Kristalle (191 mg, 94 %); DC [Dichlormethan/Methanol 10.1]: $R_f$ = 0,35; Schmp. = 203°C (Zers.).

**3.31) N-{N$^\varepsilon$-[O-(3,4-di-O-Methyl-β-D-galactopyranosyl)-4-hydroxy-phenylamino-thiocarbonyl]-lysyl-D-alanyl}-batracylin:**

Aus Verbindung 3.31.a (182,2 mg, 0,18 mmol) wird wie in Beispiel 24 beschrieben die Fluorenyl-9-methoxycarbonyl-Gruppe abgespalten. Nach Einengen im Vakuum und Lösen in Methanol/Dichlormethan 1:1 wird das Produkt durch Zugabe von Diethylether ausgefällt. Man erhält gelbe Kristalle (127,1 mg, 89 %); DC [Methanol]: $R_f$ = 0,46; Schmp. = 158°C.

**In Analogie zu den Beispiel 3.31.a und 3.31 werden aus dem Peptidkonjugat 2.5 (je 157 mg, 0,2 mmol) die folgenden Glycokonjugate hergestellt:**

**Beispiel 3.32**

**N-{N$^\varepsilon$-[O-(3-O-(Piperidyl-N)-carbonylmethyl-β-D-galactopyranosyl)-4-hydroxy-phenylamino-thiocarbonyl]-lysyl-D-alanyl}-batracylin:**

Edukt: Kohlenhydrat 1.42 (75,5 mg, 0,22 mmol)
Reinigung der Zwischenstufe wie in Beispiel 3.31.a beschrieben; man erhält gelbe Kristalle (191,2 mg, 91 %); DC [Dichlormethan/Methanol 10:1]: $R_f$ = 0,28; Schmp. = 208°C (Zers.).
Reinigung des Endprodukts wie in Beispiel 3.31 beschrieben ergibt gelbe Kristalle (155,8 mg, 88 %); DC [Methanol]: $R_f$ = 0,47; Schmp. = 120°C (Zers.).

**Beispiel 3.33**

**N-{N$^\varepsilon$-[O-(3-O-Carboxymethyl-β-D-galactopyranosyl)-4-hydroxy-phenyl-amino-thiocarbonyl]-lysyl-D-alanyl}-batracylin, Natriumsalz:**

Edukt: Kohlenhydrat 1.43 (77,3 mg, 0,22 mmol)
Reinigung der Zwischenstufe wie in Beispiel 3.31.a beschrieben; man erhält gelbe Kristalle (192 mg, 90 %); DC [Ethanol/Methanol 1:1]: $R_f$ = 0,05; Schmp. = 211-213°C (Zers.).
Reinigung des Endprodukts wie in Beispiel 3.31 beschrieben ergibt gelbe Kristalle (110,6 mg, 66 %); DC [Methanol]: $R_f$ = 0,33; Schmp. = 233-235°C (Zers.).

**Beispiel 3.34**

**N-{N$^\varepsilon$-[O-(3-O-Carbamoylmethyl-β-D-galactopyranusyl)-4-hydroxy-phenyl-amino-thiocarbonyl]-lysyl-D-alanyl}-batracylin:**

Edukt: Kohlenhydrat 1.44 (72,2 mg, 0,22 mmol)
Reinigung der Zwischenstufe wie in Beispiel 3.31.a beschrieben; man erhält gelbe Kristalle (159,2 mg, 76 %); DC [Dichlormethan/Methanol 10:1]: $R_f$ = 0,04; Schmp. = 177°C (Zers.).
Reinigung des Endprodukts wie in Beispiel 3.31 beschrieben ergibt gelbe Kristalle (125,1 mg, 76 %); DC [Methanol]: $R_f$ = 0,48, Schmp = 106°C (Zers.).

**Beispiele 4.1 - 4.12**

**Allgemeine Formel**

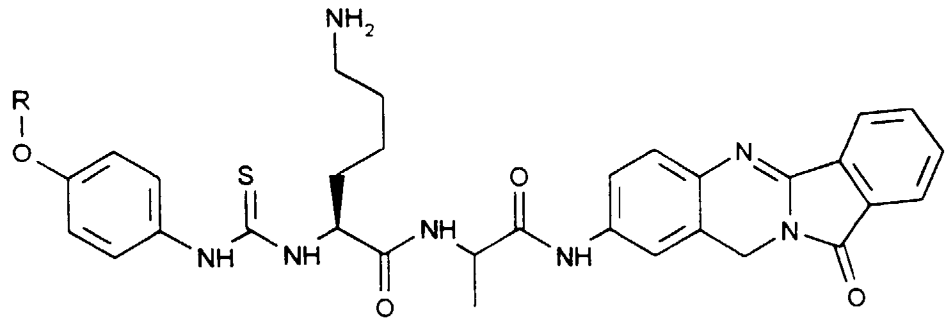

**Beispiel 4.1**

**4.1.a) N-{$N^{\alpha}$-[O-(β-L-Fucosyl)-4-hydroxy-phenylamino-thiocarbonyl]-$N^{\varepsilon}$- (fluorenyl-9-methoxycarbonyl)-lysyl-D-alanyl}-batracylin:**

140 mg (0,55 mmol) p-Aminophenyl-β-L-fucosid werden nach der Vorschrift in Beispiel 3.1.a zunächst ins Senföl überführt und anschließend mit 430 mg (0,55 mmol) N-[$N^{\varepsilon}$-(Fluorenyl-9-methoxycarbonyl)-lysyl-D-alanyl]-batracylin Trifluoracetat (Beispiel 2.5) in Gegenwart von 375 µl Ethyldiisopropylamin gekuppelt. Nach Fällung des Rohproduktes aus Methanol/Dichlormethan reinigt man durch Flash-Chromatographie (Acetonitril/Wasser 10:1). Nach Verrühren des Rückstandes mit Ether erhält man 358 mg (67 %) des Zielproduktes. [DC: Acetonitril/Wasser 10:1 $R_f$ = 0,48].

**4.1) N-{Nα-[O-(β-L-Fucosyl)-4-hydroxy-phenylamino-thiocarbonyl]-lysyl-D-alanyl}-batracylin:**

356 mg (0,37 mmol) der Verbindung aus Beispiel 4.1.a werden in 10 ml Dimethylformamid und 5 ml Piperidin gelöst und 1h bei 20°C gerührt. Man engt ein und chromatographiert den Rückstand mit Dichlormethan/Methanol/Ammoniak (17 %ig) 15:6:0,6. Das Zielprodukt wird in 46%iger Ausbeute erhalten [DC Dichlormethan/Methanol/Ammoniak (17%ig) 15:4:0,5 $R_f$ = 0,11]

**In Analogie zu den Beispielen 4.1 werden aus dem teilgeschützten Peptidkonjugat 2.5 folgende Glycokonjugate hergestellt:**

**Beispiel 4.2**

**N-{$N^{\alpha}$-[O-(3-O-Carboxymethyl-β-L-fucosyl)-4-hydroxy-phenylaminothiocarbonyl]-lysyl-D-alanyl}-batracylin, Natriumsalz:**

Edukt: Kohlenhydrat aus Beispiel 1.10
Chromatographische Reinigung der Zwischenstufe mit Dichlormethan/Methanol/Ammoniak (17%ig) 15:3:0,3; später im gleichen System 15:4:0,5. Reinigung des Endproduktes auf der Betain-Stufe durch Verrühren mit Wasser; anschließend Überführung ins Natriumsalz mit 0,1N Natronlauge und Gefriertrocknung aus Dioxan/Wasser. Ausb.: 65%; Schmp.: 220°C.

**Beispiel 4.3**

**N-{$N^{\alpha}$-[O-(3-O-Methyl-β-L-fucosyl)-4-hydroxy-phenylamino-thiocarbonyl]-lysyl-D-alanyl}-batracylin:**

Edukt: Kohlenhydrat aus Beispiel 1.2
Reinigung der Zwischenstufe durch Fällung aus Dichlormethan mit Ether; Reinigung des Endproduktes durch mehrmalige Fällung aus Dimethylformamid mit Ether. Ausb.: 88% [DC: Acetonitril/Wasser/Eisessig 5:1:0,2 $R_f$ = 0,28].

**Beispiel 4.4**

**N-{N$^{\alpha}$-[O-(4-O-Methyl-β-L-fucosyl)-4-hydroxy-phenylamino-thiocarbonyl]- lysyl-D-alanyl}-batracylin:**

Edukt. Kohlenhydrat aus Beispiel 1.4
Reinigung der Zwischenstufe durch mehrmalige Fällung aus Dichlormethan/Methanol 1:1 mit Ether; säulenchromatographische Reinigung des Endproduktes [Dichlormethan/Methanol/Ammoniak (17%ig) 15:8:0,8], Fällung aus Dichlormethan/Methanol 1:1 mit Ether. Ausb.: 74% [DC: Dichlormethan/Methanol/Ammoniak (17%ig) 10:10:1 $R_f$ = 0,19].

**Beispiel 4.5**

**N-{N$^{\alpha}$-[O-(3,4-di-O-Methyl-β-D-galactopyranosyl)-4-hydroxy-phenylaminothiocarbonyl]-lysyl-D-alanyl}-batracylin**

**4.5.a) N-{N$^{\alpha}$-[O-(3,4-di-O-Methyl-β-D-galactopyranosyl)-4-hydroxy-phenyl-amino-thiocarbonyl-N$^{\varepsilon}$-(fluorenyl-9-methoxycarbonyl)]-lysyl-D-alanyl}-batracylin:**

Eine Lösung von Verbindung 1.31 (79 mg, 0,22 mmol) in Dioxan/Wasser 1:1 (10 ml) wird unter Rühren mit Thiophosgen (33,5 ml, 0,44 mmol) versetzt. Nach 10 min. engt man im Vakuum ein und trocknet den Rückstand für 1 h im Ölpumpenvakuum. Das erhaltene Isothiocyanat wird in absolutem Dimethylformamid (10 ml) gelöst und mit Verbindung 2.6 (157 mg, 0,2 mmol) und mit Ethyldiisopropylamin (0,5 ml) versetzt. Man rührt für 16 h bei Raumtemperatur und engt dann im Vakuum ein. Durch mehrmaliges Umfällen des Rückstands aus Dichlormethan/Methanol 1:1 mittels Diethylether und abschließendes Waschen mit wenig eiskaltem Methanol erhält man gelbe Kristalle (198 mg, 98 %); DC [Dichlormethan/Methanol 10:1]: $R_f$ = 0,23; Schmp. = 175°C.

**4.5) N-{N$^{\alpha}$-[O-(3,4-di-O-Methyl-β-D-galactopyranosyl)-4-hydroxy-phenylamino-thiocarbonyl]-lysyl-D-alanyl}-batracylin;**

Aus Verbindung 4.5.a (172,1 mg, 0,17 mmol) wird wie in Beispiel 2.4 beschrieben die Fluorenyl-9-methoxycarbonyl-Gruppe abgespalten Nach Einengen im Vakuum und Lösen in Methanol/Dichlormethan 1:1 wird das Produkt durch Zugabe von Diethylether ausgefällt. Man erhält gelbe Kristalle (114,5 mg, 85 %); DC [Dichlormethan/Methanol 1:1]: $R_f$ = 0,16; Schmp. = 206°C (Zers.).

**In Analogie zu den Beispiel 4.5.a und 4.5 werden aus dem Peptidkonjugat 2.6 (je 157 mg, 0,2 mmol) die folgenden Glycokonjugate hergestellt:**

**Beispiel 4.6**

**N-{N$^{\alpha}$-[O-(β-D-Galactopyranosyl)-4-hydroxy-phenylamino-thiocarbonyl]-lysyl-D-alanyl}-batracylin:**

Edukt: Kohlenhydrat 1.23 (59,7 mg, 0,22 mmol)
Reinigung der Zwischenstufe wie in Beispiel 4.5.a beschrieben; man erhält gelbe Kristalle (185,8 mg, 94 %); DC [Dichlormethan/Methanol 10:1]: $R_f$ = 0,09; Schmp. = 182°C (Zers.).
Reinigung des Endprodukts wie in Beispiel 4.5 beschrieben ergibt gelbe Kristalle (134,3 mg, 88 %); DC [Dichlormethan/Methanol 1:1]: $R_f$ = 0,04; Schmp. = 221°C (Zers.).

**Beispiel 4.7**

**N-{N$^{\alpha}$-[O-(3-O-Methyl-β-D-galactopyranosyl)-4-hydroxy-phenylamino-thiocarbonyl]-lysyl-D-alanyl}-batracylin:**

Edukt: Kohlenhydrat 1.25 (62,8 mg, 0,22 mmol)
Reinigung der Zwischenstufe wie in Beispiel 4.5.a beschrieben; man erhält gelbe Kristalle (193,5 mg, 97 %): DC [Dichlormethan/Methanol 10:1]: $R_f$ = 0,27; Schmp. = 178°C (Zers.).
Reinigung des Endprodukts durch Flashchromatographie [Dichlormethan/Methanol 2:1 → 1:1] ergibt gelbe Kristalle (130,5 mg, 84 %); DC [Dichlormethan/Methanol 1:1]: $R_f$ = 0,09; Schmp. = 206°C (Zers.).

**Beispiel 4.8**

**N-{N$^{\alpha}$-[O-(3-O-Methoxycarbonylmethyl-β-D-galactopyranosyl)-4-hydroxy-phenylamino-thiocarbonyl]-lysyl-D-alanyl}-batracylin:**

Edukt: Kohlenhydrat 1.42 (75,5 mg, 0,22 mmol)
Reinigung der Zwischenstufe wie in Beispiel 4.5.a beschrieben, man erhält gelbe Kristalle (209,8 mg, 99 %); DC [Dichlormethan/Methanol 10:1]: $R_f$ = 0,32; Schmp. = 235°C (Zers.).
Reinigung des Endprodukts wie in Beispiel 4.5 beschrieben ergibt gelbe Kristalle (164,3 mg, 99 %); DC [Dichlormethan/Methanol 1:1]: $R_f$ = 0,05; Schmp. = 217°C (Zers.).

**Beispiel 4.9**

**N-{N$^{\alpha}$-[O-(3-O-Carboxymethyl-β-D-galactopyranosyl)-4-hydroxy-phenyl-amino-thiocarbonyl]-lysyl-D-alanyl}-batracylin, Natriumsalz:**

Edukt: Kohlenhydrat 1.43 (77,3 mg, 0,22 mmol)
Reinigung der Zwischenstufe wie in Beispiel 4.5.a beschrieben; man erhält gelbe Kristalle (210,3 mg, 99 %); DC [Dichlormethan/Methanol 10:1]: $R_f$ = 0,02; Schmp. = 185°C.
Nach Reinigung des Produkts wie in Beispiel 4.5 beschrieben wird der Rückstand in Wasser (10 ml) suspendiert und die Suspension unter Rühren tropfenweise mit 0,05N-Natronlauge versetzt, bis eine klare Lösung entsteht (pH < 10). Durch Lyophilisieren der filtrierten Lösung erhält man einen gelben amorphen Feststoff (150,8 mg, 90 %); DC [Dichlormethan/Methanol 1:1]: $R_f$ = 0,04.

**Beispiel 4.10**

**N-{N$^{\alpha}$-[O-(3-O-Carbamoylmethyl-β-D-galactopyranosyl)-4-hydroxy-phenylamino-thiocarbonyl]-lysyl-D-alanyl}-batracylin:**

Edukt: Kohlenhydrat 1.44 (72,2 mg, 0,22 mmol)
Reinigung der Zwischenstufe wie in Beispiel 4.5.a beschrieben, man erhält gelbe Kristalle (152,7 mg, 73 %); DC [Dichlormethan/Methanol 10:1]: $R_f$ = 0,11; Schmp. = 229°C (Zers.).
Nach Reinigung des Produkts wie in Beispiel 4.5 beschrieben wird der Rückstand in Wasser/Dioxan 1:1 (20 ml) suspendiert. Durch Lyophilisieren der filtrierten Lösung erhält man einen gelben amorphen Feststoff (98,4 mg, 61 %); DC [Di-chlormethan/Methanol 1:1]: $R_f$ = 0,10; $[\alpha]^{20}$ = +44,9° (c = 0,2 /$H_2O$).

**Beispiel 4.11**

**N-{N$^{\alpha}$[O-(α-D-Mannopyranosyl)-4-hydroxy-phenylamino-thiocarbonyl]-lysyl-D-alanyl}-batracylin:**

Edukt: Kohlenhydrat 1.39 (59,7 mg, 0,22 mmol)
Reinigung der Zwischenstufe wie in Beispiel 4.5.a beschrieben; man erhält gelbe Kristalle (179,6 mg, 91 %); DC [Dichlormethan/Methanol 10:1]: $R_f$ = 0,07; Schmp. = 176°C (Zers.).
Reinigung des Endprodukts wie in Beispiel 4.5 beschrieben ergibt gelbe Kristalle (137,5 mg, 90 %); DC [Dichlormethan/Methanol 1:1]: $R_f$ = 0,09; Schmp. = 213°C (Zers.).

**Beispiel 4.12**

**N-{N$^{\alpha}$-[O-(3-O-Methyl-α-D-mannopyranosyl)-4-hydroxy-phenylamino-thio-carbonyl]-lysyl-D-alanyl}-batracylin:**

Edukt: Kohlenhydrat 1.40 (62,8 mg, 0,22 mmol)
Reinigung der Zwischenstufe wie in Beispiel 4.5.a beschrieben; man erhält gelbe Kristalle (94,2 mg, 48 %); DC [Dichlormethan/Methanol 10:1]: $R_f$ = 0,13; Schmp. = 173°C (Zers.).
Reinigung des Endprodukts wie in Beispiel 4.5 beschrieben ergibt gelbe Kristalle (66,6 mg, 43 %); DC [Dichlormethan/Methanol 1:1]; $R_f$ = 0,07; Schmp = 215°C (Zers.).

**Beispiele 5.1 - 5.23**

Allgemeine Formel

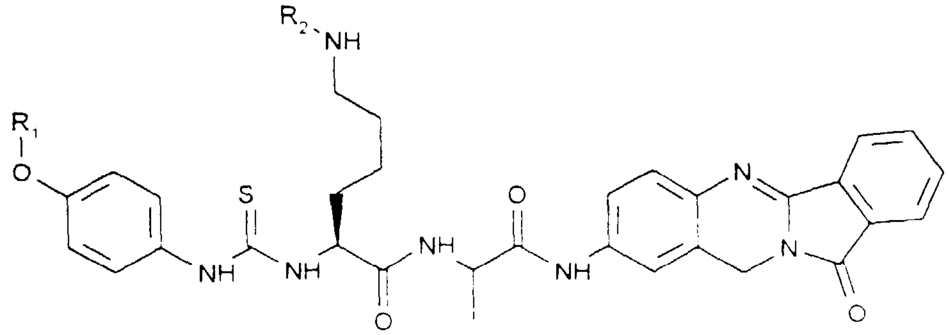

**Beispiel 5.1**

**N-{N$^{\alpha}$-[O-(3-O-Carboxymethyl-β-L-fucosyl)-4-hydroxyphenylaminothiocarbonyl]-N$^{\varepsilon}$-[O-(3-O-methyl-β-L-fucosyl)-4-hydroxyphenylaminothiocarbonyl]-lysyl-D-alanyl}-batracylin:**

50 mg (0,16 mmol) p-Aminophenyl-3-O-carboxymethyl-β-L-fucosid (Beispiel 1.10) werden in 10 ml Dioxan/Wasser 1:1 unter Rühren mit 30 µl (0,18 mmol) Thiophosgen versetzt. Nach 10 min engt man ein und trocknet 1 h im Hochvakuum. Das erhaltene Senföl wird anschließend in absolutem Dimethylformamid mit 109 mg (0,144 mmol) des Konjugats aus Beispiel 3.4 in Gegenwart von 82 µl Ethyldiisopropylamin gekuppelt. Man engt ein und reinigt durch Flash-Chromatographie [Dichlormethan/Methanol/Ammoniak(17%ig) 15:4:0,5]. Die nach Einengen erhaltene Substanz wird aus Wasser lyophilisiert. Ausb.: 89 mg (56 %) [DC: Dichlormethan/Methanol/Ammoniak (17%ig) 15:6:0,6 $R_f$ = 0,22]

**In Analogie zu Beispiel 5.1 werden folgende gemischt substituierten Konjugate hergestellt:**

**Beispiel 5.2**

**N-{N$^{\alpha}$-[O-(3-O-Methyl-β-L-fucosyl)-4-hydroxyphenylamino-thiocarbonyl]-N$^{\varepsilon}$-[O-(3-O-carboxymethyl-β-L-fucosyl)-4-hydroxyphenylamino-thiocarbonyl]-lysyl-D-alanyl}-batracylin:**

Edukte: Kohlenhydrat aus Beispiel 1.2, Konjugat aus Beispiel 3.10 Reinigung durch Fällung des Rohproduktes aus Methanol mit Ether. Ausb.: 78% [DC: Acetonitril/Wasser/Eisessig 5:1:0,2 $R_f$ = 0,45].

**Beispiel 5.3**

**N-{N$^{\alpha}$-[O-(3-O-Methyl-β-L-fucosyl)-4-hydroxyphenylamino-thiocarbonyl]-N$^{\varepsilon}$-[4-hydroxyphenylamino-thiocarbonyl]-lysyl-D-alanyl}-batracylin:**

Edukte: Konjugat aus Beispiel 4.3, 4-Hydroxy-anilin
Reinigung durch Flashchromatographie [Dichlormethan/Methanol/Ammoniak (17%ig) 15:2:0,2]; Ausb.: 60% [DC: Dichlormethan/Methanol/Ammoniak (17%ig) 15:2:0,2 $R_f$ =0,31].

**Beispiel 5.4**

**N-{N$^{\alpha}$-[O-(3-O-Carboxymethyl-β-L-fucosyl)-4-hydroxyphenylaminothiocarbonyl]-N$^{\varepsilon}$-[4-hydroxyphenylamino-thiocarbonyl]-lysyl-D-alanyl}-batracylin:**

Edukte: Konjugat aus Beispiel 4.2, 4-Hydroxy-anilin
Reinigung durch Flashchromatographi e [Di chl ormethan/Methanol/Ammoni ak (17%ig) 15:4:0,5]. Anschließend wird der Rückstand mit Methanol/Ether verrührt. Ausb.: 55% [DC: Dichlormethan/Methanol/Ammoniak (17%ig) 15:6:0,6 $R_f$ =0,3].

**Beispiel 5.5**

**N-{N$^{\alpha}$-[O-(4-O-Methyl-ß-L-fucosyl)-4-hydroxyphenylamino-thiocarbonyl]-N$^{\varepsilon}$-[4-hydroxyphenylamino-thiocarbonyl]-lysyl-D-alanyl}-batracylin:**

Edukte: Konjugat aus Beispiel 4.4, 4-Hydroxy-anilin
Reinigung durch Flashchromatographie [Dichlormethan/Methanol/Ammoniak (17%ig) 15:2:0,2]. Anschließend wird der Rückstand aus Methanol/Dichlormethan mit Ether gefällt. Ausb.: 49%; Schmp.: 128°C [DC: Dichlormethan/Methanol/Ammoniak (17%ig) 15:2:0,2 $R_f$ = 0,26].

**Beispiel 5.6**

**N-{N$^{\alpha}$-[4-Hydroxyphenylamino-thiocarbonyl]-N$^{\varepsilon}$-[O-(4-O-methyl-β-L-fucosyl)-4-hydroxyphenylamino-thiocarbonyl]-lysyl-D-alanyl}-batracylin:**

Edukte: Konjugat aus Beispiel 3.11, 4-Hydroxy-anilin
Reinigung durch Flashchromatographie [Dichlormethan/Methanol/Ammoniak (17%ig) 15:2:0,2]. Anschließend wird der Rückstand aus Wasser/Dioxan lyophilisiert. Ausb.: 50%[DC: Dichlormethan/Methanol/Ammoniak (17%ig) 15:2:0,2 $R_f$ = 0,29].

**Beispiel 5.7**

**N- {N$^{\alpha}$-[4-Hydroxyphenylamino-thiocarbonyl]-N$^{\varepsilon}$-[O-(3-O-methyl-β-L-fucosyl)-4-hydroxyphenylamino-thiocarbonyl]-lysyl-D-alanyl}-batracylin:**

Edukte: Konjugat aus Beispiel 3.4, 4-Hydroxy-anilin
Rückstand aus Methanol mit Ether fällen. Ausb.: 76% [DC: Dichlormethan/Methanol/Ammoniak (17%ig) 15:2:0,2 $R_f$ = 0,26].

**Beispiel 5.8**

**N-{N$^{\alpha}$-[O-(3-O-Methyl-β-L-fucosyl)-4-hydroxyphenylamino-thiocarbonyl]-N$^{\varepsilon}$-(4-hydroxyethylamino-2-[O-(3-O-methyl-β-L-fucosyl)-4-hydroxyphenylamino]triazin-6-yl]]-lysyl-D-alanyl}-batracylin:**

Edukte: Konjugat aus Beispiel 8.10, Kohlenhydrat aus Beispiel 1.2 Reinigung durch Flashchromatographie [Dichlormethan/Methanol/Ammoniak (17%ig) 15:2:0,2]. Ausb.: 9% FAB-MS: m/z = 1165 = M+1 [DC: Dichlormethan/Methanol/Ammoniak (17%ig) 15:3:0,3 $R_f$ = 0,27].

**Beispiel 5.9**

**N-{N$^{\alpha}$-[O-(3-O-Carboxymethyl-β-L-fucosyl)-4-hydroxyphenylaminothiocarbonyl]-N$^{\varepsilon}$-[acetyl]-lysyl-D-alanyl}-batracylin:**

Edukte: N-[N$^{\varepsilon}$-(Acetyl)-lysyl-D-alanyl]-batracylin, Kohlenhydrat aus Beispiel 1.10
Reinigung durch Flashchromatographie [Dichlormethan/Methanol/Ammoniak (17%ig) 15:2:0,2; später im gleichen System 15:4:0,5] Anschließend wird der Rückstand aus Wasser gefriergetrocknet. Ausb.: 46% [DC: Acetonitril/Wasser/Eisessig 5:1:0,2 $R_f$ = 0,31].

**Beispiel 5.10**

**N-{N$^{\alpha}$-[O-(4-O-Methyl-β-L-fucosyl)-4-hydroxyphenylamino-thiocarbonyl]-N$^{\varepsilon}$-[acetyl]-lysyl-D-alanyl}-batracylin:**

51 mg (0,067 mmol) des Konjugats aus Beispiel 4.4 werden in 5 ml Dimethylformamid mit 10 μl Acetanhydrid versetzt und 30 min bei Raumtemperatur gerührt. Man engt ein und fällt aus Methanol mit Ether. Ausb.: 46 mg (86 %) [DC: Acetonitril/Wasser/Eisessig 5:1:0,2 $R_f$ = 0,6].

Beispiel 5.11

**N-{N$^{\alpha}$-[O-(β-L-Fucosyl)-4-hydroxyphenylamino-thiocarbonyl]-N$^{\varepsilon}$-[succinyl]-lysyl-D-alanyl}-batracylin, Natriumsalz:**

20 mg (0,027 mmol) des Konjugats aus Beispiel 4.1 werden in 2 ml Dimethylformamid mit 3 mg Bernsteinsäureanhydrid versetzt und 6 h bei Raumtemperatur gerührt. Man engt ein und fällt aus Methanol mit Ether. Man nimmt den Rückstand in Wasser auf und fügt 27 µl einer 0,1N Natronlauge hinzu. Ausb.: 20 mg (86 %); [DC: Dichlormethan/Methanol/Eisessig 80:20:2 $R_f$ = 0,2].

Beispiel 5.12

**N-{N$^{\alpha}$-[O-(3-O-Methoxycarbonylmethyl-β-D-galactopyranosyl)-4-hydroxyphenylamino-thiocarbonyl]-N$^{\varepsilon}$-[O-(3,4-di-O-methyl-β-D-galactopyranosyl)-4-hydroxy-phenylamino-thiocarbonyl]-lysyl-D-alanyl}-batracylin:**

Das Kohlenhydrat 1.42 (37,7 mg, 0,11 mmol) wird wie in Beispiel 3.31.a beschrieben mit dem Peptidkonjugat 3.31 (79 mg, 0,1 mmol) umgesetzt. Nach Einengen im Vakuum und Flashchromatographie [Dichlormethan/Methanol 20:1 → 10:1] erhält man gelbe Kristalle (52,9 mg, 45 %); DC [Dichlormethan/Methanol 10:1]: $R_f$ = 0,14, Schmp. = 178°C (Zers.).

Beispiel 5.13

**N-{N$^{\alpha}$-[O-(3-O-Carboxymethyl-β-D-galactopyranosyl)-4-hydroxy-phenylamino-thiocarbonyl]-N$^{\varepsilon}$-[O-(3,4-di-O-methyl-β-D-galactopyranosyl)-4-hydroxyphenylamino-thiocarbonyl]-lysyl-D-alanyl}-batracylin, Natriumsalz:**

Das Kohlenhydrat 1.43 (38,6 mg, 0,11 mmol) wird wie in Beispiel 3.31.a beschrieben mit dem Peptidkonjugat 3.31 (79 mg, 0,1 mmol) umgesetzt. Nach Einengen im Vakuum und Lösen in Methanol/Dichlormethan 1:1 wird das Produkt durch Zugabe von Diethylether ausgefällt. Der Rückstand wird in Wasser (10 ml) suspendiert und die Suspension unter Rühren tropfenweise mit 0,05N-Natronlauge versetzt, bis eine klare Lösung entsteht (pH <10). Durch Lyophilisieren der filtrierten Lösung erhält man einen gelben amorphen Feststoff (87,9 mg, 74 %); DC [Ethanol]: $R_f$ = 0,17; $[\alpha]^{20}$ = +56,0° (c = 0,1 /$H_2O$).

Beispiel 5.14

**N-{N$^{\alpha}$-[O-(3-O-Carbamoylmethyl-β-D-galactopyranosyl)-4-hydroxy-phenylamino-thiocarbonyl]-N$^{\varepsilon}$-[O-(3,4-di-O-methyl-β-D-galactopyranosyl)-4-hydroxyphenylamino-thiocarbonyl]-lysyl-D-alanyl}-batracylin:**

Das Kohlenhydrat 1.44 (36,1 mg, 0,11 mmol) wird wie in Beispiel 3.31.a beschrieben mit dem Peptidkonjugat 3.31 (79 mg, 0,1 mmol) umgesetzt. Nach Einengen im Vakuum und Lösen in Methanol/Dichlormethan 1:1 wird das Produkt durch Zugabe von Diethylether ausgefällt. Man erhält gelbe Kristalle (45,9 mg, 39 %); DC [Ethanol]: $R_f$ = 0,38; Schmp. = 219°C (Zers.).

Beispiel 5.15

**N-{N$^{\alpha}$-[O-(3,4-di-O-Nlethyl-β-D-galactopyranosyl)-4-hydroxy-phenylamino-thiocarbonyl]-N$^{\varepsilon}$-[O-(3-O-(piperidyl-N)-carbonylmethyl-β-D-galactopyranosyl)-4-hydroxy-phenylamino-thiocarbonyl]-lysyl-D-alanyl}-batracylin:**

Das Kohlenhydrat 1.31 (39,5 mg, 0,11 mmol) wird wie in Beispiel 3.31.a beschrieben mit dem Peptidkonjugat 3.32 (88,7 mg, 0,1 mmol) umgesetzt. Nach Einengen im Vakuum und Flashchromatographie [Dichlormethan/Methanol 20:1 → 10:1] erhält man gelbe Kristalle (38,8 mg, 32 %); DC [Dichlormethan/Methanol 5:1]: $R_f$ = 0,79; Schmp. = 205°C (Zers.).

**Beispiel 5.16**

**N-{N$^{\alpha}$-[O-(3-O-Carboxymethyl-β-D-galactopyranosyl)-4-hydroxy-phenylamino-thiocarbonyl]-N$^{\varepsilon}$-[O-(3-O-(piperidyl-N)-carbonylmethyl-β-D-galactopyranosyl)-4-hydroxy-phenylamino-thiocarbonyl]-lysyl-D-alanyl}-batracylin, Natriumsalz:**

Das Kohlenhydrat 1.43 (38,6 mg, 0,11 mmol) wird wie in Beispiel 3.31.a beschrieben mit dem Peptidkonjugat 3.32 (88,7 mg, 0,1 mmol) umgesetzt. Nach Einengen im Vakuum und Lösen in Methanol/Dichlormethan 1:1 wird das Produkt durch Zugabe von Diethylether ausgefällt. Der Rückstand wird in Wasser (10 ml) suspendiert und die Suspension unter Rühren tropfenweise mit 0,05N-Natronlauge versetzt, bis eine klare Lösung entsteht (pH <10). Durch Lyophilisieren der filtrierten Lösung erhält man einen gelben amorphen Feststoff (90,3 mg, 71 %); DC [Ethanol]: $R_f$ = 0,05; $[\alpha]^{20}$ = +39,0° (c = 0,1 /$H_2O$).

**Beispiel 5.17**

**N-{N$^{\alpha}$-[O-(3-O-Carbamoylmethyl-β-D-galactopyranosyl)-4-hydroxy-phenylamino-thiocarbonyl]-N$^{\varepsilon}$-[O-(3-O-(piperidyl-N)-carbonylmethyl-β-D-galactopyranosyl)-4-hydroxy-phenylamino-thiocarbonyl]-lysyl-D-alanyl}-batracylin:**

Das Kohlenhydrat 1.44 (36,1 mg, 0,11 mmol) wird wie in Beispiel 3.31.a beschrieben mit dem Peptidkonjugat 3.32 (88,7 mg, 0,1 mmol) umgesetzt. Nach Einengen im Vakuum und Lösen in Methanol/Dichlormethan 1:1 wird das Produkt durch Zugabe von Diethylether ausgefällt. Man erhält gelbe Kristalle (44,8 mg, 36 %), DC [Ethanol]: $R_f$ = 0,06; Schmp. = 223°C (Zers.).

**Beispiel 5.18**

**N-{N$^{\alpha}$-[O-(3,4-di-O-Methyl-β-D-galactopyranosyl)-4-hydroxy-phenylaminothiocarbonyl]-N$^{\varepsilon}$-[O-(3-O-carboxymethyl-β-D-galactopyranosyl)-4-hydroxy-phenylamino-thiocarbonyl]-lysyl-D-alanyl}-batracylin, Natriumsalz:**

Das Kohlenhydrat 1.31 (39,5 mg, 0,11 mmol) wird wie in Beispiel 3.31.a beschrieben mit dem Peptidkonjugat 3.33 (84,2 mg, 0,1 mmol) umgesetzt. Nach Einengen im Vakuum wird mit Methanol/Dichlormethan 1:1 (20 ml) versetzt und das Produkt durch Zugabe von Diethylether ausgefällt. Der Rückstand wird in Wasser (10 ml) suspendiert und die Suspension unter Rühren tropfenweise mit 0,05N-Natronlauge versetzt, bis eine klare Lösung entsteht (pH <10). Durch Lyophilisieren der filtrierten Lösung erhält man einen gelben amorphen Feststoff (80,7 mg, 68 %); DC [Ethanol]: $R_f$ = 0,09; $[\alpha]^{20}$ = +35,0° (c = 0,1 /$H_2O$).

**Beispiel 5.19**

**N-{N$^{\alpha}$-[O-(3-O-Methoxycarbonylmethyl-β-D-galactopyranosyl)-4-hydroxy-phenylamino-thiocarbonyl]-N$^{\varepsilon}$-[O-(3-O-carboxymethyl-β-D-galactopyranosyl)-4-hydroxy-phenylamino-thiocarbonyl]-lysyl-D-alanyl}-batracylin, Natriumsalz:**

Das Kohlenhydrat 1.42 (37,7 mg, 0,11 mmol) wird wie in Beispiel 3.31.a beschrieben mit dem Peptidkonjugat 3.33 (84,2 mg, 0,1 mmol) umgesetzt. Nach Einengen im Vakuum wird mit Methanol/Dichlormethan 1:1 (20 ml) versetzt und das Produkt durch Zugabe von Diethylether ausgefällt. Der Rückstand wird in Wasser (10 ml) suspendiert und die Suspension unter Rühren tropfenweise mit 0,05N-Natronlauge versetzt, bis eine klare Lösung entsteht (pH <10). Durch Lyophilisieren der filtrierten Lösung erhält man einen gelben amorphen Feststoff (87,5 mg, 71 %); DC [Ethanol]: $R_f$ = 0,10, $[\alpha]^{20}$ = +24,6° (c = 0,11 / $H_2O$).

**Beispiel 5.20**

**N-{N$^{\alpha}$-[O-(3-O-Carbamoylmethyl-β-D-galactopyranosyl)-4-hydroxy-phenylamino-thiocarbonyl]-N$^{\varepsilon}$-[O-(3-O-carboxymethyl-β-D-galactopyranosyl)-4-hydroxy-phenylamino-thiocarbonyl]-lysyl-D-alanyl}-batracylin, Natriumsalz:**

Das Kohlenhydrat 1.44 (36,1 mg, 0,11 mmol) wird wie in Beispiel 3.31.a beschrieben mit dem Peptidkonjugat 3.33 (84,2 mg, 0,1 mmol) umgesetzt. Nach Einengen im Vakuum wird mit Methanol/Dichlormethan 1:1 (20 ml) versetzt und das Produkt durch Zugabe von Diethylether ausgefällt. Der Rückstand wird in Wasser (10 ml) suspendiert und die

Suspension unter Rühren tropfenweise mit 0,05N-Natronlauge versetzt, bis eine klare Lösung entsteht (pH <10). Durch Lyophilisieren der filtrierten Lösung erhält man einen gelben amorphen Feststoff (78,6 mg, 65 %); DC [Ethanol]: $R_f$ = 0,11; $[\alpha]^{20}$ = -44,0° (c = 0,13 / $H_2O$).

**Beispiel 5.21**

**N-{N$^{\alpha}$-[O-(3,4-di-O-Methyl-β-D-galactopyranosyl)-4-hydroxy-phenylaminothiocarbonyl]-N$^{\varepsilon}$-[O-(3-O-carbamoylmethyl-β-D-galactopyranosyl)-4-hydroxyphenylamino-thiocarbonyl]-lysyl-D-alanyl}-batracylin:**

Das Kohlenhydrat 1.31 (39,5 mg, 0,11 mmol) wird wie in Beispiel 3.31.a beschrieben mit dem Peptidkonjugat 3.34 (81,9 mg, 0,1 mmol) umgesetzt. Nach Einengen im Vakuum und Flashchromatographie [Ethanol → Ethanol/Methanol 2:1] erhält man gelbe Kristalle (17,4 mg, 15 %); DC [Ethanol]: $R_f$ = 0,20; Schmp. >290 °C (Zers.).

**Beispiel 5.22**

**N-{N$^{\alpha}$-[O-(3-O-Methoxycarbonylmethyl-β-D-galactopyranosyl)-4-hydroxyphenylamino-thiocarbonyl]-N$^{\varepsilon}$-[O-(3-O-carbamoylmethyl-β-D-galacto-pyranosyl)-4-hydroxy-phenylamino-thiocarbonyl]-lysyl-D-alanyl}-batracylin:**

Das Kohlenhvdrat 1.42 (37,7 mg, 0,11 mmol) wird wie in Beispiel 3.31.a beschrieben mit dem Peptidkonjugat 3.34 (81,9 mg, 0,1 mmol) umgesetzt. Nach Einengen im Vakuum und Lösen in Methanol/Dichlormethan 1:1 wird das Produkt durch Zugabe von Diethylether ausgefällt. Man erhält gelbe Kristalle (72 mg, 60 %), DC [Ethanol]: $R_f$ = 0,21, Schmp. = 222 °C (Zers.).

**Beispiel 5.23**

**N-{N$^{\alpha}$-[O-(3-O-Carboxymethyl-β-D-galactopyranosyl)-4-hydroxy-phenylamino-thiocarbonyl]-N$^{\varepsilon}$-[O-(3-O-carbamoylmethyl-β-D-galactopyranosyl)-4-hydroxy-phenylamino-thiocarbonyl]-lysyl-D-alanyl}-batracylin, Natriumsalz:**

Das Kohlenhydrat 1.43 (38,6 mg, 0,11 mmol) wird wie in Beispiel 3.31.a beschrieben mit dem Peptidkonjugat 3.34 (81,9 mg, 0,1 mmol) umgesetzt. Nach Einengen im Vakuum und Lösen in Methanol/Dichlormethan 1:1 wird das Produkt durch Zugabe von Diethylether ausgefällt. Der Rückstand wird in Wasser (10 ml) suspendiert und die Suspension unter Rühren tropfenweise mit 0,05N-Natronlauge versetzt, bis eine klare Lösung entsteht (pH < 10). Durch Lyophylisieren der filtrierten Lösung erhält man einen gelben amorphen Feststoff (78,2 mg, 65 %); DC [Ethanol]: $R_f$ = 0,07; $[\alpha]^{20}$ = +33,0° (c = 0,1 /$H_2O$).

**Beispiele 6.1 - 6.89**

**Allgemeine Formel**

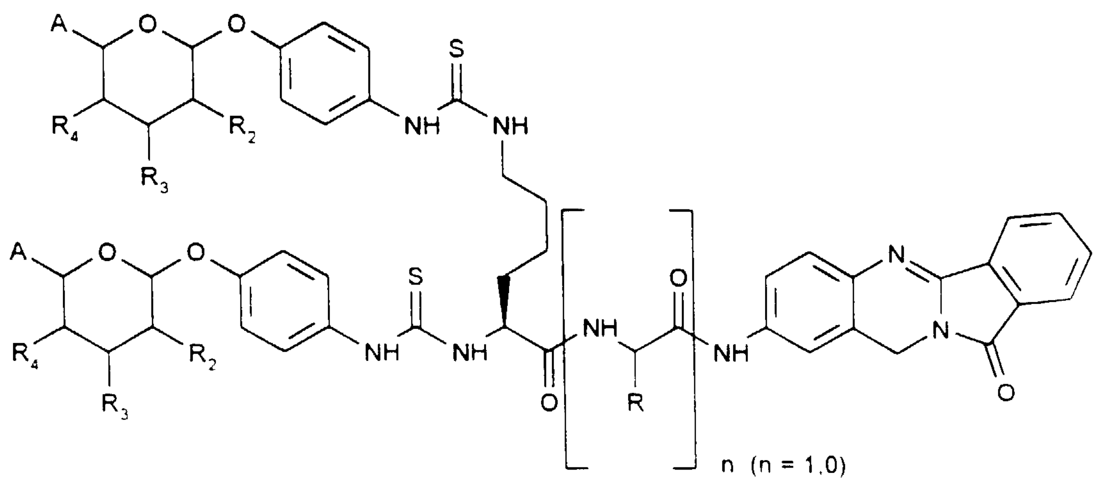

n (n = 1,0)

**Beispiel 6.1**

**N-{N$^{\alpha}$,N$^{\varepsilon}$-bis-[O-(2-O-Methyl-β-L-fucosyl)-4-hydroxy-phenylaminothiocarbonyl]-lysyl-D-alanyl}-batracylin:**

Eine Lösung von Verbindung 1.1 (50 mg, 0,19 mmol) in Dioxan/Wasser 1:1 (10 ml) wird unter Rühren mit Thiophosgen (30 µl, 0,4 mmol) versetzt. Nach 10 min. engt man im Vakuum ein und trocknet den Rückstand für 1 h im Ölpumpenvakuum. Das erhaltene Isothiocyanat wird in absolutem Dimethylformamid (10 ml) gelöst und mit Verbindung 2.13 (61 mg, 0,09 mmol) und mit Ethyldiisopropylamin (0,5 ml) versetzt. Man rührt für 16 h bei Raumtemperatur, engt dann im Vakuum ein und reinigt durch Flash-Chromatographie [Dichlormethan/Methanol/Ammoniak (17%ig) 15: 2:0,2]. Der Rückstand wird aus Methanol mit Ether gefällt. Man erhält 48mg (50%) des Zielproduktes als gelbe Kristalle.

**In Analogie zu Beispiel 6.1 werden aus dem Peptidkonjugat in Beispiel 2.13 bzw. dem L-Alanyl-Isomer (Beispiel 2.2) folgende Glycokonjugate hergestellt:**

**Beispiel 6.2**

**N-{N$^{\alpha}$,N$^{\varepsilon}$-bis-[O-(3-O-Methyl-β-L-fucosyl)-4-hydroxy-phenylaminothiocarbonyl]-lysyl-D-alanyl}-batracylin:**

Edukt: 100 mg (0,38 mmol) Kohlenhydrat aus Beispiel 1.2
Reinigung durch Flashchromatographie [Dichlormethan/Methanol/Ammoniak (17 %ig) 15:0,5:0,05; später 15:1:0,1 im gleichen System; anschließend Fällung aus Dichlormethan/Methanol/Ether. Ausb.: 59 %. Schmp.: 178°C (Zersetzung) [DC: Acetonitril/Wasser 10:1 $R_f$ =0,51].

**Beispiel 6.3**

**N-{N$^{\alpha}$,N$^{\varepsilon}$-bis-[O-(4-O-Methyl-β-L-fucosyl)-4-hydroxy-phenylaminothiocarbonyl]-lysyl-D-alanyl}-batracylin:**

Edukt: 115 mg (0,44 mmol) Kohlenhydrat aus Beispiel 1.4
Reinigung durch Flashchromatographie [Dichlormethan/Methanol/Ammoniak (17%ig) 15:2:0,2; anschließend Fällung aus Dichlormethan/Methanol (1:1) /Ether
Ausb.: 58 %. Schmp.: 176°C (Zersetzung) [DC: Acetonitril/Wasser 10:1 $R_f$ = 0,52].

**Beispiel 6.4**

**N-{$N^{\alpha}$,$N^{\varepsilon}$-bis-[O-(3-O-Methyl-β-L-fucosyl)-4-hydroxy-phenylaminothiocarbonyl]-lysyl-alanyl}-batracylin:**

Edukt: 115 mg (0,44 mmol) Kohlenhydrat aus Beispiel 1.2
Reinigung durch Fällung aus Dichlormethan/Methanol 1:1/Ether. Ausb.: 93%. Schmp.: 192°C (Zersetzung) [DC: Acetonitril/Wasser 10:1 $R_f$ = 0,46].

**Beispiel 6.5**

**N-{$N^{\alpha}$,$N^{\varepsilon}$-bis-[O-(3-O-Methyl-α-L-fucosyl)-4-hydroxy-phenylaminothiocarbonyl]-lysyl-D-alanyl}-batracylin:**

Edukt: 100 mg (0,38 mmol) Kohlenhydrat aus Beispiel 1.3
Reinigung durch Flashchromatographie [Dichlormethan/Methanol/Ammoniak (17%ig) 15:1:0,1; Fällung aus Dichlormethan/Methanol (1:1) / Ether. Schmp.: 178°C (Zersetzung); FAB-MS: m/z = 1071 = M+1.

**Beispiel 6.6**

**N-{$N^{\alpha}$,$N^{\varepsilon}$-bis-[O-(3-O-n-Propyl-β-L-fucosyl)-4-hydroxy-phenylaminothiocarbonyl]-lysyl-D-alanyl}-batracylin:**

Edukt: 38 mg (0,127 mmol) Kohlenhydrat aus Beispiel 1.5
Reinigung durch Flashchromatographie [Dichlormethan/Methanol/Ammoniak (17%ig) 15:1:0,1; Fällung aus Dichlormethan/Methanol (1:1)/ Ether. Ausb.: 42%. Schmp. 167-170°C (Zersetzung), [DC: Dichlormethan/Methanol/Ammoniak (17%ig) $R_f$ = 0,34].

**Beispiel 6.7**

**N-{$N^{\alpha}$,$N^{\varepsilon}$-bis-[O-(3-Desoxy-β-L-fucosyl)-4-hydroxy-phenylaminothiocarbonyl]-lysyl-D-alanyl}-batracylin:**

Edukt: 40 mg (0,167 mmol) Kohlenhydrat aus Beispiel 1.6
Reinigung durch Flashchromatographie [Dichlormethan/Methanol/Ammoniak (17%ig) 15:2:0,2; Fällung aus Methanol/ Ether. Ausb.:81%, [DC: Acetonitril/Wasser 10:1 $R_f$ = 0,46].

**Beispiel 6.8**

**N-{$N^{\alpha}$,$N^{\varepsilon}$-bis-[O-(3,4-Didesoxy-β-L-fucosyl)-4-hydroxy-phenylaminothiocarbonyl]-lysyl-D-alanyl}-batracylin:**

Edukt: 41mg (0,183mmol) Kohlenhydrat aus Beispiel 1.7
Reinigung durch Flashchromatographie [Dichlormethan/Methanol 95:5]; Gefriertrocknung aus Wasser/Dioxan. Ausb.: 60%; [DC: Dichlormethan/Methanol 9:1 $R_f$ = 0,22].

**Beispiel 6.9**

**N-{$N^{\alpha}$,$N^{\varepsilon}$-bis-[O-(3-Hydroxyethyl-β-L-fucosyl)-4-hydroxy-phenylaminothiocarbonyl]-lysyl-D-alanyl}-batracylin:**

Edukt: 75mg (0,25mmol) Kohlenhydrat aus Beispiel 1.12
Reinigung durch Flashchromatographie [Dichlormethan/Methanol/Ammoniak (17%ig) 15:2:0,2; später im gleichen System 15:4:0,5. Fällung aus Methanol/Ether. Ausb.: 66%; [DC: Acetonitril/Wasser 10:1 $R_f$ = 0,28].

**Beispiel 6.10**

**N-{$N^{\alpha}$,$N^{\varepsilon}$-bis-[O-(2-Hydroxyethyl-β-L-fucosyl)-4-hydroxy-phenylaminothiocarbonyl]-lysyl-D-alanyl}-batracylin:**

Edukt: 50mg (0,167mmol) Kohlenhydrat aus Beispiel 1.19
Reinigung durch Flashchromatographie [Dichlormethan/Methanol/Ammoniak (17%ig) 15.3:0,3; Fällung aus Methanol/ Ether; Gefriertrocknung aus Wasser/ Dioxan. Ausb.: 72%; [DC: Acetonitril/Wasser/Eisessig 5:1:0,2 $R_f$ = 0,39].

**Beispiel 6.11**

**N-{$N^{\alpha}$,$N^{\varepsilon}$-bis-[O-(2-O-Carboxymethyl-β-L-fucosyl)-4-hydroxy-phenylaminothiocarbonyl]-lysyl-D-alanyl}-batracylin, Di-Natriumsalz:**

Edukt: 50mg (0,16mmol) Kohlenhydrat aus Beispiel 1.13
Reinigung durch Flashchromatographie [Dichlormethan/Methanol/Ammoniak (17%ig) 15:8:0,8; später im gleichen System 15:10:1. Rückstand mit Ether digerieren, anschließend Gefriertrocknung aus Wasser/Dioxan. Überführung in das Di-Natriumsalz mit 2 Äquivalenten einer 0,1N Natronlauge, anschließend Gefriertrocknung aus Wasser. Ausb.: 49% [DC: Acetonitril/Wasser/Eisessig 5:1:0,5 $R_f$ = 0,38]. MS-FAB: $FAB^-$: m/z = 1157 = M - $2Na^+$ + $H^+$.

**Beispiel 6.12**

**N-{$N^{\alpha}$,$N^{\varepsilon}$-bis-[O-(3-O-Carboxymethyl-β-L-fucosyl)-4-hydroxy-phenylaminothiocarbonyl]-lysyl-D-alanyl}-batracylin, Di-Natriumsalz:**

Edukt: 200 mg (0,64 mmol) Kohlenhydrat aus Beispiel 1.10
Reinigung durch Flashchromatographie [Dichlormethan/Methanol/Ammoniak (17%ig) 15:4:0,5; später im gleichen System 15:8.0,8; und schließlich 15:10:1. Rückstand mit Ether digerieren, anschließend Gefriertrocknung aus Wasser/Dioxan 1:1. Überführung in das Di-Natriumsalz mit 2 Äquivalenten einer 0,1N Natronlauge, anschließend Gefriertrocknung aus Wasser. Ausb.: 59 %, [DC: Dichlormethan/Methanol/Ammoniak(17%ig) $R_f$ = 0,1].

**Beispiel 6.13**

**N-{$N^{\alpha}$,$N^{\varepsilon}$-bis-[O-(3-O-Carbamoylmethyl-β-L-fucosyl)-4-hydroxy-phenylaminothiocarbonyl]-lysyl-D-alanyl}-batracylin:**

Edukt: 60 mg (0,19 mmol) Kohlenhydrat aus Beispiel 1.18
Reinigung durch Flashchromatographie [Dichlormethan/Methanol/Ammoniak (17 %ig) 15:3:0,3]. Rückstand aus Dichlormethan/Methanol (1:1) mit Ether fällen, absaugen und anschließend Gefriertrocknung aus Wasser/Dioxan 1:1. Ausb.: 36%[DC: Acetonitril/Wasser/Eisessig 5:1:0,2 $R_f$ = 0,46]. Schmp.: 190° (Zers.).

**Beispiel 6.14**

**N-{$N^{\alpha}$,$N^{\varepsilon}$-bis-[O-(β-L-Fucosyl)-4-hydroxy-phenylamino-thiocarbonyl]-lysyl-D-alanyl}-batracylin:**

Edukt: 100mg (0,39mmol) p-Aminophenyl-β-L-fucosid
Reinigung durch Flashchromatographie [Dichlormethan/Methanol/Ammoniak (17%ig) 15:2:0,2; später im gleichen System 15:4:0,5]. Rückstand aus Dimethylformamid mit Ether fällen, absaugen. Ausb.: 48 %; Schmp.: 195°-198°C.

**Beispiel 6.15**

**N-{$N^{\alpha}$,$N^{\varepsilon}$-bis-[O-(α-L-Rhamnosyl)-4-hydroxy-phenylamino-thiocarbonyl]-lysyl-D-alanyl}-batracylin:**

Edukt: 158 mg (0,61 mmol) Kohlenhydrat aus Beispiel 1.21
Reinigung durch Flashchromatographie [Dichlormethan/Methanol/Ammoniak (17%ig) 15:3:0,3; später im gleichen System 15:4:0,5]. Rückstand lyophilisieren aus Wasser/Dioxan Ausb.: 87%. [DC: Dichlormethan/Methanol/Ammoniak (17%ig) 15:4:0,5 $R_f$ = 0,25].

**Beispiel 6.16**

**N-{$N^{\alpha}$,$N^{\varepsilon}$-bis-[O-(3-O-Carboxymethyl-α-L-rhamnosyl)-4-hydroxy-phenylamino-thiocarbonyl]-lysyl-D-alanyl}-batracylin, Di-Natriumsalz:**

Edukt: 200mg (0,64mmol) Kohlenhydrat aus Beispiel 1.22
Reinigung durch Flashchromatographie [Dichlormethan/Methanol/Ammoniak (17%ig) 15:4:0,5; später im gleichen System 15:8:0,8; und schließlich 15:10:1]. Rückstand mit Ether digerieren, anschließend Gefriertrocknung aus Wasser/Dioxan 1:1.

**In Analogie zu Beispiel 6.1 werden aus unterschiedlichen Peptidkonjugaten des Batracylins, die am Aminoterminus einen vollständig deblockierten Lysin-Baustein enthalten, folgende Glycokonjugate hergestellt:**

**Beispiel 6.17**

**N-{N$^{\alpha}$,N$^{\varepsilon}$-bis-[O-(3-O-Carboxymethyl-β-L-fucosyl)-4-hydroxy-phenylaminothiocarbonyl]-lysyl-glycyl}-batracylin, Di-Natriumsalz:**

Edukte: 32mg (0,lmmol) Kohlenhydrat aus Beispiel 1.10
32mg (0,045mmol) N-[Lysyl-glycyl]-batracylin, Di-Trifluoracetat (Beispiel 2.9)
Reinigung durch Flashchromatographi e [Di chl ormethan/Methanol/Ammoni ak (17%ig) 15:8:0,8; später im gleichen System 15:15:1,5]. Fällung aus Dimethylformamid/Methanol (1:1) mit Ether, anschließend Gefriertrocknung aus Wasser/Dioxan. Überführung in das Di-Natriumsalz mit 2 Äquivalenten einer 0,1N Natronlauge, anschließend Gefriertrocknung aus Wasser. Ausb.: 25 %, [DC: Dichlormethan/Methanol/Ammoniak (17%ig) 15:8:0,8 $R_f$ = 0,19] FAB-MS: m/z = 1189 = M+1.

**Beispiel 6.18**

**N-{N$^{\alpha}$,N$^{\varepsilon}$-bis-[O-(3-O-Methyl-β-L-fucosyl)-4-hydroxy-phenylaminothiocarbonyl]-lysyl-glycyl}-batracylin:**

Edukte: 60mg (0,22mmol) Kohlenhydrat aus Beispiel 1.2
66mg (0,1mmol) N-[Lysyl-glycyl]-batracylin, Di-Trifluoracetat (Beispiel 2.9)
Reinigung durch Flashchromatographie [Dichlormethan/Methanol/Eisessig 90:10:1]; Fällung aus Methanol mit Ether, anschließend Gefriertrocknung aus Wasser/Dioxan. Ausb.: 68% [DC: Dichlormethan/Methanol/Eisessig 80:20:2 $R_f$ = 0,62].

**Beispiel 6.19**

**N-{N$^{\alpha}$,N$^{\varepsilon}$-bis-[O-(β-L-Fucosyl)-4-hydroxy-phenylamino-thiocarbonyl]-lysyl-lysyl}-batracylin:**

**6.19.a) N-{N$^{\alpha}$,N$^{\varepsilon}$-bis-(O-(β-L-Fucosyl)-4-hydroxy-phenylamino-thiocarbonyl]-lysyl-N$^{\varepsilon}$-(fluorenyl-9-methoxycarbonyl)-lysyl}-batracylin:**

Edukte: 297 mg (1mmol) p-Aminophenyl-β-L-fucosid
66 mg (0,1 mmol) N-[Lysyl-N$^{\varepsilon}$-(fluorenyl-9-methoxycarbonyl)-lysyl]-batracylin, Di-Trifluoracetat (Beispiel 2.15)
206 µl Ethyldiisopropylamin
Reinigung durch zweimalige Fällung aus Methanol/Dichlormethan (1:1) mit Ether, Waschen des Filterrückstandes mit Ether. Ausb.: 89% [DC: Dichlormethan/Methanol/Ammoniak (17%ig) 15:4:0,5 $R_f$ = 0,31].

**6.19) N-{N$^{\alpha}$,N$^{\varepsilon}$-bis-[O-(β-L-fucosyl)-4-hydroxy-phenylamino-thiocarbonyl]-lysyl-lysyl}-batracylin:**

515 mg (0,39 mmol) der Verbindung aus Beispiel 6.19.a werden in 5 ml Dimethylformamid und 5 ml Piperidin gelöst und 30 min bei 20°C gerührt. Der Ansatz wird eingeengt und der Rückstand mit Ether digeriert. Man saugt ab und nimmt den Filterrückstand in Dimethylformamid auf. Nach Fällung mit Ether und Nachwaschen des Filterrückstandes verbleiben 335 mg (78 %) vom kristallinen Zielprodukt. FAB-MS:m/z = 1100 = M+1.

**Beispiel 6.20**

**N-{N$^{\alpha}$,N$^{\varepsilon}$-bis-[O-(3-O-Methyl-β-L-fucosyl)-4-hydroxy-phenylaminothiocarbonyl]-lysyl-diaminopropionyl}-batracylin:**

**6.20.a) N-{N$^{\alpha}$,N$^{\varepsilon}$-bis-[O-(3-O-Methyl-β-L-fucosyl)-4-hydroxy-phenylaminothiocarbonyl]-lysyl-N$^{\beta}$-(fluorenyl-9-methoxycarbonyl)-diaminopropionyl}-batracylin:**

Synthese analog zu Beispiel 6.19.
Edukte: 60 mg (0,22 mmol) Kohlenhydrat aus Beispiel 1.2
100 mg (0,11 mmol) N-[Lysyl-N$^{\beta}$-(fluorenyl-9-methoxycarbonyl)-diamino-propionyl]-batracylin, Di-Trifluoracetat (Beispiel 2.16)

76 µl Ethyldiisopropylamin
Reinigung durch zweimalige Fällung aus Methanol mit Ether, Waschen des Filterrückstandes mit Ether. Ausb.: 105 mg (73 %).

**6.20) N-{$N^\alpha$,$N^\varepsilon$-bis-[O-(3-O-Methyl-β-L-fucosyl)-4-hydroxy-phenylamino-thiocarbonyl]-lysyl-diaminopropionyl}-batracylin:**

103 mg (0,079 mmol) der Verbindung aus Beispiel 6.20.a werden analog zu Beispiel 6.19 mit Piperidin deblockiert. Reinigung durch Flashchromatographie (Dichlormethan/Methanol/Ammoniak (17%ig) 15:3:0,3; später im gleichen System 15:6:0,6). Gefriertrocknung aus Wasser/Dioxan. Ausb.: 21 %; [DC: Acetonitril/Wasser/Eisessig 5:1: 0,2 $R_f$ = 0,32].

**Beispiel 6.21**

**N-{$N^\alpha$,$N^\varepsilon$-bis-[O-(3-O-Carboxymethyl-β-L-fucosyl)-4-hydroxy-phenylaminothiocarbonyl]-lysyl-diaminopropionyl}-batracylin, Di-Natriumsalz:**

Diese Verbindung wurde analog zu Beispiel 6.20 über 2 Stufen, ausgehend von dem Kohlenhydrat aus Beispiel 1.10 und dem Peptidkonjugat aus Beispiel 2.16, hergestellt. Eine chromatographische Reinigung kann entfallen, da Nebenprodukte durch Digerieren mit Methanol und Ether entfernt werden können. Anschließend erfolgt die Überführung in das Di-Natriumsalz mit 2 Äquivalenten einer 0,1N Natronlauge. Ausb.: 66 % über 2 Stufen. [DC: Acetonitril/Wasser/Eisessig 10:3:1,5 $R_f$ = 0,3].

**Beispiel 6.22**

**N-{$N^\alpha$,$N^\varepsilon$-bis-[O-(3-O-Methyl-β-L-fucosyl)-4-hydroxy-phenylaminothiocarbonyl]-lysyl-seryl}-batracylin:**

Edukt: 60 mg (0,22 mmol) Kohlenhvdrat aus Beispiel 1.2
76 mg (0,11 mmol) N-[Lysyl-seryl]-batracylin, Di-Trifluoracetat (Beispiel 2.10)
Reinigung durch Flashchromatographie [Dichlormethan/Methanol/Ammoniak (17%ig) 15:2:0,2]; Fällung aus Dichlormethan/Methanol/Ether. Ausb.: 26%. [DC: Acetonitril/Wasser 10:1 $R_f$ = 0,39].

**Beispiel 6.23**

**N- {$N^\alpha$,$N^\varepsilon$-bis-[O-(3-O-Methyl-β-L-fucosyl)-4-hydroxy-phenylaminothiocarbonyl]-lysyl-D-seryl}-batracylin:**

Edukt: 60 mg (0,22 mmol) Kohlenhydrat aus Beispiel 1.2
69 mg (0,11 mmol) N-[Lysyl-D-seryl]-batracylin, Di-Hydrobromid (Beispiel 2.11)
Reinigung durch Flashchromatographie [Dichlormethan/Methanol/Ammoniak (17%ig) 15:2:0,2]; Gefriertrocknung aus Dioxan/Wasser. Ausb.: 29%. [DC: Acetonitril/Wasser 10:1 $R_f$ = 0,36].

**Beispiel 6.24**

**N-{$N^\alpha$,$N^\varepsilon$-bis-[O-(3-O-Methyl-β-L-fucosyl)-4-hydroxy-phenylaminothiocarbonyl]-lysyl}-batracylin:**

Edukt: 80 mg (0,3 mmol) Kohlenhydrat aus Beispiel 1.2
53 mg (0,14 mmol) N-[Lysyl]-batracylin (Beispiel 2.17)
Reinigung durch Flashchromatographie [Dichlormethan/Methanol/Ammoniak (17%ig) 15:1:0,1, später im gleichen System 15:2:0,2]; Fällung aus Methanol/ Ether. Ausb.: 26 %. [DC: Dichlormethan/Methanol/Ammoniak (17%ig) 15:2:0,2 $R_f$ = 0,22].

**Beispiel 6.25**

**N-{$N^\alpha$,$N^\varepsilon$-bis-[O-(3-O-Carboxymethyl-β-L-fucosyl)-4-hydroxy-phenylaminothiocarbonyl]-lysyl}-batracylin:**

Edukt: 60 mg (0,19 mmol) Kohlenhydrat aus Beispiel 1.10
24 mg (0,063 mmol) N-[Lysyl]-batracylin (Beispiel 2.17)
Reinigung durch Flashchromatographie [Dichlormethan/Methanol/Ammoniak (17%ig) 15:2:0,2]; später im gleichen System 15:4:0,5; Gefriertrocknung aus Wasser. Ausb.: 26%. [DC: Acetonitril/Wasser/Eisessig 5:1:0,2 $R_f$ = 0,25].

**Beispiel 6.26**

**N-{N$^{\alpha}$,N$^{\varepsilon}$-bis-[O-(3,4-di-O-Methyl-β-D-galactopyranosyl)-4-hydroxy-phenylamino-thiocarbonyl]-lysyl}-batracylin:**

Eine Lösung von Verbindung 1.31 (79 mg, 0,22 mmol) in Dioxan/Wasser 1:1 (10 ml) wird unter Rühren mit Thiophosgen (33,5 µl, 0,44 mmol) versetzt. Nach 10 min. engt man im Vakuum ein und trocknet den Rückstand für 1 h im Ölpumpenvakuum. Das erhaltene Isothiocyanat wird in absolutem Dimethylformamid (10 ml) gelöst und mit Verbindung 2.17 (37,7 mg, 0,1 mmol) und mit Ethyldiisopropylamin (0,5 ml) versetzt. Man rührt für 16 h bei Raumtemperatur, engt dann im Vakuum ein und reinigt durch Flashchromatographie [Dichlormethan/Methanol 30:1 → 20:1 → 10:1]. Man erhält gelbe Kristalle (56,7 mg, 53 %); DC [Ethylacetat/Ethanol 2:1]: $R_f$ = 0,72; Schmp. = 125 °C (Zers.).

**Beispiel 6.27**

**N-{N$^{\alpha}$,N$^{\varepsilon}$-bis-[O-(3-O-Methoxycarbonylmethyl-β-D-galactopyranosyl)-4-hydroxy-phenylamino-thiocarbonyl]-lysyl}-batracylin:**

Verbindung 1.42 (75,5 mg, 0,22 mmol) wird wie in Beispiel 6.26 beschrieben mit dem Peptidkonjugat 2.17 (37,7 mg, 0,1 mmol) umgesetzt. Reinigung durch Flash-chromatographie [Dichlormethan/Methanol 30:1 → 20:1 → 10:1] ergibt gelbe Kristalle (51,1 mg, 44 %); DC [Ethanol]: $R_f$ = 0,80; Schmp. = 176°C (Zers.)

**Beispiel 6.28**

**N-{N$^{\alpha}$,N$^{\varepsilon}$-bis-[O-(3-O-Carboxymethyl-β-D-galactopyranosyl)-4-hydroxyphenylamino-thiocarbonyl]-lysyl}-batracylin, Di-Natriumsalz:**

Verbindung 1.43 (77,3 mg. 0,22 mmol) wird wie in Beispiel 6.26 beschrieben mit dem Peptidkonjugat 2.17 (37,7 mg, 0,1 mmol) umgesetzt. Reinigung durch Umfällen aus Methanol/Dichlormethan 1:1 mit Diethylether ergibt gelbe Kristalle (53 mg, 47 %); DC [Methanol]: $R_f$ = 0,75; Schmp. = >260°C (Zers.).

**Beispiel 6.29**

**N-{N$^{\alpha}$,N$^{\varepsilon}$-bis-[O-(3-O-Carbamoylmethyl-β-D-galactopyranosyl)-4-hydroxy-phenylamino-thiocarbonyl]-lysyl}-batracylin:**

Verbindung 1.44 (72,2 mg, 0,22 mmol) wird wie in Beispiel 6.26 beschrieben mit dem Peptidkonjugat 2.17 (37,7 mg, 0,1 mmol) umgesetzt. Reinigung durch Umfällen aus Methanol/Dichlormethan 1:1 mit Diethylether ergibt gelbe Kristalle (55,6 mg, 48 %); DC [Ethanol]: $R_f$ = 0,09; Schmp. = 206°C (Zers.).

**Beispiel 6.30**

**N-{N$^{\alpha}$,N$^{\varepsilon}$-bis[O-(3-O-Methyl-β-D-galactopyranosyl)-4-hydroxy-phenylaminothiocarbonyl]-lysyl-seryl}-batracylin:**

Verbindung 1.25 (62,8 mg, 0,22 mmol) wird wie in Beispiel 6.26 beschrieben mit dem Peptidkonjugat 2.10 (69,3 mg, 0,1 mmol) umgesetzt. Nach Einengen im Vakuum nimmt man mit Dichlormethan/Methanol 1:1 (10 ml) auf, fällt das Produkt durch Zugabe von Diethylether (15 ml) aus und wäscht mit wenig eiskaltem Methanol. Man erhält gelbe Kristalle (46 mg, 41 %); DC [Ethylacetat/Ethanol 2:1]: $R_f$ = 0,12; Schmp. = 190-191°C.

**Beispiel 6.31**

**N-{N$^{\alpha}$,N$^{\varepsilon}$-bis-[O-(β-L-Fucopyranosyl)-4-hydroxy-phenylamino-thiocarbonyl]-lysyl-seryl}-batracylin:**

p-Aminophenyl-β-L-fucopyranosid (56,2 mg, 0,22 mmol) wird wie in Beispiel 6.26 beschrieben mit dem Peptidkonjugat 2.10 (69,3 mg, 0.1 mmol) umgesetzt. Reinigung durch Flashchromatographie [Ethylacetat → Ethanol] und Umfällen aus Methanol/Dichlormethan 1:1 mit Diethylether ergibt gelbe Kristalle (73,8 mg, 70 %); DC [Ethanol]: $R_f$ =

0,15; Schmp. = 123 °C.

**Beispiel 6.32**

**N-{N$^{\alpha}$,N$^{\varepsilon}$-bis-[O-(3,4-di-O-Methyl-β-D-galactopyranosyl)-4-hydroxy-phenylamino-thiocarbonyl]-lysyl-seryl}-batracylin:**

Verbindung 1.31 (79 mg, 0,22 mmol) wird wie in Beispiel 6.26 beschrieben mit dem Peptidkonjugat 2.10 (69,3 mg, 0,1 mmol) umgesetzt. Reinigung durch Flashchromatographie [Ethylacetat → Ethylacetat/Ethanol 7:1] ergibt gelbe Kristalle (43,8 mg, 38 %); DC [Ethylacetat/Ethanol 2:1]: $R_f$ = 0,31; Schmp. = 176°C.

**Beispiel 6.33**

**N-{N$^{\alpha}$,N$^{\varepsilon}$-bis-[O-(3-O-Methoxycarbonylmethyl-β-D-galactopyranosyl)-4-hydroxy-phenylamino-thiocarbonyl]-lysyl-seryl}-batracylin;**

Verbindung 1.42 (75,5 mg, 0,22 mmol) wird wie in Beispiel 6.26 beschrieben mit dem Peptidkonjugat 2.10 (69,3 mg, 0,1 mmol) umgesetzt Reinigung durch Flash-chromatographie [Ethylacetat → Ethylacetat/Ethanol 3:1] ergibt gelbe Kristalle (46,2 mg, 37 %); DC [Ethylacetat/Ethanol 2:1]: $R_f$ = 0,12; Schmp. = 161°C.

**Beispiel 6.34**

**N-{N$^{\alpha}$,N$^{\varepsilon}$-bis-[O-(3-O-Carboxymethyl-β-D-galactopyranosyl)-4-hydroxyphenylamino-thiocarbonyl]-lysyl-seryl}-batracylin, Di-Natriumsalz:**

Verbindung 1.43 (77,3 mg, 0,22 mmol) wird wie in Beispiel 6.26 beschrieben mit dem Peptidkonjugat 2.10 (69,3 mg, 0,1 mmol) umgesetzt und gereinigt. Man erhält gelbe Kristalle (39,2 mg, 31 %); DC [Methanol]: $R_f$ = 0,77, Schmp. = 213-215°C (Zers.).

**Beispiel 6.35**

**N-{N$^{\alpha}$,N$^{\varepsilon}$-bis-[O-(3-O-Carbamoylmethyl-β-D-galactopyranosyl)-4-hydroxy-phenylamino-thiocarbonyl]-lysyl-seryl}-batracylin:**

Verbindung 1.44 (72,2 mg, 0,22 mmol) wird wie in Beispiel 6.26 beschrieben mit dem Peptidkonjugat 2.10 (69,3 mg, 0,1 mmol) ungesetzt Reinigung durch Flashchromatographie [Ethylacetat/Ethanol 2:1] ergibt gelbe Kristalle (66,1 mg, 53 %); DC [Ethylacetat/Ethanol 2:1]: $R_f$ = 0,14; Schmp. = 192 °C (Zers.).

**Beispiel 6.36**

**N-{N$^{\alpha}$,N$^{\varepsilon}$-bis-[O-(3-O-Methyl-α-D-mannopyranosyl)-4-hydroxy-phenylaminothiocarbonyl]-lysyl-seryl}-batracylin:**

Verbindung 1.40 (62,8 mg, 0,22 mmol) wird wie in Beispiel 6.26 beschrieben mit dem Peptidkonjugat 2.10 (69,3 mg, 0,1 mmol) umgesetzt und gereinigt. Man erhält gelbe Kristalle (48,9 mg, 44 %); DC [Ethylacetat/Ethanol 2:1]: $R_f$ = 0,10; Schmp. = 204°C.

**Beispiel 6.37**

**N-{N$^{\alpha}$,N$^{\varepsilon}$-bis-[O-(2,3-di-O-Methyl-α-D-mannopyranosyl)-4-hydroxyphenylamino-thiocarbonyl]-lysyl-seryl}-batracylin:**

Verbindung 1.41 (66 mg, 0,22 mmol) wird wie in Beispiel 6.26 beschrieben mit dem Peptidkonjugat 2.10 (69,3 mg, 0,1 mmol) umgesetzt. Reinigung durch Flashchromatographie [Ethylacetat → Ethylacetat/Ethanol 7:1] ergibt gelbe Kristalle (52 mg, 45 %); DC [Ethylacetat/Ethanol 2:1]: $R_f$ = 0,28; Schmp. = 164-165°C.

**Beispiel 6.38**

**N-{$N^{\alpha}$,$N^{\varepsilon}$-bis-[O-(4-O-(β-D-galactopyranosyl)-β-D-glucopyranosyl)-4-hydroxyphenylamino-thiocarbonyl]-lysyl-seryl}-batracylin:**

Verbindung 1.56 (95,4 mg, 0,22 mmol) wird wie in Beispiel 6 26 beschrieben mit dem Peptidkonjugat 2.10 (69,3 mg, 0,1 mmol) umgesetzt. Nach Einengen im Vakuum wird das Produkt mit heißem Methanol (50 ml) gründlich gewaschen Man erhält gelbe Kristalle (61,6 mg, 44 %); DC [Methanol]: $R_f$ = 0,32; Schmp = 222°C (Zers.).

**Beispiel 6.39**

**N-{$N^{\alpha}$,$N^{\varepsilon}$-bis-[O-(β-L-Fucopyranosyl)-4-hydroxy-phenylamino-thiocarbonyl]-lysyl-D-seryl}-batracylin:**

p-Aminophenyl-β-L-fucopyranosid (56,2 mg, 0,22 mmol) wird wie in Beispiel 6.26 beschrieben mit dem Peptidkonjugat 2.11 (62,6 mg, 0,1 mmol) umgesetzt. Reinigung durch Flashchromatographie [Ethylacetat → Ethanol] und Umfällen des Rückstandes aus Methanol/Dichlormethan 1:1 mit Diethylether ergibt gelbe Kristalle (50,9 mg, 48 %); DC [Ethanol]: $R_f$ = 0,14; Schmp. = 133°C.

**Beispiel 6.40**

**N-{$N^{\alpha}$,$N^{\varepsilon}$-bis-[O-(3,4-di-O-Methyl-β-D-galactopyranosyl)-4-hydroxyphenylamino-thiocarbonyl]-lysyl-D-seryl}-batracylin:**

Verbindung 1.31 (79 mg, 0,22 mmol) wird wie in Beispiel 6.26 beschrieben mit dem Peptidkonjugat 2.11 (62,6 mg, 0,1 mmol) umgesetzt. Reinigung durch Flash-chromatographie [Ethylacetat → Ethylacetat/Ethanol 5:1] ergibt gelbe Kristalle (53,8 mg, 47 %); DC [Ethylacetat/Ethanol 2:1]: $R_f$ = 0,38; Schmp. = 145-146°C.

**Beispiel 6.41**

**N-{$N^{\alpha}$,$N^{\varepsilon}$-bis-[O-(3-O-Methoxycarbonylmethyl-β-D-galactopyranosyl)-4-hydroxy-phenylamino-thiocarbonyl]-lysyl-D-seryl}-batracylin:**

Verbindung 1.42 (75,5 mg, 0,22 mmol) wird wie in Beispiel 6.26 beschrieben mit dem Peptidkonjugat 2.11 (62,6 mg, 0,1 mmol) umgesetzt. Reinigung durch Flashchromatographie [Ethylacetat → Ethylacetat/Ethanol 3:1] ergibt gelbe Kristalle (52,4 mg, 42 %); DC [Ethylacetat/Ethanol 2:1]: $R_f$ = 0,12; Schmp = 168°C.

**Beispiel 6.42**

**N-{$N^{\alpha}$,$N^{\varepsilon}$-bis-[O-(3-O-Carboxymethyl-β-D-galactopyranosyl)-4-hydroxyphenylamino-thiocarbonyl]-lysyl-D-seryl}-batracylin, Di-Natriumsalz:**

Verbindung 1.43 (77,3 mg, 0,22 mmol) wird wie in Beispiel 6.26 beschrieben mit dem Peptidkonjugat 2.11 (62,6 mg, 0,1 mmol) umgesetzt und gereinigt. Man erhält gelbe Kristalle (69,2 mg, 55 %); DC [Methanol]: $R_f$ = 0,71; Schmp. = 214-216°C (Zers.).

**Beispiel 6.43**

**N-{$N^{\alpha}$,$N^{\varepsilon}$-bis-[O-(3-O-Carbamoylmethyl-β-D-galactopyranosyl)-4-hydroxyphenylamino-thiocarbonyl]-lysyl-D-seryl}-batracylin:**

Verbindung 1.44 (72,2 mg, 0,22 mmol) wird wie in Beispiel 6.26 beschrieben mit dem Peptidkonjugat 2.11 (62,6 mg, 0,1 mmol) umgesetzt. Reinigung durch Flashchromatographie [Ethylacetat/Ethanol 2:1] ergibt gelbe Kristalle (46,4 mg, 38 %); DC [Ethylacetat/Ethanol 2:1]: $R_f$ = 0,10; Schmp. = 173°C.

**Beispiel 6.44**

**N-{$N^{\alpha}$,$N^{\varepsilon}$-bis-[O-(β-L-Fucopyranosyl)-4-hydroxy-phenylamino-thiocarbonyl]-lysyl-D-asparagyl}-batracylin:**

p-Aminophenyl-β-L-fucopyranosid (56,2 mg, 0,22 mmol) wird wie in Beispiel 6.26 beschrieben mit dem Peptidkonjugat 2.7 (72,1 mg, 0,1 mmol) umgesetzt. Reinigung durch Umfällen aus Methanol/Dichlormethan 1:1 mit Diethylether ergibt gelbe Kristalle (69,4 mg, 64 %); DC [Ethanol]: $R_f$ = 0,14; Schmp. = 185-187°C.

**Beispiel 6.45**

**N-{$N^{\alpha}$,$N^{\varepsilon}$-bis-[O-(3,4-di-O-Methyl-β-D-galactopyranosyl)-4-hydroxyphenylamino-thiocarbonyl]-lysyl-D-asparagyl}-batracylin:**

Verbindung 1.31 (79 mg, 0,22 mmol) wird wie in Beispiel 6.26 beschrieben mit dem Peptidkonjugat 2.7 (72,1 mg, 0,1 mmol) umgesetzt. Reinigung durch Flash-chromatographie [Ethylacetat/Essigsäure 200:1 → Ethylacetat/Ethanol 3:1] ergibt gelbe Kristalle (99,5 mg, 85 %); DC [Ethanol]: $R_f$ = 0,59; Schmp. = 149°C (Zers.).

**Beispiel 6.46**

**N-{$N^{\alpha}$,$N^{\varepsilon}$-bis-[O-(3-O-Carboxymethyl-β-D-galactopyranosyl)-4-hydroxyphenylamino-thiocarbonyl]-lysyl-D-asparagyl}-batracylin, Di-Natriumsalz:**

Verbindung 1.43 (77,3 mg, 0,22 mmol) wird wie in Beispiel 6.26 beschrieben mit dem Peptidkonjugat 2.7 (72,1 mg, 0,1 mmol) umgesetzt. Reinigung durch Umfällen aus Methanol/Dichlormethan 1:1 mit Diethylether ergibt gelbe Kristalle (93,4 mg, 73 %); Schmp. = 220°C (Zers.).

**Beispiel 6.47**

**N-{$N^{\alpha}$,$N^{\varepsilon}$-bis-[O-(3-O-Carbamoylmethyl-β-D-galactopyranosyl)-4-hydroxyphenylamino-thiocarbonyl]-lysyl-D-asparagyl}-batracylin:**

Verbindung 1.44 (72,2 mg, 0,22 mmol) wird wie in Beispiel 6.26 beschrieben mit dem Peptidkonjugat 2.7 (72,1 mg, 0,1 mmol) umgesetzt. Reinigung durch Flashchromatographie [Ethylacetat/Ethanol/Essigsäure 400:100:2] ergibt gelbe Kristalle (69,7 mg, 57 %), DC [Ethanol]: $R_f$ = 0,11: Schmp. = 111°C.

**Beispiel 6.48**

**N-{$N^{\alpha}$,$N^{\varepsilon}$-bis-[O-(β-L-Fucopyranosyl)-4-hydroxy-phenylamino-thiocarbonyl]-lysyl-D-asparagyl}-batracylin, Natriumsalz:**

Verbindung 6.44 (21,7 mg, 20 mmol) wird in Wasser (10 ml) suspendiert und die Suspension unter Rühren tropfenweise mit 0,05N-Natronlauge versetzt, bis eine klare Lösung entsteht (pH <10). Durch Lyophilisieren der filtrierten Lösung erhält man einen gelben amorphen Feststoff (20,6 mg, 93 %).

**Beispiel 6.49**

**N-{$N^{\alpha}$,$N^{\varepsilon}$-bis-[O-(3,4-di-O-Methyl-β-D-galactopyranosyl)-4-hydroxy-phenyl-amino-thiocarbonyl]-lysyl-D-asparagyl}-batracylin, Natriumsalz:**

Verbindung 6.45 (23,5 mg, 20 μmol) wird wie in Beispiel 6.48 beschrieben umgesetzt und aufgearbeitet. Man erhält einen gelben amorphen Feststoff (23,9 mg, 100 %).

**Beispiel 6.50**

**N-{$N^{\alpha}$,$N^{\varepsilon}$-bis-[O-(3-O-Carboxymethyl-β-D-galactopyranosyl)-4-hydroxyphenylamino-thiocarbonyl]-lysyl-D-asparagyl}-batracylin, Tri-Natriumsalz:**

Verbindung 6.46 (25,6 mg, 20 μmol) wird in Wasser (10 ml) gelöst und die Lösung unter Rühren tropfenweise

mit 0,05N-Natronlauge versetzt, bis pH 8 erreicht ist. Durch Lyophilisieren der filtrierten Lösung erhält man einen gelben amorphen Feststoff (24 mg, 92 %).

**Beispiel 6.51**

**N-{Nα,Nε-bis-[O-(3-O-Carbamoylmethyl-β-D-galactopyranosyl)-4-hydroxyphenylamino-thiocarbonyl]-lysyl-D-asparagyl}-batracylin, Natriumsalz:**

Verbindung 6.47 (24,7 mg, 20 µmol) wird wie in Beispiel 6.48 beschrieben umgesetzt und aufgearbeitet. Man erhält einen gelben amorphen Feststoff (23,0 mg, 92 %).

**Beispiel 6.52**

**N-{Nα,Nε-bis-[O-(β-L-Fucopyranosyl)-4-hydroxy-phenylamino-thiocarbonyl]-lysyl-D-glutamyl}-batracylin:**

p-Aminophenyl-β-L-fucopyranosid (56,2 mg, 0,22 mmol) wird wie in Beispiel 6.26 beschrieben mit dem Peptidkonjugat 2.8 (66,8 mg, 0,1 mmol) umgesetzt. Reinigung durch Flashchromatographie [Ethylacetat/Essigsäure 200:1 → Ethylacetat/Ethanol/Essigsäure 10:1:0,1] ergibt gelbe Kristalle (39,5 mg, 36 %); DC [Ethanol]: $R_f$ = 0,09; Schmp. = 138-139°C (Zers.).

**Beispiel 6.53**

**N-{Nα,Nε-bis-[O-(3-O-Carboxymethyl-β-D-galactopyranosyl)-4-hydroxyphenylamino-thiocarbonyl]-lysyl-D-glutamyl}-batracylin, Di-Natriumsalz:**

Verbindung 1.43 (77,3 mg, 0,22 mmol) wird wie in Beispiel 6.26 beschrieben mit dem Peptidkonjugat 2.8 (66,8 mg, 0,1 mmol) umgesetzt und gereinigt. Man erhält gelbe Kristalle (97,4 mg, 75 %); Schmp. = 180°C (Zers.).

**Beispiel 6.54**

**N-{Nα,Nε-bis-[O-(3-O-Carboxymethyl-β-D-galactopyranosyl)-4-hydroxyphenylamino-thiocarbonyl]-lysyl-D-glutamyl}-batracylin, Tri-Natriumsalz:**

Verbindung 6.53 (25,6 mg, 20 µmol) wird wie in Beispiel 6.50 beschrieben umgesetzt und aufgearbeitet. Man erhält einen gelben amorphen Feststoff (24,3 mg, 92 %); $[\alpha]^{20}$ = +20,0° (c = 0,26 / $H_2O$).

**Beispiel 6.55**

**N-{Nα,Nε-bis-[O-(β-L-Fucopyranosyl)-4-hydroxy-phenylamino-thiocarbonyl]-lysyl-glycyl}-batracylin:**

p-Aminophenyl-β-L-fucopyranosid (56,2 mg, 0,22 mmol) wird wie in Beispiel 6.26 beschrieben mit dem Peptidkonjugat 2.9 (66,2 mg, 0,1 mmol) umgesetzt Reinigung durch Flashchromatographie [Ethylacetat → Ethanol] ergibt gelbe Kristalle (62,2 mg, 60 %); DC [Ethanol]: $R_f$ = 0,12; Schmp. = 176°C.

**Beispiel 6.56**

**N-{Nα,Nε-bis-[O-(3-O-Methyl-β-D-galactopyranosyl)-4-hydroxy-phenylaminothiocarbonyl]-lysyl-glycyl}-batracylin:**

Verbindung 1.25 (62,8 mg, 0,22 mmol) wird wie in Beispiel 6.26 beschrieben mit dem Peptidkonjugat 2.9 (66,2 mg, 0,1 mmol) umgesetzt. Reinigung durch Flash-chromatographie [Ethylacetat/Ethanol 10:1 → 2:1] ergibt gelbe Kristalle (56,2 mg, 52 %); DC [Ethylacetat/Ethanol 2:1]: $R_f$ = 0,22; Schmp. = 197°C.

**Beispiel 6.57**

**N-{$N^{\alpha}$,$N^{\varepsilon}$-bis-[O-(3,4-di-O-Methyl-β-D-galactopyranosyl)-4-hydroxyphenylamino-thiocarbonyl]-lysyl-glycyl}-batracylin:**

Verbindung 1.31 (79 mg, 0,22 mmol) wird wie in Beispiel 6.26 beschrieben mit dem Peptidkonjugat 2.9 (66,2 mg, 0,1 mmol) umgesetzt. Reinigung durch Flash-chromatographie [Ethylacetat/Ethanol 10:1 → 3:1] ergibt gelbe Kristalle (26,2 mg, 23 %); DC [Ethylacetat/Ethanol 2:1]: $R_f$ = 0,39; Schmp. = 209°C.

**Beispiel 6.58**

**N-{$N^{\alpha}$,$N^{\varepsilon}$-bis[O-(3-O-Methoxycarbonylmethyl-β-D-galactopyranosyl)-4-hydroxy-phenylamino-thiocarbonyl]-lysyl-glycyl}-batracylin;**

Verbindung 1.42 (75,5 mg, 0,22 mmol) wird wie in Beispiel 6.26 beschrieben mit dem Peptidkonjugat 2.9 (66,2 mg, 0,1 mmol) umgesetzt. Reinigung durch Flash-chromatographie [Ethylacetat/Ethanol → 10:1] ergibt gelbe Kristalle (22 mg, 18 %); DC [Ethylacetat/Ethanol 2:1]: $R_f$ = 0,06. Schmp. = 194-195°C (Zers.).

**Beispiel 6.59**

**N-{$N^{\alpha}$,$N^{\varepsilon}$-bis-[O-(3-O-Carboxymethyl-β-D-galactopyranosyl)-4-hydroxyphenylamino-thiocarbonyl]-lysyl-glycyl}-batracylin, Di-Natriumsalz:**

Verbindung 1.43 (77,3 mg, 0,22 mmol) wird wie in Beispiel 6.26 beschrieben mit dem Peptidkonjugat 2.9 (66,2 mg, 0,1 mmol) umgesetzt. Nach Einengen im Vakuum wird das Produkt gründlich mit Methanol, Dichlormethan und Diethylether gewaschen. Man erhält gelbe Kristalle (86,8 mg, 71 %); Schmp. = 230-232°C (Zers.).

**Beispiel 6.60**

**N-{$N^{\alpha}$,$N^{\varepsilon}$-bis-[O-(3-O-Carbamoylmethyl-β-D-galactopyranosyl)-4-hydroxyphenylamino-thiocarbonyl]-lysyl-glycyl}-batracylin:**

Verbindung 1.44 (72,2 mg, 0,22 mmol) wird wie in Beispiel 6.26 beschrieben mit dem Peptidkonjugat 2.9 (66,2 mg, 0,1 mmol) umgesetzt. Nach Einengen im Vakuum und Waschen mit Methanol, Dichlormethan und Diethylether erhält man gelbe Kristalle (40,9 mg, 35 %); DC [Ethylacetat/Ethanol 2:1]: $R_f$ = 0,05; Schmp. = 214-216°C (Zers.).

**Beispiel 6.61**

**N-{$N^{\alpha}$,$N^{\varepsilon}$-bis-[O-(3-O-Methyl-α-D-mannopyranosyl)-4-hydroxy-phenylaminothiocarbonyl]-lysyl-glycyl}-batracylin:**

Verbindung 1.40 (62,8 mg, 0,22 mmol) wird wie in Beispiel 6.26 beschrieben mit dem Peptidkonjugat 2.9 (66,2 mg, 0,1 mmol) umgesetzt. Reinigung durch Flash-chromatographie [Ethylacetat/Ethanol 10:1 → 2:1] ergibt gelbe Kristalle (72.2 mg, 66 %); DC [Ethylacetat/Ethanol 2:1]: $R_f$ = 0,18; Schmp. = 175°C.

**Beispiel 6.62**

**N-{$N^{\alpha}$,$N^{\varepsilon}$-bis-[O-(2,3-di-O-Methyl-α-D-mannopyranosyl)-4-hydroxy-phenylamino-thiocarbonyl]-lysyl-glycyl}-batracylin:**

Verbindung 1.41 (66 mg, 0,22 mmol) wird wie in Beispiel 6.26 beschrieben mit dem Peptidkonjugat 2.9 (66,2 mg, 0,1 mmol) umgesetzt. Reinigung durch Flash-chromatographie [Ethylacetat/Ethanol 10:1 → 3:1] ergibt gelbe Kristalle (66,6 mg, 60 %); DC [Ethylacetat/Ethanol 2:1]: $R_f$ = 0,41; Schmp. = 173°C.

**Beispiel 6.63**

**N-{N$^{\alpha}$,N$^{\varepsilon}$-bis-[O-(β-L-Fucopyranosyl)-4-hydroxy-phenylamino-thiocarbonyl]-lysyl-D-threonyl}-batracylin:**

p-Aminophenyl-β-L-fucopyranosid (56,2 mg, 0,22 mmol) wird wie in Beispiel 6.26 beschrieben mit dem Peptidkonjugat 2.12 (64 mg, 0,1 mmol) umgesetzt. Reinigung durch Flashchromatographie [Ethylacetat → Ethanol] und Umfallen des Produkts aus Methanol/Dichlormethan 1:1 mit Diethylether ergibt gelbe Kristalle (30,5 mg, 28 %); DC [Ethanol]: $R_f$ = 0,10; Schmp. = 172°C.

**Beispiel 6.64**

**N-{N$^{\alpha}$,N$^{\varepsilon}$-bis-[O-(β-D-Galactopyrranosyl)-4-hydroxy-phenylaminothiocarbonyl]-lysyl-D-alanyl}-batracylin:**

Verbindung 1.23 (59,7 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 10:1 → 1:1] ergibt gelbe Kristalle (68,3 mg, 64 %); DC [Dichlormethan/Methanol 1:1]: $R_f$ = 0,49, Schmp. = 222-224°C (Zers.).

**Beispiel 6.65**

**N-{N$^{\alpha}$,N$^{\varepsilon}$-bis-[O-(2-O-Methyl-β-D-galactopyranosyl)-4-hydroxy-phenylaminothiocarbonyl]-lysyl-D-alanyl}-batracylin:**

Verbindung 1.24 (62,8 mg, 0,22 mmol) wird wie in Beispiel 6.26 beschrieben mit dem Peptidkonjugat 2.13 (67,7 mg, 0,1 mmol) umgesetzt. Reinigung durch Umfällen aus Methanol/Dichlormethan 1:1 mit Diethylether ergibt gelbe Kristalle (69,6 mg, 63 %); DC [Ethanol]: $R_f$ = 0,24; Schmp. = 208°C (Zers.).

**Beispiel 6.66**

**N-{N$^{\alpha}$,N$^{\varepsilon}$-bis-[O-(3-O-Methyl-β-D-galactopyranosyl)-4-hydroxy-phenylaminothiocarbonyl]-lysyl-D-alanyl}-batracylin:**

Verbindung 1.25 (62,8 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 15:1 → 10:1 → 5:1] ergibt gelbe Kristalle (94,3 mg, 85 %); DC [Dichlormethan/Methanol 5:1]: $R_f$ = 0,16; Schmp. = 212°C (Zers.).

**Beispiel 6.67**

**N-{N$^{\alpha}$,N$^{\varepsilon}$-bis-[O-(4-O-Methyl-β-D-galactopyranosyl)-4-hydroxy-phenylaminothiocarbonyl]-lysyl-D-alanyl}-batracylin:**

Verbindung 1.26 (62,8 mg, 0,22 mmol) wird wie in Beispiel 6.26 beschrieben mit dem Peptidkonjugat 2.13 (67,7 mg, 0,1 mmol) umgesetzt. Reinigung durch Flashchromatographie [Dichlormethan/Methanol 15:1 → 10:1 → 5:1] ergibt gelbe Kristalle (52,6 mg, 48 %); DC [Dichlormethan/Methanol]: $R_f$ = 0,88, Schmp. = 192°C (Zers.).

**Beispiel 6.68**

**N-{N$^{\alpha}$,N$^{\varepsilon}$-bis-[O-(6-O-Methyl-β-D-galactopyranosyl)-4-hydroxy-phenylaminothiocarbonyl]-lysyl-D-alanyl}-batracylin:**

Verbindung 1.27 (62,8 mg, 0,22 mmol) wird wie in Beispiel 6.26 beschrieben mit dem Peptidkonjugat 2.13 (67,7 mg, 0,1 mmol) umgesetzt. Reinigung durch Umfällen aus Methanol/Dichlormethan 1:1 mit Diethylether ergibt gelbe Kristalle (96,7 mg, 88 %); DC [Dichlormethan/Methanol 5:1]: $R_f$ = 0,04; Schmp. = 210°C (Zers.).

**Beispiel 6.69**

**N-{$N^{\alpha}$,$N^{\varepsilon}$-bis-[O-(2,3-di-O-Methyl-β-D-galactopyranosyl)-4-hydroxyphenylamino-thiocarbonyl]-lysyl-D-alanyl}-batracylin:**

Verbindung 1.28 (65,9 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 20:1] ergibt gelbe Kristalle (57,4 mg, 51 %); DC [Dichlormethan/Methanol 5:1]: $R_f$ = 0,40; Schmp. = 148°C (Zers.).

**Beispiel 6.70**

**N-{$N^{\alpha}$,$N^{\varepsilon}$-bis-[O-(2,4-di-O-Methyl-β-D-galactopyranosyl)-4-hydroxyphenylamino-thiocarbonyl]-lysyl-D-alanyl}-batracylin:**

Verbindung 1.29 (65,9 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 20:1 → 15: → 10:1] ergibt gelbe Kristalle (74,2 mg, 65 %); DC [Dichlormethan/Methanol 5:1]: $R_f$ = 0,40; Schmp = 140°C (Zers.).

**Beispiel 6.71**

**N-{$N^{\alpha}$,$N^{\varepsilon}$-bis-[O-(2,6-di-O-Methyl-β-D-galactopyranosyl)-4-hydroxyphenylamino-thiocarbonyl]-lysyl-D-alanyl}-batracylin:**

Verbindung 1.30 (65,9 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 20:1 → 15:1] ergibt gelbe Kristalle (43,9 mg, 40 %); DC [Dichlormethan/Methanol 5:1]: $R_f$ = 0,43; Schmp. = 229°C (Zers.).

**Beispiel 6.72**

**N-{$N^{\alpha}$,$N^{\varepsilon}$-bis-[O-(3,4-di-O-Methyl-β-D-galactopyranosyl)-4-hydroxyphenylamino-thiocarbonyl]-lysyl-D-alanyl}-batracylin:**

Verbindung 1.31 (79 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 10:1] ergibt gelbe Kristalle (66,2 mg, 59 %); DC [Dichlormethan/Methanol/Ammoniak (25 %) 15:3:0,2]: $R_f$ = 0,39; Schmp. = 184°C.

**Beispiel 6.73**

**N-{$N^{\alpha}$,$N^{\varepsilon}$-bis-[O-(3,6-di-O-Methyl-β-D-galactopyranosyl)-4-hydroxy-phenylamino-thiocarbonyl]-lysyl-D-alanyl}-batracylin:**

Verbindung 1.32 (65,9 mg, 0.22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 15:1 → 10:1] ergibt gelbe Kristalle (57,1 mg, 50 %); DC [Dichlormethan/Methanol 5:1]: $R_f$ = 0.42, Schmp. = 190°C (Zers.).

**Beispiel 6.74**

**N-{$N^{\alpha}$,$N^{\varepsilon}$-bis-[O-(4,6-di-O-Methyl-β-D-galactopyranosyl)-4-hydroxyphenylamino-thiocarbonyl]-lysyl-D-alanyl}-batracylin:**

Verbindung 1.33 (79 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 20:1 → 10:1 → 5:1] ergibt gelbe Kristalle (47,0 mg, 42 %); DC [Dichlormethan/Methanol 5:1]: $R_f$ = 0,39; Schmp. = 169 °C.

**Beispiel 6.75**

**N-{N$^{\alpha}$,N$^{\varepsilon}$-bis-[O-(2,3,4-tri-O-Methyl-β-D-galactopyranosyl)-4-hydroxyphenylamino-thiocarbonyl]-lysyl-D-alanyl}-batracylin:**

Verbindung 1.34 (69 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 30:1] ergibt gelbe Kristalle (83,8 mg, 72 %); DC [Dichlormethan/Methanol 10:1]: $R_f$ = 0,36; Schmp. = 165°C (Zers.).

**Beispiel 6.76**

**N-{N$^{\alpha}$,N$^{\varepsilon}$-bis-[O-(2,3,6-tri-O-Methyl-β-D-galactopyranosyl)-4-hydroxyphenylamino-thiocarbonyl]-lysyl-D-alanyl}-batracylin:**

Verbindung 1.35 (69 mg, 0,22 mmol) wird wie in Beispiel 6.26 beschrieben mit dem Peptidkonjugat 2.13 (67,7 mg, 0,1 mmol) umgesetzt. Reinigung durch Umfallen aus Methanol/Dichlormethan 1:1 mit Diethylether ergibt gelbe Kristalle (105 mg, 91 %), DC [Dichlormethan/Methanol 5:1]: $R_f$ = 0,48; Schmp. = 194°C (Zers.).

**Beispiel 6.77**

**N-{N$^{\alpha}$,N$^{\varepsilon}$-bis-[O-(2,4,6-tri-O-Methyl-β-D-galactopyranosyl)-4-hydroxyphenylamino-thiocarbonyl]-lysyl-D-alanyl}-batracylin:**

Verbindung 1.36 (69 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 30:1 → 10:1] ergibt gelbe Kristalle (67 mg, 58 %); DC [Dichlormethan/Methanol 5:1]: $R_f$ = 0,54; Schmp. = 228°C (Zers.).

**Beispiel 6.78**

**N-{N$^{\alpha}$,N$^{\varepsilon}$-bis-[O-(3,4,6-tri-O-Methyl-β-D-galactopyranosyl)-4-hydroxyphenylamino-thiocarbonyl]-lysyl-D-alanyl}-batracylin:**

Verbindung 1.37 (69 mg, 0,22 mmol) wird wie in Beispiel 6.26 beschrieben mit dem Peptidkonjugat 2.13 (67,7 mg, 0,1 mmol) umgesetzt. Reinigung durch Umfällen aus Methanol/Dichlormethan 1:1 mit Diethylether ergibt gelbe Kristalle (109,3 mg, 94 %); DC [Dichlormethan/Methanol 5:1]: $R_f$ = 0,52; Schmp. = 180°C (Zers.).

**Beispiel 6.79**

**N-{N$^{\alpha}$,N$^{\varepsilon}$-bis-[O-(2,3,4,6-tetra-O-Methyl-β-D-galactopyranosyl)-4-hydroxyphenylamino-thiocarbonyl]-lysyl-D-alanyl}-batracylin:**

Verbindung 1.38 (69 mg, 0,22 mmol) wird wie in Beispiel 6.26 beschrieben mit dem Peptidkonjugat 2.13 (67,7 mg, 0,1 mmol) umgesetzt. Reinigung durch Umfällen aus Methanol/Dichlormethan 1:1 mit Diethylether ergibt gelbe kristalle (99,2 mg, 84 %); DC [Dichlormethan/Methanol 10:1] $R_f$ = 0.73; Schmp. = 188°C (Zers.).

**Beispiel 6.80**

**N-{N$^{\alpha}$,N$^{\varepsilon}$-bis-[O-(3-O-Methoxycarbonylmethyl-β-D-galactopyranosyl)-4-hydroxy-phenylamino-thiocarbonyl]-lysyl-D-alanyl}-batracylin:**

Verbindung 1.42 (75,5 mg, 0,22 mmol) wird wie in Beispiel 6.26 beschrieben mit dem Peptidkonjugat 2.13 (67,7 mg, 0,1 mmol) umgesetzt. Reinigung durch Umfällen aus Methanol/Dichlormethan 1:1 mit Diethylether ergibt gelbe Kristalle (22 mg, 18 %); DC [Dichlormethan/Methanol 5:1]: $R_f$ = 0,33; Schmp. = 194-195 °C (Zers.).

**Beispiel 6.81**

**N-{$N^{\alpha}$,$N^{\varepsilon}$-bis-[O-(3-O-Carboxymethyl-β-D-galactopyranosyl)-4-hydroxyphenylamino-thiocarbonyl]-lysyl-D-alanyl}-batracylin, Di-Natriumsalz:**

Verbindung 1.43 (77,3 mg, 0,22 mmol) wird wie in Beispiel 6.26 beschrieben mit dem Peptidkonjugat 2.13 (67,7 mg, 0,1 mmol) umgesetzt und gereinigt. Man erhält gelbe Kristalle (99,7 mg, 81 %); DC [Methanol]: $R_f$ = 0,80; Schmp. = 230°C (Zers.).

**Beispiel 6.82**

**N-{$N^{\alpha}$,$N^{\varepsilon}$-bis-[O-(3-O-Carbamoylmethyl-β-D-galactopyranosyl)-4-hydroxy-phenylamino-thiocarbonyl]-lysyl-D-alanyl}-batracylin:**

Verbindung 1.44 (72,2 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 7:1] ergibt gelbe Kristalle (29,4 mg, 25 %); DC [Dichlormethan/Methanol 3:1]: $R_f$ = 0,23; Schmp. = 201-202°C.

**Beispiel 6.83**

**N-{$N^{\alpha}$,$N^{\varepsilon}$-bis-[O-(3,4-Didesoxy-β-D-galactopyranosyl)-4-hydroxy-phenyl-amino-thiocarbonyl]-lysyl-D-alanyl}-batracylin:**

Verbindung 1.52 (52,6 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 17:1] ergibt gelbe Kristalle (38,8 mg, 37 %); DC [Dichlormethan/Methanol/Ammoniak (25 %) 15:3:0,2]: $R_f$ = 0,40; Schmp. = 175°C.

**Beispiel 6.84**

**N-{$N^{\alpha}$,$N^{\varepsilon}$-bis-[O-(α-D-Mannopyranosyl)-4-hydroxy-phenylaminothiocarbonyl]-lysyl-D-alanyl}-batracylin:**

Verbindung 1.39 (59,7 mg, 0,22 mmol) wird wie in Beispiel 6.26 beschrieben mit dem Peptidkonjugat 2.13 (67,7 mg, 0,1 mmol) umgesetzt. Reinigung durch Flash-chromatographie [Dichlormethan/Methanol 10:1 → 1:1] ergibt gelbe Kristalle (52 mg, 48 %); DC [Dichlormethan/Methanol 1:1]: $R_f$ = 0,19; Schmp. = 196°C.

**Beispiel 6.85**

**N-{$N^{\alpha}$,$N^{\varepsilon}$-bis-[O-(3,4-di-O-Methyl-β-D-galactopyranosyl)-4-hydroxyphenylamino-thiocarbonyl]-lysyl-alanyl}-batracylin:**

Verbindung 1.31 (79 mg, 0,22 mmol) wird wie in Beispiel 6.26 beschrieben mit dem Peptidkonjugat 2.2 (67,7 mg, 0,1 mmol) umgesetzt. Reinigung durch Flash-chromatographie [Dichlormethan/Methanol 10:1] ergibt gelbe Kristalle (77,6 mg, 69 %); DC [Dichlormethan/ Methanol/Ammoniak (25 %) 15:3:0,2]: $R_f$ = 0,33; Schmp. = 186°C.

**Beispiel 6.86**

**N-{$N^{\alpha}$,$N^{\varepsilon}$-bis-[O-(4-O-(β-D-Galactopyranosyl)-β-D-glucopyranosyl)-4-hydroxyphenylamino-thiocarbonyl]-lysyl-D-alanyl}-batracylin:**

Verbindung 1.56 (95,4 mg, 0,22 mmol) wird wie in Beispiel 6.26 beschrieben mit dem Peptidkonjugat 2.13 (67,7 mg, 0,1 mmol) umgesetzt. Nach Einengen im Vakuum wird der Rückstand mit heißem Methanol (50 ml) gründlich gewaschen. Man erhält gelbe Kristalle (102,8 mg, 73 %); DC [Methanol]: $R_f$ = 0,27; Schmp. = 225-226°C (Zers.).

**Beispiel 6.87**

**N-{N$^{\alpha}$,N$^{\varepsilon}$-bis-[O-(4-O-(3'-Sulfat-β-D-galactopyranosyl)-β-D-glucopyranosyl)-4-hydroxy-phenylamino-thiocarbonyl]-lysyl-D-alanyl}-batracylin, Di-Natriumsalz:**

Verbindung 1.57 (117,8 mg, 0,22 mmol) wird wie in Beispiel 6.26 beschrieben mit dem Peptidkonjugat 2.13 (67,7 mg, 0,1 mmol) umgesetzt. Reinigung durch Umfällen aus Methanol/Dichlormethan 1:1 mit Diethylether ergibt gelbe Kristalle (152,8 mg, 95 %); Schmp. = 232°C (Zers.).

**Beispiel 6.88**

**N-{N$^{\alpha}$,N$^{\varepsilon}$-bis-[O-(4-O-(3'-O-Methyl-β-D-galactopyranosyl)-β-D-glucopyranosyl)-4-hydroxy-phenylamino-thiocarbonyl]-lysyl-D-alanyl}-batracylin:**

Verbindung 1.58 (98,4 mg, 0,22 mmol) wird wie in Beispiel 6.26 beschrieben mit dem Peptidkonjugat 2.13 (67,7 mg, 0,1 mmol) umgesetzt. Reinigung durch Umfällen aus Methanol/Dichlormethan 1:1 mit Diethylether ergibt gelbe Kristalle (118,2 mg, 83 %); DC [Dichlormethan/Methanol 1:1]. $R_f$ = 0,58; Schmp. = 221°C (Zers.).

**Beispiel 6.89**

**N-{N$^{\alpha}$,N$^{\varepsilon}$-bis-[O-(2-O-Methyl-4-O-(3'-O-methyl-β-D-galactopyranosyl)-β-D-glucopyranosyl)-4-hydroxy-phenylamino-thiocarbonyl]-lysyl-D-alanyl}-batracylin:**

Verbindung 1.59 (101,5 mg, 0,22 mmol) wird wie in Beispiel 6.26 beschrieben mit dem Peptidkonjugat 2.13 (67,7 mg, 0,1 mmol) umgesetzt. Reinigung durch Flashchromatographie [Dichlormethan/Methanol 15:1 → 10:1 → 5:1] ergibt gelbe Kristalle (101,3 mg, 70 %); [Dichlormethan/Methanol 2:1]: $R_f$ = 0,58; Schmp. = 233°C (Zers.).

**Beispiele 7.1 - 7.13**

**Allgemeine Formel**

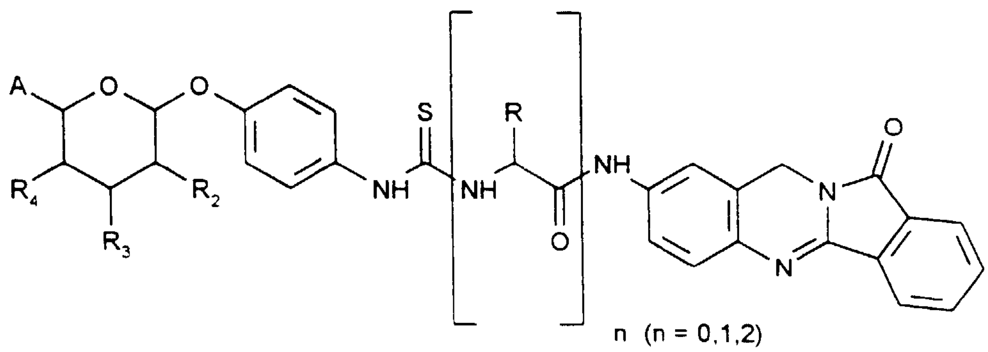

n (n = 0,1,2)

**Ausgehend von anderen Aminosäure- oder Peptidkonjugaten des Batracylins oder von Batracylin werden in Analogie zu Beispiel 4.1.a) folgende Glycokonjugate hergestellt:**

**Beispiel 7.1**

**N-{N-[O-(3-O-Methyl-β-L-fucosyl)-4-hydroxy-phenylamino-thiocarbonyl]-D-alanyl}-batracylin:**

Edukte: Kohlenhydrat aus Beispiel 1.2. Aminosäurekonjugat aus Beispiel 2.3
Reinigung durch Flashchromatographie (Dichlormethan/Methanol/Ammoniak (17 %ig) 15:1:0,1). Gefriertrocknung aus Dioxan/Wasser. Ausb.: 42 % [DC: Dichlormethan/Methanol/Ammoniak (17 %ig) 15:2:0,2 $R_f$ = 0,31].

**Beispiel 7.2**

**N-{N-[O-(3-O-Carboxymethyl-β-L-fucosyl)-4-hydroxy-phenylamino-thiocarbonyl]-D-alanyl}-batracylin, Natriumsalz:**

Edukte: Kohlenhydrat aus Beispiel 1.10, Aminosäurekonjugat aus Beispiel 2.3
Reinigung durch Flashchromatographie (Dichlormethan/Methanol/Ammoniak (17 %ig) 15:3:0,3). Gefriertrocknung aus Dioxan/Wasser und anschließende Überführung ins Natriumsalz mit 0,1N Natronlauge. Ausb.: 43 % [DC: Dichlormethan/Methanol/Ammoniak (17%ig) 15:4:0,5 $R_f$ = 0,14].

**Beispiel 7.3**

**N-{N-[O-(β-L-Fucosyl)-4-hydroxy-phenylamino-thiocarbonyl)-seryl-D-alanyl}-batracylin:**

Edukte: p-Aminophenyl-β-L-fucosid, Aminosäurekonjugat aus Beispiel 2.18
Reinigung durch Flashchromatographie (Dichlormethan/Methanol/Ammoniak (17 %ig) 15:3:0,3). Gefriertrocknung aus Dioxan/Wasser. Ausb.: 93% [DC: Dichlormethan/Methanol/Ammoniak (17 %ig) 15:3:0,3 $R_f$ = 0,28] FAB-MS: m/z = 705 = M+1.

**Beispiel 7.4**

**N-{N-[O-(β-L-Fucosyl)-4-hydroxy-phenylamino-thiocarbonyl]-D-alanyl-D-alanyl}-batracylin:**

Edukte: p-Aminophenyl-β-L-fucosid, Aminosäurekonjugat aus Beispiel 2.19
Reinigung durch Digerieren mit Methanol und Fällen aus Methanol/Dichlormethan mit Ether. Ausb.: 65 % [DC: Acetonitril/Wasser/Eisessig 5:1:0,2 $R_f$ = 0,78]

**Beispiel 7.5**

**N-{N-[O-(β-L-Fucosyl)-4-hydroxy-phenylamino-thiocarbonyl]-glutamyl-D-alanyl}-batracylin:**

Edukte: p-Aminophenyl-β-L-fucosid, Aminosäurekonjugat aus Beispiel 2.20
Reinigung durch Flashchromatographie (Dichlormethan/Methanol/Ammoniak (17%ig) 15:4:0,5; später im gleichen System 15:6:0,6). Gefriertrocknung aus Dioxan/Wasser. Ausb.: 92 % [DC: Dichlormethan/Methanol/Ammoniak (17 %ig) 15:8:0,8 $R_f$ = 0,55].

**Beispiel 7.6**

**N-[O-(3-O-Carboxymethyl-β-L-fucosyl)-4-hydroxy-phenylamino-thiocarbonyl]-batracylin:**

250 mg (1 mmol) Batracylin werden in 50 ml Dioxan gelöst und nach Zugabe von 184 µl Thiophosgen 2 h bei 20°C gerührt. Man engt ein, digeriert mit Ether und filtriert. Der Filterrückstand wird 16 h im Hochvakuum getrocknet und anschließend in 30 ml Dimethylformamid aufgenommen. Man fügt 312 mg (1 mmol) des Kohlenhydrats aus Beispiel 1.10 und 500 µl Ethyl-diisopropylamin zu und behandelt 4 h mit Ultraschall. Anschließend wird eingeengt und durch Flash-Chromatographie (Dichlormethan/Methanol/Ammoniak (17 %ig) 15:2:0,2: spater im gleichen System 15:6:0,6) gereinigt. Die relevanten Fraktionen werden eingeengt und aus Dioxan/Wasser lyophilisiert. Ausb.: 363 mg (60 %) [DC: Dichlormethan/Methanol/Ammoniak (17 %ig) 15:6:0,6 $R_f$ = 0,38].

**Beispiel 7.7**

**N-[O-(3-O-Methyl-β-L-fucosyl)-4-hydroxy-phenylamino-thiocarbonyl]-batracylin:**

Darstellung in Analogie zu Beispiel 7.6 ausgehend von Batracylin und dem Kohlenhydrat aus Beispiel 1.2.
Reinigung durch Flashchromatographie (Dichlormethan/Methanol/Ammoniak (17 %ig) 15:1:0,1; Ausb.: (58 %) [DC: Dichlormethan/Methanol/Ammoniak (17 %ig) 15:2:0,2 $R_f$ = 0,39]. FAB-MS: m/z = 561 = M+1.

**Beispiel 7.8**

**N-{N-[O-(3,4-di-O-Methyl-β-D-galactopyranosyl)-4-hydroxy-phenylamino-thiocarbonyl]-D-alanyl}-batracylin:**

Verbindung 1.31 (37,5 mg, 0,11 mmol) wird wie in Beispiel 6.26 beschrieben mit dem Peptidkonjugat 2.3 (32 mg, 0,1 mmol) umgesetzt. Reinigung durch Flashchromatographie [Dichlormethan/Methanol 30:1] ergibt gelbe Kristalle (49,3 mg, 75 %); DC [Ethylacetat/ Ethanol 2:1]: $R_f$ = 0,81; Schmp. = 185°C (Zers.).

**Beispiel 7.9**

**N-{N-[O-(2,3,4-tri-O-Methyl-β-D-galactopyranosyl)-4-hydroxy-phenylaminothiocarbonyl]-D-alanyl}-batracylin:**

Verbindung 1.34 (34,5 mg, 0,11 mmol) wird wie in Beispiel 6.26 beschrieben mit dem Peptidkonjugat 2.3 (32 mg, 0,1 mmol) umgesetzt. Reinigung durch Flash-chromatographie [Dichlormethan/Methanol 40:1 → 15:1] ergibt gelbe Kristalle (54,9 mg, 81 %); DC [Dichlormethan/Methanol 20:1]: $R_f$ = 0,15; Schmp. = 190°C (Zers.).

**Beispiel 7.10**

**N-{N-[O-(3-O-Methoxycarbonylmethyl-β-D-galactopyranosyl)-4-hydroxyphenylamino-thiocarbonyl]-D-alanyl}-batracylin:**

Verbindung 1.42 (37,8 mg, 0,11 mmol) wird wie in Beispiel 6.26 beschrieben mit dem Peptidkonjugat 2.3 (32 mg, 0,1 mmol) umgesetzt. Reinigung durch Flashchromatographie [Dichlormethan/Methanol 20:1 → 10:1] ergibt gelbe Kristalle (44,3 mg, 63 %); DC [Ethanol]: $R_f$ = 0,85; Schmp. = 195°C (Zers.).

**Beispiel 7.11**

**N-{N-[O-(3-O-Carboxymethyl-β-D-galactopyranosyl)-4-hydroxy-phenylaminothiocarbonyl]-D-alanyl}-batracylin, Natriumsalz:**

Verbindung 1.43 (38,6 mg, 0,11 mmol) wird wie in Beispiel 6.26 beschrieben mit dem Peptidkonjugat 2.3 (32 mg, 0,1 mmol) umgesetzt. Reinigung durch Umfällen aus Methanol/Dichlormethan 1:1 mit Diethylether ergibt gelbe Kristalle (63,8 mg, 89 %); DC [Ethanol]: $R_f$ = 0,15; Schmp. = 217°C (Zers.).

**Beispiel 7.12**

**N-{N-[O-(3-O-Carbamoylmethyl-β-D-galactopyranosyl)-4-hydroxyphenylamino-thiocarbonyl]-D-alanyl}-batracylin:**

Verbindung 1.44 (37,8 mg, 0,11 mmol) wird wie in Beispiel 6.26 beschrieben mit dem Peptidkonjugat 2.3 (32 mg, 0,1 mmol) umgesetzt. Reinigung durch Flash-chromatographie [Dichlormethan/Methanol 20:1 → 10:1 → 5:1] ergibt gelbe Kristalle (20 mg, 29 %); DC [Ethanol]: $R_f$ = 0,15; Schmp. = 184°C (Zers.).

**Beispiel 7.13**

**N-{N-[O-(β-L-Fucopyranosyl)-4-hydroxy-phenylamino-thiocarbonyl]-D-alanyl}-batracylin:**

p-Aminophenyl-β-L-fucopyranosid (28,1 mg, 0,11 mmol) wird wie in Beispiel 6.26 beschrieben mit dem Peptidkonjugat 2.3 (32 mg, 0,1 mmol) umgesetzt Reinigung durch Flashchromatographie [Ethylacetat/Petrolether 2:1 → 5:1] ergibt gelbe Kristalle (49,8 mg, 81 %); DC [Ethanol]: $R_f$ = 0,50; Schmp. = 173°C.

**Beispiele 8.1 - 8.12**

**Allgemeine Formel**

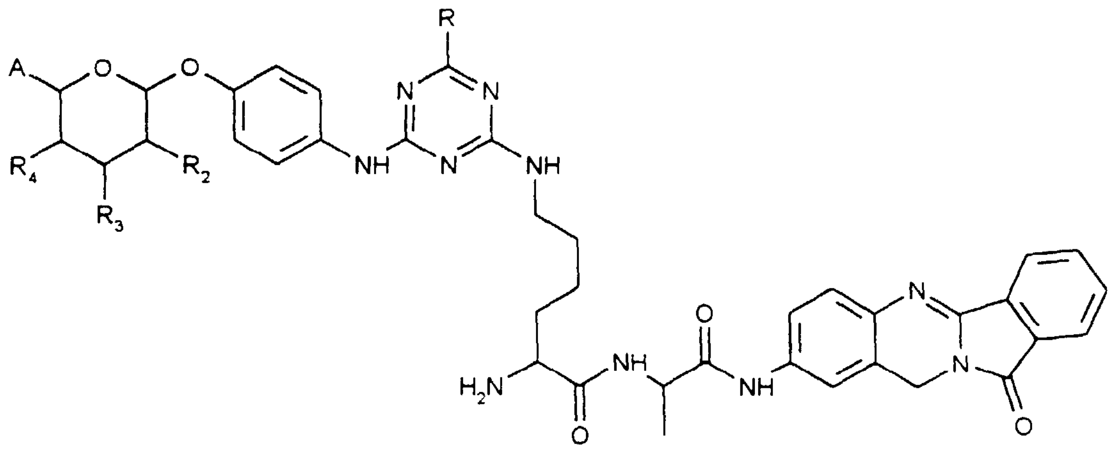

**Beispiel 8.1**

**N-{N$^{\varepsilon}$-[2-Chlor-4-[O-(3-O-methyl-β-L-fucosyl)-4-hydroxy-phenylamino]triazin-6-yl]-lysyl-D-alanyl}-batracylin, Trifluoracetat**

**8.1.a) N-{N$^{\alpha}$-[tert-Butoxycarbonyl]-N$^{\varepsilon}$-[2-Chlor-4-[O-(3-O-methyl-β-L-fucosyl)-4-hydroxy-phenylamino]-triazin-6-yl]-lysyl-D-alanyl}-batracylin:**

265 mg (0,98 mmol) p-Aminophenyl-3-O-methyl-β-L-fucosid (Beispiel 1.2) und 181mg (0,98 mmol) Cyanurchlorid werden in 50 ml Dioxan/Wasser 1:1 aufgenommen, auf -5°C abgekühlt und nach Zugabe von 83 mg Natriumhydrogencarbonat 15 min bei dieser Temperatur gerührt. Dann fügt man 538 mg (0,98 mmol) N-[N$^{\alpha}$-(tert-Butoxycarbonyl)-lysyl-D-alanyl]-batracylin (Beispiel 2.4) gelöst in 14 ml Dimethylformamid und weitere 83 mg Natriumhydrogencarbonat zu und läßt auf Raumtemperatur kommen. Nach 16 h Rühren bei 20°C engt man ein und behandelt den Rückstand mit Wasser. Man saugt ab, trocknet den Filterrückstand im Hochvakuum und erhält 890 mg (96 %) des Zielproduktes. [DC: Dichlormethan/Methanol 10.1 $R_f$ = 0,26].

**8.1) N-{N$^{\varepsilon}$-[2-Chlor-4-[O-(3-O-methyl-β-L-fucosyl)-4-hydroxy-phenylamino)triazin-6-yl]-lysyl-D-alanyl}-batracylin, Trifluoracetat:**

100 mg (0,11 mmol) der Verbindung aus Beispiel 8.1.a werden in einem Gemisch aus 5 ml wasserfreier Trifluoressigsäure und 5 ml Dichlormethan 30 min bei 0°C gerührt. Man engt ein und reinigt durch Flash-Chromatographie (Dichlormethan/Methanol/Ammoniak 17 %ig) 15:1,5:0,15. Anschließende Fällung aus Methanol/Ether führt zu 41 mg (95 %) des Zielproduktes. [DC: Dichlormethan/Methanol/Ammoniak (17 %ig) 15:2:0,2 $R_f$ = 0,26] FAB-MS: m/z = 829 = M+1.

**Beispiel 8.2**

**N-{N$^{\varepsilon}$-[4-Hydroxyethylamino-2-[O-(3-O-methyl-β-L-fucosyl)-4-hydroxyphenylamino]-triazin-6-yl]-lysyl-D-alanyl}-batracylin, Trifluoracetat**

**8.2.a) N-{N$^{\alpha}$-[tert-Butoxycarbonyl]-N$^{\varepsilon}$-[4-hydroxyethylamino-2-[O-(3-O-methyl-β-L-fucosyl)-4-hydroxy-phenylamino]-triazin-6-yl]-lysyl-D-alanyl}- batracylin:**

100 mg (0,11 mmol) der Verbindung aus Beispiel 8.1.a werden in 3 ml Dioxan gelöst. Man gibt 60 mg Kaliumcarbonat und 6,5 ml einer 0,1N Lösung von Ethanolamin in Dioxan zu und rührt 18 h bei 80°C. Anschließend wird

eingeengt und der Rückstand mit Wasser verrührt. Man filtriert und reinigt den Filterrückstand durch Flashchromatographie (Dichlormethan/Methanol/Ammoniak (17 %ig) 15:1:0,1). Nach Einengen der relevanten Fraktionen und Trocknen im Hochvakuum erhält man 83 mg (80 %) der Zielverbindung. [DC: Dichlormethan/Methanol/Ammoniak (17 %ig) 15:2:0,2 $R_f$ = 0,23].

**8.2) N-{N$^{\varepsilon}$-[4-Hydroxyethylamino-2-[O-(3-O-methyl-β-L-fucosyl)-4-hydroxyphenylamino]-triazin-6-yl]-lysyl-D-alanyl}-batracylin, Trifluoracetat:**

67 mg (0,07 mmol) der Verbindung aus Beispiel 8.2.a werden analog zu Beispiel 8.1 deblockiert. Die Reinigung erfolgt durch Flashchromatographie (Dichlormethan/Methanol/Ammoniak (17 %ig) 15:2:0,2). Anschließende Fällung aus Methanol/Ether führt zum Zielprodukt in 90 %iger Ausbeute. FAB-MS: m/z = 854 = M+1.

**In Analogie zu Beispiel 8.1 werden folgende Glycokonjugate hergestellt:**

**<u>Beispiel 8.3</u>**

**N-{N$^{\varepsilon}$-[2-Chlor-4-[O-(2-O-methyl-β-L-fucosyl)-4-hydroxy-phenylamino]-triazin-6-yl]-lysyl-D-alanyl}-batracylin, Trifluoracetat:**

Edukt: Kohlenhydrat aus Beispiel 1.1;
Ausb.: 76%; FAB-MS: m/z = 829 = M+1.

**<u>Beispiel 8.4</u>**

**N-{N$^{\varepsilon}$-[2-Chlor-4-[O-(β-L-fucosyl)-4-hydroxy-phenylamino]-triazin-6-yl]-lysyl-D-alanyl}-batracylin, Trifluoracetat:**

Edukt: p-Aminophenyl-β-L-fucosid;
Ausb.: 36 %; FAB-MS: m/z = 815 = M+1.
[DC: Dichlormethan/Methanol/Ammoniak (17 %ig) 15:4:0,
$R_f$ = 0,44].

**<u>Beispiel 8.5</u>**

**N-{N$^{\varepsilon}$-[2-Chlor-4-[O-(3-desoxy-β-L-fucosyl)-4-hydroxy-phenylamino]-triazin-6-yl]-lysyl-D-alanyl}-batracylin, Trifluoracetat:**

Edukt: Kohlenhydrat aus Beispiel 1.6;
Ausb.: 56 %; FAB-MS: m/z = 799 = M+1;
[DC: Acetonitril/Wasser/Eisessig 5:1:0,2 $R_f$ = 0,54].

**In Analogie zu den Beispielen 8.1.a, 8.2.a und 8.2 werden aus dem Batracylin-Peptidkonjugat in Beispiel 2.4 bzw. aus dem entsprechend herzustellendeii L-Alanyl-Isomeren folgende Glycokonjugate hergestellt:**

**<u>Beispiel 8.6</u>**

**N-{N$^{\varepsilon}$-[4-Hydroxyethylamino-2-[O-(2-O-methyl-β-L-fucosyl)-4-hydroxyphenylamino]-triazin-6-yl]-lysyl-D-alanyl}-batracylin, Trifluoracetat:**

Edukt: Kohlenhydrat aus Beispiel 1.1;
Ausb.: 78 %; FAB-MS: m/z = 854 = M+1;
[DC: Dichlormethan/Methanol/Ammoniak (17 %ig) 15:4:0,5
$R_f$ = 0,33].

**Beispiel 8.7**

**N-{N$^{\varepsilon}$-[4-Hydroxyethylamino-2-[O-(4-O-methyl-β-L-fucosyl)-4-hydroxyphenylamino]-triazin-6-yl]-lysyl-D-alanyl}-batracylin, Trifluoracetat:**

Edukt: Kohlenhydrat aus Beispiel 1.4;
Ausb.: 38 %;
[DC: Dichlormethan/Methanol/Ammoniak (17%ig) 15:4:0,5
$R_f$ = 0,4].

**Beispiel 8.8**

**N-{N$^{\varepsilon}$-[4-Hydroxyethylamino-2-[O-(3-desoxy-β-L-fucosyl)-4-hydroxyphenylamino]-triazin-6-yl]-lysyl-D-alanyl}-batracylin, Trifluoracetat:**

Edukt: Kohlenhydrat aus Beispiel 1.6;
Ausb.: 77 %; FAB-MS: m/z = 824 = M+1;
[DC: Dichlormethan/Methanol/Ammoniak (17 %ig) 15:4:0,5
$R_f$ = 0,37].

**Beispiel 8.9**

**N-{N$^{\varepsilon}$[4-Hydroxyethylamino-2-(O-(β-L-fucosyl)-4-hydroxy-phenylamino]- triazin-6-yl]-lysyl-D-alanyl}-batracylin, Trifluoracetat:**

Edukte: p-Aminophenyl-β-L-fucosid;
Ausb.: 52 %; FAB-MS: m/z = 840 = M+1;
[DC: Dichlormethan/Methanol/Ammoniak (17 %ig) 15:4:0,5
$R_f$ = 0,30].

**Beispiel 8.10**

**N-{N$^{\varepsilon}$-[4-Hydroxyethylamino-2-[O-(3-O-methyl-β-L-fucosyl)-4-hydroxyphenylamino]-triazin-6-yl]-lysyl-alanyl}-batracylin, Trifluoracetat:**

Edukt: Kohlenhydrat aus Beispiel 1.2;
Ausb.: 88 %;
[DC: Dichlormethan/Methanol/Ammoniak (17 %ig) 15:3:0,3
$R_f$ = 0,35].

**Beispiel 8.11**

**N-{N$^{\varepsilon}$-[4-Hydroxyethylamino-2-[O-(2-O-methyl-β-L-fucosyl)-4-hydroxyphenylamino]-triazin-6-yl]-lysyl-alanyl}-batracylin, Trifluoracetat:**

Edukt: Kohlenhydrat aus Beispiel 1.1;
Ausb.: 58 %;
[DC: Dichlormethan/Methanol/Ammoniak (17 %ig) 15:4:0,5
$R_f$ = 0,40].

**Beispiel 8.12**

**N-{N$^{\varepsilon}$-[4-Hydroxyethylamino-2-[O-(4-O-methyl-β-L-fucosyl)-4-hydroxyphenylamino]-triazin-6-yl]-lysyl-alanyl}-batracylin, Trifluoracetat:**

Edukt: Kohlenhydrat aus Beispiel 1.4;
Ausb.: 66 %;
FAB-MS: m/z = 854 = M+1;

[DC: Dichlormethan/Methanol/Ammoniak (17 %ig) 15:4:0,5
$R_f$ = 0,37] .

**Beispiel 9.1**

**N-[D-Alanyl]-chinolon-a, Trifluoracetat:**

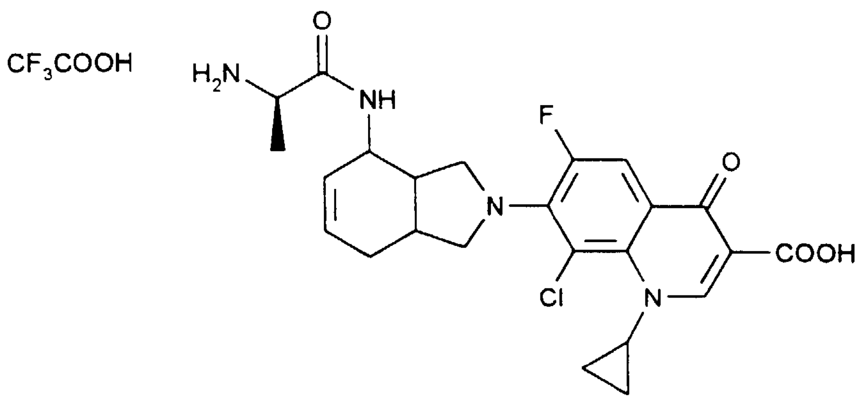

**9.1.a) N-[N-(tert-Butoxycarbonyl)-D-alanyl]-chinolon-a:**

N-(tert-Butoxycarbonyl)-D-alanin (3,6 g, 19,2 mmol) und 2-Isobutoxy-1-isobutoxycarbonyl-1,2-dihydro-chinolin (5,8 g, 19,2 mmol) werden in 200 ml Dimethylformamid gelöst. Nach 8 h Rühren bei Raumtemperatur setzt man Chinolon-a (4 g, 9,6 mmol) und 3,3 ml Ethyldiisopropylamin zu und behandelt den Ansatz 10 h mit Ultraschall. Man engt ein, nimmt den Rückstand in Dichlormethan auf und fällt mit Ether. Nach Filtration, Waschen mit Ether und Trocknen im Hochvakuum erhält man 4,58 g (81 %) vom Zielprodukt, welches ohne weitere Reinigung umgesetzt wird.

**9.1) N-[D-Alanyl]-chinolon-a, Trifluoracetat:**

4,56 g (7,75 mmol) der Verbindung aus Beispiel 9.1.a werden in 50 ml Dichlormethan und 50 ml wasserfreier Trifluoressigsäure bei 0°C gelöst und 1 h bei dieser Temperatur gerührt. Man engt ein, destilliert mit Dichlormethan nach und fällt aus Methanol mit Ether. Man erhält 4,07 g (87 %) des kristallinen Zielproduktes. [DC: Acetonitril/Wasser/Eisessig 5:1:0,2 $R_f$ = 0,34]

**Beispiel 9.2**

**N-[Alanyl]-chinolon-a, Trifluoracetat:**

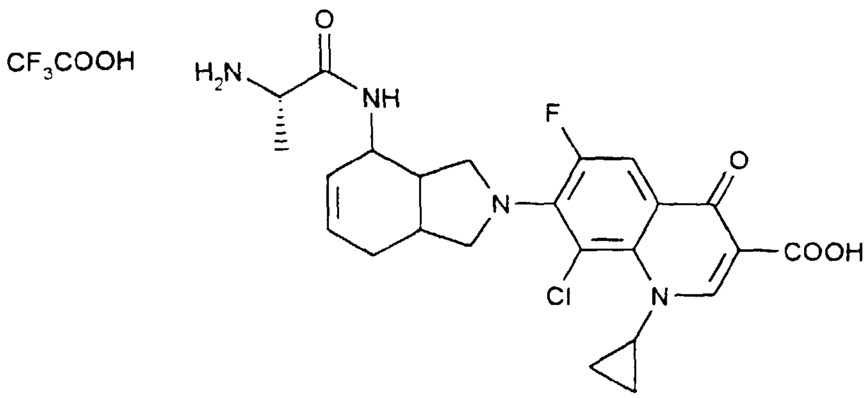

Synthese verläuft identisch mit der des Isomeren in Beispiel 9.1.

**Beispiel 9.3**

**N-[Lysyl-D-alanyl]-chinolon-a, Di-Trifluoracetat**

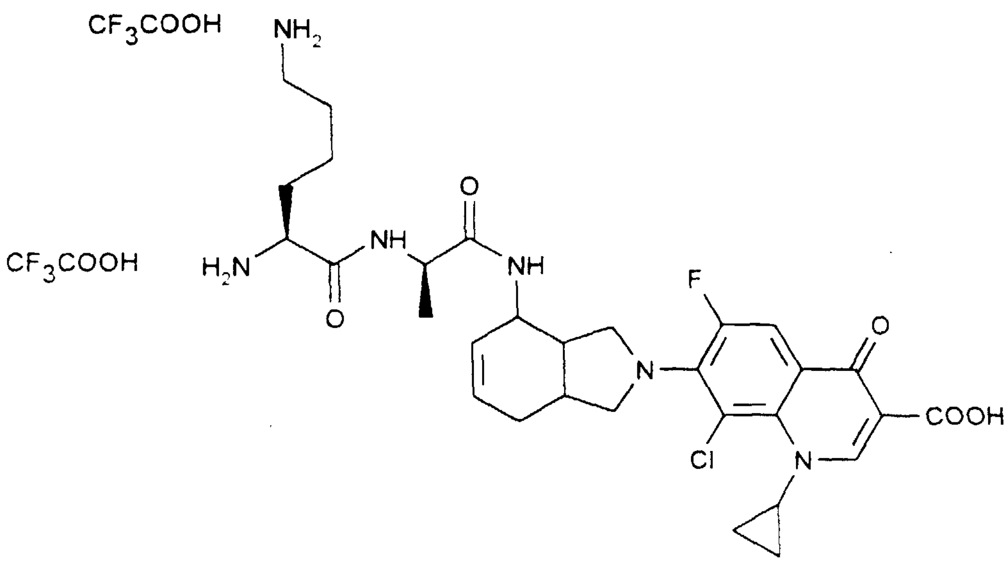

**9.3.a) N-[$N^{\alpha}$,$N^{\varepsilon}$-bis-(tert-Butoxycarbonyl)-lysyl-D-alanyl]-chinolon-a:**

341 mg (0,984 mmol) $N^{\alpha}$,$N^{\varepsilon}$-bis-(tert-Butoxycarbonyl)-lysin werden in 10 ml DMF gelöst und bei 0°C mit 200 mg (1,48 mmol) N-Hydroxybenzotriazol und 227 mg (1,18 mmol) N'-(3-Dimethylaminopropyl)-N-ethylcarbodiimid Hydrochlorid versetzt. Nach 3 h Rühren bei 10°C gibt man 432 mg (0,82 mmol) der Verbindung aus Beispiel 9.1 hinzu und rührt weitere 2 h bei 20°C. Man engt ein und verrührt den Rückstand dreimal mit 50 ml Wasser. Anschließend wird der Rückstand in Dichlormethan aufgenommen und über Natriumsulfat getrocknet. Die Fällung mit Ether führt zu 516 mg (78 %) des Zielproduktes. [DC: Acetonitril/Wasser 10:1 $R_f$ = 0,55].

**9.3) N-[Lysyl-D-alanyl]-chinolon-a, Di-Trifluoracetat:**

Deblockierung von 512 mg (0,63 mmol) der Verbindung aus Beispiel 9.3.a in Analogie zu Beispiel 9.1. Fällung aus Essigester mit Ether. Ausb.: 479 mg (90 %); [DC: Acetonitril/Wasser/Eisessig 10:3:1,5 $R_f$ = 0,3].

**Beispiel 9.4**

**N-[Lysyl-alanyl]-chinolon-a, Di-Trifluoracetat:**

Synthese verläuft identisch mit der des Isomeren in Beispiel 9.3.

**Beispiel 9.5**

**N-(N$^{\alpha}$-(tert-Butoxycarbonyl)-lysyl-D-alanyl]-chinolon-a**

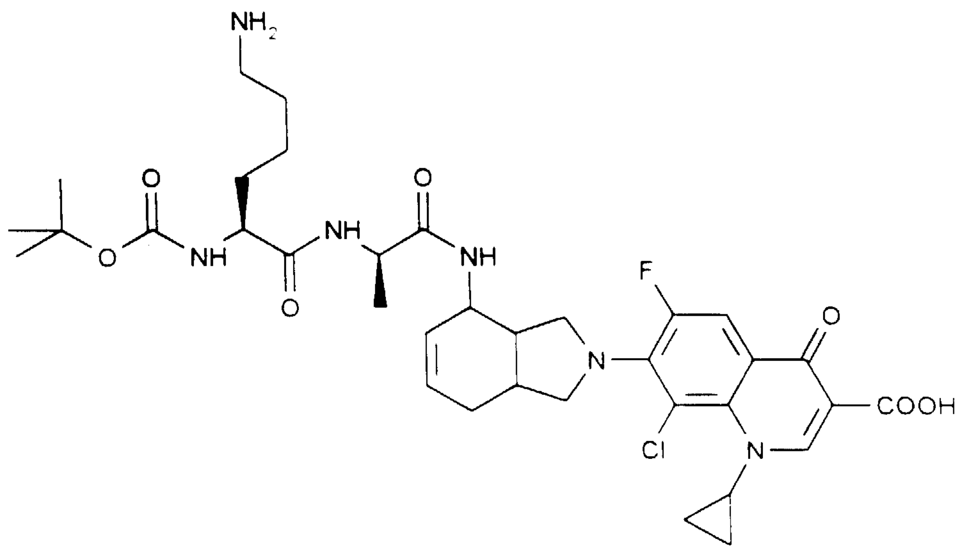

**9.5.a) N-[N$^{\alpha}$-(tert-Butoxycarbonyl)-N$^{\varepsilon}$-(fluorenyl-9-methoxycarbonyl)-lysyl-D-alanyl]-chinolon-a:**

1,57 g (3,36 mmol) N$^{\alpha}$-(tert-Butoxy-carbonyl)-N$^{\varepsilon}$-(fluorenyl-9-methoxycarbonyl)-lysin werden in 25 ml Dimethylformamid gelöst und bei 0°C mit 600 mg (5,04 mmol) N-Hydroxysuccinimid und 820 mg (4,03 mmol) N,N'-Dicyclohexylcarbodiimid versetzt. Nach 3 h filtriert man den entstandenen Harnstoff ab, gibt zum Filtrat 1,5 g (2,86 mmol) der Verbindung aus Beispiel 9.1 und rührt 16 h bei Raumtemperatur. Restlicher Harnstoff wird abfiltriert und das Filtrat durch Flash-Chromatographie [Dichlormethan/Methanol 97,5:2,5; später im gleichen System 90:10] gereinigt. Anschließend fällt man aus Dichlormethan/Methanol 1:1 mit Ether. Ausb.: 1,5 g (56 %) [DC: Dichlormethan/ Methanol 9: 1 $R_f$ 0,47].

**9.5) N-[N$^{\alpha}$-(tert-Butoxycarbonyl)-lysyl-D-alanyl]-chinolon-a:**

Fmoc-Abspaltung aus Beispiel 9.5a) mit Piperidin in Dimethylformamid. Fällung des Rohproduktes aus Dimethylformamid mit Ether. Ausb.: 72 % [DC: Acetonitril/Wasser/Eisessig 5:1:0,2 $R_f$ = 0,43]

**Beispiel 9.6**

**N-[N$^{\varepsilon}$-(fluorenyl-9-methoxycarbonyl)-lysyl-D-alanyl]-chinolon-a, Trifluoracetat**

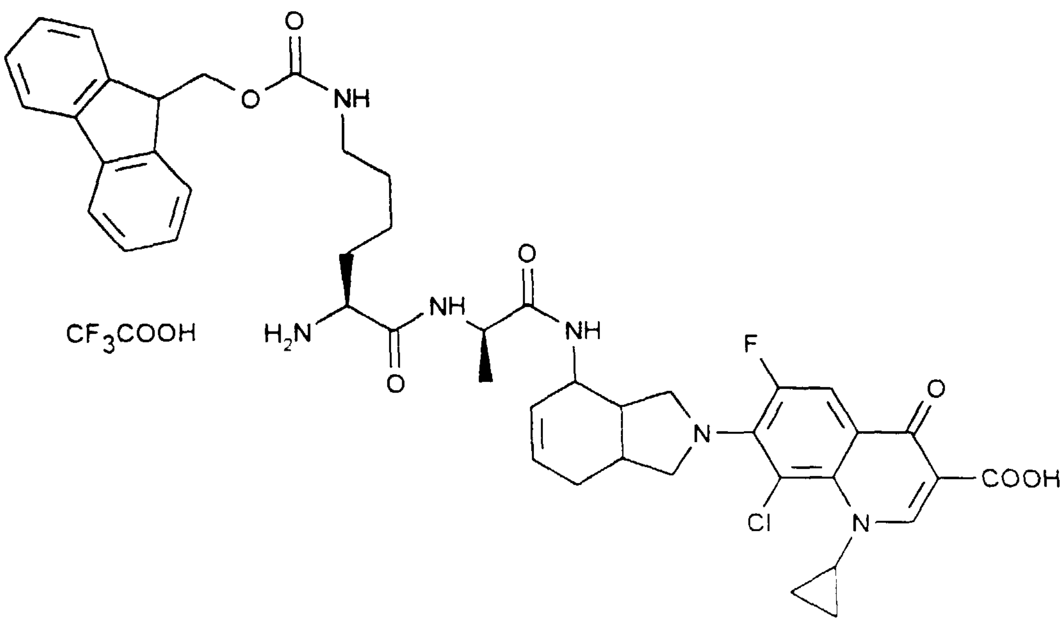

Boc-Abspaltung aus Beispiel 9.5.a) in Analogie zu Beispiel 9.1. Fällung des Rohproduktes aus Methanol/Ether. Ausb.: 80 % [DC: Dichlormethan/Methanol/ Ammoniak (17 %ig) 15:4:0,5 $R_f$ = 0,36].

**Beispiel 9.7**

**N-[N$^{\alpha}$-(tert-Butoxycarbonyl)-lysyl-alanyl]-chinolon-a:**

Synthese verläuft identisch mit der des Isomeren in Beispiel 9.5.

**Beispiel 9.8**

**N-[Lysyl]-chinolon-a, Di-Trifluoracetat**

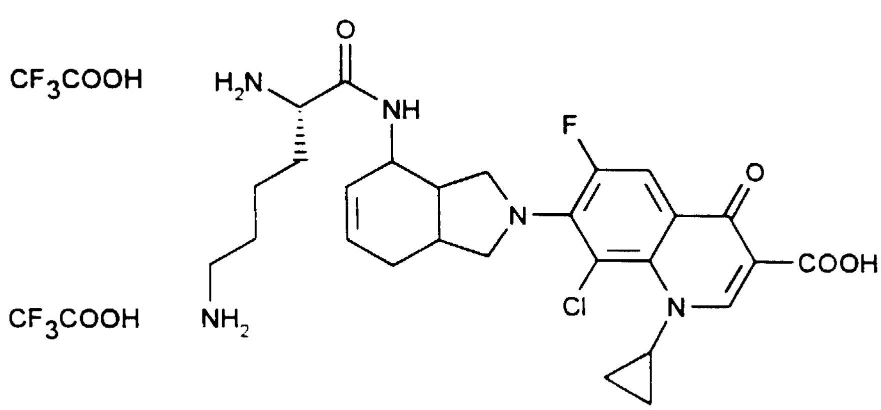

**9.8.a) N-[$N^{\alpha}$,$N^{\varepsilon}$-bis-(tert-Butoxycarbonyl)-lysyl]-chinolon-a:**

1317 mg (3,8 mmol) $N^{\alpha}$,$N^{\varepsilon}$-bis-(tert-Butoxycarbonyl)-lysin werden nach der Vorschrift in Beispiel 9.1.a mit Chinolon-a verknüpft. Die Reinigung erfolgt durch Flashchromatographie [Dichlormethan/Methanol/Ammoniak (17%ig) 15:1:0,1]. Man erhält 1010 mg (71 %) des Zielproduktes.

**9.8) N-[Lysyl]-chinolon-a, Di-Trifluoracetat:**

1005 mg (1,347 mmol) der Verbindung aus Beispiel 9.8.a werden analog zu Beispiel 9.1 deblockiert. Nach Fällung aus Dichlormethan/Methanol 1:1 mit Ether erhält man 966 mg (93 %) des kristallinen Zielproduktes. [DC: Acetonitril/Wasser/Eisessig 10:5:3 $R_f$ = 0,33].

__Beispiel 9.9__

**N-[D-Lysyl]-chinolon-a, Di-Trifluoracetat:**

Synthese verläuft identisch mit der des Isomeren in Beispiel 9.8.

__Beispiel 9.10__

**N-[$N^{\alpha}$-(tert-Butoxycarbonyl)-lysyl]-chinolon-a**

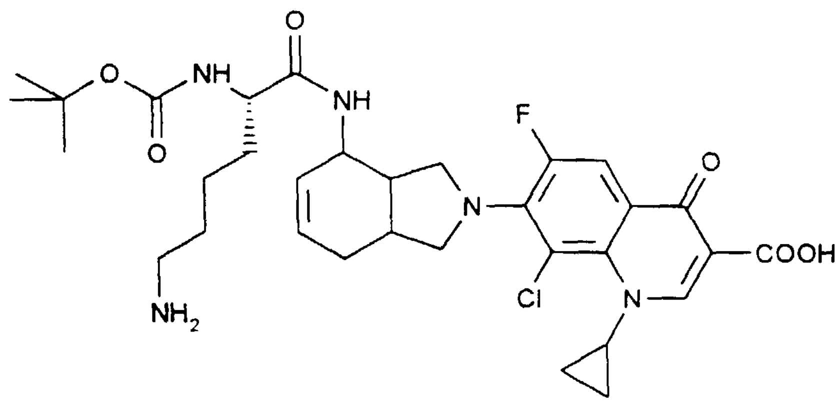

**9.10.a) N-[$N^{\alpha}$-(tert-Butoxycarbonyl)-$N^{\varepsilon}$-(fluorenyl-9-methoxycarbonyl)-lysyl]-chinolon-a:**

1350 mg (2,88 mmol) $N^{\alpha}$-(tert-Butoxycarbonyl)-$N^{\varepsilon}$-(fluorenyl-9-methoxycarbonyl)-lysin werden nach der Vorschrift in Beispiel in 9.8.a mit Chinolon-a verknüpft. Die Reinigung erfolgt durch Flashchromatographie [Dichlormethan/Methanol/Ammoniak (17 %ig) 15:1:0,1; später im gleichen System 15:2.0,2]. Man erhält 1025 mg (82 %) des Zielproduktes.

**9.10) N-[$N^{\alpha}$-(tert-Butoxycarbonyl)-lysyl]-chinolon-a:**

Fmoc-Abspaltung aus Beispiel 9.10.a in Analogie zu Beispiel 9.5. Zweimalige Fällung des Rohproduktes aus Methanol mit Ether. Ausb.: 86% [DC: Acetonitril/Wasser/ Eisessig 5:1:0,2 $R_f$ = 0,48].

**Beispiel 9.11**

**N-[N$^{\varepsilon}$-(fluorenyl-9-methoxycarbonyl)-lysyl]-chinolon-a, Trifluoracetat:**

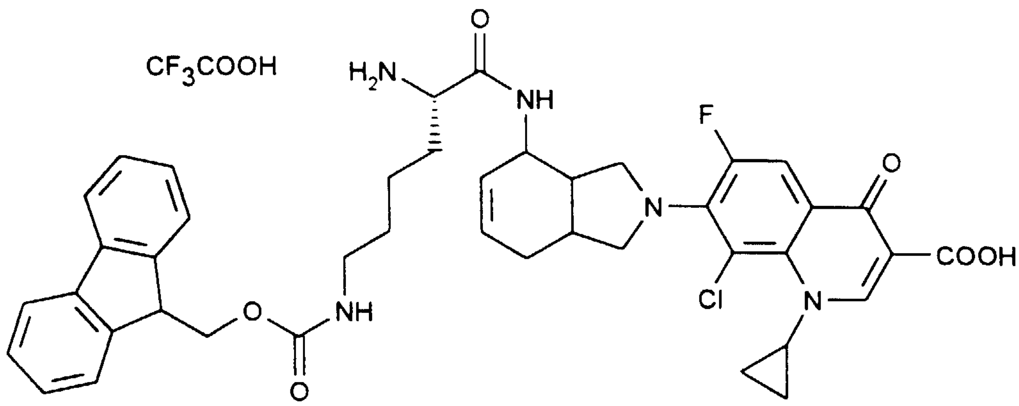

Boc-Abspaltung aus Beispiel 9.10.a in Analogie zu Beispiel 9.1. Zweimalige Fällung des Rohproduktes aus Methanol/Ether. Gefriertrocknung aus Dioxan/Wasser. Ausb.: 92 % [DC: Dichlormethan/Methanol/Ammoniak (17 %ig) 15:4:0,5 $R_f$ = 0,44].

**Beispiele 10.1- 10.3**

**Allgemeine Formel**

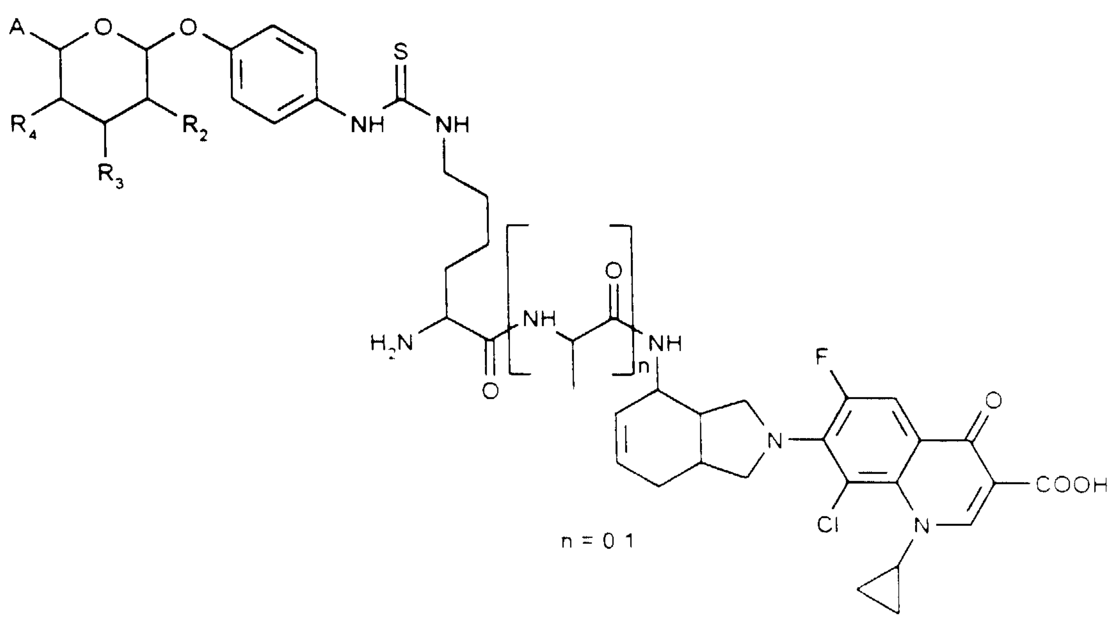

**Beispiel 10.1**

**N-{N$^{\varepsilon}$-[O-(3-O-Carboxymethyl-β-L-fucosyl)-4-hydroxy-phenylaminothiocarbonyl]-lysyl-D-alanyl}-chinolon-a:**

**10.1.a) N-{N$^{\alpha}$-(tert-Butoxycarbonyl)-N$^{\varepsilon}$-[O-(3-O-carboxymethyl-β-L-fucosyl)-4-hydroxy-phenylamino-thiocarbonyl]-lysyl-D-alanyl}-chinolon-a:**

78 mg (0,25 mmol) p-Aminophenyl-3-O-carboxymethyl-β-L-fucosid (Beispiel 1.10) werden in 15 ml Dioxan/Wasser 1:1 unter Rühren mit 47 µl (0,28 mmol) Thiophosgen versetzt. Nach 10 min Rühren bei 20°C engt man ein und trocknet 1h im Hochvakuum. Das erhaltene Senföl wird anschließend in absolutem Dimethylformamid mit 180 mg (0,25 mmol) N-[N$^{\alpha}$-(tert-Butoxycarbonyl)-lysyl-D-alanyl]-chinolon-a (Beispiel 9.5) in Gegenwart von 86 µl Ethyldiisopropylamin gekuppelt. Nach zweimaliger Fällung des Rohproduktes aus Dichlormethan/Ether, anschließendem Verrühren mit Wasser und Gefriertrocknung aus Dioxan/Wasser erhält man 210 mg (78 %) des Zielproduktes. [DC: Acetonitril/Wasser/Eisessig 5:1:0,2 $R_f$ = 0,62].

**10.1) N-{N$^{\varepsilon}$-[O-(3-O-Carboxymethyl-β-L-fucosyl)-4-hydroxy-phenylaminothiocarbonyl]-lysyl-D-alanyl}-chinolon-a:**

208 mg (0,193 mmol) der Verbindung aus Beispiel 10.1.a werden in 10 ml Dichlormethan mit 10 ml wasserfreier Trifluoressigsäure 1 h bei 0°C gerührt Man engt ein, destilliert mit 15 ml Methanol nach und chromatographiert mit Dichlormethan/Methanol/Ammoniak (17 %ig) 10:10:0,8 Nach anschließender Fällung aus Dimethylformamid mit Ether erhält man 52 mg (28 %) des Zielproduktes. [DC: Acetonitril/Wasser/Eisessig 5:1:0,2 $R_f$ = 0,53].

**In Analogie zu Beispiel 10.1 werden aus den teilgeschützten Peptidkonjugaten in Beispiel 9.5, 9.7 bzw. 9.10 folgende Glycokonjugate hergestellt:**

**Beispiel 10.2**

**N-{N$^{\varepsilon}$-[O-(3-O-Methyl-β-L-fucosyl)-4-hydroxy-phenylamino-thiocarbonyl]-lysyl-alanyl}-chinolon-a:**

Edukte: Kohlenhydrat aus Beispiel 1.2; Peptidkonjugat aus Beispiel 9.7
Reinigung der Zwischenstufe durch mehrmalige Fällung aus Methanol mit Ether. Flashchromatographische Reinigung der Endstufe mit Dichlormethan/Methanol/Ammoniak (17 %ig) 15:4:0,5; später im gleichen System mit 15:8:0,8. Ausb.: 20% [DC: Dichlormethan/Methanol/Ammoniak (17 %ig) 15:8:0,8 $R_f$ = 0,15].

**Beispiel 10.3**

**N-{N$^{\varepsilon}$-[O-(3-O-Methyl-β-L-fucosyl)-4-hydroxy-phenylamino-thiocarbonyl]-lysyl}-chinolon-a:**

Edukte: Kohlenhydrat aus Beispiel 1.2; Aminosäurekonjugat aus Beispiel 9.10
Reinigung der Zwischenstufe durch Fällung aus Methanol mit Ether. Flashchromatographische Reinigung der Endstufe mit Dichlormethan/Methanol/Ammoniak (17 %ig) 15:8:0,8. Ausb.: 39 %; [DC: Acetonitril/Wasser/Eisessig 5:1:0,2 $R_f$ = 0,33].

**Beispiele 11.1-11.18**

**Allgemeine Formel**

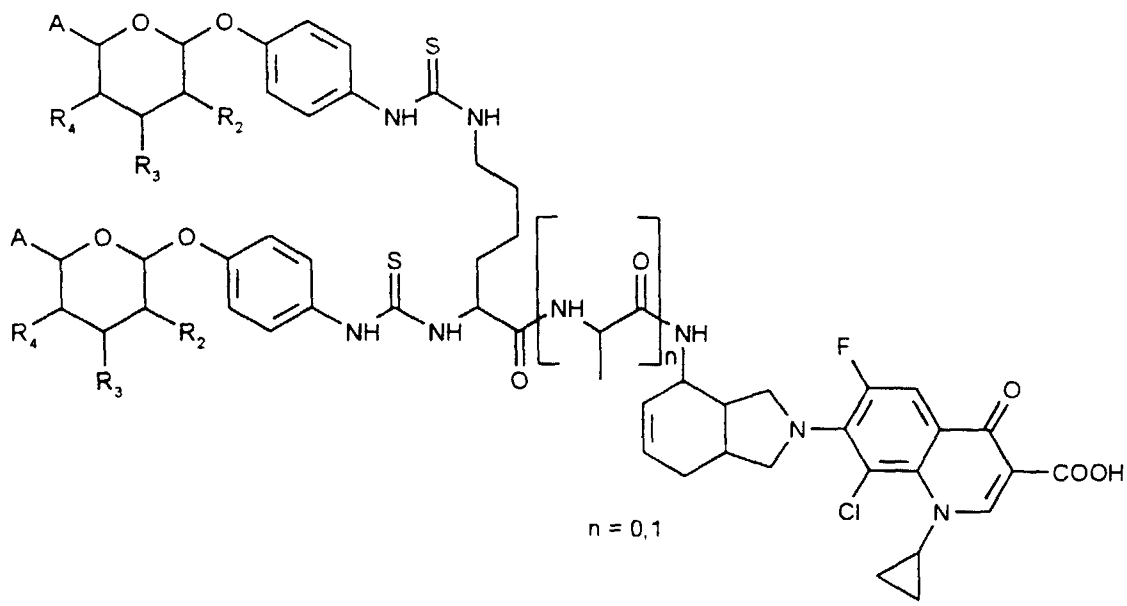

n = 0,1

**Beispiel 11.1**

**N-(N$^{\alpha}$,N$^{\varepsilon}$-bis-[O-(3-O-Methyl-β-L-fucosyl)-4-hydroxy-phenylaminothiocarbonyl]-lysyl-D-alanyl}-chinolon-a:**

50 mg (0,19 mmol) p-Aminophenyl-3-O-methyl-β-L-fucosid (Beispiel 1.2) werden nach der Vorschrift in Beispiel 10.1.a zunächst ins Senföl überführt und anschließend in 5 ml Dimethylformamid mit 68 mg (0,08 mmol) N-[Lysyl-D-alanyl]-chinolon-a, Di-Trifluoracetat (Beispiel 9.3) in Gegenwart von 55 µl Ethyldiisopropylamin gekuppelt. Man rührt 16 h bei Raumtemperatur, engt ein und reinigt durch Flash-Chromatographie [Dichlormethan/Methanol/Eisessig 85: 15:1,5] Anschließend erhält man durch Fällung aus Methanol mit Ether 62 mg (63 %) des Zielproduktes. [DC: Dichlormethan/Methanol/Eisessig 80:20:2 $R_f$ = 0,5] MS-MALDI: m/z = 1242 = M+1.

**In Analogie zu Beispiel 11.1 werden aus den Peptid-Konjugaten in den Beispielen 9.3, 9.4, 9.8 bzw. 9.9 folgende Glycokonjugate hergestellt:**

**Beispiel 11.2**

**N-{N$^{\alpha}$,N$^{\varepsilon}$-bis-[O-(3-O-Methyl-β-L-fucosyl)-4-hydroxy-phenylaminothiocarbonyl]-lysyl-alanyl}-chinolon-a:**

Edukte: 50 mg (0,19 mmol) Kohlenhydrat aus Beispiel 1.2; 0,08 mmol Peptidkonjugat aus Beispiel 9.4 Reinigung durch Flashchromatographie [Dichlormethan/Methanol/Eisessig 90:10:1] und Fällung aus Methanol mit Ether. Ausb.: 79 %. [DC: Acetonitril/Wasser/ Eisessig 5:1:0,2 $R_f$ = 0,42].

**Beispiel 11.3**

**N-{N$^{\alpha}$,N$^{\varepsilon}$-bis-[O-(3-O-Carboxymethyl-β-L-fucosyl)-4-hydroxy-phenylaminothiocarbonyl]-lysyl-alanyl}-chinolon-a:**

Edukte: 52 mg (0,166 mmol) Kohlenhydrat aus Beispiel 1.10; 0,07 mmol Peptidkonjugat aus Beispiel 9.4 Reinigung durch Flashchromatographie [Dichlormethan/Methanol/Eisessig 80:20:2] und Verrühren des Rückstandes mit Methanol. [DC: Acetonitril/Wasser/Eisessig 10:3:1,5 $R_f$ = 0,62].

**Beispiel 11.4**

**N-{$N^{\alpha}$,$N^{\varepsilon}$-bis-[O-(3-O-Carboxymethyl-β-L-fucosyl)-4-hydroxy-phenylaminothiocarbonyl]-lysyl-D-alanyl}-chinolon-a:**

Edukte. 44 mg (0,14 mmol) Kohlenhydrat aus Beispiel 1.10; 0,06 mmol Peptidkonjugat 9.3
Reinigung durch Flashchromatographie [Dichlormethan/Methanol/Eisessig 80:20:2] und Verrühren des Rückstandes mit Methanol. Ausb.: 57 %; [DC: Acetonitril/Wasser/Eisessig 10:3:1,5 $R_f$ = 0,62]

**Beispiel 11.5**

**N-{$N^{\alpha}$,$N^{\varepsilon}$-bis-[O-(α-L-Rhamnosyl)-4-hydroxy-phenylamino-thiocarbonyl]-lysyl-alanyl}-chinolon-a:**

Edukte: 44 mg (0,166 mmol) Kohlenhydrat aus Beispiel 1.21; 0,07 mmol Peptidkonjugat aus Beispiel 9.4
Reinigung durch Flashchromatographie [Dichlormethan/Methanol/Eisessig 80:20:1] und Verrühren des Rückstandes mit Methanol/Ether. Ausb.: 90 mg (89 %); [DC: Acetonitril/Wasser/Eisessig 5:1:0,2 $R_f$ = 0,51] MS-ESI: m/z = 1212 = M+1.

**Beispiel 11.6**

**N-{$N^{\alpha}$,$N^{\varepsilon}$-bis-[O-(3-O-Methyl-β-L-fucosyl)-4-hydroxy-phenylaminothiocarbonyl]-lysyl}-chinolon-a:**

Edukte: 70 mg (0,258 mmol) Kohlenhydrat aus Beispiel 1.2; 0,11 mmol Aminosäurekonjugat aus Beispiel 9.8
Reinigung durch Flashchromatographie [Dichlormethan/Methanol/Eisessig 90:10:1] und Fällung aus Dichlormethan/ Methanol 1:1 mit Ether. Verrühren des Rückstandes mit Wasser. Ausb.: 41 %. [DC: Acetonitril/Wasser/Eisessig 5:1: 0,2 $R_f$ = 0,65].

**Beispiel 11.7**

**N-{$N^{\alpha}$,$N^{\varepsilon}$-bis-[O-(3-O-Methyl-β-L-fucosyl)-4-hydroxy-phenylaminothiocarbonyl]-D-lysyl}-chinolon-a:**

Edukte: 50 mg (0,19 mmol) Kohlenhydrat aus Beispiel 1.2; 0,08 mmol Aminosäurekonjugat aus Beispiel 9.9
Reinigung durch Flashchromatographie [Dichlormethan/Methanol/Eisessig 90:10:1] und Fällung aus Dichlormethan/ Methanol 1:1 mit Ether. Verrühren des Rückstandes mit Wasser. Ausb.: 67 %. [DC: Acetonitril/Wasser/Eisessig 5:1: 0,2 $R_f$ = 0,65] MS-FAB: m/z = 1169 = M+1.

**Beispiel 11.8**

**N-{$N^{\alpha}$,$N^{\varepsilon}$-bis-[O-(3-O-Carboxymethyl-β-L-fucosyl)-4-hydroxy-phenylaminothiocarbonyl]-lysyl}-chinolon-a, Di-Natriumsalz:**

Edukte: 50 mg (0,16 mmol) Kohlenhydrat aus Beispiel 1.10; 0,07 mmol Aminosäurekonjugat aus Beispiel 9.8
Nach Einengen des Reaktionsansatzes, Aufnehmen in 10 ml Dimethylformamid und Zugabe von 8ml einer 0,1N Natronlauge wird 2 h bei 20°C gerührt. Nach erneutem Einengen nimmt man in Wasser auf und stellt den pH-Wert auf 5 ein. Man lyophilisiert und digeriert mit Methanol und anschließend mit Methanol/Ether. So erhält man 68 mg (65 %) der Zielverbindung. [DC: Acetonitril/Wasser/Eisessig 5:1:0,2 $R_f$ = 0,26].

**Beispiel 11.9**

**N-{$N^{\alpha}$,$N^{\varepsilon}$-bis-[O-(3-O-Carboxymethyl-β-L-fucosyl)-4-hydroxy-phenylaminothio-carbonyl)-D-lysyl}-chinolon-a, Di-Natriumsalz:**

Edukte: 100 mg (0,32 mmol) Kohlenhydrat aus Beispiel 1.10; 0,13 mmol Aminosäurekonjugat aus Beispiel 12.9
Nach Einengen des Reaktionsansatzes, Aufnehmen in 10 ml Dimethylformamid und Zugabe von 16 ml einer 0,1N

Natronlauge wird 2 h bei 20°C gerührt. Nach erneutem Einengen nimmt man in Wasser auf und stellt den pH-Wert auf 5 ein. Man lyophilisiert und digeriert mit Methanol und anschließend mit Methanol/Ether. So erhält man 160 mg (77 %) der Zielverbindung. Schmp.: 218-220°C [DC: Acetonitril/Wasser/Eisessig 10:3:1,5 $R_f$ = 0,69].

**Beispiel 11.10**

**N-{$N^{\alpha}$,$N^{\varepsilon}$-bis-[O-(3-O-Methyl-α-L-fucosyl)-4-hydroxy-phenylaminothiocarbonyl]-D-lysyl}-chinolon-a:**

Edukte 50 mg (0,19 mmol) Kohlenhvdrat aus Beispiel 1.3;
0,08 mmol Aminosäurekonjugat aus Beispiel 9.9 Darstellung analog zu Beispiel 11.7. Reinigung durch Flashchromatographie [Dichlormethan/Methanol/Ammoniak (17 %) 10:10:1] und anschließende Fällung aus Dichlormethan/Methanol 1:1 mit Ether. Ausb.: 66 %. [DC: Acetonitril/Wasser/Eisessig 5:1:0,2: $R_f$ = 0,62].

**Beispiel 11.11**

**N-{$N^{\alpha}$,$N^{\varepsilon}$-bis-[O-(α-L-rhamnosyl)-4-hydroxy-phenylamino-thiocarbonyl]-D-lysyl}-chinolon-a:**

Edukte: 50 mg (0,19 mmol) Kohlenhydrat aus Beispiel 1.21; 0,08 mmol Aminosäurekonjugat aus Beispiel 9.9
Darstellung analog zu Beispiel 11.7. Ausb.: 57 %. [DC: Acetonitril/Wasser/Eisessig 5:1:0,2: $R_f$ = 0,63].

**Beispiel 11.12**

**N-{$N^{\alpha}$,$N^{\varepsilon}$-bis-[O-(3-O-Methyl-β-L-fucosyl)-4-hydroxy-phenylaminothiocarbonyl]-D-lysyl}-chinolon-a, Natriumsalz:**

58mg (0,05mmol) der Verbindung aus Beispiel 11.7 werden in Wasser suspendiert und mit einem Äquivalent einer 0,1N Natronlauge ins Natriumsalz überführt. Nach Gefriertrocknung erhält man 60mg der Zielverbindung.

**Beispiel 11.13**

**N-{$N^{\alpha}$,$N^{\varepsilon}$-bis-[O-(3-O-Methyl-b-D-galactopyranosyl)-4-hydroxy-phenylaminothiocarbonyl]-lysyl}-chinolon-a, Natriumsalz:**

Eine Lösung von Verbindung 1.25 (62,8 mg, 0,22 mmol) in Dioxan/Wasser 1:1 (10 ml) wird unter Rühren mit Thiophosgen (33,5 ml, 0,44 mmol) versetzt. Nach 10 min. engt man im Vakuum ein und trocknet den Ruckstand für 1 h im Ölpumpenvakuum. Das erhaltene Isothiocyanat wird in absolutem Dimethylformamid (10 ml) gelöst und mit Verbindung 9.8 (77,4 mg, 0,1 mmol) und mit Ethyldiisopropylamin (0,5 ml) versetzt. Man rührt für 16 h bei Raumtemperatur, engt dann im Vakuum ein und reinigt durch Flashchromatographie [Dichlormethan/Methanol/Ammoniak (25 %) 10:10: 1 → Methanol/Ammoniak (25 %) 20:1] Man erhält gelbe Kristalle, die in Wasser (10 ml) suspendiert werden Die Suspension wird unter Rühren tropfenweise mit 0,05N-Natronlauge versetzt, bis eine klare Lösung entsteht (pH < 10). Durch Lyophilisieren der filtrierten Lösung erhält man einen gelben amorphen Feststoff (39,7 mg, 32 %); $[a]_D^{20}$ = +25,8° (c = 0,26 / $H_2O$).

**Beispiel 11.14**

**N-{$N^{\alpha}$,$N^{\varepsilon}$-bis-[O-(4-O-(3'-O-Methyl-b-D-galactopyranosyl)-b-D-glucopyranosyl)-4-hydroxy-phenylamino-thiocarbonyl]-lysyl}-chinolon-a, Natriumsalz:**

Verbindung 1.58 (98,4 mg, 0,22 mmol) wird wie in Beispiel 11.13 beschrieben mit dem Peptidkonjugat 9.8 (77,4 mg, 0,1 mmol) umgesetzt und gereinigt. Man erhält einen gelben amorphen Feststoff (62,5 mg, 40 %); $[a]_D^{20}$ = +12,9° (c = 0,26 / $H_2O$).

**Beispiel 11.15**

**N-{N$^{\alpha}$,N$^{\varepsilon}$-bis-[O-(2-O-Methyl-4-O-(3'-O-methyl-b-D-galactopyranosyl)-b-D-glucopyranosyl)-4-hydroxy-phenyl-amino-thiocarbonyl]-lysyl}-chinolon-a, Natriumsalz:**

Verbindung 1.59 (101,5 mg, 0.22 mmol) wird wie in Beispiel 11.13 beschrieben mit dem Peptidkonjugat 9.8 (77,4 mg, 0,1 mmol) umgesetzt. Reinigung durch Umfallen aus Methanol/Dichlormethan 1:1 mit Diethylether und Auskochen mit Ethanol ergibt gelbe Kristalle, die wie beschrieben ins Natriumsalz überführt werden. Man erhält einen gelben amorphen Feststoff (51,1 mg, 32 %); $[a]_D^{20}$ = +27,9° (c = 0,24 / $H_2O$).

**Beispiel 11.16**

**N-{N$^{\alpha}$,N$^{\varepsilon}$-bis-[O-(3-O-Methyl-b-D-galactopyranosyl)-4-hydroxy-phenylaminothiocarbonyl]-D-lysyl}-chinolon-a, Natriumsalz:**

Verbindung 1.25 (62,8 mg, 0,22 mmol) wird wie in Beispiel 11.13 beschrieben mit dem Peptidkonjugat 9.9 (77,4 mg. 0,1 mmol) umgesetzt und gereinigt Man erhält einen gelben amorphen Feststoff (77,3 mg, 63 %); $[a]_D^{20}$ = -23,8° (c = 0,63 / $H_2O$).

**Beispiel 11.17**

**N-{N$^{\alpha}$,N$^{\varepsilon}$-bis-[O-(3-O-Methyl-a-D-mannopyranosyl)-4-hydroxy-phenylaminothiocarbonyl]-D-lysyl}-chinolon-a, Natriumsalz:**

Verbindung 1.40 (62,8 mg, 0,22 mmol) wird wie in Beispiel 11.13 beschrieben mit dem Peptidkonjugat 9.9 (77,4 mg, 0,1 mmol) umgesetzt und gereinigt. Man erhält einen gelben amorphen Feststoff (33,6 mg, 27 %); $[a]_D^{20}$ = +0,7° (c = 0,28 / $H_2O$).

**Beispiel 11.18**

**N-{N$^{\alpha}$,N$^{\varepsilon}$-bis-[O-(4-O-(3'-O-Methyl-b-D-galactopyranosyl)-b-D-glucopyranosyl)-4-hydroxy-phenylamino-thio-carbonyl]-D-lysyl}-chinolon-a, Natriumsalz:**

Verbindung 1.58 (98,4 mg, 0,22 mmol) wird wie in Beispiel 11.13 beschrieben mit dem Peptidkonjugat 9.9 (77,4 mg, 0,1 mmol) umgesetzt und gereinigt. Man erhält einen gelben amorphen Feststoff (63,0 mg, 41 %); $[a]_D^{20}$ = -21,8° (c = 0,22 / $H_2O$).

**Beispiele 12.1 - 12.15**

**Allgemeine Formel**

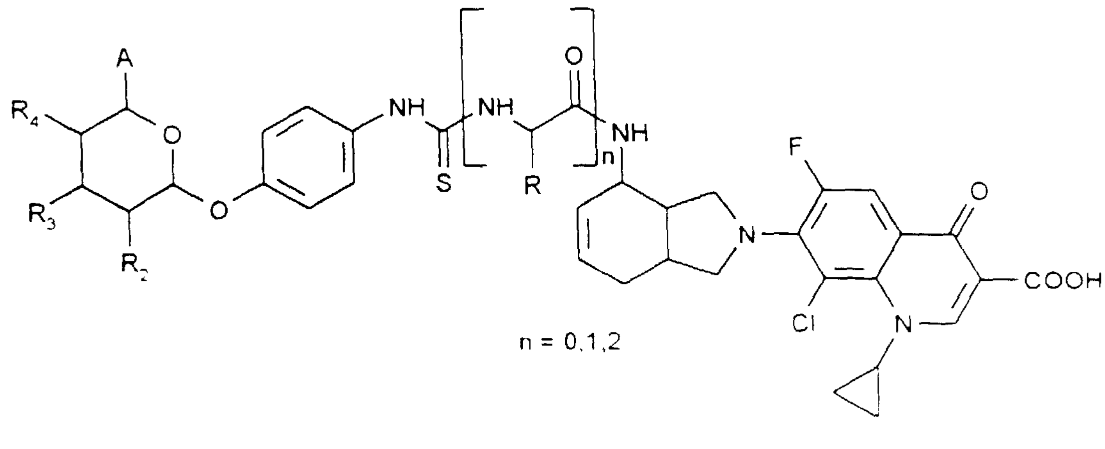

**Beispiel 12.1**

**N-{N'-[O-(3-O-Methyl-β-L-fucosyl)-4-hydroxy-phenylamino-thiocarbonyl]-D-alanyl}-chinolon-a:**

447 mg (1,66 mmol) p-Aminophenyl-3-O-methyl-β-L-fucosid (Beispiel 1.2) werden nach der Vorschrift in Beispiel 10.1.a zunächst ins Senföl überführt und anschließend in 40 ml Dimethylformamid mit 1 g (1,66 mmol) N-[D-Alanyl]-chinolon-a, Trifluoracetat (Beispiel 9.1) in Gegenwart von 568 µl Ethyldiisopropylamin gekuppelt. Man rührt 2 h bei Raumtemperatur, engt ein und reinigt durch mehrfache Fällung aus Dichlormethan/Methanol 1:1 mit Ether. Anschließend verrührt man den Filterrückstand noch zweimal mit Wasser. Man erhält 876 mg (66 %) des Zielproduktes. Schmp.: 198°C; [DC: Acetonitril/Wasser/Eisessig 5:1:0,2 $R_f$ = 0,63].

**In Analogie zu Beispiel 12.1 werden aus den Aminosäure-Konjugaten in den Beispielen 9.1 bzw. 9.2 folgende Glycokonjugate hergestellt:**

**Beispiel 12.2**

**N-{N'-[O-(3-O-Methyl-β-L-fucosyl)-4-hydroxy-phenylamino-thiocarbonyl]-alanyl}-chinolon-a:**

Edukt: 25 mg (0,092 mmol) Kohlenhydrat aus Beispiel 1.2
Reinigung durch Flashchromatographie [Dichlormethan/Methanol/Eisessig 90:10:1], Fällung aus Dichlormethan/Methanol 1:1 mit Ether und Verrühren des Filterrückstandes mit Wasser. Ausb.: 53 mg (52%) [DC: Acetonitril/Wasser/Eisessig 5:1:0,2 $R_f$ = 0,65].

**Beispiel 12.3**

**N-{N'-[O-(3-O-Carboxymethyl-β-L-fucosyl)-4-hydroxy-phenylaminothiocarbonyl]-D-alanyl}-chinolon-a, Mono-Natriumsalz:**

Edukte: 523 mg (1,67 mmol) Kohlenhydrat aus Beispiel 1.10;
Edukt: 840 mg (1,39 mmol) der Verbindung aus Beispiel 12,1. Nach 6 h Reaktionszeit wird eingeengt und mit Wasser verrührt. Flashchromatographie [Dichlormethan/Methanol/Ammoniak (17 %ig) 15:8:0,8; später im gleichen System 10: 1:1] schließt sich eine Gefriertrocknung aus Dioxan/Wasser an. Anschließend nimmt man in Wasser auf und setzt ein Äquivalent einer 0,1N Natronlauge zu und lyophilisiert erneut. Ausb.: 525 mg (45%). [DC: Acetonitril/Wasser/Eisessig 5:1:0,2 $R_f$ = 0,39].

**Beispiel 12.4**

**N-{N'-(O-(α-L-Rhamnosyl)-4-hydroxy-phenylamino-thiocarbonyl]-D-alanyl}-chinolon-a:**

Edukt: 20 mg (0,076 mmol) Kohlenhydrat aus Beispiel 1.21
Reinigung durch Flashchromatographie [Dichlormethan/Methanol/Eisessig 90:10:1] und Fällung aus Methanol mit Ether. Ausb.: 20mg (34%). [DC: Acetonitril/Wasser/Eisessig 5:1:0,2 $R_f$ = 0,42].

**Aus teilgeschützten N-(Lysyl)-chinolon-a-Konjugaten bzw. N-(lysyl-D-alanyl)-chinolon-a-Konjugaten werden folgende Glycokonjugate hergestellt:**

**Beispiel 12.5**

**N-{$N^{\alpha}$-[O-(3-O-Methyl-β-L-fucosyl)-4-hydroxy-phenylamino-thiocarbonyl)-lysyl}-chinolon-a:**

**12.5.a) N-{$N^{\alpha}$-[O-(3-O-Methyl-β-L-fucosyl)-4-hydroxy-phenylaminothiocarbonyl]-$N^{\varepsilon}$-[fluorerlyl-9-methoxycarbonyl)-lysyl}-chirlolon-a:**

92 mg (0,34 mmol) p-Aminophenyl-3-O-methyl-β-L-fucosid (Beispiel 1.2) werden nach der Vorschrift in Beispiel 10.1.a zunächst ins Senföl überführt und anschließend in 20 ml Dimethylformamid mit 300 mg (0,34mmol) N-[$N^{\varepsilon}$-(Fluorenyl-9-methoxycarbonyl)-lysyl]-chinolon-a, Trifluoracetat (Beispiel 9.11) in Gegenwart von 116 µl Ethyldiisopropylamin gekuppelt. Man rührt 16 h bei Raumtemperatur, engt ein und reinigt durch Fällung aus Dichlormethan mit Ether Anschließend verrührt man den Filterrückstand noch mit Wasser und lyophilisiert aus Dioxan/Wasser. Man erhält

290 mg (79 %) des Zielproduktes [DC Acetonitril/Wasser 10:1 $R_f$ = 0,6].

**12.5) N-{N$^{\alpha}$-[O-(3-O-Methyl-β-L-fucosyl)-4-hydroxy-phenylaminothiocarbonyl]-lysyl}-chinolon-a:**

288 mg (0,267 mmol) der Verbindung aus Beispiel 12.5.a werden in 20 ml Dichlormethan gelöst und mit 8 ml Piperidin versetzt. Nach 30 min Rühren bei 20°C engt man ein und fällt aus Dichlormethan mit Ether. Man reinigt durch Flashchromatographie [Dichlormethan/Methanol/Ammoniak (17 %ig) 10:10:2]. Der Rückstand wird mit Ether verrührt und aus Wasser lyophilisiert. Man erhält 90 mg (39 %) des Zielproduktes. [DC: Dichlormethan/Methanol/Ammoniak (17 %ig) 10:10:5 $R_f$ = 0,4].

**In Analogie zu Beispielen 12.5 werden aus den Konjugaten in Beispiel 9.11 bzw. 9.6 folgende Glycokonjugate hergestellt:**

**Beispiel 12.6**

**N-{N$^{\alpha}$-[O-(3-O-Carboxymethyl-β-L-fucosyl)-4-hydroxy-phenylaminothiocarbonyl]-lysyl}-chinolon-a, Di-Natriumsalz:**

Edukte: 63 mg (0,2 mmol) Kohlenhydrat aus Beispiel 1.10;
158 mg (0,18 mmol) Verbindung aus Beispiel 9.11
Reinigung der Zwischenstufe durch Flashchromatographie [Dichlormethan/Methanol/Ammoniak (17%ig) 15:4:0,5; später im gleichen System 10:10:1] und der Endstufe durch mehrfaches Verrühren mit Methanol und Waschen des Filterrückstandes mit Ether. Ausb.: 44 %. Anschließend wird in Wasser suspendiert und mit 2 Äquivalenten einer 0,1N Natronlauge das Di-Natriumsalz hergestellt und die Lösung lyophilisiert. [DC: Acetonitril/Wasser/Eisessig 10:3:1,5 $R_f$ = 0,34].

**Beispiel 12.7**

**N-{N$^{\alpha}$-[O-(3-O-Carboxymethyl-β-L-fucosyl)-4-hydroxy-phenylaminothiocarbonyl)-lysyl-D-alanyl}-chinolon-a:**

Edukte: 147 mg (0,47 mmol) Kohlenhydrat aus Beispiel 1.10;
448 mg (0,47 mmol) Verbindung aus Beispiel 9.6
Reinigung der Zwischenstufe durch zweimalige Fällung aus Dichlormethan/Methanol 1:1 mit Ether; Verrühren des Filterrückstandes mit Wasser (Ausb.: 92%). Reinigung der Endstufe durch Flashchromatographie [Dichlormethan/Methanol/Ammoniak (17 %ig) 10:10:2]; Fällung aus Dimethylformamid mit Ether. Ausb.: 59 % [DC: Acetonitril/Wasser/Eisessig 10:3:1,5 $R_f$ = 0,4].

**Beispiel 12.8**

**N-{N$^{\alpha}$-[O-(3-O-Methyl-b-D-galactopyranosyl)-4-hydroxy-phenylamino-thiocarbonyl)-lysyl}-chinolon-a, Hydrochlorid:**

Edukte: Verbindung 1.25 (62,8 mg, 0,22 mmol);
Peptidkonjugat 9,11 (180,0 mg, 0,2 mmol)
Reinigung der Zwischenstufe durch Flashchromatographie [Dichlormethan/Methanol 10:1 → 7:1 → 2:1]. Man erhält gelbe Kristalle (145,7 mg, 67 %); DC [Dichlormethan/Methanol 5:1]: $R_f$ = 0,48. Anschließend wird wie in Beispiel 4.5 beschrieben die Fluorenyl-9-methoxycarbonyl-Gruppe abgespalten und gereinigt. Man erhält gelbe Kristalle, die in Wasser (10 ml) suspendiert werden. Die Suspension wird unter Rühren tropfenweise mit 0,1N-Salzsäure versetzt, bis eine klare Lösung entsteht (pH > 3). Durch Lyophilisieren der filtrierten Lösung erhält man einen gelben amorphen Feststoff (119,4 mg, 66 %); $[a]_D^{20}$ = +33,8° (c = 0,28 / $H_2O$).

**Beispiel 12.9**

**N-{N$^{\alpha}$-[O-(3,6-di-O-Methyl-b-D-galactopyranosyl)-4-hydroxy-phenylaminothiocarbonyl]-lysyl}-chinolon-a, Hydrochlorid:**

Edukte: Verbindung 1.32 (65,9 mg, 0,22 mmol);
Peptidkonjugat 9.11 (180,0 mg, 0,2 mmol)

Reinigung der Zwischenstufe durch Flashchromatographie [Dichlormethan/Methanol 10:1 → 7:1 → 1:1]. Man erhält gelbe Kristalle (115,0 mg, 52 %); DC [Dichlormethan/Methanol 5:1]: $R_f$ = 0,44. Anschließend wird wie in Beispiel 4.5 beschrieben die Fluorenyl-9-methoxycarbonyl-Gruppe abgespalten und gereinigt. Man erhält gelbe Kristalle, die in Wasser (10 ml) suspendiert werden Die Suspension wird unter Ruhren tropfenweise mit 0,1N-Salzsäure versetzt, bis eine klare Lösung entsteht (pH > 3). Durch Lyophilisieren der filtrieren Lösung erhält man einen gelben amorphen Feststoff (94,3 mg, 51 %); $[a]_D^{20}$ = +44,2° (c = 0,34 / $H_2O$).

## Beispiel 12.10

**N-{N$^{\alpha}$-[O-(3-O-Methyl-a-D-mannopyranosyl)-4-hydroxy-phenylamino-thiocarbonyl)-lysyl}-chinolon-a, Hydrochlorid:**

Edukt: Verbindung 1.40 (62,8 mg, 0,22 mmol)
Peptidkonjugat 9.11 (180,0 mg, 0,2 mmol)
Reinigung der Zwischenstufe durch Flashchromatographie [Dichlormethan/Methanol 10:1 → 5:1 → 1:1]. Man erhält gelbe Kristalle (96,7 mg, 44 %); DC [Dichlormethan/Methanol 5:1]: $R_f$ = 0,47. Anschließend wird wie in Beispiel 4.5 beschrieben die Fluorenyl-9-methoxycarbonyl-Gruppe abgespalten und gereinigt. Man erhält gelbe Kristalle, die in Wasser (10 ml) suspendiert werden. Die Suspension wird unter Rühren tropfenweise mit 0,1N-Salzsäure versetzt, bis eine klare Lösung entsteht (pH > 3). Durch Lyophilisieren der filtrierten Lösung erhält man einen gelben amorphen Feststoff (78,2 mg, 43 %); $[a]_D^{20}$ = -157,2° (c = 0,30 / $H_2O$).

## Beispiel 12,11

**N-{N$^{\alpha}$-[O-(4-O-(3'-O-Methyl-b-D-galactopyranosyl)-b-D-glucopyranosyl)-4-hydroxy-phenylamino-thiocarbonyl]-lysyl}-chinolon-a, Hydrochlorid:**

Edukt: Verbindung 1.58 (98,4 mg, 0,22 mmol)
Peptidkonjugat 9.11 (180,0 mg, 0,2 mmol)
Reinigung der Zwischenstufe durch Flashchromatographie [Dichlormethan/Methanol/Ammoniak (25 %) 20:10:1 → 10:10:1 → Methanol/Ammoniak (25 %) 20:1]. Man erhält beige Kristalle (132,1 mg, 53 %); DC [Dichlormethan/Methanol/Ammoniak (25 %) 10:10:3]: $R_f$ = 0,60 Anschließend wird wie in Beispiel 4.5 beschrieben die Fluorenyl-9-methoxycarbonyl-Gruppe abgespalten und gereinigt. Man erhält gelbe Kristalle, die in Wasser (10 ml) suspendiert werden. Die Suspension wird unter Rühren tropfenweise mit 0,1N-Salzsäure versetzt, bis eine klare Lösung entsteht (pH > 3). Durch Lyophilisieren der filtrierten Lösung erhält man einen gelben amorphen Feststoff (90,0 mg, 42 %), $[a]_D^{20}$ = +192,2° (c = 0,27 / $H_2O$).

**Ausgehend von unsubstituiertem Chinolon-a werden nach der Vorschrift in Beispiel 12.1 folgende Glycokonjugate hergestellt:**

## Beispiel 12.12

**N-[O-(3-O-Carboxymethyl-β-L-fucosyl)-4-hydroxy-phenylamino-thiocarbonyl]-chinolon-a, Di-Natriumsalz:**

Edukte: 78,5 mg (0,25 mmol) Kohlenhydrat aus Beispiel 1.10;
70 mg (0,167 mmol) Chinolon-a
Nach 6 h Reaktionszeit wird eingeengt, in Dimethylformamid aufgenommen und mit 4 ml einer 0,1N Natronlauge 1 h gerührt. Reinigung durch Flashchromatographie [Dichlormethan/Methanol/Ammoniak (17%ig) 15:4:0,5. Man stellt mit einer 0,1N Natronlauge auf pH=7 ein und lyophilisiert. Ausb.: 60 mg (44 %). [DC : Acetonitril/Wasser/Eisessig 5:1:0,2 $R_f$ = 0,42] FAB-MS: m/z = 773 = M-2$Na^+$+3$H^+$

## Beispiel 12.13

**N-[O-(3-O-Methyl-β-L-fucosyl)-4-hydroxy-phenylamino-thiocarbonyl)-chinolon-a:**

Edukte: 32 mg (0,12 mmol) Kohlenhydrat aus Beispiel 1.2;
50 mg (0,12 mmol) Chinolon-a
2 h Reaktionszeit; Reinigung durch Flashchromatographie [Dichlormethan/Methanol/Eisessig 90:10:1]; Fällung aus Dichlormethan/Methanol 1:1 mit Ether. Ausb.: 59 mg (51 %). [DC: Acetonitril/Wasser 10:1 $R_f$ = 0,43].

**Beispiel 12.14**

**N-{N$^{\alpha}$-[O-(3-O-Methyl-β-L-fucosyl)-4-hydroxy-phenylamino-thiocarbonyl)-lysyl}-chinolon-a, Hydrochlorid:**

86 mg (0,1mmol) der Verbindung aus Beispiel 12.5 werden in Wasser aufgenommen und mit einem Äquivalent 0,1N Salzsäure in das Salz überführt Nach Gefrier-trocknung erhält man 88mg der Zielverbindung.

**Beispiel 12.15**

**N-{N$^{\alpha}$-[O-(3-O-Methyl-β-L-fucosyl)-4-hydroxy-phenylamino-thiocarbonyl]-diamino-propionoyl)-chinolon-a, Hydrochlorid:**

Ausgehend von N$^{\alpha}$-(tert-Butoxycarbonyl)-N$^{\varepsilon}$-(fluorenyl-9-methoxycarbonyl)-L-di-aminopropionsäure und Chinolon-a wird in Analogie zu Beispiel 12.14 über mehrere Stufen das Glycokonjugat 12.15 hergestellt [DC: Acetonitril/Wasser/Eisessig 5:1:0,2 $R_f$ = 0,3].

**Beispiele 13:**

Allgemeine Formel

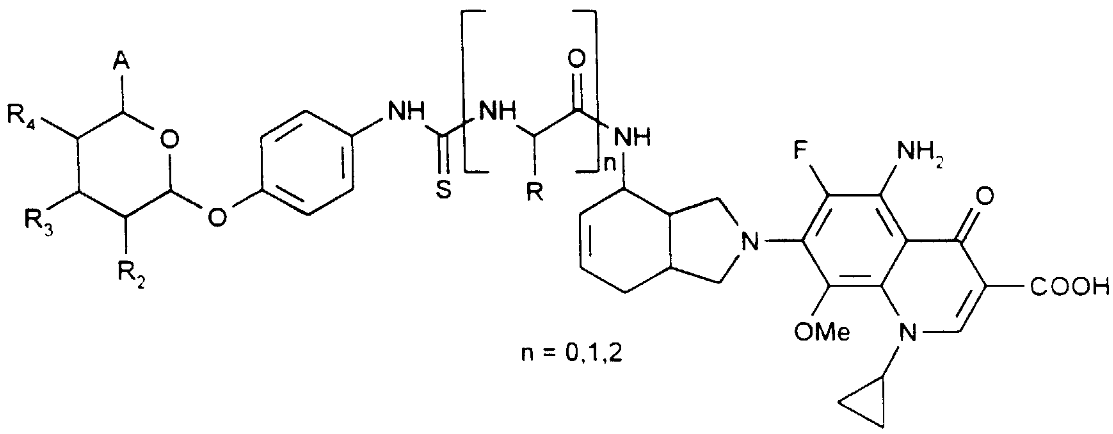

n = 0,1,2

**Beispiel 13.1**

**N-{N'-[O-(3-O-Methyl-β-L-fucosyl)-4-hydroxy-phenylamino-thiocarbonyl]-D-alanyl}-chinolon-b:**

**13.1.a) Chinolon b: 4-Amino-7-[(3aRS, 4RS, 7aSR)-4-amino-1,3,3a,4,7,7a-hexahydro-isoindol-2-yl)-1-cyclopropyl-6-fluor-1,4-dihydro-8-methoxy-4-oxo-3-chinolincarbonsäure**

310 mg (1 mmol) 5-Amino-1-cydopropyl-6,7-difluor-1,4-dihydro-8-methoxy-4-oxo-3-chinolincarbonsäure werden in einer Mischung aus 4 ml Acetonitril und 2 ml Dimethylformamid mit 170 mg (1.5 mmol) 1,4-Diazabicyclo [2.2.2]octan und 152 mg (1,1 mmol) (3aRS, 4RS, 7aSR)-4-Amino-1,3,3a,4,7,7a-hexahydro-isoindol versetzt und 1 Stunde unter Rückfluß erhitzt. Die Mischung wird im Vakuum eingeengt, der Rückstand mit etwa 20 ml Wasser verrührt und der ausgefallene Rückstand abgesaugt und bei 100°C im Vakuum getrocknet.
Ausbeute: 301 mg (70 % der Theorie),
Schmelzpunkt: 237-239°C (unter Zersetzung).

**13.1.b) N-[D-Alanyl)-chinolon-b, Trifluoracetat:**

Ausgehend von der Verbindung 13.1.a und N-(tert.-Butoxycarbonyl)-D-alanin wird in Analogie zu Beispiel 9.1 die Zielverbindung dargestellt.

**13.1) N-{N'-[O-(3-O-Methyl-β-L-fucosyl)-4-hydroxy-phenylaminothiocarbonyl]-D-alanyl}-chinolon-b:**

Ausgehend von der Verbindung 13.1.b und p-Aminophenyl-3-O-methyl-β-L-fucosid (Beispiel 1.2) wird in Analogie zu Beispiel 12.1 die Zielverbindung dargestellt.

**Beispiele 14:**

Allgemeine Formel

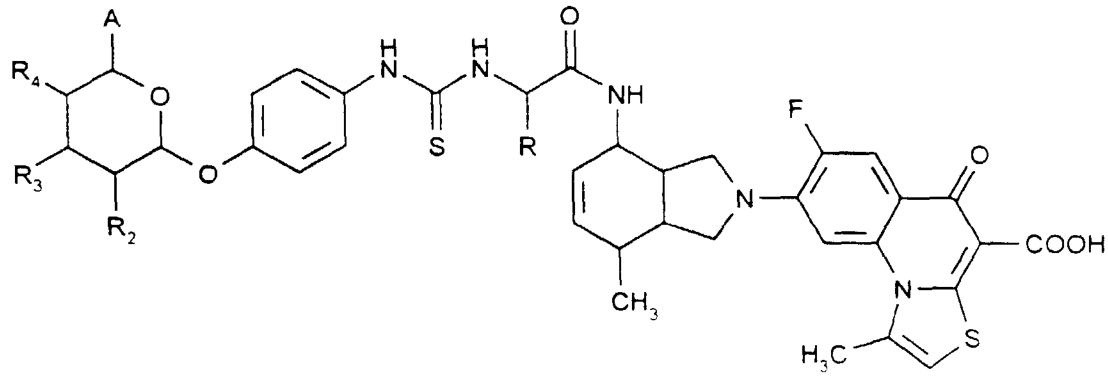

**Chinolon c: 8-(2-Amino-5-methyl-8-azabicyclo[4.3.0]non-3-en-8-yl)-1-methyl-7-fluoro-5-oxo-5H-thiazolo[3,2-a] chinolin-4-carbonsäure**

**Beispiel 14.1**

**N-{N$^{\alpha}$-[O-(3-O-Methyl-b-D-galactopyranosyl)-4-hydroxy-phenylamino-thiocarbonyl)-lysyl}-chinolon-c, Hydrochlorid:**

**14.1.a) N-[N$^{\varepsilon}$-(Fluorenyl-9-methoxycarbonyl)-lysyl]-chinolon-c, Trifluoracetat:**

Edukte: N$^{\alpha}$-(tert-Butoxycarbonyl)-N$^{\varepsilon}$-(fluorenyl-9-methoxycarbonyl)-lysin (1,4 g, 3,0 mmol); Chinolon-c (820 mg, 1,9 mmol)

Die Herstellung des Zwischenprodukts erfolgt analog zu Beispiel 9.1.a. Durch Umfällen aus Ethanol/Diethylether erhält man hellgelbe Kristalle (1,37 g, 82 %), aus denen in Analogie zu Beispiel 9.1.b die Verbindung 14.1.a freigesetzt wird Man erhält orange Kristalle (1,25 g, 74 %); DC [Dichlormethan/Methanol/Ammoniak (25 %) 30:10:1]: $R_f$ = 0,7; Schmp. = 180 °C.

**14.1)N-{N$^{\alpha}$-[O-(3-O-Methyl-b-D-galactopyranosyl)-4-hydroxy-phenylaminothiocarbonyl]-lysyl}-chinolon-c, Hydrochlorid:**

In Analogie zu Beispiel 12.5 wird Verbindung 1.25 (62,8 mg, 0,22 mmol) mit dem Peptidkonjugat 14.1.a (178,4 mg, 0,2 mmol) umgesetzt. Die Reinigung der Zwischenstufe erfolgt durch Flashchromatographie [Dichlormethan/Methanol/Ammoniak (25 %) 30:6:1 → 30:10:1]. Man erhält hellgelbe Kristalle (97,0 mg, 44 %), DC [Dichlormethan/Methanol/Ammoniak (25 %) 30:10:1]: $R_f$ = 0,23 Anschließend wird wie beschrieben die Fluorenyl-9-methoxycarbonyl-Gruppe abgespalten und gereinigt. Man erhält gelbe Kristalle, die in Wasser (10 ml) suspendiert werden. Die Suspension wird unter Rühren tropfenweise mit 0,1N-Salzsaure versetzt, bis eine klare Lösung entsteht (pH > 3). Durch Lyophilisieren der filtrierten Lösung erhält man einen gelben amorphen Feststoff (75,8 mg, 41 %); $[a]_D^{20}$ = +12,5° (c = 0,27 / $H_2O$).

**In Analogie zu Beispiel 14.1 werden aus dem Peptidkonjugat 14.1.a die folgenden Gylcokonjugate hergestellt:**

**Beispiel 14.2**

**N-{N$^{\alpha}$-[O-(3-O-Methyl-b-L-fucopyranosyl)-4-hydroxy-phenylamino-thiocarbonyl]-lysyl}-chinolon-c, Hydrochlorid:**

Edukte: Verbindung 1.2 (59,5 mg, 0,22 mmol); Peptidkonjugat 14.1.a (178,4 mg, 0,2 mmol)
Reinigung der Zwischenstufe durch Flashchromatographie [Dichlormethan/Methanol/Ammoniak (25 %) 30:6:1 → 30:10:1]. Man erhält hellgelbe Kristalle (146,6 mg, 67 %); DC [Dichlormethan/Methanol/Ammoniak (25 %) 30:6:1]: $R_f$ = 0,48. Anschließend wird wie beschrieben die Fluorenyl-9-methoxycarbonyl-Gruppe abgespalten und in das Hydrochlorid überführt. Man erhält einen gelben amorphen Feststoff (107,7 mg, 60 %); $[a]^{20}_{D}$ = +51,6° (c = 0,36 /$H_2O$).

**Beispiel 14.3**

**N-{N$^{\alpha}$-[O-(3-O-Carboxymethyl-β-L-fucosyl)-4-hydroxy-phenylaminothiocarbonyl)-lysyl}-chinolon-c, Di-Natriumsalz:**

Ausgehend von Verbindung 14.1.a wird in Analogie zu Beispiel 12.6 über mehrere Stufen das Glycokonjugat 14.4 hergestellt [FAB-MS: m/z = 911 = M-2Na+3H].

**Beispiel 14.4**

**N-{N$^{\alpha}$-[O-(3-O-Methyl-β-L-fucopyranosyl)-4-hydroxy-phenylaminothiocarbonyl)-D-lysyl}-chinolon-c, Hydrochlorid:**

Das Konjugat wird analog zum Isomer in Beispiel 14.2 hergestellt [FAB-MS: m/z = 867 = M+H].

**Beispiele 15:**

Allgemeine Formel

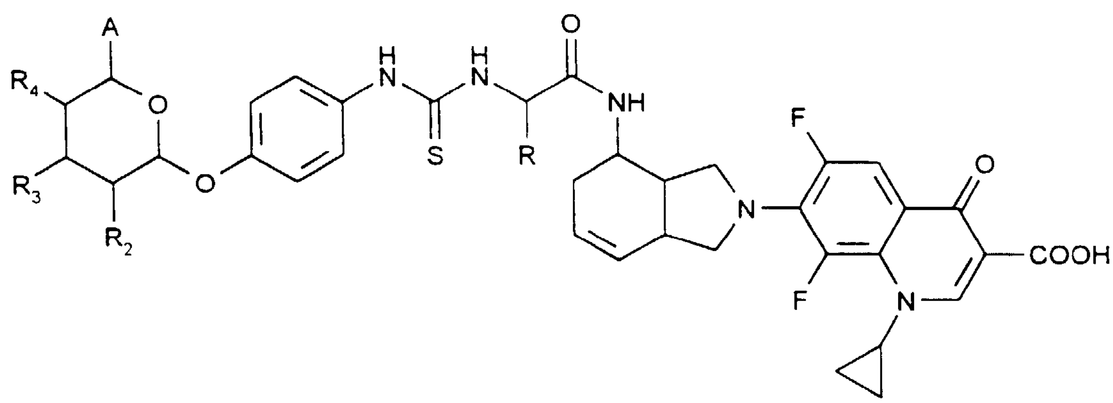

**Chinolon d: 4-(2-Amino-8-azabicyclo[4.3.0]non-4-en-8-yl)-1-cyclopropyl-6,8-difluoro-1,4-dihydro-4-oxo-chinolin-3-carbonsäure**

**Beispiel 15.1**

**N-{N$^{\alpha}$-[O-(3-O-Methyl-b-D-galactopyranosyl)-4-hydroxy-phenylamino-thiocarbonyl]-lysyl}-chinolon-d, Hydrochlorid:**

**15.1.a) N-[N$^{\varepsilon}$-(Fluorenyl-9-methoxycarbonyl)-lysyl]-chinolon-d, Trifluoracetat:**

N$^{\alpha}$-(tert-Butoxycarbonyl)-N$^{\varepsilon}$-(fluorenyl-9-methoxycarbonyl)-lysin (1,4 g, 3,0 mmol) wird wie in Beispiel 9.1.a beschrieben mit Chinolon-d, Hydrochlorid (1,28 mg, 2,8 mmol) umgesetzt. Durch Umfällen aus Dichlormethan/Methanol 1:1 mit Diethylether erhält man beige Kristalle (1,97 g, 83 %), aus denen in Analogie zu Beispiel 9.1.b die Verbindung 15.1.a freigesetzt wird Man erhält beige Kristalle (1,7 g, 70 %); DC [Dichlormethan/Methanol/Ammoniak (25 %) 28:14:1]: $R_f$ = 0.60; Schmp. = 215 °C.

**15.1)N-{N$^{\alpha}$-[O-(3-O-Methyl-b-D-galactopyrartosyl)-4-hydroxy-phenylaminothiocarbonyl]-lysyl}-chinolon-d, Hydrochlorid:**

In Analogie zu Beispiel 12.5 wird Verbindung 1.25 (62,8 mg, 0,22 mmol) mit dem Peptidkonjugat 15.1.a (173,2 mg, 0,2 mmol) umgesetzt. Die Reinigung der Zwischenstufe erfolgt durch Flashchromatographie [Dichlormethan/Methanol/Ammoniak (25 %) 28:14:1 → Methanol/Ammoniak (25 %) 20:1] Man erhält beige Kristalle (140,8 mg, 65 %); DC [Dichlormethan/Methanol/Ammoniak (25 %) 28:14:1]: $R_f$ = 0,06. Anschließend wird wie beschrieben die Fluorenyl-9-methoxy-carbonyl-Gruppe abgespalten und gereinigt. Man erhält beige Kristalle, die in Wasser (10 ml) suspendiert werden. Die Suspension wird unter Rühren tropfenweise mit 0,1N-Salzsäure versetzt, bis eine klare Lösung entsteht (pH > 3). Durch Lyophilisieren der filtrierten Lösung erhält man einen gelben amorphen Feststoff (102,6 mg, 57 %); $[a]_D^{20}$ = -49,0° (c = 0,26 / $H_2O$).

**In Analogie zu Beispiel 15.1 wird aus dem Peptidkonjugat 15.1.a das folgende Gylcokonjugate hergestellt:**

**Beispiel 15.2**

**N-{N$^{\alpha}$-[O-(4-O-(3'-O-Methyl-b-D-galactopyranosyl)-b-D-glucopyranosyl)-4-hydroxy-phenylamino-thiocarbonyl]-lysyl}-chinolon-d, Hydrochlorid:**

Edukt: Verbindung 1.58 (98,4 mg, 0,22 mmol);
Peptidkonjugat 15.1.a (173,2 mg, 0,2 mmol)
Reinigung der Zwischenstufe durch Flashchromatographie [Dichlormethan/Methanol/Ammoniak (25 %) 20:10:1 → 10:10:1 → Methanol/Ammoniak (25 %) 20:1]. Man erhält beige Kristalle (106,5 mg, 43 %); DC [Dichlormethan/Methanol/Ammoniak (25 %) 10:10:3]: $R_f$ = 0,51. Anschließend wird wie beschrieben die Fluorenyl-9-methoxycarbonyl-Gruppe abgespalten und in das Hydrochlorid überführt. Man erhält einen gelben amorphen Feststoff (82,0 mg, 39 %); $[a]_D^{20}$ = +22,8° (c = 0,29 / $H_2O$).

**Beispiele 16: Glycokonjugate mit Melphalan**

Allgemeine Formel

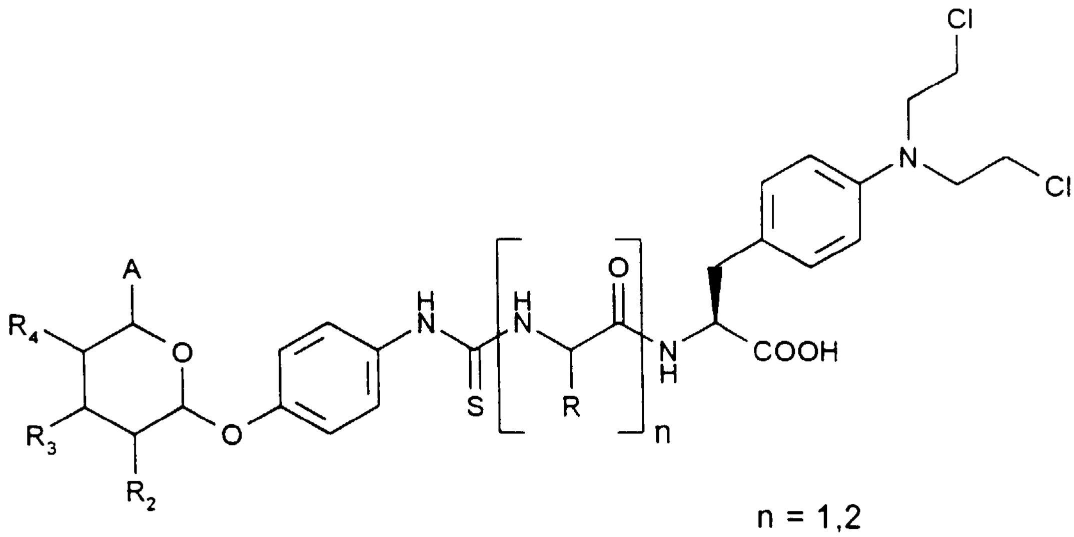

n = 1,2

**Beispiel 16.1**

**N-{N'-[O-(3-O-Methyl-β-L-fucopyranosyl)-4-hydroxy-phenylamino-thioarbonyl]-D-alany}-melphalan:**

**16.1.a) N-tert-Butoxycarbonyl-D-alanyl-melphalan:**

114 mg (0,6mmol) N-tert-Butoxycarbonyl-D-alanin werden in 10ml DMF gelöst und bei 0°C mit 138mg N'-(3-Dimethylaminopropyl)-N-ethyl-carbodiimid, Hydrochlorid und mit 1-Hydroxy-benzotriazol versetzt. Nach 10min gibt man 153mg Melphalan hinzu und rührt 16h bei Raumtemperatur. Man engt ein und verteilt zwischen Dichlormethan und Wasser. Die organische Phase wird gewaschen, über Natriumsulfat getrocknet, eingeengt und dann mit Dichlormethan/Methanol/Ammoniak (17%ig) 15:2:0,2 → 15:4:0,5 flashchromatographiert. Man erhält 134mg (56%) der Zielverbindung [DC: Dichlormethan/Methanol/Ammoniak (17%ig) 15:4:0,5 $R_f$ = 0,45).

**16.1) N-{N'-[O-(3-O-Methyl-β-L-fucopyranosyl)-4-hydroxy-phenylamino-thioarbonyl]-D-alany}-melphalan:**

Schutzgruppenabspaltung und Kopplung mit dem Kohlenhydrat werden wie in den Beispielen 9.1 bzw 12.1 beschrieben durchgeführt. [DC: Acetonitril/Wasser 10:1 $R_f$ = 0,26; FAB-MS: m/z = 685 = M-H].

**Beispiel 16.2**

**N-{N'-[O-(3-O-Methyl-β-L-fucopyranosyl)-4-hydroxy-phenylaminothiocarbonyl]-alanyl-alanyl}-melphalan:**

Diese Verbindung kann über mehrere Stufen in Analogie zu Beispiel 16.1 hergestellt werden [DC: Acetonitril/Wasser 10:1 $R_f$ = 0,2, FAB-MS: m/z = 756 = M-H].

**Beispiele 17: Glycokonjugate mit Doxorubicin (Adriamycin)**

Allgemeine Formel

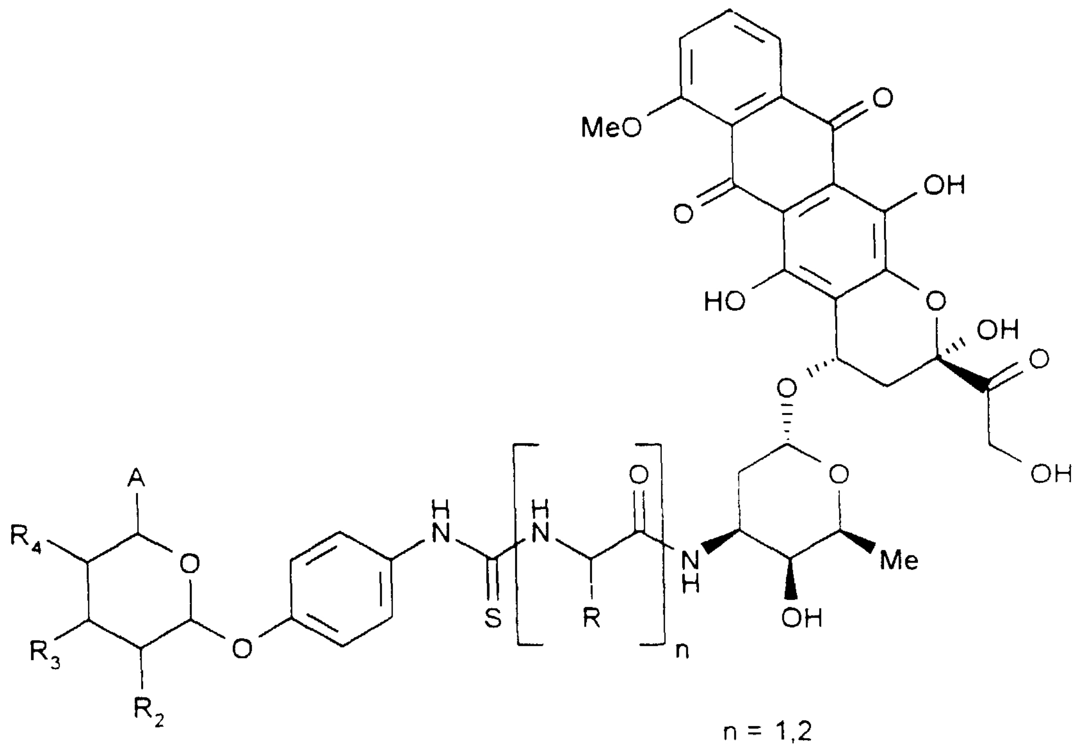

n = 1,2

**Beispiel 17.1**

**N-{N'-[O-(3-O-Methyl-β-L-fucopyranosyl)-4-hydroxy-phenylaminothiocarbonyl]-alanyl-alanyl}-doxorubicin:**

**17.1.a) N-[O-(3-O-Methyl-β-L-fucopyranosyl)-4-hydroxy-phenylamino-thio-carbonyl]-alanyl-alanin:**

160mg (1mmol) Alanyl-Alanin werden in 20ml Dioxan/Wasser 1:1 aufgenommen und mit 1ml Hünig-Base versetzt. 1,2 mmol p-Aminophenyl-3-O-methyl-β-L-fucosid (Beispiel 1.2) werden nach Vorschrift 10.1.a zunächst in das Senföl umgewandelt und anschließend zu der Lösung des Dipeptids gegeben. Man läßt 16h bei Raumtemp. rühren, engt dann ein und reinigt den Rückstand durch Flash-Chromatographie (Acetonitril/Wasser 15:1). Nach Einengen der entsprechenden Fraktionen wird das Produkt aus Methanol/Ether ausgefällt. Ausb.: 267mg (57%).

**17.1) N-{N'-[O-(3-O-Methyl-β-L-fucopyranosyl)-4-hydroxy-phenylamino-thiocarbonyl]-alanyl-alanyl}-doxorubicin:**

48mg (0,1mmol) der Verbindung aus Beispiel 17.1.a werden in 10ml DMF gelöst und mit 23,1mg N'-(3-Dimethylaminopropyl)-N-ethyl-carbodiimid, Hydrochlorid sowie mit 21mg 1-Hydroxy-benzotriazol versetzt. Nach 5min gibt man 30mg Doxorubicin und 35μl Hünig-Base hinzu und rührt 30min bei Raumtemperatur. Man engt ein und reinigt durch Flash-Chromatographie (Dichlormethan/Methanol 88:12). Die entsprechenden Fraktionen werden eingeengt und aus Dioxan/Wasser lyophilisiert. Man erhält 20mg (40%) der Zielverbindung. [DC: Dichlormethan/Methanol 10:1 $R_f$ = 0,17; ESI: m/z = 997 = M+H].

**Beispiel 17.2**

**N-{$N^{\alpha}$,$N^{\varepsilon}$-bis-[O-(3-O-Methyl-β-L-fucopyranosyl)-4-hydroxy-phenylaminothiocarbonyl]-D-lysyl-alanyl}-doxorubicin:**

**17.2.a) $N^{\alpha}$,$N^{\varepsilon}$-bis-[O-(3-O-Methyl-β-L-fucopyranosyl)-4-hydroxy-phenylaminothiocarbonyl]-D-lysyl-alanin:**

580mg (1,31mmol) des Bis-Trifluoracetats von D-Lysyl-alanin werden wie in Beispiel 17.1.a beschrieben in Gegenwart von 1,3ml Hünig-Base mit 2,2 Äquivalenten des Kohlenhydrats aus Beispiel 1.2 verknüpft. Die flashchromatographische Reinigung erfolgt mit Acetonitril/Wasser 10:1. Man erhält 446mg (41%) der Zielverbindung.

**17.2) N-{$N^{\alpha}$,$N^{\varepsilon}$-bis-[O-(3-O-Methyl-β-L-fucopyranosyl)-4-hydroxyphenylamino-thiocarbonyl]-D-lysyl-alanyl}-doxorubicin:**

Die Verknüpfung von 59mg der Verbindung aus Beispiel 17.2.a mit 20mg Doxorubicin erfolgt wie in Beispiel 17.1 beschrieben. Man erhält 15mg des Konjugats. [DC: Dichlormethan/Methanol 85:15 $R_f$ = 0,43; FAB-MS: m/z = 1365 = M+H].

**Beispiele 18: Glycokonjugate mit Camptothecin**

Allgemeine Formel

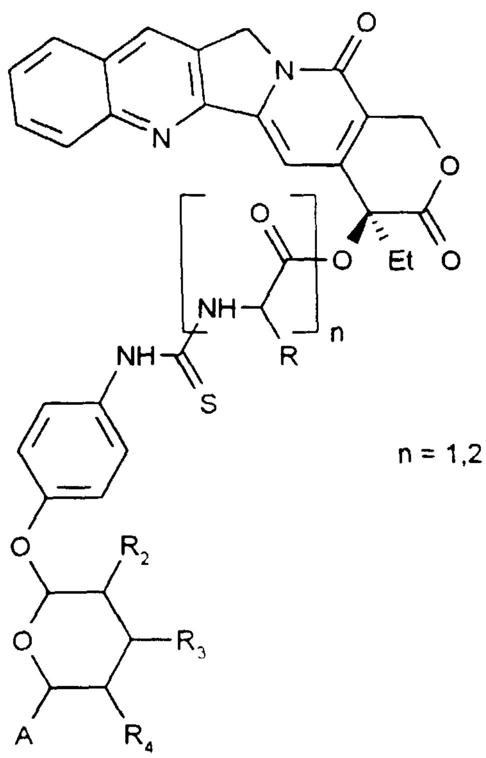

n = 1,2

**Beispiel 18.1**

**20-O-{$N^{\alpha}$,$N^{\varepsilon}$-bis-[O-(3-O-Methyl-β-L-fucopyranosyl)-4-hydroxy-phenylaminothiocarbonyl]-lysyl-alanyl}-camptothecin:**

**18.1.a) 20-O-(Alanyl)-camptothecin, Trifluoracetat:**

500mg (1,44mmol) Camptothecin werden in 20ml DMF gelöst und dann mit 50mg 4-Dimethylaminopyridin und N-tert-Butoxycarbonyl-alanin-N-carboxy-anhydrid versetzt. Nach 3h werden weitere 775mg N-tert-Butoxycarbonyl-alanin-N-carboxy-anhydrid zugegeben und die Suspension 16h mit Ultraschall behandelt. Man engt ein, nimmt das Rohmaterial in 50ml Dichlormethan auf und gibt bei 0°C 5ml Trifluoressigsäure zu. Nach 30min Rühren wird erneut eingeengt und das Produkt durch Flash-Chromatographie gereinigt (Acetonitril/Wasser 20:1). Die entsprechenden Fraktionen werden gesammelt, eingeengt und aus Dioxan/Wasser lyophilisiert. Man erhält 712mg (93%) der Zielverbindung [FAB-MS: m/z = 420 = M+H].

**18.1.b) 20-O-(Lysyl-alanyl)-camptothecin, Bis-Trifluoracetat:**

Das Konjugat aus Beispiel 18.1.a wird nach Standard-Vorschrift mit $N^{\alpha}$ ,$N^{\varepsilon}$-bis-(tert- Butoxycarbonyl)-lysin verknüpft und anschließend deblockiert. Man erhält die Ziel verbindung in 65%iger Ausbeute.

**18.1) 20-O-{$N^{\alpha}$,$N^{\varepsilon}$-bis-[O-(3-O-Methyl-β-L-fucopyranosyl)-4-hydroxyphenylamino-thiocarbonyl]-lysyl-alanyl}-camptothecin:**

In Analogie zur Vorschrift in Beispiel 11.1 wird p-Aminophenyl-3-O-methyl-β-L-fucosid (Beispiel 1.2) mit dem Konjugat aus Beispiel 18.1.b verknüpft.
Ausb.: 40% [DC: Acetonitril/Wasser 10:1 $R_f$ = 0,44]

**Beispiel 18.2**

**20-O-{$N^{\alpha}$-[O-(3-O-Carboxymethyl-β-L-fucopyranosyl)-4-hydroxy-phenyl-amino-thiocarbonyl)-lysyl-alanyl}-camptothecin:**

**18.2.a) 20-O-[$N^{\varepsilon}$-(Fluorenyl-9-methoxycarbonyl)-lysyl-alanyl]-camptothecin, Trifluoracetat:**

Das Konjugat aus Beispiel 18.1.a wird nach Standard-Vorschrift mit $N^{\alpha}$ -(tert-Butoxycarbonyl)-$N^{\varepsilon}$-(fluorenyl-9-methoxycarbonyl)-lysin verknüpft und anschließend an der α-Aminofunktion deblockiert. Man erhält die Zielverbindung in 24%iger Ausbeute. [DC: Acetonitril/Wasser 20:1 $R_f$ = 0,15]

**18.2.b) 20-O-{$N^{\alpha}$-[O-(3-O-Carboxymethyl-β-L-fucopyranosyl)-4-hydroxy-phenylamino-thiocarbonyl]-$N^{\varepsilon}$-[fluorenyl-9-methoxycarbonyl]-lysyl-alanyl}-camptothecin:**

Die Verbindung aus Beispiel 18.1.a wird in Analogie zu Beispiel 12.6 bzw. 12.5 mit dem Kohlenhydratderivat aus Beispiel 1.10 modifiziert. Das Rohprodukt läßt sich durch Digerieren mit Wasser reinigen, wird anschließend aus Dioxan/Wasser lyophilisiert und ohne weitere Charakterisierung in die nächste Stufe eingesetzt.

**18.2) 20-O-{$N^{\alpha}$-(O-(3-O-Carboxymethyl-β-L-fucopyranosyl)-4-hydroxyphenylamino-thiocarbonyl]-lysyl-alanyl}-camptothecin:**

Das Konjugat 18.2.b wird mit Piperidin in DMF deblockiert. Nach 30min engt man ein und digeriert den Rückstand zweimal mit Dichlormethan. Dann wird in DMF aufgenommen und mit Methanol/Ether gefällt. Das Produkt wird abgesaugt, mit Ether gewaschen und dann aus Dioxan/Wasser lyophilisiert. Ausb.: 86% [DC: Acetonitril/Wasser/Eisessig 5:1:0,2 $R_f$ = 0,17]

Beispiel 18.3

**20-O-{N$^{\alpha}$-[O-(3-O-Carboxymethyl-β-L-fucopyranosyl)-4-hydroxy-phenyl-amino-thiocarbonyl]-lysyl-alanyl}-camptothecin, Natriumsalz:**

62mg (0,074mmol) des Konjugats aus Beispiel 18.2 werden in Dioxan/Wasser aufgenommen und mit einem Äquivalent einer 0,1N-Natronlauge ins Natriumsalz überführt. Ausb.: quant. [DC: Acetonitril/Wasser/Eisessig 5:1:0,2 $R_f$ = 0,17]

**In Analogie zu den Beispielen 18.1 und 18.2 werden folgende Glycokonjugate von Camptothecin hergestellt:**

Beispiel 18.4

**20-O-{N$^{\alpha}$,N$^{\varepsilon}$-bis-[O-(3-O-Methyl-β-L-fucopyranosyl)-4-hydroxy-phenylaminothiocarbonyl]-lysyl-D-alanyl}-camptothecin**

Beispiel 18.5

**20-O-{N$^{\alpha}$,N$^{\varepsilon}$-bis-(O-(3-O-Methyl-β-L-fucopyranosyl)-4-hydroxy-phenylaminothiocarbonyl]-lysyl-valinyl}-camptothecin**

Beispiel 18.6

**20-O-{N$^{\alpha}$-[O-(3-O-Carboxymethyl-β-L-fucopyranosyl)-4-hydroxy-phenylamino-thiocarbonyl]-lysyl-valinyl}-camptothecin**

Beispiel 18.7

**20-O-{N$^{\alpha}$-[O-(3-O-Methyl-β-D-galactopyranosyl)-4-hydroxy-phenylamino-thiocarbonyl]-lysyl-valinyl}-camptothecin**

Beispiel 18.8

**20-O-{N$^{\alpha}$-[O-(3-O-Methyl-β-D-galactopyranosyl)-4-hydroxy-phenylamino-thiocarbonyl]-lysyl-alanyl}-camptothecin**

Beispiel 18.9

**20-O-{N$^{\alpha}$(O-(3-O-Carboxymethyl-β-L-fucopyranosyl)-4-hydroxy-phenylaminothiocarbonyl]-lysyl-valinyl}-camptothecin, Hydrochlorid**

Die Verbindung 18.6 wird mit einem Equivalent 0,01 N Salzsäure in das Hydrochlorid überführt.

Beispiel 18.10

**20-O-{N$^{\alpha}$[O-(3-O-Carboxymethyl-β-L-fucopyranosyl)-4-hydroxy-phenylamino-thiocarbonyl]-lysyl-alanyl}-camptothecin, Hydrochlorid**

Die Verbindung 18.2 wird mit einem Equivalent 0,01 N Salzsäure in das Hydrochlorid überführt.

Beispiel 18.11

**20-O-{N$^{\alpha}$[O-(3-O-Carboxymethyl-β-L-fucopyranosyl)-4-hydroxy-phenylaminothiocarbonyl]-lysyl-phenylalanyl}-camptothecin, Hydrochlorid**

Beispiel 18.12

**20-O-{N$^{\alpha}$,N$^{\varepsilon}$-bis-[O-(3-O-Carboxymethyl-β-L-fucopyranosyl)-4-hydroxyphenylamino-thiocarbonyl]-lysyl-alanyl}-camptothecin, Natriumsalz**

**Beispiel 18.13**

**20-O-{N$^{\alpha}$,N$^{\varepsilon}$-bis-[O-(3-O-Carboxymethyl-β-L-fucopyranosyl)-4-hydroxyphenylamino-thiocarbonyl]-lysyl-valinyl}-camptothecin, Natriumsalz**

**Beispiel 18.14**

**20-O-{N$^{\alpha}$(O-(3-O-Methyl-β-L-fucopyranosyl)-4-hydroxy-phenylamino-thiocarbonyl]-lysyl-alanyl}-camptothecin, Hydrochlorid**

## Patentansprüche

1. Verbindungen der allgemeinen Formel

$$K - Sp - L - AA1 - AA2 - C \qquad (I)$$

mit

K = unsubstituierter oder regioselektiv modifizierter Kohlenhydratrest,
Sp = gegebenenfalls substituiertes Arylen oder Alkylen,
L =

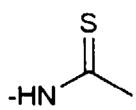

oder

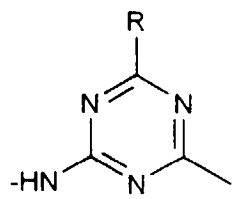

mit R = Chlor oder Hydroxyalkylamino,
AA1 = ist ein Aminosäure-Rest in der D- oder L-Konfiguration, der gegebenenfalls Schutzgruppen oder eine zweite Gruppierung K-Sp-L- tragen kann, in der K, Sp, L unabhängig von der anderen Gruppierung K-Sp-L- die oben angegebenen Bedeutungen haben können, oder eine direkte Bindung,
AA2 = ist ein Aminosäure-Rest in der D- oder L-Konfiguration, der gegebenenfalls Schutzgruppen oder eine zweite Gruppierung K-Sp-L- tragen kann, in der K, Sp, L unabhängig von der anderen Gruppierung K-Sp-L- die oben angegebenen Bedeutungen haben können, oder eine direkte Bindung,

und

C = ein cytotoxischer Rest oder der Rest eines Cytostatikums oder eines Cytostatikum-Derivats, das zusätzlich eine Amino- oder Hydroxygruppe tragen kann,

und deren Isomere und Salze.

2. Verbindungen der allgemeinen Formel (I) gemäß Anspruch 1, worin K ein Kohlenhydratrest der Formel (II) ist

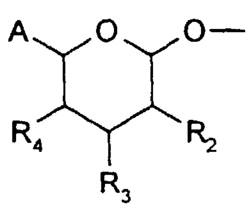

(II),

wobei

A = Methyl, Hydroxymethyl, Carboxy sowie davon abgeleitete Ester und Amide, Alkoxymethyl, Acyloxymethyl oder Carboxyalkyloxymethyl sowie davon abgeleitete Ester und Amide; A kann auch $CH_2$-B sein, wobei B wiederum ein über das anomere Zentrum verknüpfter Kohlenhydratrest der allgemeinen Formel (II) sein kann,

$R_2$, $R_3$, $R_4$ = einzeln oder zusammen gleich H, Hydroxy, Alkyloxy, Carboxyalkyloxy sowie davon abgeleitete Ester und Amide, Hydroxyalkyloxy, Aminoalkyloxy, Acyloxy, Carboxyalkylcarbonyloxy, Sulfato, Phosphato, Halogen oder ein weiterer, im gleichen Rahmen modifizierter und über das anomere Zentrum verknüpfter Kohlenhydratrest (II), wobei $R_2$ zusätzlich auch ein Amino oder Acylamino sein kann, oder zwei der Reste $R_2$, $R_3$, $R_4$ zusammen für eine Epoxygruppe stehen,

und Sp, L, AA1, AA2 und C wie in Anspruch 1 definiert sind,
und deren Isomere und Salze.

**3.** Verbindungen der Formel (I) gemäß Anspruch 1, worin

Sp = Arylenrest, der ortho-, meta- oder para-ständig mit K und L modifiziert ist und darüber hinaus noch 1 bis 4 weitere Substituenten tragen kann, die unabhängig oder gleich H, Methyl, Methoxy, Hydroxy, Carboxy, Methyloxycarbonyl, Cyano, Nitro, Halogen, Sulfonyl oder Sulfonamid sein können oder Sp kann auch ein linearer oder verzweigter Alkylen-Rest sein,

und K, L, AA1, AA2 und C wie in Anspruch 1 definiert sind,
und deren Isomere und Salze.

**4.** Verbindungen der allgemeinen Formel (I) gemäß Anspruch 1, worin C ein Nucleosid, ein Endiin-Antibiotikum, ein cytotoxisches Peptidantibiotikum, eine Chinolon- oder Naphthyridoncarbonsäure oder Batracylin, 5-Fluorouracil, Cytosinarabinosid, Methotrexat, Etoposid, Camptothecin, Daunomycin, Doxorubicin, Taxol, Vinblastin, Vincristin, Dynemicin, Calicheamycin, Esperamycin, Quercetin, Suramin, Erbstatin, Cyclophosphamid, Mitomycin C, Melphalan, Cisplatin, Bleomycin, Staurosporin oder ein anderer antineoplastisch aktiver Wirkstoff sein kann,
und K, Sp, L, AA1, AA2 wie in Anspruch 1 definiert sind,
und deren Isomere und Salze.

**5.** Verbindungen der allgemeinen Formel (I) gemäß Anspruch 1, worin AA1 ein Aminosäure-Rest abgeleitet von Lysin, Alanin, Asparaginsäure, Glutaminsäure, Glycin, Ornithin, Tyrosin, Valin oder Serin in der D- oder L-Konfiguration ist, der gegebenenfalls mit einer weiteren Gruppierung K-Sp-L- verknüpft ist, oder worin AA1 eine Bindung bedeutet
und K, Sp, L, AA2 und C wie in Anspruch 1 definiert sind,
und deren Isomere und Salze

**6.** Verbindungen der allgemeinen Formel (I) gemaß Anspruch 1, worin AA2 ein Aminosaure-Rest abgeleitet von Lysin, Alanin, Glycin, Ornithin, Diaminopropionsäure oder Serin in der D- oder L-Konfiguation ist, der gegebenenfalls mit einer weiteren Gruppierung K-Sp-L- verknüpft ist, oder worin AA2 eine Bindung bedeutet
und K, Sp, L, AA1 und C wie in Anspruch 1 definiert sind,
und deren Isomere und Salze.

**7.** Verbindungen der allgemeinen Formel (I) gemäß Anspruch 1, worin C für einen Rest der Formel (III) steht

T-Q (III)

in welcher

Q einen Rest der Formeln

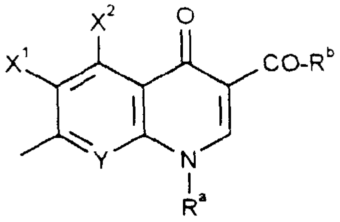
,
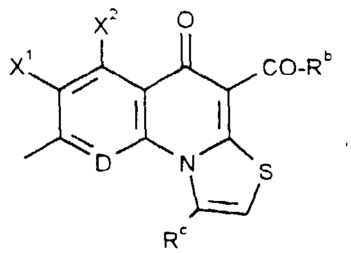
,
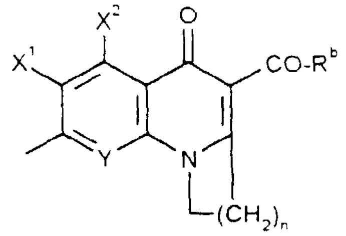

oder

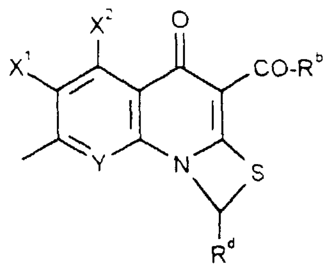

bedeutet, worin

$R^a$ für gegebenenfalls durch Halogen oder Hydroxy ein- oder zweifach substituiertes Alkyl mit 1 bis 4 Kohlenstoffatomen, Vinyl, gegebenenfalls durch 1 oder 2 Fluoratome substituiertes Cycloalkyl mit 3 bis 6 Kohlenstoffatomen, Bi-cyclo[1.1.1]pent-1-yl, 1,1-Dimethylpropargyl, 3-Oxetanyl, Methoxy, Amino, Methylamino, Dimethylamino, gegebenenfalls durch Halogen, Amino oder Hydroxy ein- oder zweifach substituiertes Phenyl steht, oder auch gemeinsam mit $R^e$ eine dort beschriebene Brücke bilden kann,
$R^b$ für Hydroxy, Alkoxy mit 1 bis 3 Kohlenstoffatomen, Nitromethyl,
$R^c$ für Wasserstoff oder Methyl steht, oder gemeinsam mit $R^g$ eine dort beschriebene Brücke bilden kann,
$R^d$ für Wasserstoff, $CH_3$, $CH_2F$ oder $=CH_2$,
$X^1$ für Wasserstoff, Halogen oder Nitro,
$X^2$ für Wasserstoff, Halogen, Amino, Hydroxy, Methoxy, Mercapto, Methyl, Halogenmethyl oder Vinyl,
Y für N oder C-$R^c$ steht, worin

$R^c$ für Wasserstoff, Halogen, $CF_3$, $OCH_3$, $OCHF_2$, $CH_3$, CN, $CH=CH_2$ oder $C\equiv CH$ steht oder auch gemeinsam mit $R^a$ eine Brücke der Struktur $-{}^*O-CH_2-CH-CH_3$, $-{}^*S-CH_2-CH_2-$, $-{}^*S-CH_2-CH-CH_3$, $-{}^*CH_2-CH_2-CH-CH_3$ oder $-{}^*O-CH_2-N-R^f$, wobei das mit * markierte Atom mit dem Kohlenstoffatom von Y verknüpft ist und worin

$R^f$ Wasserstoff, Methyl oder Formyl bedeutet,

bilden kann, und
D für N oder C-$R^g$ steht, worin

$R^g$ für Wasserstoff, Halogen, $CF_3$, $OCH_3$, $OCHF_2$ oder $CH_3$ steht oder auch gemeinsam mit * $R^c$ eine Brucke der Struktur $- {}^*O-CH_2-$, $-{}^*NH-CH_2-$, $-{}^*N(CH_3)-CH_2-$, $- {}^*N(C_2H_5)-CH_2-$, $- {}^*N(C_3H_5)-CH_2-$ oder $- {}^*S-CH_2-$bilden kann, wobei das mit * markierte Atom mit dem Kohlenstoffatom von D verknüpft ist.

n 1, 2 oder 3 und

T einen Rest der Formel

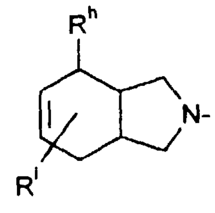 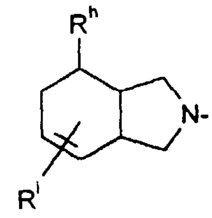 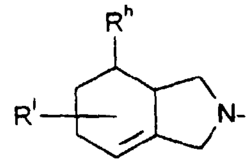

bedeutet, worin

$R^h$ für O-,

$-N-R^k$, $CH_2-O-$ oder $-CH_2-N-R^k$

steht, wobei

$R^k$ Wasserstoff oder Methyl,

$R^i$ für Wasserstoff, $C_1$-$C_3$-Alkyl oder Cyclopropyl steht,

und worin K, Sp, L, AA1, AA2 wie in Anspruch 1 definiert sind, und deren Isomere und Salze.

**8.** Verbindungen der Formel (I) gemäß Anspruch 7, in welcher

Q einen Rest der Formel

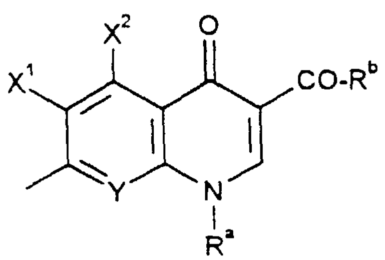

oder

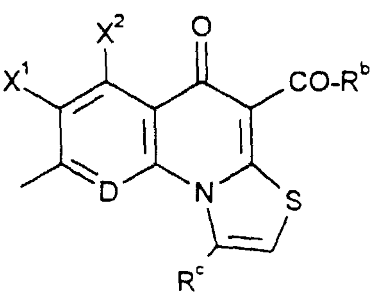

bedeutet, worin

$R^a$ für gegebenenfalls durch 1 Fluoratom substituiertes Alkyl mit 2 bis 4 Kohlenstoffatomen, gegebenenfalls durch 1 Fluoratom substituiertes Cyclopropyl, gegebenenfalls durch Fluor ein- oder zweifach substituiertes Phenyl steht oder gemeinsam mit $R^e$ eine dort beschriebene Brücke bilden kann,

$R^b$ für Hydroxy oder Alkoxy mit 1 oder 2 Kohlenstoffatomen,

$R^c$ für Wasserstoff oder Methyl steht oder gemeinsam mit $R^g$ eine dort beschriebene Brücke bilden kann,

$X^1$ für Fluor,

$X^2$ für Wasserstoff oder Amino,

Y für N oder C-$R^c$ steht, worin

$R^c$ für Wasserstoff, Fluor, Chlor, $CF_3$, $OCH_3$, $OCHF_2$, oder C≡CH steht oder auch gemeinsam mit $R^a$ eine Brücke der Struktur -*O-$CH_2$-CH-$CH_3$ oder -*O-$CH_2$-N-$R^f$, wobei das mit * markierte Atom mit dem Kohlenstoffatom von Y verknüpft ist und worin

$R^f$ Methyl bedeutet,

bilden kann,

D für N oder C-$R^g$ steht, worin

$R^g$ für Wasserstoff, Fluor, Chlor, $CF_3$, $OCH_3$ oder $CH_3$ steht oder auch gemeinsam mit $R^e$ eine Brücke der Struktur -*$OCH_2$-, -*NH-$CH_2$-, -*N($CH_3$)-$CH_2$- oder -*S-$CH_2$- bilden kann, wobei das mit * markierte Atom mit dem Kohlenstoffatom von D verknüpft ist, und

T einen Rest der Formeln

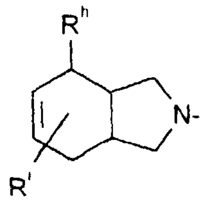

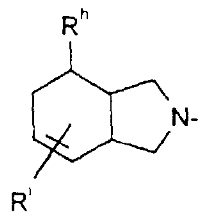

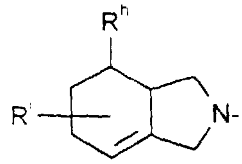

bedeutet, worin

$R^h$ für

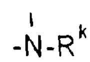

steht, worin

$R^k$ für Wasserstoff oder Methyl steht,

$R^i$ für Wasserstoff oder Methyl steht,

und deren Isomere und Salze.

**9.** Arzneimittel enthaltend eine oder mehrere Verbindungen aus den Ansprüchen 1 bis 8.

**10.** Verwendung von Verbindungen der Formel (I) gemäß den Ansprüchen 1 bis 8 zur Herstellung von Arzneimitteln.

## Claims

**1.** Compounds of the general formula

$$K - Sp - L - AA1 - AA2 - C \qquad (I)$$

where

K = an unsubstituted or regioselectively modified carbohydrate radical,

Sp = optionally substituted arylene or alkylene,

L =

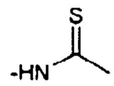 or 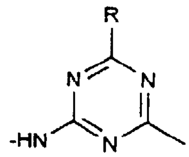

where R = chlorine or hydroxyalkylamino,

AA1 is an amino acid radical in the D or L configuration, which can optionally carry protective groups or a second grouping K-Sp-L-, in which K, Sp and L, independently of the other grouping K-Sp-L-, can have the abovementioned meanings, or a direct bond,

AA2 is an amino acid radical in the D or L configuration, which can optionally carry protective groups or a second grouping K-Sp-L-, in which K, Sp and L, independently of the other grouping K-Sp-L-, can have the abovementioned meanings, or a direct bond,

and

C = a cytotoxic radical or the radical of a cytostatic or of a cytostatic derivative, which can additionally carry an amino or hydroxyl group,

and isomers and salts thereof.

**2.** Compounds of the general formula (I) according to Claim 1, wherein K is a carbohydrate radical of the formula (II)

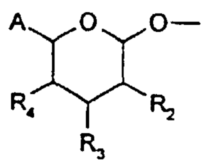 (II),

wherein

A = methyl, hydroxymethyl, carboxyl and esters and amides derived therefrom, alkoxymethyl, acyloxymethyl or carboxyalkyloxymethyl and esters and amides derived therefrom; A can also be $CH_2$-B, wherein B in turn can be a carbohydrate radical of the general formula (II) linked via the anomeric centre,

$R_2$, $R_3$, $R_4$ = individually or together at the same time, H, hydroxyl, alkyloxy, carboxyalkyloxy and esters and amides derived therefrom, hydroxyalkyloxy, aminoalkyloxy, acyloxy, carboxyalkylcarbonyloxy, sulphato, phosphato, halogen or another carbohydrate radical (II) modified in the same framework and linked via the anomeric centre, $R_2$ can additionally also be amino or acylamino, or two of the radicals $R_2$, $R_3$ and $R_4$ together represent an epoxide group,

and Sp, L, AA1, AA2 and C are defined as in Claim 1,
and isomers and salts thereof.

**3.** Compounds of the formula (I) according to Claim 1, wherein

Sp = an arylene radical which is modified with K and L in the ortho-, meta- or para-position and furthermore can also carry 1 to 4 further substituents which, independently of one another or in an identical manner, can be H, methyl, methoxy, hydroxyl, carboxyl, methoxycarbonyl, cyano, nitro, halogen, sulphonyl or sulphonamide, or Sp can also be a linear or branched alkylene radical,

and K, L, AA1, AA2 and C are defined as in Claim 1,
and isomers and salts thereof.

**4.** Compounds of the general formula (I) according to Claim 1, wherein C can be a nucleoside, an endiine antibiotic, a cytotoxic peptide antibiotic, a quinolone- or naphthyridonecarboxylic acid or batracyline, 5-fluorouracil, cytosine arabinoside, methotrexate, etoposide, camptothecin, daunomycin, doxorubicin, taxol, vinblastine, vincristine, dynemycin, calicheamycin, esperamycin, quercetin, suramin, erbstatin, cyclophosphamide, mitomycin C, melphalan, cisplatin, bleomycin, staurosporin or another active compound having an antineoplastic action,
and K, Sp, L, AA1 and AA2 are defined as in Claim 1,
and isomers and salts thereof.

**5.** Compounds of the general formula (I) according to Claim 1, wherein AA1 is an amino acid radical derived from lysine, alanine, aspartic acid, glutamic acid, glycine, ornithine, tyrosine, valine or serine in the D or L configuration, which is optionally linked with a further grouping K-Sp-L-, wherein AA1 denotes a bond
and K, Sp, L, AA1, AA2 and C are defined as in Claim 1,
and isomers and salts thereof.

**6.** Compounds of the general formula (I) according to Claim 1, wherein AA2 is an amino acid radical derived from lysine, alanine, glycine, ornithine, diaminopropionic acid or serine in the D or L configuration, which is optionally linked with a further grouping K-Sp-L-, or wherein AA2 denotes a bond
and K, Sp, L, AA1 and C are defined as in Claim 1,
and isomers and salts thereof.

**7.** Compounds of the general formula (I) according to Claim 1, wherein C represents a radical of the formula (III)

$$T\text{-}Q \qquad (III)$$

in which

Q denotes a radical of the formulae

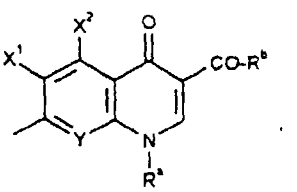
,
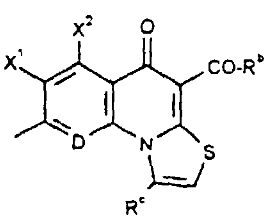
,
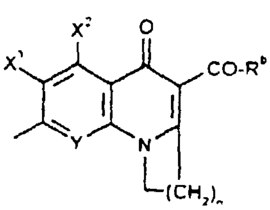

or

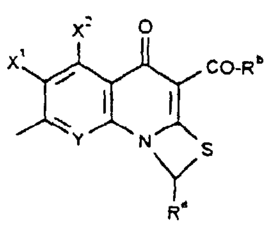

wherein

$R^a$ represents alkyl which has 1 to 4 carbon atoms and is optionally mono- or disubstituted by halogen or hydroxyl, vinyl, cycloalkyl which has 3 to 6 carbon atoms and is optionally substituted by 1 or 2 fluorine atoms, bicyclo[1.1.1]pent1-yl, 1,1-dimethylpropargyl, 3-oxetanyl, methoxy, amino, methylamino, dimethylamino or phenyl which is optionally mono-or disubstituted by halogen, amino or hydroxyl, or, together with $R^e$, can also form a bridge described for that radical,

$R^b$ represents hydroxyl, alkoxy having 1 to 3 carbon atoms or nitromethyl,
$R^c$ represents hydrogen or methyl or, together with $R^g$, can also form a bridge described for that radical,
$R^d$ represents hydrogen, $CH_3$, $CH_2F$ or $=CH_2$,
$X^1$ represents hydrogen, halogen or nitro,
$X^2$ represents hydrogen, halogen, amino, hydroxyl, methoxy, mercapto, methyl, halogenomethyl or vinyl,
Y represents N or C-$R^e$, wherein

$R^e$ represents hydrogen, halogen, $CF_3$ , $OCH_3$ , $OCHF_2$ , $CH_3$, CN, $CH=CH_2$ or CeCH, or, together with $R^a$, can also form a bridge of the structure-*O-$CH_2$-CH-$CH_3$,-*S-$CH_2$-$CH_2$-, -*S-$CH_2$-CH-$CH_3$, -*$CH_2$-$CH_2$-CH-$CH_3$ or -*O-$CH_2$-N-$R^f$, wherein the atom marked with * is linked with the carbon atom of Y and wherein
$R^f$ denotes hydrogen, methyl or formyl,

and
D represents N or C-$R^g$, wherein

$R^g$ represents hydrogen, halogen, $CF_3$, $OCH_3$, $OCHF_2$ or $CH_3$, or, together with $R^c$, can also form a bridge of the structure -*O-$CH_2$-, -*NH-$CH_2$- , -*N($CH_3$)-$CH_2$-, -*N($C_2H_5$)-$CH_2$-,-*N($C_3H_5$)-$CH_2$-or -*S-$CH_2$-, wherein the atom marked with * is linked with the carbon atom of D,

n denotes 1, 2 or 3 and

T denotes a radical of the formula

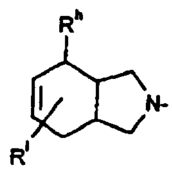
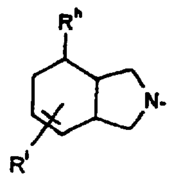
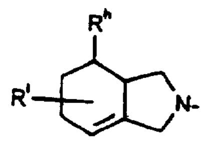

wherein

$R^h$ represents O-,

$$N-R^k,\quad CH_2-O-\ \text{or}\ -CH_2-N-R^k,$$

wherein

$R^k$ represents hydrogen or methyl and
$R^i$ represents hydrogen, $C_1$-$C_3$-alkyl or cyclopropyl,

and wherein K, Sp, L, AA1 and AA2 are defined as in Claim 1,
and isomers and salts thereof.

**8.** Compounds of the formula (I) according to Claim 7, in which

Q denotes a radical of the formula

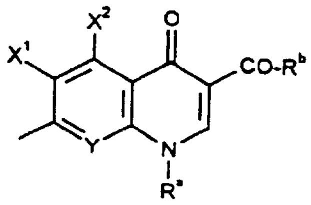

or

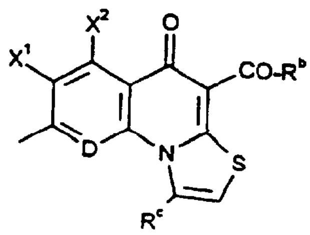

wherein

$R^a$ represents alkyl which has 2 to 4 carbon atoms and is optionally substituted by 1 fluorine atom, cyclopropyl which is optionally substituted by 1 fluorine atom or phenyl which is optionally mono- or disubstituted by fluorine or, together with $R^e$, can form a bridge described for that radical,

$R^b$ represents hydroxyl or alkoxy having 1 or 2 carbon atoms,

$R^c$ represents hydrogen or methyl, or, together with $R^g$, can form a bridge described for that radical,

$X^1$ represents fluorine,

$X^2$ represents hydrogen or amino,

Y represents N or C-$R^e$, wherein

$R^e$ represents hydrogen, fluorine, chlorine, $CF_3$, $OCH_3$, $OCHF_2$ or C≡CH, or, together with $R^a$, can form a bridge of the structure -*O-$CH_2$-CH-$CH_3$ or -*O-$CH_2$-N-$R^f$, wherein the atom marked with * is linked with the carbon atom of Y and wherein

$R^f$ denotes methyl,

D represents N or C-$R^g$, wherein

$R^g$ represents hydrogen, fluorine, chlorine, $CF_3$, $OCH_3$ or $CH_3$, or, together with $R^c$, also form a bridge of the structure -*O-$CH_2$-, -*NH-$CH_2$-, -*N($CH_3$)-$CH_2$- or -*S-$CH_2$-, wherein the atom marked with * is linked with the carbon atom of D, and

T denotes a radical of the formulae

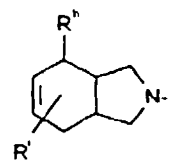 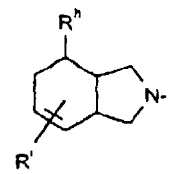 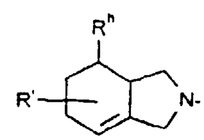

wherein

$R^h$ represents

$$-\overset{|}{N}-R^k,$$

wherein

$R^k$ represents hydrogen or methyl, and

$R^i$ represents hydrogen or methyl,

and isomers and salts thereof.

**9.** Medicaments comprising one or more compounds from Claims 1 to 8.

**10.** Use of compounds of the formula (I) according to Claims 1 to 8 for the preparation of medicaments.

**Revendications**

**1.** Composés répondant à la formule générale

$$K - Sp - L - AA1 - AA2 - C \qquad (I)$$

dans laquelle

K représente un radical d'hydrate de carbone non substitué ou modifié de manière régiosélective,
Sp représente un groupe arylène ou un groupe alkylène le cas échéant substitué
L représente un groupe

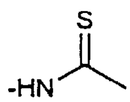 ou un groupe 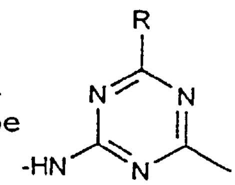

dans lesquels R représente un atome de chlore ou un groupe hydroxyalkylamino,

AA1 représente un résidu d'acide aminé dans la configuration D ou L, qui peut le cas échéant porter des groupes de protection ou un second groupement K-Sp-L- dans lequel K, Sp, L peuvent avoir, indépendamment de l'autre groupement K-Sp-L-, les significations indiquées ci-dessus, ou encore une liaison directe,

AA2 représente un résidu d'acide aminé dans la configuration D ou L, qui peut le cas échéant porter des groupes de protection ou un second groupement K-Sp-L- dans lequel K, Sp, L peuvent avoir, indépendamment de l'autre groupement K-Sp-L-, les significations indiquées ci-dessus, ou encore une liaison directe,

et

C représente un radical cytotoxique ou le radical d'un agent cytostatique ou d'un dérivé d'un agent cytostatique, qui peut porter en outre un groupe amino ou un groupe hydroxyle,

ainsi que leurs isomères et leurs sels.

**2.** Composés répondant à la formule générale (I) selon la revendication 1, dans lesquels K représente un radical d'hydrate de carbone répondant à la formule (II)

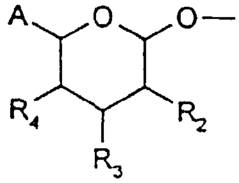 (II),

dans laquelle

A représente un groupe méthyle, un groupe hydroxyméthyle, un groupe carboxyle, ainsi que des esters et des amides qui en dérivent, un groupe alcoxyméthyle, un groupe acyloxyméthyle ou un groupe carboxyalcoxyméthyle, ainsi que des esters et des amides qui en dérivent; A peut égale-

ment représenter un groupe $CH_2$-B où B peut représenter à nouveau un radical d'hydrate de carbone répondant à la formule générale (II), lié via le centre anomère,

$R_2$, $R_3$, $R_4$ représentent, à titre individuel ou ensemble de manière identique, un atome d'hydrogène, un groupe hydroxyle, un groupe alcoxy, un groupe carboxyalcoxy, ainsi que des esters et des amides qui en dérivent, un groupe hydroxyalcoxy, un groupe aminoalcoxy, un groupe acyloxy, un groupe carboxyalkyl-carbonyloxy, un groupe sulfato, un groupe phosphato, un atome d'halogène ou encore un autre radical d'hydrate de carbone (il) modifié dans le même cadre et lié via le centre anomère, $R_2$ pouvant représenter en outre également un groupe amino ou un groupe acylamino, ou bien deux des radicaux $R_2$, $R_3$, $R_4$ représentent ensemble un groupe époxy,

et Sp, L, AA1, AA2 et C sont tels que définis à la revendication 1,
ainsi que leurs isomères et leurs sels.

**3.** Composés répondant à la formule (I) selon la revendication 1, dans lesquels

Sp représente un radical arylène qui a été modifié en position ortho, en position méta ou en position para avec K et avec L et qui peut porter en outre encore de 1 à 4 substituants supplémentaires qui peuvent représenter de manière indépendante ou de manière identique un atome d'hydrogène, un groupe méthyle, un groupe méthoxy, un groupe hydroxyle, un groupe carboxyle, un groupe méthyloxycarbonyle, un groupe cyano, un groupe nitro, un atome d'halogène, un groupe sulfonyle ou un groupe sulfonamide, ou bien
Sp peut également représenter un radical alkylène linéaire ou ramifié,

et K, L, AA1, AA2 et C sont tels que définis à la revendication 1,
ainsi que leurs isomères et leurs sels.

**4.** Composés répondant à la formule générale (I) selon la revendication 1, dans lesquels C peut représenter un nucléoside, un antibiotique, plus précisément l'endiine, un antibiotique peptidique cytotoxique, un acide quinolone- ou naphtyridone-carboxylique ou encore la batracyline, le 5-fluorouracile, le cytosinarabinoside, le méthotrexate, l'étoposide, la camptothécine, la daunomycine, la doxorubicine, le taxol, la vinblastine, la vincristine, la dynémicine, la calichéamycine, l'espéramycine, la quercétine, la suramine, l'erbstatine, le cyclophosphamide, la mitomycine C, le melphalan, le cisplatine, la bléomycine, la staurosporine ou une autre substance active antinéoplasique,
et K, Sp, L, AA1, AA2 sont tels que définis à la revendication 1,
ainsi que leurs isomères et leurs sels.

**5.** Composés répondant à la formule générale (I) selon la revendication 1, dans lesquels AA1 représente un résidu d'acide aminé dérivé de la lysine, de l'alanine, de l'acide aspartique, de l'acide glutamique, de la glycine, de l'ornithine, de la tyrosine, de la valine ou de la sérine dans la configuration D ou L, qui est le cas échéant relié à un autre groupement K-Sp-L-, ou dans lesquels AA1 représente une liaison,
et K, Sp, L, AA2 et C sont tels que définis à la revendication 1,
ainsi que leurs isomères et leurs sels.

**6.** Composés répondant à la formule générale (I) selon la revendication 1, dans lesquels AA2 représente un résidu d'acide aminé dérivé de la lysine, de l'alanine, de la glycine, de l'ornithine, de l'acide diaminopropionique ou de la sérine dans la configuration D ou L, qui le cas échéant est relié à un autre groupement K-Sp-L-, ou dans lesquels AA2 représente une liaison,
et K, Sp, L, AA1 et C sont tels que définis à la revendication 1,
ainsi que leurs isomères et leurs sels.

**7.** Composés répondant à la formule générale (I) selon la revendication 1, dans lesquels C représente un radical répondant à la formule (III)

$$T\text{-}Q \qquad (III)$$

dans laquelle

Q représente un radical répondant aux formules

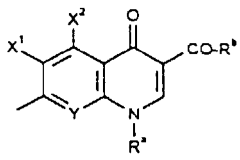, 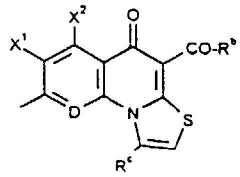, 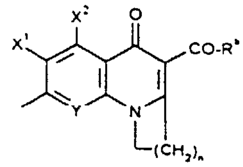

ou

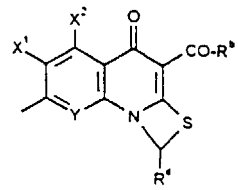

dans lesquelles

- $R^a$ représente un groupe alkyle contenant de 1 à 4 atomes de carbone portant le cas échéant un ou deux substituants halogéno ou hydroxyle identiques ou différents, un groupe vinyle, un groupe cycloalkyle contenant de 3 à 6 atomes de carbone portant le cas échéant un ou deux substituants fluoro, un groupe bicyclo[1.1.1]pent-1-yle, un groupe 1,1-diméthylpropargyle, un groupe 3-oxétanyle, un groupe méthoxy, un groupe amino, un groupe méthylamino, un groupe diméthylamino, un groupe phényle portant le cas échéant un ou deux substituants halogéno, amino ou hydroxyle identiques ou différents, ou bien peut également former de manière conjointe avec $R^e$, un pont décrit dans la définition de ce radical,
- $R^b$ représente un groupe hydroxyle, un groupe alcoxy contenant de 1 à 3 atomes de carbone, un groupe nitrométhyle,
- $R^c$ représente un atome d'hydrogène ou un groupe méthyle ou bien peut également former de manière conjointe avec $R^g$, un pont décrit dans la définition de ce radical,
- $R^d$ représente un atome d'hydrogène, un groupe $CH_3$, un groupe $CH_2F$ ou un groupe $=CH_2$,
- $X^1$ représente un atome d'hydrogène, un atome d'halogène ou un groupe nitro,
- $X^2$ représente un atome d'hydrogène, un atome d'halogène, un groupe amino, un groupe hydroxyle, un groupe méthoxy, un groupe mercapto, un groupe méthyle, un groupe halogénométhyle ou un groupe vinyle,
- Y représente un atome d'azote ou un groupe C-$R^e$ où

  - $R^e$ représente un atome d'hydrogène, un atome d'halogène, un groupe $CF_3$, un groupe $OCH_3$, un groupe $OCHF_2$, un groupe $CH_3$, un groupe CN, un groupe $CH=CH_2$ ou un groupe $C\equiv CH$ ou bien peut également former de manière conjointe avec $R^a$, un pont de structure $-{}^*O-CH_2-CH-CH_3-$, $-{}^*S-CH_2-CH_2-$, $-{}^*S-CH_2-CH-CH_3$, $-{}^*CH_2-CH_2-CH-CH_3$ ou $-{}^*O-CH_2-N-R^f$, l'atome marqué avec * étant lié à l'atome de carbone de Y, et où
  - $R^f$ représente un atome d'hydrogène, un groupe méthyle ou un groupe formyle,

  et
- D représente un atome d'azote ou un groupe C-$R^g$ où

  - $R^g$ représente un atome d'hydrogène, un atome d'halogène, un groupe $CF_3$, un groupe $OCH_3$, un groupe $OCHF_2$ ou un groupe $CH_3$, ou bien peut également former de manière conjointe avec $R^e$, un pont de structure $-{}^*O-CH_2-$, $-{}^*NH-CH_2-$, $-{}^*N(CH_3)-CH_2-$, $-{}^*N(C_2H_5)-CH_2-$, $-{}^*N(C_3H_5)-CH_2-$ ou $-{}^*S-CH_2-$, l'atome marqué avec * étant lié à l'atome de carbone de D,

- n est égal à 1, 2 ou 3, et

T représente un radical répondant aux formules

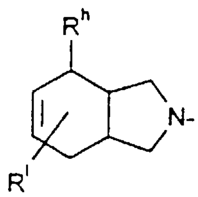 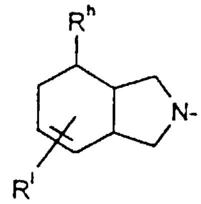 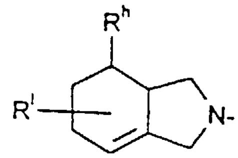

dans lesquelles

$R^h$ représente un atome d'oxygène, un groupe

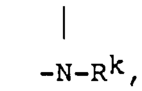

un groupe $CH_2$-O-ou un groupe

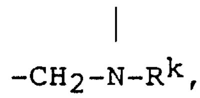

où

$R^k$ représente un atome d'hydrogène ou un groupe méthyle,
$R^i$ représente un atome d'hydrogène, un groupe alkyle en $C_1$-$C_3$ ou un groupe cyclopropyle,

et dans lesquels K, Sp, L, AA1, AA2 sont tels que définis à la revendication 1,
ainsi que leurs isomères et leurs sels.

**8.** Composés répondant à la formule (III) selon la revendication 7, dans lesquels

Q représente un radical répondant aux formules

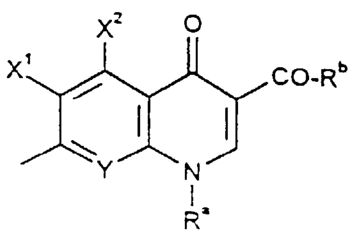

ou

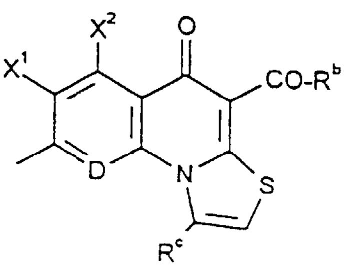

dans lesquelles

$R^a$ représente un groupe alkyle contenant de 2 à 4 atomes de carbone portant le cas échéant un substituant fluoro, un groupe cyclopropyle portant le cas échéant un substituant fluoro, un groupe phényle portant le cas échéant un ou deux substituants fluoro, ou bien peut également former de manière conjointe avec $R^e$, un pont décrit dans la définition de ce radical,
$R^b$ représente un groupe hydroxyle ou un groupe alcoxy contenant 1 ou 2 atomes de carbone,
$R^c$ représente un atome d'hydrogène ou un groupe méthyle ou bien peut former de manière conjointe avec $R^g$, un pont décrit dans la définition de ce radical,

$X^1$ représente un atome de fluor,
$X^2$ représente un atome d'hydrogène ou un groupe amino,
Y représente un atome d'azote ou un groupe C-$R^e$ où

$R^e$ représente un atome d'hydrogène, un atome de fluor, un atome de chlore, un groupe $CF_3$, un groupe $OCH_3$, un groupe $OCHF_2$ ou un groupe C≡CH ou bien peut également former de manière conjointe avec $R^a$, un pont de structure -*O-$CH_2$-CH-$CH_3$ ou -* O-$CH_2$-N-$R^f$,
l'atome marqué avec * étant lié à l'atome de carbone de Y, et où
$R^f$ représente un groupe méthyle,

D représente un atome d'azote ou un groupe C-$R^g$ où

$R^g$ représente un atome d'hydrogène, un atome de fluor, un atome de chlore, un groupe $CF_3$, un groupe $OCH_3$ ou un groupe $CH_3$, ou bien peut également former de manière conjointe avec $R^e$, un pont de structure -*$OCH_2$-, -*NH-$CH_2$-, -*N($CH_3$)-$CH_2$- ou -* S-$CH_2$-, l'atome marqué avec * étant lié à l'atome de carbone de D,

et
T représente un radical répondant aux formules

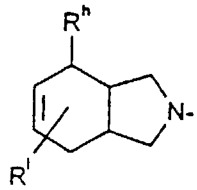 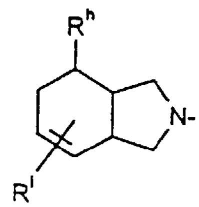 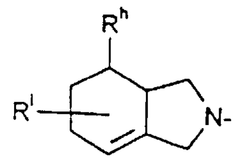

dans lesquelles

$R^h$ représente un groupe

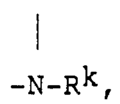

où $R^k$ représente un atome d'hydrogène ou un groupe méthyle,
$R^i$ représente un atome d'hydrogène ou un groupe méthyle,

ainsi que leurs isomères et leurs sels.

**9.** Médicament contenant un ou plusieurs composés selon les revendications 1 à 8.

**10.** Utilisation de composés répondant à la formule (I) selon les revendications 1 à 8 pour la préparation de médicaments.

Fig. 1

Knochenmark-Stammzellen-Proliferation